(19) Europäisches Patentamt

European Patent Office

Office européen des brevets

(11) **EP 1 695 981 A1**

(12) **EUROPÄISCHE PATENTANMELDUNG**

(43) Veröffentlichungstag:
30.08.2006 Patentblatt 2006/35

(51) Int Cl.:
*C07K 14/435* (2006.01)    *G01N 33/50* (2006.01)

(21) Anmeldenummer: 05090045.5

(22) Anmeldetag: 25.02.2005

(84) Benannte Vertragsstaaten:
AT BE BG CH CY CZ DE DK EE ES FI FR GB GR
HU IE IS IT LI LT LU MC NL PL PT RO SE SI SK TR
Benannte Erstreckungsstaaten:
AL BA HR LV MK YU

(71) Anmelder: Forschungsverbund Berlin e.V.
12489 Berlin (DE)

(72) Erfinder:
• **Freund, Christian**
  **D- 14055 Berlin (DE)**
• **Zimmermann, Jürgen**
  **Jamaica Plain, MA 02130 (US)**

(74) Vertreter: **Hertin, Paul W.**
**Anwaltssozietät Hertin**
**Kurfürstendamm 54/55**
**10707 Berlin (DE)**

Bemerkungen:
Das Sequenzprotokoll, das als Anlage zu den
Anmeldungsunterlagen mitveröffenlicht ist, ist nach
dem Anmeldetag eingereicht worden. Der Anmelder
hat erklärt, dass dieses nicht über den Inhalt der
Anmeldung in der ursprünglich eingereichten
Fassung hinausgeht.

(54) **Verfahren zum Redox-Potential-abhängigen Nachweis von Targetmolekülen durch wechselwirkende Polypeptide**

(57)    Die Erfindung betrifft eine Aminosäuresequenz gemäß SEQ ID Nr. 1, eine Proteinerkennungsdomäne umfassend diese Aminosäuresequenz und die Verwendung der Aminosäuresequenz oder der Proteinerkennungsdomäne zur Identifizierung von Redox-abhängigen Protein-Protein- oder Protein-Lipid-Wechselwirkungen und ein Verfahren zur Detektion von Target-Molekülen (Moleküle, die von SEQ ID Nr. 1 gebunden werden) in Abhängigkeit vom Redoxpotential; die Erfindung betrifft auch die Verwendung der Aminosäuresequenz zur Herstellung einer Aminosäuresequenz-/Polypeptidbibliothek; weiterhin betrifft die Erfindung einen Kit, der die Aminosäuresequenz bzw. die Proteinerkennungsdomäne umfasst; ausserdem betrifft die Erfindung Antikörper gegen Aminosäuresequenzen von SEQ ID Nr. 1 oder der Polypeptidbibliothek, sowie die Verwendung der Aminosäuresequenzen in pharmazeutischen Formulierungen.

SEQUENCE LISTING

<110> Forschungsverbund Berlin e.V.

<120> Verfahren zum Redox-Potential-abhaengigen Nachweis von Targetmolekuelen durch wechselwirkende Peptide

<130> XII 849-05

<160> 137

<170> PatentIn version 3.3

<210> 1
<211> 94
<212> PRT
<213> Homo sapiens

<400> 1

Glu Lys Lys Glu Gln Lys Glu Lys Glu Lys Lys Glu Gln Glu Ile Lys
1               5                   10                  15

Lys Lys Phe Lys Leu Thr Gly Pro Ile Gln Val Ile His Leu Ala Lys
            20                  25                  30

Ala Cys Cys Asp Val Lys Gly Gly Lys Asn Glu Leu Ser Phe Lys Gln
            35                  40                  45

Gly Glu Gln Ile Glu Ile Ile Arg Ile Thr Asp Asn Pro Glu Gly Lys
        50                  55                  60

Trp Leu Gly Arg Thr Ala Arg Gly Ser Tyr Gly Tyr Ile Lys Thr Thr
65                  70                  75                  80

Ala Val Glu Ile Asp Tyr Asp Ser Leu Lys Leu Lys Lys Asp
                85                  90

<210> 2
<211> 50
<212> PRT
<213> Homo sapiens

<400> 2

Glu Leu Val Gln Lys Phe Gln Val Tyr Tyr Leu Gly Asn Val Pro Val
1               5                   10                  15

Ala Lys Pro Val Gly Val Asp Val Ile Asn Gly Ala Leu Glu Ser Val
            20                  25                  30

Leu Ser Ser Ser Ser Arg Glu Gln Trp Thr Pro Ser His Val Ser Val
            35                  40                  45

Ala Pro
        50

<210> 3

```
<211>  50
<212>  PRT
<213>  Homo sapiens

<400>  3

Gly Leu Ser Leu Ser Val Pro Ala Thr Arg Gln Val Ile Ala Asn His
1               5                   10                  15


His Met Pro Ser Ile Ser Phe Ala Ser Gly Gly Asp Thr Asp Met Thr
            20                  25                  30


Asp Tyr Val Ala Tyr Val Ala Lys Asp Pro Ile Asn Gln Arg Ala Cys
            35                  40                  45


His Ile
    50


<210>  4
<211>  50
<212>  PRT
<213>  Homo sapiens

<400>  4

Met Thr Leu Glu Glu Leu Val Ala Cys Asp Asn Ala Ala Gln Lys Met
1               5                   10                  15


Gln Thr Val Thr Ala Ala Val Glu Glu Leu Leu Val Ala Ala Gln Arg
            20                  25                  30


Gln Asp Arg Leu Thr Val Gly Val Tyr Glu Ser Ala Lys Leu Met Asn
            35                  40                  45


Val Asp
    50


<210>  5
<211>  53
<212>  PRT
<213>  Homo sapiens

<400>  5

Glu Asn Glu Glu Ala Glu Gly Asp Glu Ile Tyr Glu Asp Leu Met Arg
1               5                   10                  15


Ser Glu Pro Val Ser Met Pro Pro Lys Met Thr Glu Tyr Asp Lys Arg
            20                  25                  30


Cys Cys Cys Leu Arg Glu Ile Gln Gln Thr Glu Glu Lys Tyr Thr Asp
            35                  40                  45


Thr Leu Leu Glu Arg
        50
```

```
<210>    6
<211>    50
<212>    PRT
<213>    Homo sapiens

<400>    6
```

| | | | | | | | | | | | | | | |
|---|---|---|---|---|---|---|---|---|---|---|---|---|---|---|
Leu Glu Glu Glu Pro Ser Val Glu Thr Glu Asp Pro Ser Pro Glu Thr
1              5                     10                  15

Phe Arg Gln Leu Phe Arg Leu Phe Cys Tyr Gln Glu Val Ala Gly Pro
           20                 25                   30

Arg Glu Ala Leu Ser Arg Leu Trp Glu Leu Cys Cys Arg Trp Leu Arg
        35                40               45

Pro Glu
    50

```
<210>    7
<211>    37
<212>    PRT
<213>    Homo sapiens

<400>    7
```

Gln Glu Leu Cys Arg Gln Leu Phe Arg Gln Phe Cys Tyr Gln Asp Ser
1             5                   10               15

Pro Gly Pro Arg Glu Ala Leu Ser Arg Leu Arg Glu Leu Cys Cys Gln
           20                25               30

Trp Leu Lys Pro Glu
        35

```
<210>    8
<211>    37
<212>    PRT
<213>    Homo sapiens

<400>    8
```

Ser Glu Ala Cys Arg Gln Arg Phe Arg Gln Phe Cys Tyr Gly Asp Val
1             5                   10               15

His Gly Pro His Glu Ala Phe Ser Gln Leu Trp Glu Leu Cys Cys Arg
           20                25               30

Trp Leu Arg Pro Glu
        35

```
<210>    9
<211>    39
<212>    PRT
<213>    Homo sapiens

<400>    9
```

Cys Glu Val Phe Arg Gln Arg Phe Arg Gln Phe Gln Tyr Arg Glu Ala
1               5                   10              15

Ala Gly Pro His Glu Ala Phe Asn Lys Leu Trp Glu Leu Cys Cys Gln
                20              25              30

Trp Leu Lys Pro Lys Pro His
        35

<210> 10
<211> 49
<212> PRT
<213> Homo sapiens

<400> 10

Lys Gly Leu Glu Gly Met Ile Arg Lys Arg Ser Gly Gly His Arg Val
1               5                   10              15

Pro Gly Leu Thr Cys Cys Gly Arg Asp Gln Val Cys Tyr Arg Trp Ser
                20              25              30

Lys Arg Trp Leu Trp Lys Asp Ser Phe Leu Leu Tyr Met Cys Leu Glu
        35              40              45

Thr


<210> 11
<211> 50
<212> PRT
<213> Homo sapiens

<400> 11

Lys Gly Ile Glu Gly Met Ile Met Lys Arg Ser Gly Gly His Arg Ile
1               5                   10              15

Pro Gly Leu Asn Cys Cys Gly Gln Gly Arg Ala Cys Tyr Arg Trp Ser
                20              25              30

Lys Arg Trp Leu Ile Val Lys Asp Ser Phe Leu Leu Tyr Met Lys Pro
        35              40              45

Asp Ser
    50


<210> 12
<211> 50
<212> PRT
<213> Homo sapiens

<400> 12

Ile Leu Arg Val Gly Phe Val Leu Arg Ser Ala Arg Arg Ala Pro Cys
1               5                   10              15

Cys Arg Ile Met Val Leu Cys Ser Asp Arg Ile Leu Phe Gly His Arg
             20                  25                  30

Gly Val Asn Leu Asp Gly Asn Phe Phe Thr Val His Ala Glu Phe Lys
             35                  40                  45

Leu Lys
    50


<210>   13
<211>   49
<212>   PRT
<213>   Homo sapiens

<400>   13

Asn Thr Met Cys Gly Tyr Leu Lys Arg Lys Leu Arg Asn Ser Ser Gly
1               5                  10                  15

Trp Gln Asp Leu Trp Trp Met Cys Cys His Thr Leu Tyr Phe Tyr Arg
             20                  25                  30

Asn His Asn Glu Arg Glu Pro Leu Ala His Leu Ser Leu Met Asp Tyr
             35                  40                  45

Gly


<210>   14
<211>   50
<212>   PRT
<213>   Homo sapiens

<400>   14

Gly Trp Lys Gln Cys Cys Val Arg Tyr Leu Leu Pro Trp Ser Lys Arg
1               5                  10                  15

Trp Leu Met Val Arg Asp Ser Phe Val Ala Tyr Met Asp His Arg Thr
             20                  25                  30

Glu Gln Ile Arg Met Val Leu Leu Met Asp Arg Asp Phe Lys Val Ala
             35                  40                  45

Ala Gly
    50


<210>   15
<211>   50
<212>   PRT
<213>   Homo sapiens

<400>   15

Phe Ile Met Glu Gly Thr Leu Thr Arg Val Gly Ala Lys His Glu Arg
1               5                  10                  15

His Ile Phe Leu Phe Asp Gly Leu Met Ile Cys Cys Lys Ser Asn His
        20             25             30

Gly Gln Pro Arg Leu Pro Gly Ala Ser Ser Ala Glu Tyr Arg Leu Lys
      35           40            45

Glu Lys
    50

<210> 16
<211> 49
<212> PRT
<213> Homo sapiens

<400> 16

Val Leu Lys Gln Gly Tyr Met Met Lys Lys Gly His Arg Arg Lys Asn
1          5            10            15

Trp Thr Glu Arg Trp Phe Val Leu Lys Pro Asn Ile Ile Ser Trp Val
      20           25          30

Ser Glu Asp Leu Lys Asp Lys Lys Gly Asp Ile Leu Leu Asp Glu Asn
      35           40          45

Cys

<210> 17
<211> 50
<212> PRT
<213> Homo sapiens

<400> 17

Val Leu Lys Ala Gly Tyr Leu Glu Lys Arg Arg Lys Asp His Ser Phe
1          5            10            15

Leu Gly Phe Glu Trp Gln Lys Arg Trp Cys Ala Leu Ser Lys Thr Val
      20           25          30

Phe Tyr Tyr Tyr Gly Ser Asp Lys Asp Lys Gln Gln Lys Gly Glu Phe
      35           40          45

Ala Ile
    50

<210> 18
<211> 50
<212> PRT
<213> Homo sapiens

<400> 18

Ala Leu Glu Leu Gly Thr Val Met Thr Val Phe Ser Ala Arg Lys Ser

EP 1 695 981 A1

```
         1                5                    10        . .       15

         Thr Pro Glu Arg Arg Thr Val Gln Met Ile Met Glu Thr Arg Gln Val
                     20                  25                  30

         Ala Trp Ser Lys Thr Ala Asp Lys Ile Glu Gly Phe Leu Asp Ile Met
                 35                  40                  45

         Glu Ile
                 50


         <210>  19
         <211>  49
         <212>  PRT
         <213>  Homo sapiens

         <400>  19

         Leu Gln Tyr Phe Gly Gln Leu Leu Val Trp Asp Trp Asn Val Cys Lys
         1               5                    10                  15


         Ala Asp Ile Glu Arg Glu Tyr His Val Tyr Leu Phe Glu Lys Ile Leu
                     20                  25                  30


         Leu Cys Cys Lys Glu Met Ser Thr Leu Lys Arg Gln Ala Arg Ser Ile
                 35                  40                  45


         Ser


         <210>  20
         <211>  50
         <212>  PRT
         <213>  Homo sapiens

         <400>  20

         Ala Leu Glu Leu Gly Thr Val Met Thr Val Phe Ser Phe Arg Lys Ser
         1               5                    10                  15


         Thr Pro Glu Arg Arg Thr Val Gln Val Ile Met Glu Thr Arg Gln Val
                     20                  25                  30


         Ala Trp Ser Lys Thr Ala Asp Lys Ile Glu Gly Phe Leu Asp Ile Met
                 35                  40                  45


         Glu Ile
                 50

         <210>  21
         <211>  50
         <212>  PRT
         <213>  Homo sapiens

         <400>  21
```

Phe Ile Met Glu Gly Thr Leu Thr Arg Val Gly Ala Lys His Glu Arg
1               5                   10                  15

His Ile Phe Leu Phe Asp Gly Leu Met Ile Cys Cys Lys Ser Asn His
            20                  25                  30

Gly Gln Pro Arg Leu Pro Gly Ala Ser Asn Ala Glu Tyr Arg Leu Lys
        35                  40                  45

Glu Lys
    50


<210>  22
<211>  50
<212>  PRT
<213>  Homo sapiens

<400>  22

Tyr Tyr Lys Glu Arg Leu Leu Tyr Leu Glu Glu Gly Gln Lys Asp Ser
1               5                   10                  15

Leu Ile Asp Ser Ser Arg Val Leu Cys Cys His Gly Glu Leu Lys Asn
            20                  25                  30

Asn Arg Gly Val Lys Leu His Val Phe Leu Phe Gln Glu Val Leu Val
        35                  40                  45

Ile Thr
    50


<210>  23
<211>  50
<212>  PRT
<213>  Homo sapiens

<400>  23

Arg Leu Cys Glu Gly Thr Leu Phe Arg Lys Ile Ser Ser Arg Arg Arg
1               5                   10                  15

Gln Asp Lys Leu Trp Phe Cys Cys Leu Ser Pro Asn His Lys Leu Leu
            20                  25                  30

Gln Tyr Gly Asp Met Glu Glu Gly Ala Ser Pro Pro Thr Leu Glu Ser
        35                  40                  45

Leu Pro
    50


<210>  24
<211>  50
<212>  PRT
<213>  Homo sapiens

<400>  24

```
Asp Thr Lys His Gly Met Met Lys Phe Arg Glu Asp Arg Ser Leu Leu
1                5                10                15

Gly Leu Gly Leu Pro Ser Gly Gly Phe His Asp Arg Tyr Phe Ile Leu
            20                25                30

Asn Ser Ser Cys Leu Arg Leu Tyr Lys Glu Val Arg Ser Gln Arg Pro
            35                40                45

Trp Ser
        50
```

```
<210>  25
<211>  54
<212>  PRT
<213>  Homo sapiens

<400>  25
```

```
Leu Ser Met Gly Asp Leu Leu Leu His Thr Thr Val Ile Trp Leu Asn
1                5                10                15

Pro Pro Ala Ser Leu Gly Lys Trp Lys Lys Glu Pro Glu Leu Ala Ala
            20                25                30

Phe Val Phe Lys Thr Ala Trp Leu Val Tyr Lys Asp Gly Ser Lys Gln
            35                40                45

Lys Ile Leu Trp Glu Gln
            50
```

```
<210>  26
<211>  50
<212>  PRT
<213>  Homo sapiens

<400>  26
```

```
Gly Val Lys Leu Glu Val Asn Glu Arg Ile Leu Gly Cys Cys Thr Ser
1                5                10                15

Leu Met Gln Ala Ile Gln Val Leu Ile Val Ala Ser Lys Asp Leu Gln
            20                25                30

Arg Glu Ile Val Glu Ser Gly Arg Gly Thr Ala Ser Pro Lys Glu Phe
            35                40                45

Tyr Ala
        50
```

```
<210>  27
<211>  50
<212>  PRT
<213>  Homo sapiens
```

<400> 27

Cys Pro Ser Pro Val Leu Asn Thr Pro Trp Ile Pro Phe Gln Asn Cys
1               5                   10                  15

Cys Tyr Asn Phe Ile Ile Thr Lys Asn Arg His Met Ala Thr Thr Gln
            20                  25                  30

Asp Glu Val His Thr Lys Cys Gln Lys Leu Asn Pro Lys Ser His Ile
            35                  40                  45

Leu Ser
    50

<210>   28
<211>   30
<212>   PRT
<213>   Homo sapiens

<400>   28

Glu Asp Ala Arg Ile Met Leu Ser Arg Ala Val Glu Cys Cys Pro Thr
1               5                   10                  15

Ser Val Glu Leu Trp Leu Ala Leu Ala Arg Leu Glu Thr Tyr
            20                  25                  30

<210>   29
<211>   50
<212>   PRT
<213>   Homo sapiens

<400>   29

Arg Phe Ile Phe Asp Val Gly Thr Arg Leu Gly Leu His Tyr Asp Thr
1               5                   10                  15

Leu Ala Thr Gly Ile Ile Tyr Phe His Arg Phe Tyr Met Phe His Ser
            20                  25                  30

Phe Lys Gln Phe Pro Arg Tyr Val Thr Gly Ala Cys Cys Leu Phe Leu
            35                  40                  45

Ala Gly
    50

<210>   30
<211>   50
<212>   PRT
<213>   Homo sapiens

<400>   30

Asn Phe Leu Arg Arg Ile Ser Lys Ala Asp Asp Tyr Asp Val Gln Ser
1               5                   10                  15

Arg Thr Leu Gly Lys Tyr Leu Leu Glu Ile Thr Ile Val Asp Tyr Lys

Phe Ile Gly Met Arg Pro Ser Leu Cys Cys Ala Ser Ala Met Tyr Leu
        35              40              45

Ala Arg
    50

<210> 31
<211> 50
<212> PRT
<213> Homo sapiens

<400> 31

Met Gln Ile Ile Asp Val Ala Ser Ile Leu Gly Leu Asp Cys Asp Ile
1           5               10              15

Ser Glu His Ala Phe Gln Leu Phe Arg Asp Cys Cys Ser Ala Thr Cys
            20              25              30

Leu Arg Asn Arg Ser Val Glu Ala Leu Ala Thr Ala Cys Leu Val Gln
        35              40              45

Ala Ile
    50

<210> 32
<211> 50
<212> PRT
<213> Homo sapiens

<400> 32

Cys Phe Met Arg Arg Phe Leu Lys Ala Ala Gln Ser Glu Lys Lys Leu
1           5               10              15

Glu Leu Leu Ser Phe Phe Met Ile Glu Leu Ser Leu Val Glu Tyr Glu
            20              25              30

Met Leu Gln Phe Cys Pro Ser Met Leu Ala Ala Ala Ala Ile Tyr Thr
        35              40              45

Ala Gln
    50

<210> 33
<211> 50
<212> PRT
<213> Homo sapiens

<400> 33

Asp Phe Ile Met Tyr Cys His Thr Arg Leu Asn Leu Ser Thr Ser Thr
1           5               10              15

Leu Phe Leu Thr Phe Thr Ile Leu Asp Lys Tyr Ser Ser Arg Phe Ile
20 25 30

Ile Lys Ser Tyr Asn Tyr Gln Leu Leu Ser Leu Thr Ala Leu Trp Ile
35 40 45

Ser Ser
50

<210> 34
<211> 50
<212> PRT
<213> Homo sapiens

<400> 34

Ile Arg Ile Arg Asn Leu Cys Glu Arg Ile Gln Tyr Met Glu Gln Thr
1 5 10 15

Glu Arg Val Tyr Asn Val Phe Lys Gln Ile Leu Asp Gln Gln Thr Thr
20 25 30

Leu Phe Phe Asn Arg His Met His Gln Leu Ile Leu Cys Cys Leu Tyr
35 40 45

Gly Val
50

<210> 35
<211> 41
<212> PRT
<213> Homo sapiens

<400> 35

Met Gln Arg Glu Val Trp Met Ser Val Phe Arg Tyr Leu Ser Arg Arg
1 5 10 15

Glu Leu Cys Glu Cys Met Arg Val Cys Lys Thr Trp Tyr Lys Trp Cys
20 25 30

Cys Asp Lys Arg Leu Trp Thr Lys Ile
35 40

<210> 36
<211> 50
<212> PRT
<213> Homo sapiens

<400> 36

Tyr Ser Lys Trp Tyr Phe Glu Val Met Val Asp Glu Val Thr Pro Phe
1 5 10 15

Leu Thr Ala Gln Ala Thr His Leu Arg Val Gly Trp Ala Leu Thr Glu
20 25 30

Gly Tyr Thr Pro Tyr Pro Gly Ala Gly Glu Gly Trp Gly Gly Asn Gly
        35              40              45

Val Gly
    50


<210> 37
<211> 23
<212> PRT
<213> Homo sapiens

<400> 37

Trp Ala Ser Thr Glu Gly Tyr Ser Pro Tyr Pro Gly Gly Gly Glu Glu
1           5               10              15

Trp Gly Gly Asn Gly Val Gly
            20


<210> 38
<211> 50
<212> PRT
<213> Homo sapiens

<400> 38

Met Ala Gly Lys Ser Ser Leu Phe Lys Val Ile Leu Leu Gly Asp Gly
1           5               10              15

Gly Val Gly Lys Ser Ser Leu Met Asn Arg Tyr Val Thr Asn Lys Phe
            20              25              30

Asp Thr Gln Leu Phe His Thr Ile Gly Val Glu Phe Leu Asn Lys Asp
        35              40              45

Leu Glu
    50


<210> 39
<211> 50
<212> PRT
<213> Homo sapiens

<400> 39

Glu Phe Val Ala Ile Ala Asp Tyr Ala Ala Thr Asp Glu Thr Gln Leu
1           5               10              15

Ser Phe Leu Arg Gly Glu Lys Ile Leu Ile Leu Arg Gln Thr Thr Ala
            20              25              30

Asp Trp Trp Trp Gly Glu Arg Ala Gly Cys Cys Gly Tyr Ile Pro Ala
        35              40              45

Asn His
    50

<210> 40
<211> 50
<212> PRT
<213> Homo sapiens

<400> 40

His Arg Thr Leu Leu Tyr Gly His Ala Ile Leu Leu Arg His Ser Tyr
1               5                   10                  15

Ser Gly Met Tyr Leu Cys Cys Leu Ser Thr Ser Arg Ser Ser Thr Asp
            20                  25                  30

Lys Leu Ala Phe Asp Val Gly Leu Gln Glu Asp Thr Thr Gly Glu Ala
        35                  40                  45

Cys Trp
        50


<210> 41
<211> 50
<212> PRT
<213> Homo sapiens

<400> 41

Lys Met Phe Val Gly Gln Val Pro Arg Thr Trp Ser Glu Lys Asp Leu
1               5                   10                  15

Arg Glu Leu Phe Glu Gln Tyr Gly Ala Val Tyr Glu Ile Asn Val Leu
            20                  25                  30

Arg Asp Arg Ser Gln Asn Pro Pro Gln Ser Lys Gly Cys Cys Phe Val
        35                  40                  45

Thr Phe
        50


<210> 42
<211> 50
<212> PRT
<213> Homo sapiens

<400> 42

Arg Ile Ile Val Glu Asn Leu Phe Tyr Pro Val Thr Leu Asp Val Leu
1               5                   10                  15

His Gln Ile Phe Ser Lys Phe Gly Thr Val Leu Lys Ile Ile Thr Phe
            20                  25                  30

Thr Lys Asn Asn Gln Phe Gln Ala Leu Leu Gln Tyr Ala Asp Pro Val
        35                  40                  45

Ser Ala

50

```
<210>   43
<211>   50
<212>   PRT
<213>   Homo sapiens

<400>   43
```

Ser Val Ile Gly Gly Asn Leu Thr Gly Ile Phe Ile His Arg Val Thr
1               5                   10                  15

Pro Gly Ser Ala Ala Asp Gln Met Ala Leu Arg Pro Gly Thr Gln Ile
            20                  25                  30

Val Met Val Asp Tyr Glu Ala Ser Glu Pro Leu Phe Lys Ala Val Leu
        35                  40                  45

Glu Asp
        50

```
<210>   44
<211>   50
<212>   PRT
<213>   Homo sapiens

<400>   44
```

His Asp Phe Gly Phe Ser Val Ser Asp Gly Leu Leu Glu Lys Gly Val
1               5                   10                  15

Tyr Val His Thr Val Arg Pro Asp Gly Pro Ala His Arg Gly Gly Leu
            20                  25                  30

Gln Pro Phe Asp Arg Val Leu Gln Val Asn His Val Arg Thr Arg Asp
        35                  40                  45

Phe Asp
        50

```
<210>   45
<211>   50
<212>   PRT
<213>   Homo sapiens

<400>   45
```

Glu Asp Phe Gly Phe Ser Val Ala Asp Gly Leu Leu Glu Lys Gly Val
1               5                   10                  15

Tyr Val Lys Asn Ile Arg Pro Ala Gly Pro Gly Asp Leu Gly Gly Leu
            20                  25                  30

Lys Pro Tyr Asp Arg Leu Leu Gln Val Asn His Val Arg Thr Arg Asp
        35                  40                  45

Phe Asp
50

<210> 46
<211> 48
<212> PRT
<213> Homo sapiens

<400> 46

Pro Ala Ile Gly Asp Ile Cys Cys Ala Gln Phe Ser Glu Asp Asp Gln
1               5                   10                  15

Trp Tyr Arg Ala Ser Val Leu Ala Tyr Ala Ser Glu Glu Ser Val Leu
            20                  25                  30

Val Gly Tyr Val Asp Tyr Gly Asn Phe Glu Ile Leu Ser Leu Met Arg
        35                  40                  45

<210> 47
<211> 50
<212> PRT
<213> Homo sapiens

<400> 47

Phe Lys Ala Glu Ile Gly Gln Pro Cys Cys Ala Phe Phe Ala Gly Asp
1               5                   10                  15

Gly Ser Trp Tyr Arg Ala Leu Val Lys Glu Ile Leu Pro Asn Gly His
            20                  25                  30

Val Lys Val His Phe Val Asp Tyr Gly Asn Ile Glu Glu Val Thr Ala
        35                  40                  45

Asp Glu
50

<210> 48
<211> 49
<212> PRT
<213> Homo sapiens

<400> 48

Tyr Arg Pro Arg Ile Gly Asp Ala Cys Cys Ala Lys Tyr Thr Ser Asp
1               5                   10                  15

Asp Phe Trp Tyr Arg Ala Val Val Leu Gly Thr Ser Asp Thr Asp Val
            20                  25                  30

Glu Val Leu Tyr Ala Asp Tyr Gly Asn Ile Glu Thr Leu Pro Leu Cys
        35                  40                  45

Arg

```
<210>  49
<211>  50
<212>  PRT
<213>  Homo sapiens

<400>  49

Lys Asp Lys Leu Lys Met Glu Val Asp Gln Leu Lys Lys Glu Val Thr
1               5                   10                  15

Leu Glu Arg Met Leu Val Ser Lys Cys Cys Glu Glu Val Arg Asp Tyr
            20                  25                  30

Val Glu Glu Arg Ser Gly Glu Asp Pro Leu Val Lys Gly Ile Pro Glu
        35                  40                  45

Asp Lys
    50


<210>  50
<211>  50
<212>  PRT
<213>  Homo sapiens

<400>  50

Met Asp Asp Arg Glu Asp Leu Val Tyr Gln Ala Lys Leu Ala Glu Gln
1               5                   10                  15

Ala Glu Arg Tyr Asp Glu Met Val Glu Ser Met Lys Lys Val Ala Gly
            20                  25                  30

Met Asp Val Glu Leu Thr Val Glu Glu Arg Asn Leu Leu Ser Val Ala
            35                  40                  45

Tyr Lys
    50


<210>  51
<211>  50
<212>  PRT
<213>  Homo sapiens

<400>  51

Asn Thr Thr Leu Arg Cys Cys Leu Ser Pro Glu Glu Asn Ala Glu Asp
1               5                   10                  15

Met Glu Val Arg Trp Phe Gln Ser Gln Phe Ser Pro Ala Val Phe Val
            20                  25                  30

Tyr Lys Gly Gly Arg Glu Arg Thr Glu Glu Gln Lys Glu Glu Tyr Arg
        35                  40                  45

Gly Arg
    50
```

```
<210>   52
<211>   50
<212>   PRT
<213>   Homo sapiens

<400>   52

Ser Arg Ala Pro His Met Ile Arg Asp Arg Lys Tyr His Leu Lys Thr
1               5                   10                  15


Tyr Arg Gln Cys Cys Val Gly Thr Glu Leu Val Asp Trp Met Met Gln
            20                  25                  30


Gln Thr Pro Cys Val His Ser Arg Thr Gln Ala Val Gly Met Trp Gln
            35                  40                  45


Val Leu
        50


<210>   53
<211>   50
<212>   PRT
<213>   Homo sapiens

<400>   53

Ala Thr Leu Thr Ile Leu His Gln Gln Thr Glu Ala Val Leu Gly Glu
1               5                   10                  15


Cys Arg Val Arg Phe Leu Ser Phe Leu Ala Val Gly Arg Asp Val His
            20                  25                  30


Thr Phe Ala Phe Ile Met Ala Ala Gly Pro Ala Ser Phe Cys Cys His
            35                  40                  45


Met Phe
        50


<210>   54
<211>   50
<212>   PRT
<213>   Homo sapiens

<400>   54

Leu Glu Cys Cys Glu Gly Leu Ala Gln Ser Ile Ile Ser Thr Val Gly
1               5                   10                  15


Gln Ala Phe Glu Leu Arg Phe Lys Gln Tyr Leu His Ser Pro Pro Lys
            20                  25                  30


Val Ala Leu Pro Pro Glu Arg Leu Ala Gly Pro Glu Glu Ser Ala Trp
            35                  40                  45


Gly Asp
```

50

<210> 55
<211> 49
<212> PRT
<213> Homo sapiens

<400> 55

Pro Asp Ser Trp Leu Cys Leu Leu Ala Ile Asp Glu Glu Glu Glu Asp
1               5                   10                  15

Asp Ile Ala Leu Gln Ile His Phe Thr Leu Ile Gln Ser Phe Cys Cys
            20                  25                  30

Asp Asn Asp Ile Asn Ile Val Arg Val Ser Gly Met Gln Arg Leu Ala
            35                  40                  45

Gln


<210> 56
<211> 53
<212> PRT
<213> Homo sapiens

<400> 56

Gly Ser Ile Gln Gln His Phe Leu Lys Pro Leu Gln Arg Phe Leu Lys
1               5                   10                  15

Pro Gln Asp Ile Glu Ile Ile Phe Ile Asn Ile Glu Asp Leu Leu Arg
            20                  25                  30

Val His Thr His Phe Leu Lys Glu Met Lys Glu Ala Leu Gly Thr Pro
            35                  40                  45

Gly Ala Leu Glu Arg
        50


<210> 57
<211> 50
<212> PRT
<213> Homo sapiens

<400> 57

Leu Arg Thr Lys Glu Gln Ile Leu Glu Leu Leu Val Leu Glu Gln Phe
1               5                   10                  15

Leu Thr Val Leu Pro Gly Glu Ile Gln Ala Arg Val Arg Glu Gln Gln
            20                  25                  30

Pro Glu Ser Gly Glu Glu Ala Val Val Leu Val Glu Gly Leu Gln Arg
            35                  40                  45

Lys Pro
        50


<210>  58
<211>  50
<212>  PRT
<213>  Homo sapiens

<400>  58

Ile His Thr Lys Glu Gln Ile Leu Glu Leu Leu Val Leu Glu Gln Phe
1               5               10              15

Leu Thr Ile Leu Pro Gly Asp Leu Gln Ala Trp Val His Glu His Tyr
            20              25              30

Pro Glu Ser Gly Glu Glu Ala Val Thr Ile Leu Glu Asp Leu Glu Arg
            35              40              45

Gly Thr
        50


<210>  59
<211>  50
<212>  PRT
<213>  Homo sapiens

<400>  59

Leu Arg Thr Lys Glu Gln Ile Leu Glu Leu Leu Val Leu Glu Gln Phe
1               5               10              15

Leu Thr Val Leu Pro Gly Glu Ile Gln Gly Trp Val Arg Glu Gln His
            20              25              30

Pro Gly Ser Gly Glu Glu Ala Val Ala Leu Val Glu Asp Leu Gln Lys
            35              40              45

Gln Pro
        50


<210>  60
<211>  50
<212>  PRT
<213>  Homo sapiens

<400>  60

Met Arg Ser Lys Glu Gln Ile Leu Glu Leu Leu Val Leu Glu Gln Phe
1               5               10              15

Leu Thr Ile Leu Pro Thr Glu Ile Glu Thr Trp Val Arg Glu His Cys
            20              25              30

Pro Glu Asn Arg Glu Arg Val Val Ser Leu Ile Glu Asp Leu Gln Arg
            35              40              45

Glu Leu
50

<210> 61
<211> 50
<212> PRT
<213> Homo sapiens

<400> 61

Gly Ala Ile Ser Phe Val Gln Leu Phe Asp Pro Gly Phe Glu Val Gln
1               5                   10                  15

Val Gly Lys Arg Ser Thr Glu Ala Arg His Gly Val Arg Ile Asp Thr
            20                  25                  30

Ser His Arg Ser Leu Ile Leu Lys Cys Ser Ser Tyr Arg Gln Ala Arg
            35                  40                  45

Trp Trp
50

<210> 62
<211> 50
<212> PRT
<213> Homo sapiens

<400> 62

Gly Ala Ile Ala Phe Val Leu Leu Val Asp Lys Glu Phe Lys Ile Lys
1               5                   10                  15

Val Gly Lys Lys Glu Thr Glu Thr Lys Tyr Gly Ile Arg Ile Asp Asn
            20                  25                  30

Leu Ser Arg Thr Leu Ile Leu Lys Cys Asn Ser Tyr Arg His Ala Arg
            35                  40                  45

Trp Trp
50

<210> 63
<211> 50
<212> PRT
<213> Homo sapiens

<400> 63

Gly Met Met Ile Asp Glu Gly Asp Thr Tyr Lys Val Met Asp Gly Glu
1               5                   10                  15

Asn Asn Val Ile Thr Leu His Asn Ala Asp Ile Ser Ile Val Phe Ala
            20                  25                  30

Ala Pro Asp Arg Ala Ser Trp Ile Glu Asp Ile Thr Glu Ala Ile Lys
            35                  40                  45

Asn Ala
50

```
<210>    64
<211>    50
<212>    PRT
<213>    Homo sapiens

<400>    64
```

Val Gly Leu Pro Thr Val Ala Asp Lys Ile Asp Asn His Glu Asn Cys
1               5                   10                  15

Phe Lys Leu Phe Tyr Gly Ser His Thr Tyr Phe Phe Arg Thr Asp Ser
                20                  25                  30

Tyr Tyr Phe Phe Glu Arg Trp Val Asp Ser Ile Phe Gln Ala Ala Ile
        35                  40                  45

Ser Arg
50

```
<210>    65
<211>    50
<212>    PRT
<213>    Homo sapiens

<400>    65
```

Gly Lys Glu Thr Glu Gly Ile Pro Thr Gly Leu Ile Ile Thr Asn Ser
1               5                   10                  15

Gln His Glu Leu His Leu Lys Cys Arg Arg Leu Gln Asp Thr Ala Thr
                20                  25                  30

Trp Lys Tyr Ile Ile Glu Gln Ala Met Gly Gly Ile Gly Asn Thr Trp
        35                  40                  45

Leu Gln
50

```
<210>    66
<211>    50
<212>    PRT
<213>    Homo sapiens

<400>    66
```

Phe Phe Met Arg Lys Val Gln Ile Asn Asp Lys Asp Asp Thr Ser Glu
1               5                   10                  15

Tyr Lys His Ala Phe Glu Ile Ile Leu Lys Asp Gly Asn Ser Val Ile
                20                  25                  30

Phe Ser Ala Lys Ser Ala Glu Glu Lys Asn Asn Trp Met Ala Ala Leu

35           40           45

Ile Ser
50

```
<210>   67
<211>   48
<212>   PRT
<213>   Homo sapiens

<400>   67
```

Cys Val Glu Ser Leu Pro Asp Lys Asp Gly Lys Lys Cys Leu Phe Leu
1               5               10              15

Val Lys Cys Phe Asp Lys Thr Phe Glu Ile Ser Ala Ser Asp Lys Lys
            20              25              30

Lys Lys Gln Glu Trp Ile Gln Ala Ile His Ser Thr Ile His Leu Leu
        35              40              45

```
<210>   68
<211>   50
<212>   PRT
<213>   Homo sapiens

<400>   68
```

Asp Gly Tyr Ser Val Arg Met Asn Asn Thr Leu Arg Lys Asp Gly Lys
1               5               10              15

Lys Asp Cys Cys Phe Glu Ile Ser Ala Pro Asp Lys Arg Ile Tyr Gln
            20              25              30

Phe Thr Ala Ala Ser Pro Lys Asp Ala Glu Glu Trp Val Gln Gln Leu
        35              40              45

Lys Phe
50

```
<210>   69
<211>   50
<212>   PRT
<213>   Homo sapiens

<400>   69
```

Lys Glu Ile Arg Pro Gly Lys Asn Ser Lys Asp Phe Glu Arg Ala Lys
1               5               10              15

Ala Val Arg His Lys Ala Asp Cys Cys Phe Thr Ile Phe Tyr Gly Thr
            20              25              30

Gln Phe Val Leu Ser Thr Leu Ser Leu Ala Thr Asp Ser Lys Glu Asp
        35              40              45

Ala Val
50

<210> 70
<211> 49
<212> PRT
<213> Homo sapiens

<400> 70

Met Asn Lys Lys Thr Lys Arg Leu Asp Ser Leu Gln Leu Lys Gly Arg
1               5                   10                  15

Ile Leu Thr Ser Asn Ile Thr Thr Trp Pro Asn His His Met Gly Ser
                20                  25                  30

Tyr Ala Ile Gln Ile Phe Trp Arg Gly Asp Pro Gln His Glu Ser Phe
            35                  40                  45

Ile

<210> 71
<211> 50
<212> PRT
<213> Homo sapiens

<400> 71

Lys Glu Ile Arg Pro Gly Lys Asn Ser Lys Asp Phe Glu Arg Ala Lys
1               5                   10                  15

Ala Val Arg Gln Lys Glu Asp Cys Cys Phe Thr Ile Leu Tyr Gly Thr
                20                  25                  30

Gln Phe Val Leu Ser Thr Leu Ser Leu Ala Ala Asp Ser Lys Glu Asp
            35                  40                  45

Ala Val
50

<210> 72
<211> 50
<212> PRT
<213> Homo sapiens

<400> 72

Phe Phe Met Arg Lys Val Gln Ile Asn Asp Lys Asp Asp Thr Asn Glu
1               5                   10                  15

Tyr Lys His Ala Phe Glu Ile Ile Leu Lys Asp Glu Asn Ser Val Ile
                20                  25                  30

Phe Ser Ala Lys Ser Ala Glu Glu Lys Asn Asn Trp Met Ala Ala Leu
            35                  40                  45

117

Ile Ser
50

<210> 73
<211> 50
<212> PRT
<213> Homo sapiens

<400> 73

Arg Ala Val Thr His Asn Glu Gln Leu Cys Tyr Gln Leu Tyr Arg Gln
1               5                   10                  15

Pro Ile Pro Val Lys Asp Leu Leu Leu Glu Asp Leu Gln Asp Gly Glu
                20                  25                  30

Val Arg Leu Gly Gly Ser Leu Arg Gly Ala Phe Ser Asn Asn Glu Arg
            35                  40                  45

Ile Lys
50

<210> 74
<211> 50
<212> PRT
<213> Homo sapiens

<400> 74

Glu Gln Leu Pro Val Ala Asp Met Arg Ala Leu Leu Thr Gly Lys Asp
1               5                   10                  15

Cys Pro His Val Arg Glu Lys Gly Ser Gly Lys Gln Asn Lys Asp Leu
                20                  25                  30

Tyr Glu Leu Ala Phe Ser Ile Ser Tyr Asp Arg Gly Glu Glu Glu Ala
            35                  40                  45

Tyr Leu
50

<210> 75
<211> 49
<212> PRT
<213> Homo sapiens

<400> 75

Gly Ala Pro Glu Thr Ser His Arg Pro Glu Lys Glu Trp Pro Ile Lys
1               5                   10                  15

Ser Leu Lys Val Tyr Leu Gly Val Lys Lys Lys Leu Arg Pro Pro Thr
                20                  25                  30

Cys Trp Gly Phe Thr Trp His Glu Thr Glu Lys His Glu Lys Gln Gln
            35                  40                  45

Trp

<210> 76
<211> 50
<212> PRT
<213> Homo sapiens

<400> 76

Lys Lys Leu Val Gly Ser His Arg Leu Ser Ile Tyr Glu Asp Trp Asp
1               5                   10                  15

Pro Phe Arg Phe Arg His Met Ile Pro Thr Glu Ala Leu Gln Val Arg
            20                  25                  30

Ala Leu Ala Ser Ala Asp Ala Glu Ala Asn Ala Val Cys Glu Ile Val
        35                  40                  45

His Val
    50

<210> 77
<211> 50
<212> PRT
<213> Homo sapiens

<400> 77

Lys Asn Ser Arg Trp Thr Glu Gly Leu Ile Ser Ala Ser Lys Ala Val
1               5                   10                  15

Gly Trp Gly Ala Thr Val Met Val Asp Ala Ala Asp Leu Val Val Gln
            20                  25                  30

Gly Arg Gly Lys Phe Glu Glu Leu Met Val Cys Ser His Glu Ile Ala
        35                  40                  45

Ala Ser
    50

<210> 78
<211> 50
<212> PRT
<213> Homo sapiens

<400> 78

Ile Arg Asp Glu Lys Glu Asn Asn Phe Val Leu Glu Gln Leu Leu Tyr
1               5                   10                  15

Phe Asn Tyr Met Ala Ser Trp Val Met Leu Gly Ile Thr Tyr Arg Asn
            20                  25                  30

Asn Ser Leu Met Trp Phe Asp Lys Thr Pro Leu Ser Tyr Thr His Trp

                35                          40                          45

Arg Ala
        50


<210>  79
<211>  45
<212>  PRT
<213>  Homo sapiens

<400>  79

Lys Val Glu Glu Thr Pro Lys Lys Cys Lys Asp Ile Ile Lys Thr Ala
1               5                   10                  15

Arg Ser Leu Leu Asn Asp Val Gln Phe Gly Gln Phe Gly Asp Asp Pro
            20                  25                  30

Lys Glu Glu Val Met Val Leu Glu Arg Ile Leu Leu Gln
            35                  40                  45


<210>  80
<211>  34
<212>  PRT
<213>  Homo sapiens

<400>  80

Leu Ile Leu Gly Lys Leu Pro Val Trp Asn Gly Asn Leu Ile His Tyr
1               5                   10                  15

Ser Gly Gly Tyr Arg Ile Ser Asp Met Arg Glu Cys Ile Glu Leu Met
            20                  25                  30

Phe Gln


<210>  81
<211>  32
<212>  PRT
<213>  Homo sapiens

<400>  81

Arg Glu Ala Gln Glu Pro Arg Thr Leu Gln Glu Ile Ser Ile Ala Ala
1               5                   10                  15

Asn Val Gln Gln Lys Glu Ile Gly Lys Tyr Ile Lys Ile Leu Gly Glu
            20                  25                  30


<210>  82
<211>  33
<212>  PRT
<213>  Homo sapiens

<400>  82

Cys Thr Ile Asn Gly Phe Lys Ser Trp Asn Lys Cys Cys Glu Leu His

| 1 | | | | 5 | | | | | 10 | | | | | 15 |
|---|---|---|---|---|---|---|---|---|---|---|---|---|---|---|

Thr Arg Tyr Ser Glu Glu His Leu Met Val Cys Ser Arg Met Met Val
              20               25                30

Glu


<210> 83
<211> 37
<212> PRT
<213> Homo sapiens

<400> 83

Lys Phe Trp Asp Ser Lys Asn Arg Met Ala Thr Leu Lys Val Leu Gln
1                5               10               15

Asn Leu Cys Cys Asn Gln Tyr Ser Ile Lys Gln Phe Thr Thr Met Glu
          20              25               30

Met His Leu Phe Lys
        35


<210> 84
<211> 50
<212> PRT
<213> Homo sapiens

<400> 84

Ala Lys Val Cys Gln Leu Glu Leu Ser Phe Arg Glu Ile Leu Asn Asn
1                5               10               15

Tyr Lys Lys Glu Ala Gln Cys Lys Pro Glu Val Phe Leu Ser Ile Tyr
          20              25               30

Ile Gly Ser Arg Asn His Asn Gly Val Leu Ile Ser Arg His Val Asp
          35                40             45

Ile Ile
     50


<210> 85
<211> 50
<212> PRT
<213> Homo sapiens

<400> 85

Asp Asp Leu Tyr Ser Tyr Gly Phe Asp Gly Leu His Leu Trp Thr Gly
1                5               10               15

His Val Ala Arg Pro Val Thr Ser Pro Gly Gln His Leu Leu Ala Pro
          20              25               30

Glu Asp Val Ile Ser Cys Cys Leu Asp Leu Ser Val Pro Ser Ile Ser
35 40 45

Phe Arg
50

<210> 86
<211> 50
<212> PRT
<213> Homo sapiens

<400> 86

Asp Asp Leu Phe Ser Tyr Gly Phe Asp Gly Leu His Leu Trp Ser Gly
1 5 10 15

Cys Ile Ala Arg Thr Val Ser Ser Pro Asn Gln His Leu Leu Arg Thr
20 25 30

Asp Asp Val Ile Ser Cys Cys Leu Asp Leu Ser Ala Pro Ser Ile Ser
35 40 45

Phe Arg
50

<210> 87
<211> 50
<212> PRT
<213> Homo sapiens

<400> 87

Val Asp Gly His Phe Val Thr Met Gln Ile Trp Asp Thr Ala Gly Gln
1 5 10 15

Glu Arg Phe Arg Ser Leu Arg Thr Pro Phe Tyr Arg Gly Ser Asp Cys
20 25 30

Cys Leu Leu Thr Phe Ser Val Asp Asp Ser Gln Ser Phe Gln Asn Leu
35 40 45

Ser Asn
50

<210> 88
<211> 5
<212> PRT
<213> Homo sapiens

<400> 88

Val Gly Lys His Val
1 5

<210> 89
<211> 6
<212> PRT

EP 1 695 981 A1

<213> Homo sapiens

<400> 89

Trp Thr Ile His Pro Ala
1               5


<210> 90
<211> 29
<212> PRT
<213> Homo sapiens

<400> 90

Tyr Thr Arg Lys Ala Ala Leu Glu Ala Gln Asn Ala Leu His Asn Met
1               5                   10                  15


Lys Val Leu Pro Gly Met His His Pro Ile Gln Met Lys
            20              25


<210> 91
<211> 20
<212> PRT
<213> Homo sapiens

<400> 91

Gln His Ala Lys Leu Ser Leu Asp Gly Gln Asn Ile Tyr Asn Ala Cys
1               5                   10                  15


Cys Thr Leu Arg
            20


<210> 92
<211> 25
<212> PRT
<213> Homo sapiens

<400> 92

Thr Thr Leu Glu Glu Ala Val Gly Leu Leu Arg Arg Val Asp Gly Phe
1               5                   10                  15


Cys Cys Leu Ser Val Lys Val Asn Thr
            20              25


<210> 93
<211> 23
<212> PRT
<213> Homo sapiens

<400> 93

Cys Cys Leu Ala Val Pro Leu Leu Ala Glu Ala Gly Asp Val Leu Glu
1               5                   10                  15


Leu Ile Ile Ser Arg Lys Pro
            20

<210> 94
<211> 23
<212> PRT
<213> Homo sapiens

<400> 94

Cys Cys Leu Val Val Pro Leu Ile Ala Glu Ser Gly Asn Lys Leu Asp
1               5                   10                  15

Leu Val Ile Ser Arg Asn Pro
                20


<210> 95
<211> 9
<212> PRT
<213> Homo sapiens

<400> 95

Leu Cys Pro Ile Ile Pro Lys Leu Leu
1               5


<210> 96
<211> 9
<212> PRT
<213> Homo sapiens

<400> 96

Leu Arg Met Ile Ser Ser Thr Phe Leu
1               5


<210> 97
<211> 9
<212> PRT
<213> Homo sapiens

<400> 97

Val Gln Pro Ile Thr Ser Ser His Leu
1               5


<210> 98
<211> 13
<212> PRT
<213> Homo sapiens

<400> 98

Asn Pro Phe Lys Glu Leu Lys Gly Gly Cys Val Ile Ser
1               5                   10


<210> 99
<211> 50
<212> PRT
<213> Homo sapiens

<400> 99

Asn Val Ile Gly Ala Arg Arg Ala Ser Trp Arg Ile Ile Ser Ser Ile
1               5                   10                  15

Glu Gln Lys Glu Glu Asn Lys Gly Gly Glu Asp Lys Leu Lys Met Ile
20              25              30

Arg Glu Tyr Arg Gln Met Val Glu Thr Glu Leu Lys Leu Ile Cys Cys
35                  40                  45

Asp Ile
50

<210> 100
<211> 31
<212> PRT
<213> Homo sapiens

<400> 100

Thr Thr Phe Val Ser Lys Asp Ser Arg Gly Ser Val Ala Leu Ile Ile
1           5               10              15

His Asn Val Thr Ala Glu Asp Asn Gly Ile Tyr Gln Cys Tyr Phe
20              25              30

<210> 101
<211> 26
<212> PRT
<213> Homo sapiens

<400> 101

Leu Glu Asp Gly Val Leu Asn His Val Asp Gln Glu His His Phe Gln
1           5               10              15

Asp Lys Tyr Leu Phe Tyr Arg Phe Leu Asp
20              25

<210> 102
<211> 30
<212> PRT
<213> Homo sapiens

<400> 102

Trp Cys Glu Pro Asn Ala Ala Ser Leu Ser Glu Ala Val Gln Ala Ala
1           5               10              15

Cys Met Leu Arg Tyr Gln Lys Cys Leu Asp Ala Arg Ser Gln
20              25              30

<210> 103
<211> 50
<212> PRT
<213> Homo sapiens

<400> 103

Glu Glu Asp Ser Leu Glu His Asn Tyr Tyr Asn Ser Ile Pro Gly Lys
1           5               10              15

Glu Pro Pro Leu Gly Gly Leu Val Asp Ser Arg Leu Ala Leu Thr Gln
                20                  25                  30

Pro Cys Ala Leu Thr Ala Leu Asp Gln Gly Pro Ser Pro Ser Leu Arg
            35                  40                  45

Asp Ala
        50


<210>    104
<211>    50
<212>    PRT
<213>    Homo sapiens

<400>    104

Leu Leu Gly Glu Pro Ala Glu Thr Gln Gly Thr Thr Glu Ala Arg Asp
1                 5                   10                  15

Leu His Cys Leu Leu Val Thr Met Pro His Thr Asp Ala Trp Lys Ser
            20                  25                  30

His Gly Leu Val Glu Val Ala Ser Tyr Cys Glu Glu Ser Arg Gly Asn
            35                  40                  45

Asn Gln
        50


<210>    105
<211>    50
<212>    PRT
<213>    Homo sapiens

<400>    105

Ala Asn Leu Tyr Gln Val Phe Ile Lys Tyr Lys Glu Arg Phe Leu Val
1                 5                   10                  15

Tyr Gly Arg Tyr Cys Ser Gln Val Glu Ser Ala Ser Lys His Leu Asp
            20                  25                  30

Arg Val Ala Ala Ala Arg Glu Asp Val Gln Met Lys Leu Glu Glu Cys
            35                  40                  45

Ser Gln
        50


<210>    106
<211>    7
<212>    PRT
<213>    Homo sapiens

<400>    106

Arg Lys His Arg Gln Arg Gly

1                  5

```
<210>  107
<211>  26
<212>  PRT
<213>  Homo sapiens

<400>  107

Asp Glu Ala Val Leu Gln Val Gln Ala His Glu His Gly Gln Glu Ile
1               5                   10                  15

Phe Gln Lys Lys Val Ser Pro Pro Gly Pro
            20              25


<210>  108
<211>  18
<212>  PRT
<213>  Homo sapiens

<400>  108

Val Lys Ala Trp Arg Gln Asp Val Pro Ser Glu Glu Ala Glu Pro Glu
1               5                   10                  15

Ala Ala


<210>  109
<211>  22
<212>  PRT
<213>  Homo sapiens

<400>  109

Glu Ile Pro Glu Gln Gln Val Asp Met His Asp Ile Leu Leu Glu Glu
1               5                   10                  15

Leu Ala Pro Val Gly Thr
            20


<210>  110
<211>  11
<212>  PRT
<213>  Homo sapiens

<400>  110

Ala Gln Glu Ile Thr Glu Leu Ala Gln Gly Pro
1               5                   10


<210>  111
<211>  11
<212>  PRT
<213>  Homo sapiens

<400>  111

Gly Gly Ala Ile Glu Glu Phe Ile Gln Lys His
1               5                   10
```

```
<210> 112
<211> 11
<212> PRT
<213> Homo sapiens

<400> 112

Pro His Arg Phe Ser Ser Ser Phe Pro Val Arg
1               5               10


<210> 113
<211> 5
<212> PRT
<213> Homo sapiens

<400> 113

Leu Gln Tyr Arg Ser
1               5


<210> 114
<211> 5
<212> PRT
<213> Homo sapiens

<400> 114

Val Leu Gln Asp Met
1               5


<210> 115
<211> 13
<212> PRT
<213> Homo sapiens

<400> 115

Lys Trp Leu Ser Gly Leu Lys Ile Leu His Gln Glu Ala
1               5               10


<210> 116
<211> 23
<212> PRT
<213> Homo sapiens

<400> 116

Leu Lys Leu Arg Asn Glu Glu Ser His Lys Leu Trp Met Ser Val Leu
1               5               10              15

Asn Arg Leu Leu Trp Lys Asn
                20


<210> 117
<211> 13
<212> PRT
<213> Homo sapiens

<400> 117
```

Asn Trp Leu Ser Gly Leu Lys Ile Leu His Gln Glu Ala
1               5               10


<210>    118
<211>    5
<212>    PRT
<213>    Homo sapiens

<400>    118

Leu Gln Tyr Arg Ser
1               5


<210>    119
<211>    40
<212>    PRT
<213>    Homo sapiens

<400>    119

Asn Phe Phe Arg Val Ser Phe Lys Asn Gly Ser Gln Ser Gln Thr His
1               5               10              15


Ser Leu Gln Ala Asn Asp Thr Phe Asn Lys Gln Gln Trp Leu Asn Cys
            20              25              30

Ile Arg Gln Ala Lys Glu Thr Val
            35              40


<210>    120
<211>    24
<212>    PRT
<213>    Homo sapiens

<400>    120

Asn Phe Ile Ala Pro Ser Lys Arg Glu Phe Tyr Leu Trp Thr Asp Gly
1               5               10              15


Leu Ser Ala Leu Leu Gly Ser Pro
            20


<210>    121
<211>    24
<212>    PRT
<213>    Homo sapiens

<400>    121

Tyr Leu Cys Cys Asp Thr Gln Met Glu Leu Arg Glu Trp Phe Ala Thr
1               5               10              15


Phe Leu Phe Val Gln His Asp Gly
            20


<210>    122
<211>    36
<212>    PRT
<213>    Homo sapiens

<400> 122

Lys Ser Glu Ser Glu Gly Arg Pro Glu Arg Val Phe His Leu Cys Cys
1               5                   10                  15

Ser Ser Pro Glu Ser Arg Lys Asp Phe Leu Lys Ala Val His Ser Ile
            20                  25                  30

Leu Arg Asp Lys
            35

<210> 123
<211> 49
<212> PRT
<213> Homo sapiens

<400> 123

Thr Ala Gln Leu Val Ala Ala Ser Lys Val Lys Ala Asp Lys Asp Ser
1               5                   10                  15

Pro Asn Leu Ala Gln Leu Gln Gln Ala Ser Arg Gly Val Asn Gln Ala
            20                  25                  30

Thr Ala Gly Val Val Ala Ser Thr Ile Ser Gly Lys Gln Ile Glu Glu
            35                  40                  45

Thr

<210> 124
<211> 43
<212> PRT
<213> Homo sapiens

<400> 124

Gly Arg Pro Thr Ile Lys Asn Glu Lys Phe Leu Ala Gly Leu Ser Thr
1               5                   10                  15

Asp Gly Phe Trp Asp Ile Gln Thr Phe Lys Val Ile Glu Glu Ala Val
            20                  25                  30

Tyr Phe His Gln His Ser Ile Leu Ala Cys Lys
            35                  40

<210> 125
<211> 8
<212> PRT
<213> Homo sapiens

<400> 125

Thr Phe Tyr Asn Glu Val Phe Val
1               5

```
<210>   126
<211>   38
<212>   PRT
<213>   Homo sapiens

<400>   126

Ile Asn Gly Cys Pro Val Gln Gly Val Phe Glu Ser Phe Asn Leu Asp
1               5                   10                  15

Gly Leu Phe Phe Pro Val Val Ser Phe Ser Ala Gly Val Lys Val Arg
                20                  25                  30

Phe Leu Leu Gly Gly His
            35


<210>   127
<211>   39
<212>   PRT
<213>   Homo sapiens

<400>   127

Ile Asn Gly Gln Pro Val Gln Gly Met Phe Glu Asn Phe Asn Ile Asp
1               5                   10                  15

Gly Leu Phe Phe Pro Val Val Ser Phe Ser Ala Gly Ile Lys Val Arg
                20                  25                  30

Phe Leu Leu Gly Gly Arg His
            35


<210>   128
<211>   50
<212>   PRT
<213>   Homo sapiens

<400>   128

Trp Lys Lys Glu Phe Ile Tyr Tyr Ala Asp Val Lys Glu Pro Glu Ser
1               5                   10                  15

Phe Pro Phe Val Ile Leu Gly Asn Lys Ile Asp Ile Ser Glu Arg Gln
                20                  25                  30

Val Ser Thr Glu Glu Ala Gln Ala Trp Cys Arg Asp Asn Gly Asp Tyr
            35                  40                  45

Pro Tyr
        50


<210>   129
<211>   50
<212>   PRT
<213>   Homo sapiens

<400>   129
```

```
Leu Asp Val Leu Asp Lys His Leu Ile Pro Ala Ala Asn Thr Gly Glu
1               5                   10                  15

Ser Lys Val Phe Tyr Tyr Lys Met Lys Gly Asp Tyr His Arg Tyr Leu
            20                  25                  30

Ala Glu Phe Ala Thr Gly Asn Asp Arg Lys Glu Ala Ala Glu Asn Ser
            35                  40                  45

Leu Val
    50
```

```
<210>   130
<211>   10
<212>   PRT
<213>   Homo sapiens

<400>   130
```

```
Trp Val Pro Tyr Ile Ser Leu Gln Glu Arg
1               5                   10
```

```
<210>   131
<211>   50
<212>   PRT
<213>   Homo sapiens

<400>   131
```

```
Arg Ala Asn Asn Gly Arg Phe Thr Leu Arg Asp Leu Leu Met Val Pro
1               5                   10                  15

Met Gln Arg Val Leu Lys Tyr His Leu Leu Leu Gln Glu Leu Val Lys
            20                  25                  30

His Thr Gln Glu Ala Met Glu Lys Glu Asn Leu Arg Leu Ala Leu Asp
            35                  40                  45

Ala Met
    50
```

```
<210>   132
<211>   14
<212>   PRT
<213>   Homo sapiens

<400>   132
```

```
Cys Ser Leu Pro Trp Asp Val Gly Ser Thr Gly Thr Ala Pro
1               5                   10
```

```
<210>   133
<211>   49
<212>   PRT
<213>   Homo sapiens

<400>   133
```

Asp Asn Met Asp Phe Ser Ser Met Thr Leu Thr Gln Ile Lys Arg Gln
1                   5                   10                  15

Glu Met Asp Ser Gln Val Arg Val Leu Glu Leu Glu Asn Glu Leu Gln
                20              25                  30

Lys Glu Arg Gln Lys Leu Gly Glu Leu Arg Lys Lys His Tyr Glu Leu
            35                  40                  45

Ala


<210> 134
<211> 25
<212> PRT
<213> Homo sapiens

<400> 134

Phe Glu Thr Ser Ala Lys Asp Ala Thr Asn Val Ala Ala Ala Phe Glu
1                   5                   10                  15

Glu Ala Val Arg Arg Val Leu Ala Thr
                20              25


<210> 135
<211> 50
<212> PRT
<213> Homo sapiens

<400> 135

Ala Tyr Lys Ala Ala Ser Asp Ile Ala Met Thr Glu Leu Pro Pro Thr
1                   5                   10                  15

His Pro Ile Arg Leu Gly Leu Ala Leu Asn Phe Ser Val Phe Tyr Tyr
                20              25                  30

Glu Ile Leu Asn Ser Pro Asp Arg Ala Cys Arg Leu Ala Lys Ala Ala
            35                  40                  45

Phe Asp
    50


<210> 136
<211> 17
<212> PRT
<213> Homo sapiens

<400> 136

Arg Asp Leu Ala Gln Cys Val Asn Glu Val Lys Arg Asp Asn Glu Thr
1                   5                   10                  15

Leu


133

```
<210>   137
<211>   45
<212>   PRT
<213>   Homo sapiens

<400>   137

Asp Ala Ile Ala Glu Leu Asp Thr Leu Ser Glu Glu Ser Tyr Lys Asp
1               5                   10                  15

Ser Thr Leu Ile Met Gln Leu Leu Arg Asp Asn Leu Thr Leu Trp Thr
                20                  25                  30

Ser Asp Met Gln Gly Asp Gly Glu Glu Gln Asn Lys Glu
            35                  40                  45
```

**Beschreibung**

Die Erfindung betrifft eine Aminosäuresequenz gemäß SEQ ID Nr. 1, eine Proteinerkennungsdomäne umfassend diese Aminosäuresequenz und die Verwendung der Aminosäuresequenz oder der Proteinerkennungsdomäne zur Identifizierung von Redox-abhängigen Protein-Protein- oder Protein-Lipid-Wechselwirkungen und ein Verfahren zur Detektion von Target-Molekülen (Moleküle, die von SEQ ID Nr. 1 gebunden werden) in Abhängigkeit vom Redoxpotential; die Erfindung betrifft zudem die Verwendung der Aminosäuresequenz als Marker zur Messung des Redoxpotentials in Zellen; weiterhin betrifft die Erfindung die Verwendung der Aminsäuresequenz für den Transport und die Redox-abhängige Freisetzung von an die Seitenkette der Cysteine von SEQ ID Nr. 1 gebundenen Molekülen; die Erfindung betrifft auch die Verwendung der Aminosäuresequenz zur Herstellung einer Aminosäuresequenz-/Polypeptidbibliothek; weiterhin betrifft die Erfindung einen Kit, der die Aminosäuresequenz bzw. die Proteinerkennungsdomäne umfasst; ausserdem betrifft die Erfindung Antikörper gegen Aminosäuresequenzen von SEQ ID Nr. 1 oder der Polypeptidbibliothek, sowie die Verwendung der Aminosäuresequenz in pharmazeutischen Formulierungen.

Oxidativer Stress und ein verändertes Redox-Gleichgewicht in Organismen, insbesondere in Zellen, sind ein wichtiger Indikator und wesentlicher Risikofaktor für zahlreiche Krankheits- und Alterungsprozesse. So ist beispielsweise beschrieben, dass bei altersbedingten neurodegenerativen Erkrankungen, wie z.B. Alzheimer, reaktive Sauerstoffspezien eine herausragende Bedeutung spielen. Weiterhin ist bekannt, dass viele entzündungshemmende Medikamente z.B. für Autoimmunerkrankungen als Antioxidantien wirken. Chronisch entzündliche Erkrankungen gehen oft mit einer Dysfunktion Redox-abhängiger-Prozesse einher, z.B. rheumatoide Arthritis, Hautkrankheiten wie Psoriasis und Hautkrebs, Multiple Sklerose, Tuberkulose und chronische Lungen- und Atemwegserkrankungen wie etwa Asthma pheumoconiosis, nasale Polypen und Lungen-Fibrose.

Es ist außerdem im Stand der Technik offenbart, dass in vielen Tumoren ein prooxidatives Milieu vorherrscht, welches die entarteten Zellen vor extrazellulären Zelltod-Signalen schützt.

Auch in HIV-infizierten Individuen kommt es zum systemischen oxidativen Stress. Daher stellt die Redox-Kontrolle eine wichtige therapeutische Strategie bei HIV-Erkrankten dar. Oxidativer Stress ist auch ein Indikator bei chronischen Nierenleiden.

Hieraus folgt, dass bei allen genannten Erkrankungen Redox-sensitive Inhibitoren als pharmazeutische Mittel zur Diagnose oder Therapie einsetzbar wären, sofern sie zur Verfügung stünden. Aber trotz der Fülle an Beispielen, die die Bedeutung von Redoxvorgängen in Krankheitsprozessen belegen, gibt es bisher keine spezifisch wirkenden Medikamente, die in ihrer Wirkung sensitiv für den Redoxzustand eines Gewebes sind und nur unter pathologischen Redox-Bedingungen wirken. Dies liegt zum einen an der noch mangelnden Kenntnis und Charakterisierung der am Redox-Signaling beteiligten Komponenten, aber auch an der Schwierigkeit, Redox-abhängige Makromoleküle wie Peptide, Proteine aber auch Lipid- bzw. Kohlenhydratstrukturen spezifisch zu inhibieren.

Aufgabe der Erfindung war es daher, ein Polypeptid bzw. eine Aminosäuresequenz bereitzustellen, mit dem Targetmoleküle, deren Inhibition Redox-Potential-abhängig schaltbar ist, detektiert-werden können und die weiterhin zur Identifizierung von Redox-abhängigen Protein-Protein- oder Protein-Lipid-Wechselwirkungen eingesetzt werden können; Aufgabe der Erfindung war es weiterhin, Sonden oder Marker bereitzustellen, die mit den Targetmolekülen spezifisch wechselwirken.

Die Erfindung löst dieses Problem durch die Bereitstellung einer Aminosäuresequenz gemäß SEQ ID Nr.1 oder einer Nukleinsäuresequenz, die für die genannte Aminosäuresequenz kodiert.

Das erfindungsgemäße Nukleinsäuremolekül ist ausgewählt aus der Gruppe umfassend:

a) ein Nukleinsäuremolekül kodierend eine Aminosäuresequenz nach SEQ ID Nr. 1 oder deren komplementären Nukleotidsequenzen,

b) ein Nukleinsäuremolekül, welches mit einer Nukleotidsequenz gemäß a) unter stringenten Bedingungen hybridisiert,

c) ein Nukleinsäuremolekül umfassend eine Nukleotidsequenz, die eine ausreichende Homologie aufweist, um zu einer Nukleotidsequenz gemäß a) oder b) funktionsanalog zu sein,

d) ein Nukleinsäuremolekül, das in Folge des genetisches Codes zu einer Nukleotidsequenz gemäß a) - c) degeneriert ist und

e) ein Nukleinsäuremolekül gemäß einer Nukleotidsequenz nach a) - d), welches durch Deletionen, Additionen, Substitutionen, Translokationen, Inversionen und/oder Insertionen modifiziert und funktionsanalog zu einer Nukleotidsequenz gemäß a) bis d) ist.

Vorteilhafte Ausgestaltungsformen ergeben sich aus den Unteransprüchen.

Die Erfindung soll im folgenden an Hand von vorteilhaften Ausführungsformen und verschiedenen Aspekten der erfindungsgemäßen technischen Lehre näher erläutert werden, wobei nach Darstellung der erfindungsgemäßen Erzeugnisse - d.h. der Moleküle, die auch als Aminosäuresequenzen, als Peptide oder Proteine oder einer Mischung dieser bezeichnet werden können - bevorzugte erfindungsgemäße Verfahren und Anwendungen beschrieben werden: (i) zunächst werden die erfindungsgemäße Aminosäuresequenz SEQ ID Nr. 1, (ii) die räumliche Struktur der Aminosäuresequenz gemäß SEQ ID Nr. 1 und (iii) Erkennungsmoleküle, die gegen diese gerichtet sind, näher erläutert, (iv) folgend werden Herstellungsverfahren der Aminosäuresequenz gemäß SEQ ID Nr. 1 beschrieben, (v) anschließend werden Verfahren zur Gewinnung bevorzugter Modifikationen und Abwandlungen (Muteine) der Aminosäuresequenz gemäß SEQ ID Nr. 1 offenbart, (vi) sowie ein Nachweis- und die Gewinnungsverfahren von modifizierten Aminosäuresequenzen gemäß SEQ ID Nr. 1, die an definierte Targetmoleküle binden, (vii) und abschließend werden vorteilhafte Verwendungen der Aminosäuresequenz gemäß SEQ ID Nr. 1 und der davon abgeleiteten Muteine sowie (viii) noch einmal Vorteile der erfindungsgemäßen Lehre erläutert.

(i) Die erfindungsgemäße Sequenz SEQ ID Nr. 1: Das Nukleinsäuremolekül kodiert die Aminosäuresequenz EKKEQQKEKEK KEQEIKKKFK LTGPIQVIHL AKACCDVKGG KNELSFKQGE QIEIIRITDN PEGKWLGRTA RGSYGYIKTT AVEIDYDSLK LKKD (= SEQ ID Nr.1). In einer bevorzugten Ausführungsform der Erfindung ist das Nukleinsäuremolekül, das eine ausreichende Homologie aufweist, um zu einer Nukleotidsequenz kodierend eine Aminosäuresequenz gemäß SEQ ID Nr. 1 oder deren komplementären Nukleotidsequenzen funktionsanalog zu sein, zumindest zu 40% homolog. Im Sinne der Erfindung heißt, zu den genannten Nukleotidsequenzen bzw. den mit diesen Nukleotidsequenzen hybridisierenden Sequenzen funktionsanalog zu sein, dass die homologen Nuklein- aber auch die kodierten Aminosäuresequenzen ein Verhalten zeigen, das Rückschlüsse auf ein Targetmolekül zulässt, dessen Inhibition Redox-Potential-abhängig schaltbar ist. In einer weiteren vorteilhaften Ausführungsform der Erfindung weist das Nukleinsäuremolekül mindestens 60%, vorzugsweise 70%, bevorzugt 80%, ganz besonders bevorzugt 90% Homologie zu einem Nukleinsäuremolekül kodierend eine Aminosäuresequenz gemäß SEQ ID Nr. 1 oder deren komplementären Nukleotidsequenzen auf, wobei dieses Nukleinsäuremolekül eine biologische Aktivität wie das Nukleinsäuremolekül kodierend eine Sequenz gemäß SEQ ID Nr. 1 oder deren komplementäre Sequenz aufweist. In einer weiteren bevorzugten Ausführungsform der Erfindung ist das Nukleinsäuremolekül eine genomische DNA, eine cDNA und/oder eine RNA.

Die Aminosäuresequenz oder das Polypeptid gemäß SEQ ID Nr. 1 zeigt überraschende Eigenschaften in Bezug auf sein Verhalten unter entweder reduzierenden oder oxidierenden Bedingungen. Insbesondere NMR-spektroskopische Untersuchungen zeigen, dass die beiden benachbarten Cysteine von SEQ ID Nr. 1 reversibel eine intramolekulare Disulfidbrücke bilden können. Das Polypeptid oder Protein liegt dabei in beiden Formen als Monomer vor, wobei sich die Struktur der durch SEQ ID Nr. 1 definierten Aminosäuresequenz unter reduzierenden oder oxidierenden Bedingungen signifikant ändert. Mittels Dithiothreitol (reduzierend) oder Wasserstoffperoxid (oxidierend) kann der Redox-Zustand eindeutig festgelegt werden, da keine weiteren Cysteine ausserhalb der benachbarten Cysteine in SEQ ID Nr. 1 vorkommen. Auch reduziertes und oxidiertes Glutathion, der intrazelluläre Redox-Puffer, eignet sich zur Einstellung einer der beiden Konformationen, es sind aber beliebige Redox-Puffer-Systeme zur Einstellung des Redox-Gleichgewichts der Aminosäuresequenz nach SEQ ID Nr. 1 denkbar. Vorteilhafte Eigenschaften der Aminosäuresequenz nach SEQ ID Nr. 1 sind die sehr gute Löslichkeit, die relativ hohe Temperaturstabilität und die geringe Tendenz zur Aggregation.

Der Gegenstand der Erfindung ist auch eine Proteinerkennungsdomäne, die die Aminosäuresequenz bzw. die von dieser abgeleiteten Mutanten oder Muteine umfasst. Die Proteinerkennungsdomäne kann durch Standardverfahren der Biochemie bzw. Genetik in funktionsanaloge Derivate und strukturhomologe Makromoleküle so umgewandelt werden, dass diese besonders effizient mit Targetmolekülen wechselwirken, deren Inhibition Redox-Potential-abhängig modifizierbar ist. Ein Target im Sinne der Erfindung sind z.B. Zucker, RNA, DNA, Aminosäuren, Vitamine, second messenger, insbesondere Proteine oder Lipide.

(ii) Die Struktur der Aminosäuresequenz gemäß SEQ ID Nr. 1: Durch die Strukturaufklärung wurde eine Domäne identifiziert, die zu einer Familie von Protein-Interaktionsdomänen gehört. Vorteilhafterweise kann durch geeignete Mutationen die Domänen so verändert werden, dass sie als Bindungspartner für beliebige andere Zielmoleküle fungieren kann. Überraschend konnte gezeigt werden, dass die Bindung abhängig vom Redox-Potential (der Umgebung) ist. Es können daher spezifische Zielmoleküle einer Variante der Domäne entwickelt werden, die in der einen Redoxform an das Zielmolekül binden und in der anderen nicht.

Durch NMR-spektroskopische Methoden wurde die dreidimensionale Struktur der Domäne bzw. der Aminosäuresequenz nach SEQ ID Nr. 1 aufgeklärt. Es stellte sich heraus, dass die Domäne durch $H_2O_2$ bzw. Dithiothreitol reversibel in eine oxidierte bzw. reduzierte Form gebracht werden kann. Beide Formen unterscheiden sich strukturell deutlich voneinander. Die strukturelle Umwandlung beruht auf der Oxidation bzw. Reduktion der beiden Cysteine in der Sequenz. Diese strukturelle Umwandlung stellt einen wichtigen Aspekt der Erfindung dar. Die Erfindung betrifft daher auch Peptidstrukturen, die mindestens zwei benachbarte Cysteine aufweisen, die sich Redox-abhängig umwandeln können.

Durch analytische Ultrazentrifugation und NMR-Methoden konnte gezeigt werde, dass sowohl die oxidierte als auch die reduzierte Form der Domäne - also der Aminosäuresequenz nach SEQ ID Nr. 1 - als Monomer vorliegt. Es handelt sich also um eine intramolekulare Reaktion, bei der zwischen den beiden benachbarten Cysteinen eine Disulfidbrücke gebildet wird; es kommt bevorzugt nicht zur Bildung von Dimeren etc. durch Bildung von intermolekularen Disulfidbrücken. Die Domäne bindet an Sequenzen in anderen Proteinen, und an saure Lipide, wie sie z.B. in biologischen Membranen vorkommen. Dabei sind die verschiedenen Domänen der erfindungsgemäßen Peptide jeweils spezifisch in ihrer Bindung für bestimmte Sequenzen, ähnlich wie ein bestimmter Antikörper spezifisch ist.

Die Strukturen der reduzierten und oxidierten Form der erfindungsgemäßen Aminosäuresequenzen, beruhen auf NOE-Restraints, die in Tabelle 3 zusammengefasst sind. Das Charakteristische der Struktur ist eine N-terminale alpha-Helix, die gegen ein beta-Faltblatt gepackt ist und über diverse hydrophobe Kontakte wechselwirkt. Insbesondere befinden sich zwei Cysteine direkt benachbart an den Positionen 34 und 35 dieser Struktur der Aminosäuresequenz gemäß SEQ ID Nr. 1 und können in der oxidierten Variante eine intramolekulare Disulfidbrücke bilden.

Diese Eigenschaft und die Veränderungen in der räumlichen Anordnung der Atome infolge des Cysteinyl-Cystein-Ringschlusses werden durch die beiden dreidimensionalen Strukturen, die auch Gegenstand der Erfindung sind, offenbart. Ein weiterer Aspekt der Erfindung ist auch das Ensemble der 3D-dimensionalen Koordinaten (siehe Beispiele). Elektronische Repräsentationen dieser Koordinaten oder schematische Repräsentationen, welche die Sekundärstrukturelemente und den Verlauf der Kette (Rückgrat-Konformation) enthalten, sind ein weiterer wesentlicher Aspekt der Erfindung. Dies bezieht sich auch auf die elektronische Repräsentation der Struktur eines Muteins, welches eine ähnliche dreidimensionale Faltung wie die Struktur des Polypeptids gemäß SEQ ID Nr. 1 aufweist.

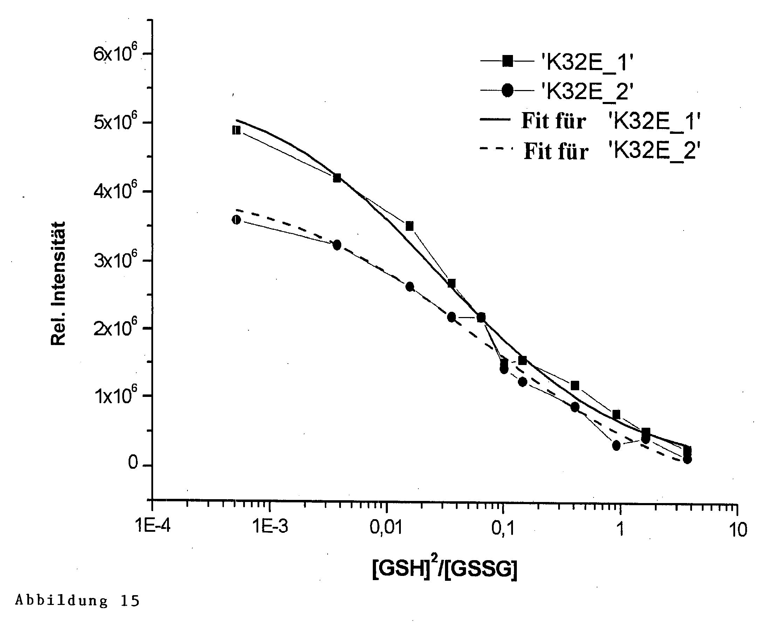

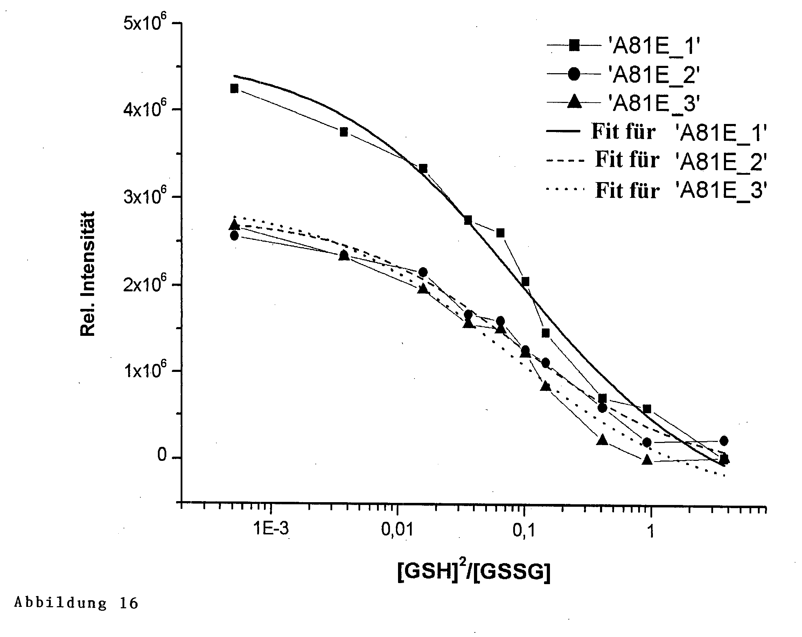

Vorteilhafterweise erlauben die Strukturen gemäß der Erfindung die Anwendung der Methode der rationalen Mutagenese, um das Redox-Potential der Domäne zu verändern. Insbesondere, aber nicht ausschliesslich, werden die Positionen 32, 48, 36-43, 59-64, 78, 80 und 81 gemäß der erfindungsgemäßen Struktur gegen eine der zwanzig natürlichen Aminosäuren ausgetauscht, die dem Fachmann bekannt sind; das Redox-Potential kann z. B. über die NMR-Spektroskopie (wie in den Beispielen angeführt) bestimmt werden. Dadurch werden Varianten des erfindungsgemäßen Proteins erzeugt, die ein spezifisches Redox-Potential besitzen und als Scaffold-Protein verwendet werden können (s. Abb. 15 und 16 und Beispielteil).

Ebenso dient die erfindungsgemäße Struktur als Grundlage für das Einführen von Mutationen, die zu grösseren "Cavities" führen, z.B. unter zu Hilfenahme des Programms VOIDOO (Uppsala Software Factory). Dadurch können gezielt Bindungstaschen, z.B. für Fluorophore, Xenon oder Lipide in das Protein eingeführt werden. Ausserdem erlaubt die Kenntnis der Struktur die Anwendung einer Methode zur Randomisierung von Aminosäuren, welche die Grundlage für die neuen Bindungseigenschaften von Sequenzen nach SEQ ID Nr. 1 oder den Muteinen bildet. Auch das Auffinden von kleinen organischen Molekülen, die an bestimmte Oberflächenstrukturen (wie z.B. hydrophobe Taschen oder elektrostatisch positiv oder negativ geladene Bereiche) binden, wird durch die erfindungsgemäße Struktur vorteilhafterweise ermöglicht.

(iii) Bevorzugte Modifikationen und Abwandlungen der Aminosäuresequenz gemäß SEQ ID Nr. 1: Es ist selbstverständlich möglich, gezielte Abwandlungen und Modifikationen der Aminosäuresequenz vorzunehmen, um eine verbesserte Funktion zu erhalten. Derartig gezielt veränderte Aminosäuresequenzen werden im Sinne der Erfindung als Muteine (siehe oben) bezeichnet. Die erfindungsgemäße Aminosäuresequenz bzw. deren Muteine können durch geeigneten Austausch, Deletion oder Addition von weiteren Aminosäuren oder durch Einfügung eines Glykosidrestes weiterhin modifiziert werden, ohne dass die Fähigkeit der Aminosäuresequenz, mit einem Targetmolekül, dessen Inhibition Redox-Potential-abhängig schlatbar ist, zu interagieren, wesentlich beeinträchtigt ist. Derartige Aminosäuresequenzen, insbesondere Muteine, werden im Sinne der Erfindung auch als funktionsanaloge Derivate oder Strukturhomologe bezeichnet.

Die Muteine sind insbesondere als Redox-sensitive Inhibitoren einsetzbar. Im Sinne der Erfindung ist jedes von der erfindungsgemäßen Aminosäuresequenz (SEQ ID Nr. 1) abgeleitete Polypeptid, welches nur im oxidierten oder reduzierten Zustand an die Target-Moleküle bindet, ein Mutein. Der Redoxzustand des Inhibitors ist sensitiv für die aktuelle Umgebung. Selbstverständlich ist die Redox-Schaltbarkeit keine notwendige Eigenschaft der Muteine. Die Muteine können auch bindende Varianten sein, die krankheitsrelevante Targets nicht Redox-abhängig (also sowohl in reduzierter als auch in oxidierter Form) binden. Auch solche Muteine sind - mit einer nicht-redox-abhängigen Erkennung - bevorzugte Marker oder Sonden der Analyse oder therapeutische Mittel zur Behandlung der oben genannten Krankheiten.

Die Muteine können auch als funktionsanaloge Sequenzen bezeichnet werden. Funktionsanaloge Sequenzen sind im Sinne der Erfindung jene Sequenzen, die der Fachmann als gleichwirkend identifizieren kann. Selbstverständlich ist es möglich, dass der Fachmann aufgrund seiner Kenntnis der im Menschen gefundenen Nukleinsäuremoleküle auch in Versuchstieren Analoge und Homologe aufgrund von Homologie-oder Analogie-Untersuchungen detektiert, mit denen Redox-Vorgänge untersucht werden können.

Funktionsanalog bedeutet insbesondere, dass die abgewandelten Strukturhomologen oder Derivate wie die Aminosäuresequenz gemäß SEQ ID Nr.1 die Fähigkeit besitzen, mit Targetmolekülen messbar zu interagieren bzw. zur Identifizierung von Redox-abhängigen Protein-Protein- oder Protein-Lipid-Wechselwirkungen eingesetzt zu werden. Das heißt, die Aminosäuresequenzen bzw. die Muteine werden durch den Austausch, die Deletion oder die Addition von Aminosäuren oder durch Hinzufügen bzw. Variationen von Zuckerresten nicht so beeinträchtigt, dass ihr Bindungsverhalten hierdurch stark nachlässt.

Für die Aminosäuresequenz nach SEQ ID Nr. 1 können z.B. A-minosäureaustausche auf der Basis, verschiedener Kriterien gemacht werden, die Hydrophobizität, Ladungseigenschaften, Polarität, Größe, die Anwesenheit einer funktionellen Gruppe (z.B. -NH2-Gruppe, aromatischer Charakter) umfassen. Die Zuordnung der Aminosäuren in bestimmte Gruppen wird der geübten Fachkraft gut möglich sein, weitere angemessene A-minosäureaustausche sind z.B. in Bowie et al. (Science 247; 1306-1310 (1990)) zu finden. Z.B. können konservative Aminosäure-Austausche gemacht werden in Bezug auf Aminosäuren, welche verwandte Seitenketten besitzen. Die natürlichen Aminosäuren werden so in vier Gruppen eingeteilt: (1) saure Aminosäuren: Apsartat und Glutatmat. (2) Basische Aminosäuren: Lysin, Arginin und Histidin, (3) nicht-polar: Alanin, Valin, Leucin, Isoleucin, Prolin, Phenylalanin, Methionin, Tryptophan, (4) ungeladen polare Aminosäuren: Glycin, Asparagin, Glutamin, Cystein, Serin, Threonin und Tyrosin. Phenylalanin, Tryptophan und Tyrosin werden manchmal auch'als Aminosäuren mit aromatischen Seitenketten als Gruppe definiert. Es ist z.B. zu erwarten, das ein isolierter Austausch von Leucin zu Isoleucin, von Aspartat zu Glutamat, von Threonin zu Serin oder ein ähnlicher konservativer isolierter Austausch nicht zu einer wesentlichen Veränderung in der Aktivität oder Bindungseigenschaft der Sequenz gemäß SEQ ID Nr. 1 führen wird.

Bevorzugte Aminosäuresequenzen - insbesondere Peptide oder Proteine - im Sinne der Erfindung sind die, die ein Cys-Cys-Motiv enthalten, welches ein reversibel einstellbares Redox-Potential im Bereich von -400 bis +200 mV besitzt, also alle Proteine mit einem Cys-Cys-Motiv, wie z.B. die in Tabelle 4 offenbarten. Auch diese Peptide können bevorzugt an den direkt benachbarten Positionen w-x-Cys-Cys-y-z (w, x, y, z: eine beliebige der zwanzig natürlichen Aminosäuren) durch eine der 20 natürlich vorkommenden Aminosäuren ersetzt sein und ein verändertes Redoxpotential mit Werten zwischen -400 und +200 mV aufweisen.

(iv) Erkennungsmoleküle, die gegen die erfindungsgemäßen Moleküle gerichtet sind: Die Erfindung betrifft auch ein Erkennungsmolekül, das gegen das Nukleinsäuremolekül, den Vektor, die Wirtszelle und/oder das Polypeptid - die Aminosäuresequenz - gerichtet sind. Erkennungssubstanzen im Sinne der Erfindung sind Moleküle, die mit den genannten Strukturen wie Nukleinsäuremolekülen oder -sequenzen, Vektoren, Wirtszellen und/oder Polypeptiden bzw. deren Fragmenten wechselwirken können; insbesondere so wechselwirken, dass eine Detektion dieser Strukturen möglich ist. Die Erkennungssubstanzen können insbesondere spezifische Nukleinsäuren sein, die an das erfindungsgemäße Nukleinsäuremolekülen binden, aber auch Antikörper, Fluoreszenzmarker, markierte Kohlenhydrate oder Lipide, Antisense-Konstrukte, cDNA oder mRNA-Moleküle bzw. deren Fragmente. Es ist selbstverständlich auch möglich, dass die Erkennungssubstanzen nicht Proteine oder Nukleinsäuren bzw. Antikörper sind, sondern gegen diese gerichtete Antikörper. Die Erkennungssubstanzen können in solch einem Fall insbesondere sekundäre Antikörper sein. In einer besonderen Ausführungsform der Erfindung sind die Erkennungsmoleküle Antikörper, Antikörperfragmente und/oder Antisensekonstrukte, z.B. RNA-Interferenzmoleküle. Die Erfindung betrifft also auch Antikörper, welche die Sequenz gemäß SEQ ID Nr. 1, die Muteine oder Fragmente dieser Sequenz binden. Zum Beispiel bezieht sich die Erfindung auf polyklonale oder monoklonale Antikörper, einschliesslich nichthumaner und humaner Antikörper, chimäre Antikörper, humanisierte Antikörper oder Fragmente von Antikörpern, die nach Immunisierung, durch Hybridomazellen oder durch Phage Display oder durch Ribosome Display gewonnen wurden (Current Protocols in Immunology, John Wiley & Sons, N. Y. (1994;) EP application 173,494 (Morrison); International Patent Application WO86/01533; Chem. Rev. 2001, 101, 3205-3218, Ronald H. Hoess, Protein Design and Phage Display);(Hanes & Plückthun, Proc. Nat. Acad. Sci. 94, 4937-4942 (1997); Roberts & Szostak 94, 12297-12302 (1997)). Techniken zur Gewinnung von Immunogenizität gegen das Protein oder Polypeptid umfassen die Konjugation an eine Träger-Substanz oder ein Träger-Molekül, die dem Fachmann wohlbekannt sind. Das Protein kann zudem in Anwesenheit eines Adjuvans für die Immunisierung verwendet werden. Standard ELISA oder Immuno-Assays mit dem Protein der SEQ ID Nr. 1 können benutzt werden um die Menge an Antikörper festzustellen. Auch kann der Antikörper (das Antikörperfragment) mit einem weiteren Molekül verknüpft werden (z.B. ein Fluorophor), welches die bessere Detektion des Antikörpers erlaubt.

(v) Herstellungsverfahren zur Generierung der Aminosäuresequenz gemäß SEQ ID Nr. 1: Die Erfindung betrifft auch einen Vektor, der ein erfindungsgemäßes Nukleinsäuremolekül umfasst. Weiterhin betrifft die Erfindung auch eine Wirtszelle, die den erfindungsgemäßen Vektor umfasst.

Bei der erfindungsgemäßen Aminosäuresequenz kann es sich beispielsweise um rekombinant hergestellte Produkte handeln. Diese Produkte können durch genetische Manipulationen hergestellt werden, bei denen die kodierende Nukleinsäure für das benötigte Produkt gewöhnlich mit Hilfe eines Plasmids oder eines viralen Vektors in einen geeigneten Mikroorganismus oder in eine geeignete Zelllinie eingeführt wird. In der Zelle wird die Nukleinsäure exprimiert und in die Aminosäuresequenz translatiert. Die gewünschte Aminosäuresequenz kann aus den Zellen durch Extraktion und Reinigung gewonnen werden. Die Proteine und Polypeptide der erfindungsgemäßen Anwendung können durch eine Reihe von Prozeßen isoliert und gereinigt werden. Diese umfassen, aber sind nicht begrenzt auf, Anionen oder Kationenaustausch-Chromatographie, High Performance Liquid Chromatography (HPLC), Ni-basierte Chromatographie Hisgetagter Proteine, Streptavidin-Tag basierte Reinigung. Die spezielle Methode zur Reinigung hängen von den speziellen Eigenschaften des Polypeptids und der Wirtszelle ab und sind für die geübte Fachkraft direkt ersichtlich.

Die Transfektion eines Vektors, der die Aminosäuresequenz gemäß SEQ ID Nr. 1 oder eines der Muteine enthält, kann in prokaryotische oder eukaryotische Zellen erfolgen. Zellen, die transfiziert werden können umfassen, aber sind nicht limitiert auf, Bakterien-Zellen (wie etwa E.coli K12-Stämme, Streptomyces, Pseudomonaden, Serratia marcescens und Salmonella typhimurium, Insektenzellen (wie z.B. Sf9-Zellen) einschliesslich Drosophila-Zellen, Pilze wie etwa Hefe (S.cerevisiae, S.pombe, Pichia pastoris), Pflanzenzellen und Säugerzellen wie z.B. Thymocyten, primäre Zellen oder Zellkulturen wie Jurkat, CHO-Zellen oder COS-Zellen.

Die Expressionsrate in E.coli BL21(DE3) ist vorteilhafterweise sehr groß (typischerweise 20 mg gereinigtes Protein pro 1 Liter Bakterienkultur). Als Konsequenz dieser ungewöhnlichen Eigenschaft können auch Muteine der Aminosäuresequenz nach SEQ ID Nr. 1 erzeugt werden, die ebenfalls stabil exprimiert werden und die ein verändertes Redoxpotential aufweisen (s. Beispiele). Eine weitere Eigenschaft der Aminosäuresequenz nach SEQ ID Nr. 1 besteht in der Bindung saurer Lipide, wie Phosphoinositolen. Weiterhin kann die Sequenz gemäß SEQ ID Nr. 1 an Position 86 in einem in vitro Kinase-Versuch phosphoryliert werden und auch in vivo kann diese Phosphorylierung stattfinden. Dadurch wird die Möglichkeit gegeben, die Domäne über phosphospezifische Antikörper oder SH2-Domänen in Kontakt zu bringen und damit analytische nachweisen oder diese zu Reinigungszwecken zu verwenden. Weiterhin können Aminosäuresequenzen nach SEQ ID Nr. 1 oder entsprechende Muteine Vorläufermoleküle aktiver Moleküle sein, d.h. aus diesen Vorläufermoleküle können durch Spaltung aktive Aminosäuresequenzen nach SEQ ID Nr. 1 oder die entsprechenden aktiven Muteine erzeugt werden.

Alternativ kann das Polypeptid der SEQ ID Nr. 1 oder die entsprechenden Muteine auch durch in vitro Translation hergestellt werden. Dabei werden prokayrotische oder eukaryotische Zellextrakte für die Translation einer cDNAgelesenen mRNA in Polypeptid-Sequenzen verwendet. Eine weitere Alternative zur Herstellung des Polypeptids besteht in der chemischen Synthese von SEQ ID Nr. 1.

Die Sequenz der erfindungsgemäßen Aminosäuresequenz SEQ ID Nr. 1 - d.h. EKKEQKEKEK KEQEIKKKFK LTGPIQVIHL AKACCDVKGG KNELSFKQGE QIEIIRITDN PEGKWLGRTA RGSYGYIKTT AVEIDYDSLK LKKD - kann z.B. als rekombinantes Peptid oder als ungetagtes oder z.B. His$_6$-getagtes Protein in dem Bakterium E. coli exprimiert werden.

Die erfindungsgemäße Aminosäuresequenz kann z.B. mit einer Membran-Translokations-Sequenz (MTS) auf der Ebene der DNA verknüpft werden, um bevorzugt Zellmembran-permeabel zu werden. Dadurch wird die Protein-Trasnduktion ermöglicht, d.h. die erfindungsgemäße Aminosäuresequenz kann vorteilhafterweise in Zellen und Gewebe eindringen (Behrens et al. Curr Gene Ther. 2003 Oct;3(5):486-94; Rojas et al., Nat Biotechnol. 16, 370-375 (1998)).

(vi) Bevorzugte Methoden zur Herstellung von Modifikationen und Abwandlungen der Aminosäuresequenz gemäß SEQ ID Nr. 1: In einer bevorzugten Ausführungsform der Erfindung kann die Modifikation des erfindungsgemäßen Polypeptides mit verschiedenen Methoden erfolgen, die insbesondere eine gezielte oder eine zufällige Modifikation ermöglichen. Dem Fachmann sind hierzu verschiedene Methoden bekannt. Dabei können gezielt Nukleotide ausgetauscht werden, so dass eine gewünschte Aminosäuresequenz oder Mutante erzeugt wird. Bevorzugt können randomisierende Methoden (z.B. error-prone-PCR, Verwendung degenerierter Oligonukleotide in der PCR oder bei der Gensynthese, DNA-shuffling, UV-Strahlung) eingesetzt werden, um eine Vielfalt von Muteinen der Aminosäuresequenz gemäß SEQ ID Nr. 1 zu erzeugen. Die Amplifizierung, Klonierung, Ligation etc. der entstehenden NukleotidSequenzen erfolgt insbesondere mit molekularbiologischen Methoden.

Bei besonders bevorzugten Methoden zur Herstellung von Muteinen werden zunächst Variationen einer Ausgangs-DNA oder - RNA erzeugt. Das Ergebnis dieser - mit molekularbiologischen Mitteln umgesetzten - kombinatorischen Methoden ist eine Mischung (Bibliothek) verschiedener Moleküle, die bevorzugt Peptide (als Peptid-/Polypeptid-Bibliothek), Proteine oder Nukleinsäuren (als Protein- oder Nukleinsäure-Bibliothek) sein können. Es haben sich im Stand der Technik verschiedene Methoden zur Generierung dieser Mischung etabliert. Die Mischung kann als Bibliothek verschiedener Moleküle in-vivo, d.h. in Organismen wie z.B. Bakterien, oder in-vitro, beispielsweise in einem Reagenzglas mit Hilfe von Enzymen oder isolierten biologischen Komponenten erzeugt werden.

Als Basis für die Erzeugung der Mischung verschiedener Moleküle dient z.B. entweder die DNA oder RNA in verschiedenen Variationen. Je nach Technik wird diese Variation dann in Proteine übersetzt oder als RNA bzw. DNA getestet. Hierzu dienen spezielle - dem Fachmann bekannte - Selektionssysteme, also Methoden, welche die Proteine oder die DNA bzw. RNA mit den gewünschten Eigenschaften selektieren. Insbesondere sei hier neben dem Phage Display das Ribosome Display (Hanes & Plückthun, Proc. Nat. Acad. Sci. 94, 4937-4942 (1997); Roberts & Szostak 94, 12297-12302 (1997)) erwähnt. Die selektierten Moleküle werden in einem weiteren Schritt angereichert und gegebenenfalls im Detail (Sequenzabfolge, Redoxpotential, pI u.ä.) analysiert. Eine bevorzugte - mit molekularbiologischen Techniken - umgesetzte kombinatorische Methode ist die Phage-Display-Technologie. Hierzu werden bevorzugt Bakteriophagen, d. h. Viren, die Bakterien infizieren, eingesetzt, die an ihrer Oberfläche Proteine exprimieren, die gewisse Veränderungen, beispielsweise zusätzliche Segmente in bestimmten Sequenzbereichen, tolerieren. Dabei werden vor allem Protein-Fusionen des GenIII- oder des GenVIII-Oberflächenproteins verwendet, so dass ein beliebiges Polypeptid auf der Oberfläche des Phagen als Fusionsprotein exprimiert wird. Die Bereitstellung der Bakteriophagen, die kombinatorischen Möglichkeiten der Phage-Display-Technologie, der Aufbau einer Phagen-Bibliothek und ihre Vermehrung sowie das Screening der gewünschten Phagen ist in der Standardliteratur ausreichend beschrieben, so dass diesbezüglich auf die entsprechende Literatur verwiesen werden kann (z. B. Chem. Rev. 2001, 101, 3205-3218, Ronald H. Hoess, Protein Design and Phage Display).

Eine weitere bekannte kombinatorische Methode ist die kombinatorische Mutagenese. Bei dieser Technik erfolgt die Variation der Aminosäuren indirekt auf der DNA-Ebene. Das Verfahren der kombinatorischen Mutagenese, insbesondere das Screening nach Aktivität, bzw. das Screening nach physikalischen Eigenschaften bzw. die Techniken der zirkulären Permutation sind dem Fachmann bekannt.

Weitere bevorzugte kombinatorische Möglichkeiten ergeben sich aus in-vitro-Systemen zur Proteinbiosynthese und der gesteuerten Evolution. Hierzu zählen die Ribosom-Display-Technologie sowie verschiedene Techniken des DNA-shufflings.

Selbstverständlich kann die Polypeptid-Bibliothek nicht nur unter Verwendung der Aminosäuresequenz gemäß SEQ ID Nr. 1 bereitgestellt werden. Bevorzugte Sequenzen sind auch die in Tabelle 4 genannten, welche CC-Motive als Teil einer gefalteten Bindungsdomäne enthalten.

(vi) Nachweis- und Gewinnungsverfahren von an Targetmolekülen bindenden Abwandlungen und Modifikationen der Aminosäuresequenz gemäß SEQ ID Nr. 1: Bevorzugt wird durch Phage Display eine Bibliothek von Varianten der Aminosäuresequenz gemäß SEQ ID Nr. 1 hergestellt. Auf Grundlage der aufgeklärten Struktur werden die Reste 36-43, 59-64, 78, 80 und 81 (Nummerierung in Bezug auf die oben stehende Sequenz) ausgewählt, die in dieser Bibliothek im Prinzip je durch alle zwanzig natürliche Aminosäuren ersetzt werden. Dies entspricht bei 17 variierten Resten einer theoretischen Größe der Bibliothek von ca. $4 * 10^{24}$ Varianten. Bevorzugt ist es, mindestens $1 * 10^8$ Varianten in der Bibliothek zu haben. Diese Bibliothek wird im Weiteren für das Screening verwendet.

Gegenstand der Erfindung ist demgemäss also auch ein Verfahren zur Detektion und Gewinnung von an Targetmolekülen bindenden Polypeptiden umfassend folgende Schritte

a) Immobilisierung eines potentiellen Targetmoleküls auf einem Träger,
b) Inkontaktbringen von Peptiden einer Polypeptid-Bibliothek mit den immobilisierten Targetmolekülen,
c) Eliminieren der nicht an die Targetmoleküle gebundenen Polypeptide,
d) Eluieren der gebundenen Polypeptide unter stringenten Bedingungen, wobei die bindenden Polypeptide gewonnen werden.

Ein Ziel- oder Targetmolekül (z.B: ein Protein, eine Nukleinsäure, ein Zucker oder ein Lipid) wird identifiziert, dessen Funktion beeinflusst werden soll. Von besonderem Interesse sind Target-Moleküle, die eine Rolle im Redox-Signaling spielen, z.B. NFkB, Phosphatasen (SHP-2), Transkriptionsfaktoren, Lipide. Bevorzugt sind auch Target-Moleküle, die nur im oxidierten oder reduzierten Zustand von einer Sequenz gemäß SEQ ID Nr. 1 gebunden werden und unterschiedliche Fluoreszenzeigenschaften im gebundenen versus nicht-gebundenem Zustand besitzen. Vorteilhafterweise wird der Einsatz Redox-Potential-abhängiger Fluoreszenzmarker in der Zelle dadurch ermöglicht.

Das Targetmolekül wird insbesondere auf einer Säule immobilisiert, die Phagenbibliothek wird über die Säule gegeben. Wenn die Bibliothek geeignete Varianten zur Bindung enthält, binden diese an das Targetmolekül, alle anderen Phagen werden direkt von der Säule gespült. Die gebundenen Phagen können anschließend unter strikteren Bedingungen (pH-Wert = 2.2) ebenfalls von der Säule gespült werden. Man erhält so eine Auswahl der ursprünglichen Bibliothek. Die enthaltenen Phagen werden vermehrt und wiederum über die Säule gegeben. Dieser Zyklus wird z.B. 5-10 Mal wiederholt. Dabei wird die Anzahl der Phagen und mithin der Varianten immer kleiner. Schließlich werden die Phagen sequenziert. Dadurch kann im Detail ermittelt werden, welche Varianten der Domäne zur Bindung an das Targetmolekül geeignet sind.

Das Screening der Bibliothek wird zunächst unter oxidierenden/reduzierenden Bedingungen durchgeführt. Nach einigen Zyklen der Auswahl wird das Screening unter den entgegengesetzten Bedingungen fortgesetzt, dabei werden jetzt die Phagen ausgewählt, die nicht binden, also sofort von der Säule eluieren. So erhält man Varianten der Domäne, die nur unter reduzierenden oder oxidierenden Bedingungen an das Zielmolekül binden.

Wichtige zelluläre Target- oder Zielstrukturen, wie etwa die Proteine, die bei der HIV-Infektion eine vermittelnde Rolle spielen, können beispielsweise mittels der erfindungsgemäßen molekularen Bibliothek gescreent werden. Das Screening der Bibliothek mit molekularen Targets, die krankheitsrelevant sind und von denen gezeigt wurde, dass sie eine wichtige Rolle in Redox-abhängigen Prozessen spielen, ist selbstverständlich nicht auf Proteine, die Infektionen - wie beispielsweise HIV-Infektion - vermitteln, beschränkt, sondern betrifft auch Proteine, die bei der Tumorentstehung, bei allergischen Krankheiten und bei Autoimmun-Erkrankungen eine entscheidende Rolle spielen. Beispiele sind die Transkriptionsfaktoren $NF_kB$ und AP-1, die in den Luft-Kanälen asthmatischer Patienten auf Grund einer veränderten Redox-Balance stimuliert werden. $NF_kB$ wird auch durch Angiotensin II stimuliert, welches oxidativen Stress, z.B. bei chronischen Nierenleiden, auslösen kann.

Weiterhin sind Redox-sensitive Phosphatasen als Zielmoleküle bevorzugt, da sie ebenfalls krankheitsrelevant sind. So ist z.B. eine Dysfunktion der SHP-2-Phosphatase mit der Entstehung bestimmter Tumore korreliert. Weiterhin sind Mutationen im Gen der SHP-2 Phosphatase als häufigste Ursache für das Noonan-Syndrom identifiziert worden. Dieses Syndrom tritt mit einer Inzidenz von etwa 1:1000 auf (in Deutschland sind 80.000 Personen betroffen) und ist nach dem Down-Syndrom die häufigste Ursache für eine Fehlbildung des Herzens. Auch bei weiteren kardiovaskulären Krankheiten (CVD) ist die vermehrte Produktion von reaktiven Sauerstoff-Spezies eine wichtige Indikation. Der geringe Effekt "unspezifischer" Anti-Oxidantien als Medikamente in klinischen Studien wird darauf zurückgeführt, dass es bisher keine spezifischen und sensitiven Bio-Marker gibt, die den Redox-Phänotyp von kardiovaskulären Krankheiten beschreiben.

(vii) Weitere vorteilhafte Verwendungen der Aminosäuresequenz gemäß SEQ ID Nr. 1 und der davon abgeleiteten Muteine: In einer bevorzugten Ausgestaltung der Erfindung kann die Aminosäuresequenz nach SEQ ID Nr. 1 oder deren Muteine als Köder-Proteine in einem Yeast-two-hybrid-System oder three-hybrid-System (z.B. U.S. Patent-Nummer 5, 283,317; Zervos et al.(1993), Cell 72: 223-232; Madura et al.(1993), J. Biol. Chem. 268: 12046-12054; Bartel et al. (1993), Biotechniques 14:920-924; Iwabuchi et al. (1993) Oncogene 8; 1693-1696; and Brent WO94/10300) verwendet werden, um andere Proteine zu identifizieren, die mit dem Protein der SEQ ID Nr. 1 oder deren Muteine interagieren und ggf. die Aktivität modulieren. Solche bindenden Proteine können auch in den Prozessen, z.B. der Signaltransduktion in Zellen, an denen das Peptid gemäß SEQ ID Nr. 1 beteiligt ist beteiligt sein. Eine weitere vorteilhafte Ausgestaltung der Erfindung ist ein Screening zur Identifizierung von Modulatoren (z.B. antisense-Moleküle, Polypeptide, Peptidomimetica, kleine Moleküle), welche an Nukleinsäuresequenzen, Polypeptide oder Proteine, die aus SEQ ID Nr. 1 oder deren Varianten bestehen oder hervorgehen, binden oder deren Aktivität, Expression oder andere Eigenschaften verändern.

Eine weitere vorteilhafte Ausgestaltung betrifft einen Assay, in welchem die Muteine mit unterschiedlichem Redox-Potential an ein Träger-Material gebunden oder in Kontakt gebracht werden (z.B. im Plattenformat), wobei die Cysteine der Muteine mit einem Marker-Moleküle (z.B. ein Fluorphor, ein Farbstoff, ein radioaktives Molekül) kovalent verknüpft sind. Dieses Marker-Molekül bleibt bevorzugt unter Bedingungen, die im Vergleich zur Cystein-Marker-Bindung (bevorzugt eine Schwefel-Schwefel-Bindung) oxidierend sind an das Mutein gebunden. Eine Änderung des Redox-Potentials durch eine Lösung mit verändertem Redox-Potential, z.B. ein zelluläres Lysat, oder durch eine Lösung mit verändertem pH-Wert oder einer Lösung mit einer Kombination aus verändertem Redox-Potential und pH-Wert, führt dann zur Freisetzung des Marker-Moleküls. Dabei kann das Marker-Molekül nur entweder im freien oder aber in an dem Peptid nach SEQ ID Nr. 1/Mutein-gebundenen Zustand eine bestimmte Eigenschaft zeigen, die dann zur Messung des Redox-Potentials der Lösung (z.B. des Zell-Lysates) geeignet ist. So kann etwa ein nur in freiem Zustand fluoreszierendes Molekül durch Inkubation mit einer Lösung, deren Redox-Potential bestimmt werden soll, freigesetzt werden. Der Anteil freien Fluorophors steht dann im Verhältnis zum Redox-Potential der Lösung. Möglich ist aber auch, dass das unter bestimmten Redox-Potential-Bedingungen freigesetzte Marker-Molekül in Wasch-Schritten entfernt wird und der Anteil des weiter gebundenen Marker-Moleküls bestimmt wird. Durch den Einsatz von SEQ ID Nr. 1 und deren Muteinen, mit einem über ein weites Spektrum variierenden Redox-Potential wird es möglich sein, durch dieses Verfahren das Redox-Potential einer Vielzahl verschiedener Lösungen, z.B. Zell-Kulturen, Seren, Körperflüssigkeiten, zu bestimmen.

In einer weiteren erfindungsgemäßen Anwendung wird eine Lipid-Modifikation der beiden Cystein-Reste des SEQ ID Nr. 1 oder der Muteine vorgenommen: Die kovalente Verknüpfung der Redox-aktiven Cysteine mit fluoreszierenden Myristoyl- oder Palmitoyl-Lipiden wird als chemische Reaktion durchgeführt. Da die Aminosäuresequenz nach SEQ ID Nr. 1 schon selbst eine Affinität zu negativ geladenen Membranen hat, führt dies zu einer erhöhten Bindung an Membranen. Wird das Protein in Zellen eingeführt (etwa durch Mikroinjektion) ist eine Membran-Rekrutierung zu erwarten. Kommt es jedoch durch Stimulation der Zellen oder durch chemisch inuzierten oxidativen Stress zu einer Veränderung des intrazellulären Redox-Potentials, so wird die Lipid-Gruppe abgespalten und die Aminosäuresequenz nach SEQ ID Nr. 1 wird von der Membran abgelöst werden wobei sich die Fluoreszenz des fluoreszierenden Lipids ändert. Die Änderung des Redoxpotentials führt also zu einer Redox-abhängigen Änderung der Fluoreszenz an der Membran. Alternativ kann das Protein zusätzlich zur Lipid-Modifkation auch Fluoreszenz-markiert werden und die Änderung des Redox-Potentials sollte dann zu einer Abnahme der Fluoreszenz an der Membran führen.

In einer weiteren erfindungsgemäßen Anwendung wird eine Methode zur Verfügung gestellt, bei der die unterschiedlichen Muteine mit variierendem Redox-Potential spezifisch durch bestimmte Antikörper oder deren Antigen-bindende Fragmente erkannt werden. Insbesondere stellt die Methode auch Antikörper zur Verfügung, die nur entweder die reduzierte oder oxidierte Form des jeweiligen Muteins (oder von SEQ ID Nr. 1 selbst) erkennt. Die Methoden zur Gewinnung der Antikörper oder deren Fragmente durch Immunisierungstechniken oder durch in vitro Methdoen (z.B. Phage Display, Ribosome Display) sind dem kundigen Fachmann bekannt und können nach publizierten Protokollen ausgeführt werden. Die Mutein- und Redox-spezifischen Antikörper können in ELISA oder RIA-Assays eingesetzt werden oder aber in Immunopräzipitationsexperimenten und auf Protein-Chips verwendet werden. Dadurch kann der Redox-Zustand von SEQ ID Nr. 1 und den Muteinen in beliebigen Lösungen (deren Redox-Potential. bestimmt werden soll, z.B. zellulären Lysaten) durch die Antikörper und nachfolgende Detektionsmethoden (wie z.B. die Verwendung eines sekundären, Enzym-gekoppelten Antikörpers) nachgewiesen werden.

In einer weiteren vorteilhaften Ausgestaltung der Erfindung werden Zellen mit der DNA des jeweiligen Muteins (in einem Vektor, mit regulatorischen Sequenzen versehen oder als Fusionsprotein) transfiziert. Durch die Nachweisreaktion mit dem Mutein- und Redox-spezifischen Antikörper kann dann der Bereich des Redoxpotentials bestimmt werden. Dabei ist der Bereich durch die Unterschiede der Redox-Potentiale der Muteine und die Spezifität der Antikörper gegeben: Als Beispiel nehme man die Reaktion eines Antikörpers mit einem Mutein, das oxidiert vorliegt, während ein anderes Mutein noch vorwiegend durch reduziertes Mutein erkennenden Antikörper nachgewiesen wird. Besitzt ersteres Mutein etwa ein Redox-Potential von -250 mV, das zweite ein Redox-Potential von -240 mV, so liegt der wahre Wert des Redox-Potentials der Lösung zwischen -240 und -250 mV. Durch Mikroinjektion oder andere Verfahren zur intrazellulären Applikation des Antikörpers kann so auch das intrazelluläre Redox-Potential von Zellen abgeschätzt werden. Wird der Mutein-spezifische Antikörper mit einem Antikörper verbunden (bispezifischer Antikörper oder bispezifisches Antikörperfragment), der eine andere Spezifität besitzt und z.B. ein mit einem bestimmten Zell-Kompartment-assoziiertem Antigen wechselwirkt, so kann in dieser besonderen Variante des Verfahrens das Redox-Potential in bestimmten Kompartmenten der Zelle (z.B. dem Endoplasmatischen Retikulum, dem Zellkern, der Plasmamembran)gemessen werden.

Die Erfindung stellt in einer weiteren vorteilhaften Ausgestaltungsform auch eine Methode zur Verfügung, um biologisch nützliche Agenzien herzustellen, wobei ausgehend von der Analyse von Strukturen der Aminosäuresequenz gemäß SEQ ID Nr. 1 solche Agenzien identifiziert werden. Solche Unter-Strukturen oder Substrukturen können z.B. Epitope für die Herstellung von Antikörpern sein und die Methode umfasst die Entwicklung von Antikörpern gegen das Epitop. Die Substrukturen können auch Mutationsstellen beinhalten, welche die Bindungseigenschaften und die biologische Aktivität des Proteins verändern und die Methode enthält das Verfahren zur Einführung dieser Mutationen. Die Substruktur kann auch einen Verknüpfungspunkt für Anbindung einer seperaten chemischen Gruppe, wie z.B. ein Peptid, ein Polypeptid, ein Festkörpermaterial ("beads", Gele, chromatographische Medien, Chips, Platten, Glasträger), einen Linker und einen Marker (z.B. ein direkter Marker, etwa ein Fluorophor, oder einen indirekten Marker wie z.B. Biotin als Teil eines spezifischen Bindungspaars beinhalten.

In einer weiteren erfindungsgemäßen Anwendung wird insbesondere aufbauend auf einer strukturbasierten Analyse eine Methode zur Verfügung gestellt, um mögliche Metallkoordinierende Aminosäuren an bestimmten Positionen einzuführen (z.B. Cystein, Histidin, Glutamat).

In einer weiteren Anwendung der Erfindung wird eine Methode zur Verfügung gestellt, die eine effiziente Zuordnung der NMR-Resonanzsignale der Muteine ermöglicht. Insbesondere für Muteine, deren dreidimensionale Struktur nicht grundlegend gegenüber der Aminosäuresequenz gemäß SEQ ID Nr. 1 verändert ist, erlaubt der Vergleich mit dem charakterisierten Protein gemäß SEQ ID Nr. 1 ein schnelles Verfahren der Resonanzzuordnung. Aufgrund dieser Resonanzzuordnung kann eine Epitop-Kartierung von Bindungspartnern der Muteine vorgenommen werden. Das Screening von Substanzbibliotheken führt so zu einer Identifizierung der jeweiligen Bindungsstelle. Aufgrund eines Homologiemodeling kann die Bindungsstelle auch als dreidimensionales Modell realistisch wiedergegeben werden.

In einer anderen erfindungsgemäßen Anwendung wird eine Methode zur Verfügung gestellt, welche die Entwicklung von Liganden (Bindungspartnern) und molekularen "Scaffolds" ermöglicht. Aufbauend auf der Struktur und den NMR-Zuordnungen ist es möglich, die Struktur eines Muteinscaffold-Komplexes aufzuklären und die chemischen Strukturen zu identifizieren, die, wenn sie verändert werden, die Bindungsaffinität, die Bindungs-Spezifität oder beides zwischen Mutein und molekularem Scaffold verändern; und die als Grundlage für die Synthese eines Liganden dienen, der eine geänderte Bindungsaffinität, Bindungsspezifität oder beides besitzt. Mit molekularem "Scaffold" ist ein Kernmolekül gemeint, an welches eine oder mehrere chemische Gruppen kovalent verknüpft werden können. Die chemischen Gruppen umfassen, sind aber nicht begrenzt auf, Hydroxyl-, Methyl-, Nitro-, Carboxyl-Gruppen. Molekulare Scaffolds binden an mindestens ein Zielmolekül (z.B. das Polypeptid gemäß SEQ ID Nr. 1 und den Liganden für SEQ ID Nr. 1 oder ein Mutein), und dieses Zielmoleküle kann bevorzugt ein Protein oder Enzym sein. Vorteilhafte Eigenschaften eines "Scaffolds" umfassen die Bindung an ein Zielmolekül-Bindungstasche, so dass ein oder mehrere Substituenten des "Scaffolds" in einer Bindungstasche des Zielmoleküls binden; "Scaffolds" besitzen bevorzugt chemisch veränderbare Gruppen, insbesondere durch chemische Synthese, so dass kombinatorische Bibliotheken erzeugt werden können; "Scaffolds besitzen bevorzugt Positionen, an welche andere chemische Gruppen angeknüpft werden können, die nicht mit der Bindung des "Scaffolds" an das Zielmolekül (z.B. dem Protein bzw. Peptid gemäß SEQ ID Nr. 1) interferieren, so dass das "Scaffold" oder Mitglieder der "Scaffold"-abgeleiteten Bibliothek modifiziert werden können, so dass der "Scaffold"-Zielmolekül-Komplex vorteilhafte Eigenschaften besitzt (z.B. aktiver Transport in Zellen oder bestimmte Organe oder die Möglichkeit den Liganden an ein chromatographisches Material für die weitere Analyse zu binden).

(viii) Vorteile der Erfindung: Es ist überraschend, dass die anmeldungsgemäßen Aminosäuresequenzen, deren Muteine bzw. Proteinerkennungsdomänen die diese umfassen, eingesetzt werden können, um spezifisch Redox-abhängige Ziel- bzw. Targetmoleküle zu detektieren bzw. zu generieren.

Ebenso stellt die Erfindung eine Methode zur Verfügung, um Antikörper oder deren Antigen-bindende Fragmente nur gegen entweder die oxidierte oder reduzierte Form des Proteins über Tier-Immunisierungen, Hybridomazell-Linien oder Phage Display zu erhalten.

Die anmeldungsgemäßen Sequenzen und Domänen bzw. die mit diesen gewonnenen Mitglieder einer Polypeptid-Bibliothek können überraschenderweise als intra- und extrazelluläre Marker in der wissenschaftlichen Analyse aber auch in der Diagnose und Therapie von Krankheiten, die mit oxidativem Stress bzw. mit einem veränderten Redox-Gleichgewicht in den Zellen assoziiert sind, eingesetzt werden. Insbesondere können zelluläre Veränderungen, die mit pathologischen Veränderungen des Redox-Gleichgewichts und mit oxidativen Modifikationen von Proteinen einhergehen, zum einen detektiert aber zum anderen auch moduliert - d.h. gezielt verändert - werden.

Hierbei ist es beispielsweise möglich, dass in einem ersten Schritt eine molekulare Bibliothek auf Basis der erfindungsgemäßen Aminosäuresequenz bzw. der erfindungsgemäßen Proteinerkennungsdomäne, die auch als Redox-Scaffolds bezeichnet werden können, erzeugt werden. Diese Bibliothek kann dann mit einer Reihe klinisch relevanter Targetmoleküle gescreent bzw. durchmustert werden, so dass die Inhibition der Targetmoleküle in-vitro bzw. in-vivo detektiert und getestet werden kann. Ausgehend von den gewonnen Ergebnissen können dann in weitergehenden klinischen Studien im humanen Bereich zur Effizienz und Einsetzbarkeit die aus der molekularen Bibliothek isolierten rekombinanten Peptide oder Proteine getestet und als pharmazeutische Mittel entwickelt werden. Selbstverständlich ist es auch möglich, die gewonnenen Proteine oder Peptide als analytisches Werkzeug für die in-vitro und in-vivo Analyse zellulärer Redoxvorgänge zu verwenden.

Neben der linearen Sequenzabfolge der erfindungsgemäßen Aminosäuresequenz stellt insbesondere die räumliche Struktur derselben einen wesentlichen Aspekt der Erfindung dar. Die Struktur ist die rationale Grundlage für die Erzeugung einer molekularen Bibliothek.

Die vorteilhaften Eigenschaften der erfindungsgemäßen Polypeptide sind die Basis dafür, dass eine molekulare Bibliothek erzeugt werden kann, die eine Vielzahl von $10^8$ bis $10^{10}$ verschiedenen Muteine der Aminosäuresequenz gemäß SEQ ID Nr. 1 enthält.

Der anmeldungsgemäßen Lehre liegt demgemäß die Kenntnis zugrunde, dass Proteine oder Peptide eine herausragende Rolle in allen biologischen Systemen spielen. Als zelluläre Exekutive können sie dabei entweder eine enzymatische Funktion wahrnehmen, eine Rolle als Struktur- oder Stützprotein spielen oder aber über vielfältige Wechselwirkungen mit DNA, Lipiden, Zuckermolekülen oder anderen Proteinen regulatorische Funktionen übernehmen. Auf Grund dieser großen Bedeutung von Proteinen ist es in Wissenschaft und Klinik entscheidend, molekulare Marker zur Untersuchung von Proteinen zur Hand zu haben. Die anmeldungsgemäßen Aminosäuresequenzen bzw. Proteinerkennungsdomänen oder die mit Hilfe dieser gewonnenen Mitglieder einer Polypeptid-Bibliothek können als solche Marker in den verschiedensten Bereichen der Medizin, Biologie oder Chemie eingesetzt werden, etwa bei mikroskopischen Untersuchung von Proteinen, bei der Identifizierung von Proteinen aus größeren Komplexen oder als Bindungsmoleküle in der Protein-Chip-Technologie. Da bekannt ist, dass eine sehr große Anzahl von Protein-vermittelten Prozessen über Mechanismen gesteuert wird, die vom Redox-Potential innerhalb der Zelle abhängig sind, können die erfindungsgemäßen Aminosäuresequenzen bzw. Proteinerkennungsdomänen vorteilhafterweise als molekulare Sonden für die Untersuchung und Steuerung dieser Mechanismen verwendet werden. Weil oxidativer Stress ein wichtiger Faktor bei der Entstehung von Tumoren ist und einen wesentlichen epigenetischen Faktor im Alterungsprozess darstellt, können die erfindungsgemäßen Erzeugnisse selbstverständlich auch als molekulare Sonden innerhalb dieser Prozesse eingesetzt werden.

Auch die Informationsverarbeitung gesunder Zellen wird in vielfacherweise durch Oxidations- oder Reduktionsprozesse (Redox-Gleichgewichte) reguliert. Dabei wirken die Redox-abhängigen Änderungen auf der Ebene von Modifikationen von Nukleinsäuren, Lipiden, Zuckern und insbesondere auch von Proteinen. Insbesondere die Charakterisierung der Veränderung von Proteinen - mittels der erfindungsgemäßen molekularen Sonden - durch die Änderung des Redox-Potentials und die Verfügbarkeit reaktiver Sauerstoffspezies (ROS) ermöglicht die Detektion, aber auch die Modifikation einer Vielzahl biologisch interessanter und medizinisch relevanter ROS-modifizierter Proteine. Die erfindungsgemäßen molekularen Sonden sind daher Marker, die das Redox-Potential in lebenden Zellen, Geweben oder Organismen zum einen charakterisieren und zum anderen modulieren können. Die erfindungsgemäßen Peptide können somit als Sonden bzw. Marker in der Analyse eingesetzt werden, um Redox-sensitive Wechselwirkungen auf Zelloberflächen oder innerhalb der Zellen zu verfolgen. Aus der Analyse der Redox-modifizierten Proteine ergibt sich auch die Möglichkeit, therapeutische Proteine zu detektieren, die krankheitsrelevante Redox-Potential-abhängige Mechanismen unterbinden können. Insbesondere Proteinwirkstoffe, welche spezifisch die bei der Oxidation veränderten pathologischen Mechanismen unterbinden können, sind mit den anmeldungsgemäßen Aminosäuresequenzen, insbesondere mit den Mitgliedern der Polypeptid-Bibliothek, detektier- und modifizierbar. Solche spezifischen Protein-Biologicals waren bisher im Stand der Technik noch nicht bekannt. Die erfindungsgemäßen molekularen Sonden, d.h. die erfindungsgemäßen Aminosäuresequenzen bzw. Peptide, insbesondere die Polypeptid-Bibliothek und deren Muteine aber auch die Proteinerkennungsdomänen sowie die funktionsanalogen Derivate und strukturhomologen Makromoleküle, weisen zahlreiche vorteilhafte Eigenschaften für den Einsatz als molekulare Marker/Sonden auf. Sie sind z.B. sehr gut wasserlöslich, thermisch stabil und tendieren nicht zur Aggregation. Weiterhin sind sie in beiden Zuständen (oxidiert, reduziert) löslich und daher in der gesamten Breite ihrer Anwendung löslich schaltbar. Sie sind sehr klein und können demgemäß gut durch Gewebe dringen. Da sie humanen Ursprungs sind, können sie in Menschen eingesetzt werden, ohne dass es zur Immunreaktion kommt. Insbesondere ist es vorteilhaft, dass ihr Redox-Zustand reversibel regulierbar ist. Das heißt, dass zwei unterschiedliche Zustände der erfindungsgemäßen Aminosäuresequenzen, Peptide oder Proteine existieren, die sich in ihrer Struktur unterscheiden und es daher erlauben, die molekulare Markierung oder therapeutische Inhibition entweder im reduzierten oder im oxidierten Zustand vorzunehmen. Des Weiteren hat sich gezeigt, dass die entsprechenden Aminosäuresequenzen sehr gut in E.coli exprimierbar sind. Die gewonnenen erfindungsgemäßen Aminosäuresequenzen sind über ein einfaches Reinigungsschema gut zu isolieren.

Eine erfindungsgemäße Anwendung ist die kovalente Verknüpfung von der Aminosäuresequenz gemäß SEQ ID Nr. 1 über einen Linker (peptidisch oder nicht-peptidisch) mit einem "caged" compound, welcher Xenon, ein Fluorophor oder andere als Sonden in der Biologie und Medizin einsetzbare Marker-Atome oder Moleküle enthält. Hyperpolarisiertes Xenon stellt als Gas ein sehr vielverprechendes und in der Medizin auch schon eingesetztes Agens zur Detektion von magentischen Resonanz-Signalen in der NMR-Spektroskopie und im MR-Imaging dar. Im Falle des hpyerpolarisierten Xenons kann die Konformationsänderung zwischen oxidiertem und reduziertem Zustand über eine Änderung des Xenon-Signals, welches entweder über die kovalente Verknüpfung des caged compounds oder aber direkt ans Protein gebunden wird, detektiert werden (Spence et al., J. Am. Chem. Soc. 126, 15287-15294 (2004); Lowery et al., Angew. Chem. Int. Ed. 43, 6320-6322 (2004)). Im letzteren Fall werden Mutationen einer hydrophoben Aminosäure, etwa Methionin, Leucin, Valin, Isoleucin, Phenylalanin, Tyrosin oder Tryptophan durch Alanin vorgenommen und eine Oligo-Tyrosin-Sequenz an den C-Terminus des Proteins anhängt.

Entsprechend der erfindungsgemäßen Anwendung kann ein auf ein spezielles Target-Molekül (wie z.B. NFkB, SHP-2) gerichtetes -Mutein eine Methode zur Verfügung stellen, bei der das Mutein in bestimmte Zellen und Gewebe eingeschleust, bzw. dort freigesetzt wird. Dazu stehen dem Fachmann verschiedene Methoden zur Verfügung, z.B. die Einbettung des Muteins in Liposomen oder Polymer-Materialien mit anschliessender Zell-spezifischer Erkennung (z.B. durch Antikörper-Moleküle) und Freisetzung des Muteins.

Die erfindungsgemäße Anwendung umfasst pharmakologische Zusammensetzungen, welche das Polypeptid SEQ ID Nr. 1 oder ein Mutein oder ein Apoprotein umfassen. So kann z.B. das durch SEQ ID Nr. 1 oder eines der Muteine definierte Polypeptid mit einem physiologisch verträglichen Medium gemischt werden. Solche Medien umfassen aber sind nicht beschränkt auf, wässrige Lösungen, Salzlösungen, Polyole (z.B. Glycerol, Propylen-Glykol, flüssiges PolyethylenGlykol) und Dextrose-Lösungen. Die optimale Konzentration der aktiven Komponenten können empirisch entsprechend dem Fachmann wohl bekannten Methoden bestimmt werden und hängen von der jeweiligen gewünschten Zusammensetzung der Pharmaka ab. Methoden zur Applikation des exogenen Polypeptids (inbesondere von SEQ ID Nr. 1 und dessen Muteinen) an die Stelle der krankheitsrelevanten Behandlung umfassen, aber sind nicht beschränkt auf intradermale, intramuskuläre, intraperitoneale, intravenöse, subkutane, orale und intranasale Anwendungen. Andere Methoden können die Gentherapie, wiederbeladbare biologisch abbaubare Partikel, virale Vektoren, nackte DNA und Polymere umfassen. Die pharmazeutische Formulierung dieser Erfindung kann auch als Teil einer kombinierten Therapie mit anderen Agenzien verabreicht werden. Die Sequenz gemäß SEQ ID Nr. 1 oder der Muteine kann auch durch die Methoden der Gentherapie oder ex vivo in Zellen für die Expression in einem Wirbeltier (einschliesslich Homo sapiens) eingeführt werden.

Im Folgenden soll die Erfindung an Hand von Beispielen näher erläutert werden, ohne auf diese Beispiele beschränkt zu sein.

Protein-Expression:

Die Sequenz der hSH3-1-Domäne des humanen ADAP-Proteins (SwissProt 015117, Aminosäuren 485 bis 579) wurde in den Vektor pET24d (Novagen) über die NcoI und XhoI-Restriktions-Schnittstellen subkloniert. Das Konstrukt enthält am N-Terminus zusätzlich die Aminosäure Methionin, und am C-Terminus die Aminosäuren Leucin und Glutamat und einen hexa-Histidin-tag.

Das Plasmid mit der ADAP-hSH3-1-Domäne wurde in den E. *coli* Stamm BL21(DE3) (Invitrogen) transformiert. Eine Vorkultur wurde in 5 ml LB-Medium, das 34 mg/ml Kanamycin enthielt, bei 37 °C, 185 rpm für 12 Stunden inkubiert.

Anschliessend wurde entweder eine 1 Liter LB-Hauptkultur so überimpft, dass eine OD$_{600}$ von 0.05 in der neuen Kultur gemessen wurde (Expression nicht-isotopenmarkierten Proteins), oder 0.5 ml dieser Vorkultur wurden in 100 ml $^{15}$N- oder $^{15}$N/$^{13}$C-markiertes Minimalmedium verdünnt. Das definierte Minimalmedium enthält 60 mM K$_2$HPO$_4$, 11 mM KH$_2$PO$_4$, pH = 7.4, 2 μM Natriumcitrat, 0.8 % (w/v) Glukose ($^{13}$C-markiert für die Expression $^{13}$C-markierten Proteins), 1 mM MgSO$_4$, 0.75 g/l NH$_4$Cl $^{15}$N-markiert für $^{15}$N-markiertes Protein) und 34 mg/ml Kanamycin. Im Falle des Wachstums in LB-Medium wurde die Kultur bei einer OD$_{600}$ von 0.5 mit 1mM IPTG versetzt und für weitere 3 Stunden wachsen gelassen. Dann wurde die Kultur bei 4° C, 6000 rpm für 20 Minuten zentrifugiert. Das Zell-Pellet wurde bei -70° C gelagert.

Im Falle des Wachstums in Minimalmedium wurde die 100 ml-Kultur bei Erreichen einer OD$_{600}$ von 2.0 (nach ca. 12h) mit weiteren 900 ml frischen Mediums verdünnt und weiter bei 37° C und 185 rpm inkubiert. Bei einer OD$_{600}$ von 0.5 wurden die Zellen mit IPTG versetzt (1mM End-Konzentration im Medium), für weitere 4.5 Stunden inkubiert und bei 4° C, 6000 rpm für 20 Minuten zentrifugiert. Das Zell-Pellet wurde bei -70° C gelagert.

Das Zell-Pellet wurde in 20 ml NiHiTrap-Puffer A (enthaltend: 20 mM NaPPi, pH = 7.4, 500 mM NaCl, 20 mM Imidazol) resuspendiert und auf Eis mit Ultraschall zehn mal bei einer Leistung von maximal 120W für 30 Sekunden behandelt, wobei zwischen jedem der Ultraschall-Schritte eine Minute gewartet wurde. Das Zell-Lysat wurde dann für 30 Minuten bei 4° C und 24500 rpm zentrifugiert. Der Überstand wurde durch einen 0.2 μm Spritzen-Filter passiert und auf eine NiHiTrap-Säule (5 ml Volumen, Pharmacia) aufgetragen. Nach dem Waschen mit 20 mM NaPP$_i$, pH = 7.4, 500 mM NaCl, 50 mM Imidazol (8 Säulenvolumen) wurde zur Elution ein linearer Gradient von 10% bis 100% NiHiTrap Puffer B (enthält 20 mM NaPP$_i$, pH = 7.4, 500 mM NaCl, 400 mM Imidazol) gefahren, mit insgesamt 28 Säulenvolumen. Die hSH3-1-Domäne eluierte in den Fraktionen 26 - 34 bei ca. 35% Puffer B. Die Detektion des eluierten Proteins erfolgte über SDS-PAGE und Coomassie-Blau-Färbung. Die relevanten Fraktionen wurden vereinigt und auf eine Protein-Konzentration von 1 mM mittels Centriprep-Zentrifugationsfiltern (MW-cutoff: 3 kDa) eingeengt. Für die NMR-Messungen fand ein Puffer-Austausch gegen 50 mM NaPP$_i$, pH = 7.0, 150 mM NaCl, 0.05 % NaN$_3$-Lösung statt. Um das Protein in die reduzierte Form zu bringen, wurde zwischen 0.2 und 4 mM DTT zugegeben, zur Überführung in die oxidierte Form wurden zwischen 0.2 und 4 mM H$_2$O$_2$ zu der Proteinlösung zugegeben.

Expression von GST-hSH3-1: Die Aminosäuren 490-579 des ADAP-Proteins (SwissProt 015117) wurden über BamH1/Xho1 in den Vektor pGEX4-T1 kloniert und das Plasmid anschliessend in E.coli BL21 (DE3) transformiert. Die Expression erfolgte wie weiter oben für das His-verknüpfte Protein beschrieben. Zur Aufreinigung wurde das Zell-Pellet in 1xPBS (140 mM NaCl, 2,7 mM KCl, 10 mM Na$_2$HPO$_4$ 1,8 mM KH$_2$PO$_4$ pH = 7.3) resuspendiert und ultraschall- behandelt (s.o.) und der filtrierte Überstand auf eine Glutathion-Sepharose Säule (GSTrap 5 ml, Pharmacia) gegeben. Das Protein wurde über einen Schritt-Gradienten (5 SV 12,5%, 5 SV 25%, 2 SV 100% Puffer B, 50 mM Tris pH = 8.0, 40 mM Glutathion red.) eluiert. Die Domäne eluierte in den Fraktionen 10 - 18 bei 25 % Puffer B. Die Detektion des eluierten Proteins erfolgte über SDS-PAGE und Coomassie-Blau-Färbung. Die relevanten Fraktionen wurden vereinigt und gegen 1xPBS dialysiert. Um den GST-Tag abzutrennen, wurden je 25 mg Protein mit 50 U Thrombin (Merck Bioscience) über Nacht bei 8° C inkubiert. Die Probe wurde dann mittels einer Vivaspin-Zentrifugeneinheit (MWC 3 kDa) auf 2 ml konzentriert und über eine Gelfiltrationssäule (Superdex 75, Pharmacia) getrennt. Die hSH3-1-Domäne eluierte nach ca 80 ml. Die Detektion der Proteine erfolgte wie oben beschrieben. Im Folgenden wurde die Domäne wie das his-verknüpfte Protein behandelt.

NMR-Spektroskopie

Zur Aufklärung der Struktur der ADAP hSH3-1-Domäne wurden eine Reihe von NMR-spektroskopischen Experimenten durchgeführt. Alle Experimente wurden bei einer Probentemperatur von 300 K mit einer Proteinkonzentration von 0,2 - 1,5 mM gemessen. Die Spektren wurden an einem Bruker Avance 600-Spektrometer mit Cryo-Probenkopf oder an einem Bruker DMX600-Spektrometer mit Triple-Resonanz-Probenkopf durchgeführt.

Für die Rückgrat-Resonanz-Zuordnung wurde ein Standard-Paar von CBCANNH/CBCACONNH-Spektren (Grzesiek et al., J. Magn. Res. 1992, 99, 201; Grzesiek et al., J. Am. Chem. Soc. 1992, 114, 6291) mit einer einheitlich $^{15}$N/$^{13}$C-markierten Probe aufgenommen und es wurde jeweils ein nahezu komplettes Rückgrat-Assignment für die oxidierte und reduzierte Form der ADAP hSH3-1-Domäne erhalten. Die Reste 1-11 und 91-102 in beiden Formen sind in den Spektren nicht sichtbar, vermutlich aufgrund von erhöhter Flexibilität und damit verbundener geänderter Relaxationseigenschaften dieser Reste. Für die Seitenketten-Zuordnung wurde insbesondere das HCCH-TOCSY-Spektrum verwendet. Es konnten mehr als 95% der Seitenketten-Resonanzen sowohl für die reduzierte als auch für die oxidierte Form des Proteins zugeordnet werden. Ausgehend von der fast vollständigen Zuordnung der Kern-Resonanzen der ADAP-hSH3-1-Domäne wurden anschließend die Signale in den $^{15}$N-NOESY-HSQC, $^{13}$C-HMQC-NOESY und 2D-$^1$H-NOESY-Spektren zugeordnet, welche die zur Strukturberechnung erforderlichen Abstands-informationen liefern (s.u.) Verwendete Software: TOPSPIN (Bruker, Rheinstetten, Deutschland); ANSIG 3.3, P. Kraulis, *ANSIG: A Program for the Assignment of Protein 1H 2D NMR spectra by Interactive Graphics,* J. Magn. Reson. (1989) v 24, pp 627-633)

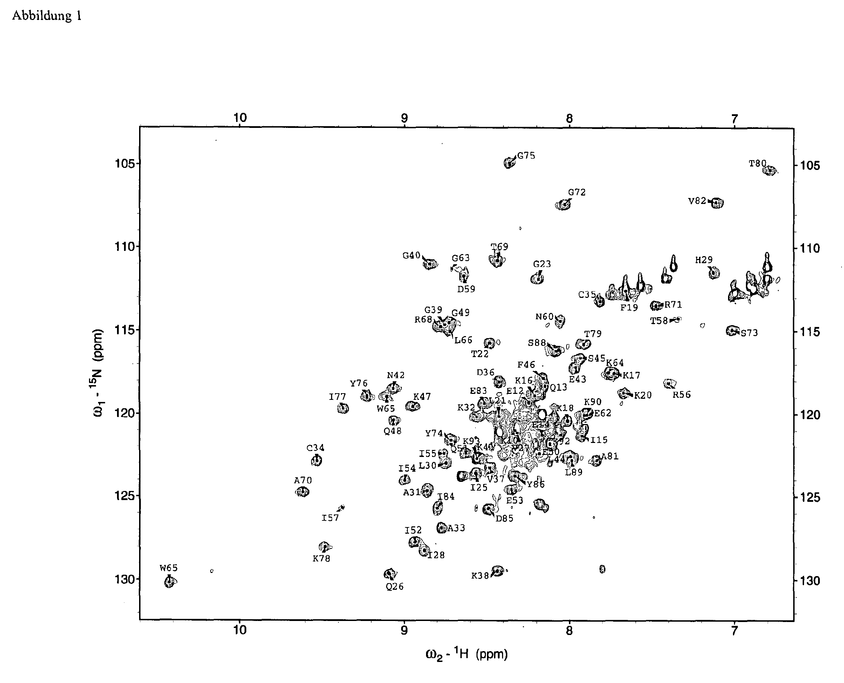

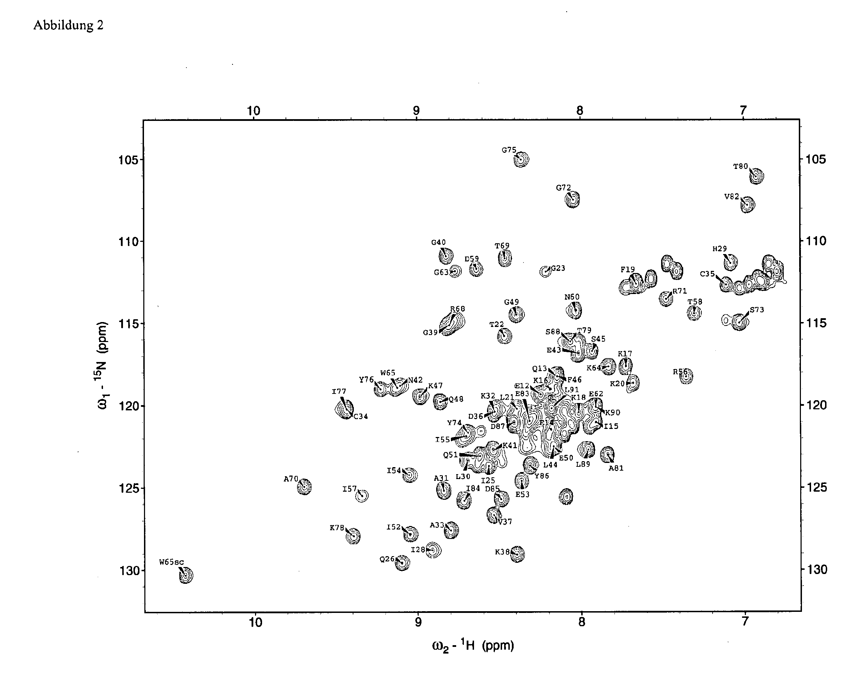

Die Rückgratzuordnung ist in Abb. 1 und 2 am Beispiel des $^{15}N$-$^1H$-*Korrelationsspektrums* ($^{15}$N-HSQC) dar- gestellt. Jedes Signal in diesem zweidimensionalen Experiment stammt von einer bestimmten Rückgrat-NH-Gruppe des Proteins (ausgenommen die NH2-Gruppen von Asparagin und Glutamin und die NH-Gruppe des Tryptophan). Die NH-Gruppe der Seitenkette des Tryptophan-Restes 65 ist mit der Erweiterung sc (side chain) gekennzeichnet.

Während beide Formen der ADAP hSH3-1-Domäne sich in Lösung ähnlich verhalten, was ihre Oligomerisation betrifft, zeigen die $^{15}$N-HSQC-Spektren der beiden Formen deutliche Unterschiede (s. Abb. 1 und 2). Dies deutet darauf hin, daß sie sich auch strukturell unterscheiden.

Die Unterschiede der beiden Spektren sind in Abb. xy dargestellt. Dazu wurde die kombinierte chemische Verschiebung für einen Rest nach Formel 2 (Hajduk et al., J. Med. Chem. 1997, 40, 3144) berechnet:

$$\Delta = |\Delta(\delta^1 H)| + 0,2 * |\Delta(\delta^{15}N)|$$

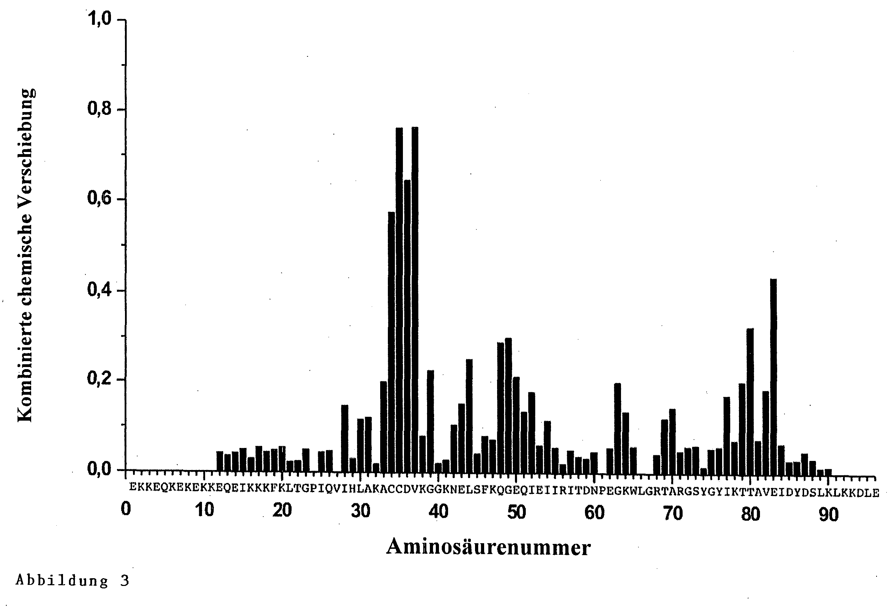

hier ist $\delta^1 H$ die chemische Verschiebung der Rückgrat-Protonen, $\delta^{15}N$ die chemische Verschiebung der Rückgrat-Stickstoffatome. Die deutlichsten Unterschiede sind in der Region um die beiden Cystein-Reste erkennbar (s. Abb. 3).

Basierend auf der Rückgratzuordnung wurden die Relaxationseigenschaften der Rückgrat-NH-Gruppen der beiden Formen des Proteins gemessen. Diese erlauben eine Aussage über die interne Flexibilität der Domäne und die Bestimmung des Oligomerisationsgrades des Proteins unter den gegeben Puffer- und Konzentrationsbedingungen.

Zu diesem Zweck wurde eine Serie von $^{15}$N $T_1$- und $T_2$-Relaxationsexperimenten (Farrow et al., Biochemistry 1997, 36, 2390) gemessen ($T_1$ und $T_2$ sind charakteristische Relaxationszeiten). Zur Messung von $T_1$ wurde der Delay (ein experimenteller Parameter) in sukzessiven Experimenten auf 14, 70, 140, 210, 280, 420, 560, 840, 1.260, 2.800 und 7.000 $\mu$s gesetzt, in den analogen $T_2$-Experimenten wurde der entsprechende Delay auf 6, 12, 24, 36, 48, 60, 120, 180, 240, 300 und 360 $\mu$s gesetzt. Die resultierenden Spektren sind einem $^{15}$N-HSQC ähnlich. Für jeden Rest des Proteins wird die Signalintensität in jedem Spektrum bestimmt und gegen den Delay aufgetragen.

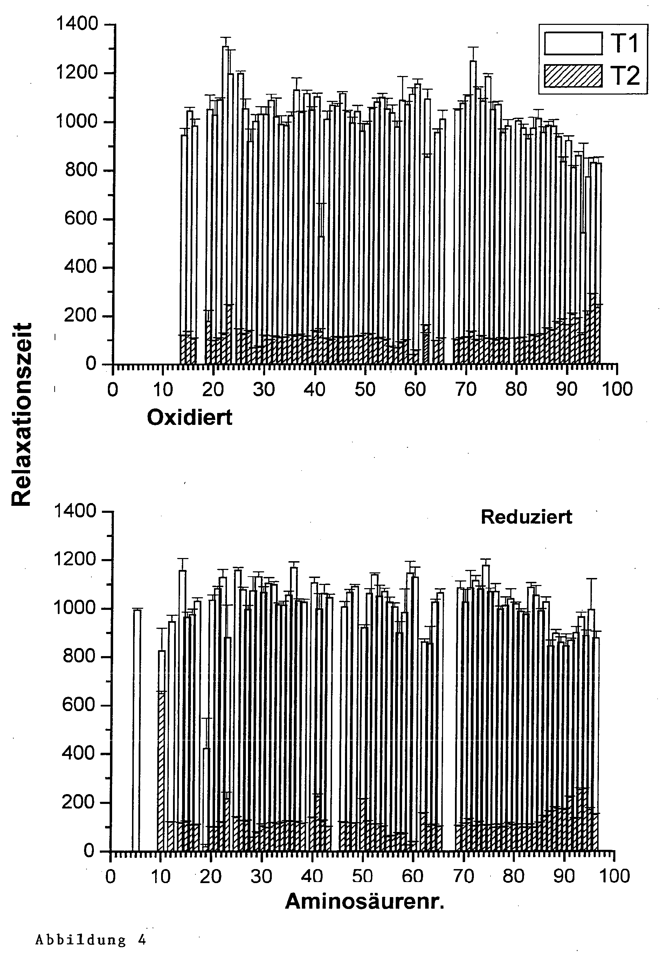

Durch Regression mit einer einfach exponentiellen Funktion werden für jeden Rest die $T_1$- und $T_2$-Relaxationszeiten bestimmt, die erhaltenen Werte sind in Abbildung 4 gezeigt. Für die Regression wurde das Programm Sparky benutzt (Version 3.1, T.D. Goddard und D. G. Kneller, SPARKY 3, University of California, San Francisco).

Aus der weitgehend einheitlichen Verteilung der $T_1$- und $T_2$-Relaxationszeiten innerhalb der beiden Formen der ADAP hSH3-1-Domäne kann geschlossen werden, daß das Protein jeweils eine stabile Domäne mit geringer interner Mobilität bildet.

Weiterhin kann aus den Werten der $T_1$- und $T_2$-Relaxationszeiten der Oligomeristaionsgrad der Proteine abgeleitet werden. Dazu wird jeweils der Mittelwert der $T_1$ und $T_2$-Relaxationszeiten berechnet und mit Hilfe der nachstehenden Formel die Korrelationszeit der Rotation der Proteine berechnet. Die Korrelationszeit ist ein Maß für das Molekulargewicht eines Körpers und erlaubt daher Rückschlüsse auf eine mögliche Oligomerisierung der Proteine(Gryk et al., J. Mol. Biol. 1998, 280, 879):

$$\tau_C = \frac{\left\{\left[6\left(T_1/T_2\right) - 7\right]\frac{1}{4}\right\}^{1/2}}{\Omega_N \bullet 2\pi} \quad (1)$$

hier sind $T_1$ und $T_2$ die mittleren $^{15}$N-Relaxationszeiten und $\Omega_N$ ist die Larmorfrequenz für $^{15}$N bei einer gegebenen magnetischen Feldstärke (hier ist $\Omega_N$ = 60.827865 MHz).

Tabelle 1 zeigt die Werte für die mittleren $T_1$- und $T_2$-Relaxationszeiten der beiden Formen der ADAP hSH3-1-Domäne sowie die daraus berechneten Korrelationszeiten $\tau_c$:

| | $T_1$ [ms] | $T_2$ [ms] | $\tau_c$ [ns] |
|---|---|---|---|
| Reduziert | 1.010,92 | 126,44 | **8,4** |
| Oxidiert | 1.012,56 | 122,39 | **8,5** |

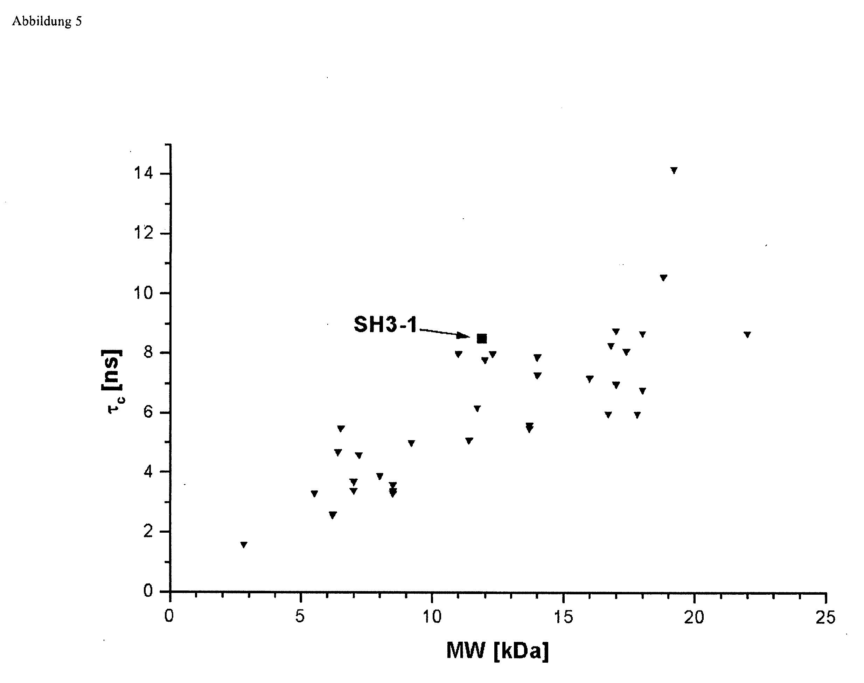

Im Rahmen der Messgenauigkeit sind die Korrelationszeiten für beiden Formen der Domäne gleich. Das erlaubt den Schluss, das beide Formen auch den gleichen Oligomerisationsgrad haben. Der Vergleich mit Korrelationszeiten anderer Proteine, deren Oligomeristaionsgrad bekannt ist, zeigt, dass die ADAP hSH3-1-Domäne sowohl in der reduzierten als auch in der oxidierten Form als Monomer vorliegt (s. Abbildung 5). Dies ist selbst bei den unphysiologisch hohen Konzentrationen der Fall, unter denen die NMR-Experimente durchgeführt wurden. Man darf mit Sicherheit davon ausgehen, daß die Domäne bei niedrigeren Konzentrationen ebenfalls als Monomer vorliegt.

Bestimmung des Redox-Potentials:

Das Redox-Potential der Umwandlung der ADAP hSH3-1-Domäne von der reduzierten in die oxidierte Form wurde durch eine NMR-Messung bestimmt. Das Protein wurde in der oxidierten Form vorgelegt (Konzentration 0,1 mM). Der Standard-NMR-Puffer (s.o.) wurde zusätzlich mit 0,2 mM oxidiertem Glutathion (GSSG) versetzt, sukzessive wurde dann reduziertes Glutathion (GSH) zugesetzt. Die Konzentrationen sind in der Tab. genannt:

| Exp.-Nr. | 1 | 2 | 3 | 4 | 5 | 6 | 7 | 8 | 9 | 10 | 11 |
|---|---|---|---|---|---|---|---|---|---|---|---|
| GSH [mM] | 0,2 | 0,5 | 1,0 | 1,5 | 2,0 | 2,5 | 3,0 | 5,0 | 7,5 | 10,0 | 15,0 |
| GSSG [mM] | 0,2 | 0,2 | 0,2 | 0,2 | 0,2 | 0,2 | 0,2 | 0,2 | 0,2 | 0,2 | 0,2 |

Über diese Serie von $^{15}$N-HSQC-Experimenten wurde dann die Intensität geeigneter Signale verfolgt. Geeignet sind hier solche Reste, deren Signale in den Spektren der ganz oxidierten und ganz reduzierten Form nicht überlappen und die auch nicht mit Signalen anderer Reste überlappen. Ausgewählt wurden daher die Reste Cys 34, Cys 35, Gln 48, Gly 49 und Thr 80.

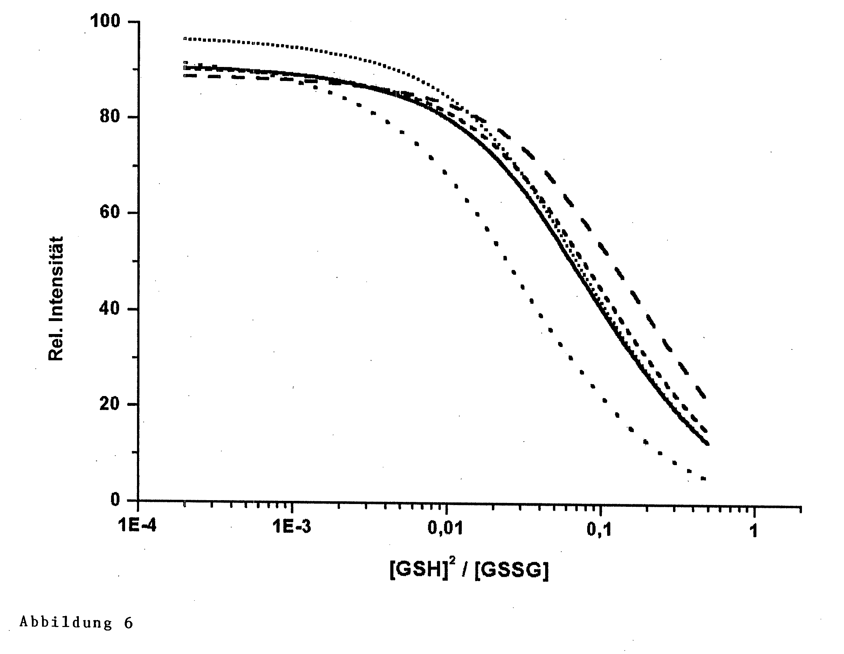

Abb. 6 zeigt den Verlauf der Signalintensitäten dieser Reste, aufgetragen gegen [GSH]$^2$/[GSSG]. Hierfür wurde die jeweils höchste Intensität willkürlich auf 100 skaliert. Die verschiedenen Reste zeigen ein vergleichbares Verhalten. Die Regression liefert einen Wert von 0,05 für den Wendepunkt der Kurven. Daraus lässt sich mit Hilfe der Nernst'schen Gleichung das Redox-Potential der Domäne berechnen:

$$E = E_0 - \frac{RT}{zF} \ln \frac{[GSH]^2}{[GSSG]}$$

hier ist $E_0$ = - 240 mV (Standard-Potential für GSH/GSSG), R = 8,314 JK$^{-1}$mol$^{-1}$ (ideale Gaskonstante), T = 300 K, z = 2 (Anzahl der übertragenen Elektronen) und F = 9,648 10$^4$ (Faraday-Konstante). Als Redox-Potential wird - 204 mV berechnet.

Dieser Wert liegt im Bereich des intrazellulären Milieus, wie es in verschiedenen Stadien der Zellreifung vorliegt. In ruhenden Zellen liegt das Redox-Potential bei ca. -150 mV, unter diesen Bedingungen wird die hSH3-1-DOmäne bevorzugt im reduzierten Zustand vorliegen. In sich teilenden Zellen liegt das Potential bei ca. -200 mV, in apoptotischen Zellen kann es auch bei -250 mV liegen. Hier wird die hSH3-1-Domäne im oxidierten Zustand vorliegen.

Bestimmung der Schmelzpunkte

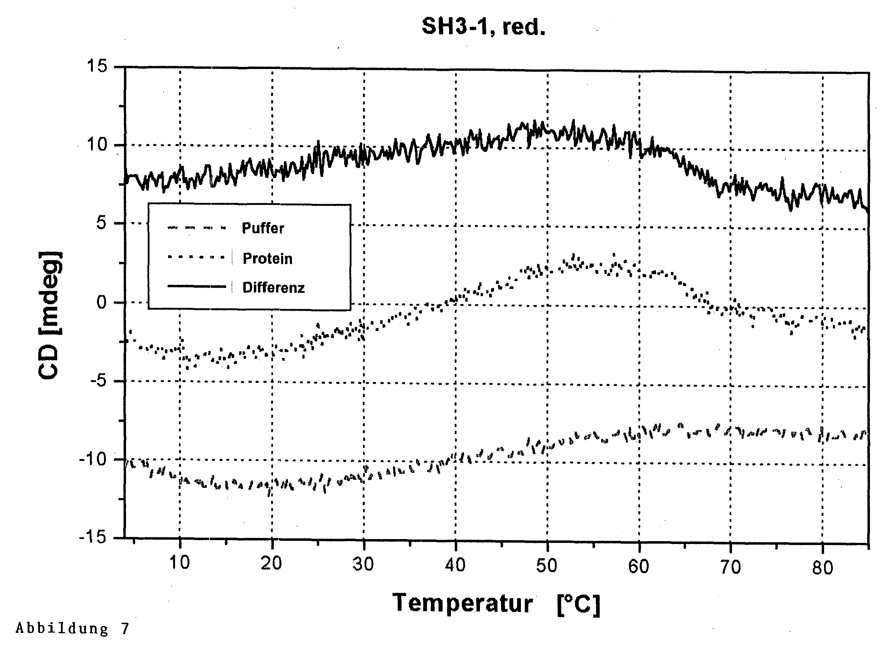

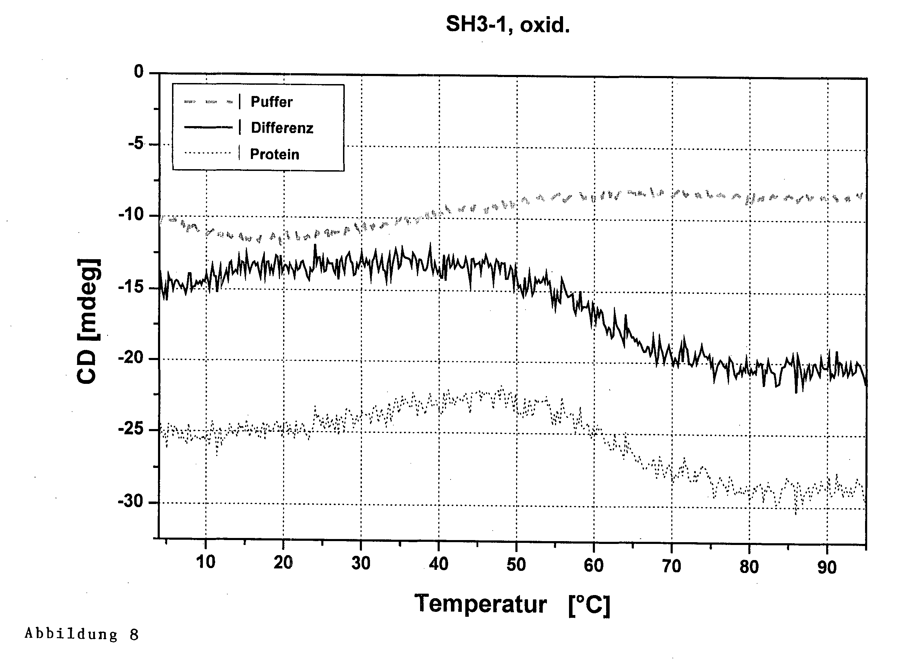

Mit Hilfe von CD-Spektroskopie wurde die TemperaturStabilität der reduzierten und oxidierten Form der ADAP hSH3-1 Domäne untersucht. Dazu wurde jeweils eine 15 μM Probe in NMR-Puffer in ein CD-Spektrometer (Jasco 720) eingebracht. Das CD-Signal wurde bei 221 nm verfolgt, während die Temperatur der Probe kontinuierlich von +4° auf +95° C erhöht wurde. Die Temperaturkurve des Puffers wurde von der so erhaltenen Kurve abgezogen. Die Ergebnisse sind in den Abb. 7 und 8 dargestellt. Beide Formen der ADAP hSH3-1 Domäne haben einen Schmelzpunkt von ca. 60° C und zeigen damit eine hohe thermische Stabilität.

Basierend auf der Auswertung der NOESY-Spektren (s.o.) wurden die Strukturen der reduzierten und oxidierten Form der ADAP hSH3-1- Domäne berechnet. Die Berechnungen wurden mit Hilfe des Programms CNS 1.1 ("Crystallography and NMR System (CNS): A new software system for macromolecular structure determination". Brunger A.T., Adams P.D., Clore G.M., Delano W.L., Gros P., Grosse-Kunstleve R.W., Jiang J.-S., Kuszewski J., Nilges N., Pannu N.S., Read R.J., Rice L.M., Simosnson T., and Warren G.L. ACTA CRYST. D54, 905-921 (1998)) auf UNIX-basierten Rechnern durchgeführt.

Folgende experimentelle Daten waren Grundlagen für die Berechnungen:

- Abstandsinformationen, basierend auf der Zuordnung der NOESY-Spektren.
  Für die reduzierte Form konnten 1119 Abstandsrestraints bestimmt werden, für die oxidierte Form waren es 999 Restraints. Eine genaue Auflistung und ein Vergleich der Restraints für beide Formen wurde in Tabelle 2 zusammengestellt .

- Restraints für die Rückgrat-Diederwinkel $\phi$ und $\psi$, diese Restraints basieren auf einer statistischen Auswertung der chemischen Verschiebungen der C$\alpha$- und C$\beta$-Kohlenstoffatome für die einzelnen Reste (TALOS, (Cornilescu et al., J. Biomol. NMR 1999, 13, 289)).
- Wasserstoffbrückenbindungen; Donor-NH-Gruppen wurden durch Austausch des wässrigen Puffers gegen $D_2O$ bestimmt, Akzeptor-Sauerstoffatome konnten während mehrerer Runden der Strukturberechnung identifiziert werden.

Die Strukturberechnung wurde unter Verwendung eines CNS-Standard-Protokolls für Torsional Angle Dynamics durchgeführt. Die Parameter der Berechnung wurden wie folgt gesetzt:

Tabelle 2

| High Temperature annealing | |
|---|---|
| Start Temperature | 50000 |
| Number of Steps | 3000 |
| Van der Waals scale Factor | 0.1 |
| NOE scale Factor | 50 |
| Dihedral Angle Scale Factor | 100 |
| MD Timestep | 0.015 |
| **First Slow Cool Annealing** | |
| Temperature | 50000 |
| Number of Steps | 20000 |
| Van der Waals Scale Factor | 1 |
| NOE Scale Factor | 50 |
| Dihedral Angle Scale Factor | 100 |
| MD Timestep | 0.015 |
| Temperature Step | 250 |
| **Second Slow Cool Annealing** | |
| Temperature | 3000 |
| Number of Steps | 10000 |
| Van der Waals Scale Factor | 1 |
| NOE Scale Factor | 50 |
| Dihedral Angle Scale Factor | 100 |
| MD Timestep | 0.005 |
| Temperature Step | 25 |
| **Final Minimization** | |
| NOE Scale Factor | 50 |
| Dihedral Angle Scale factor | 100 |
| Number of Steps | 200 |
| Number of Cycles | 10 |

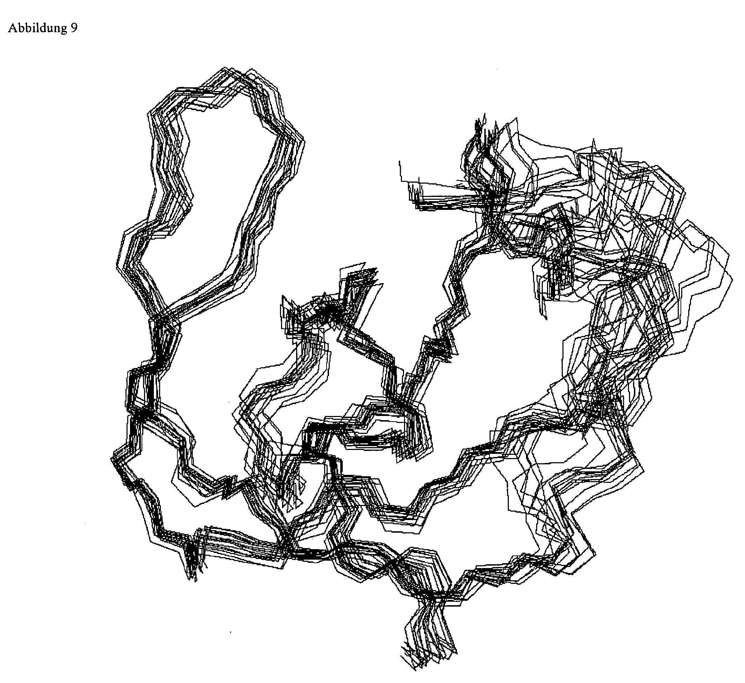

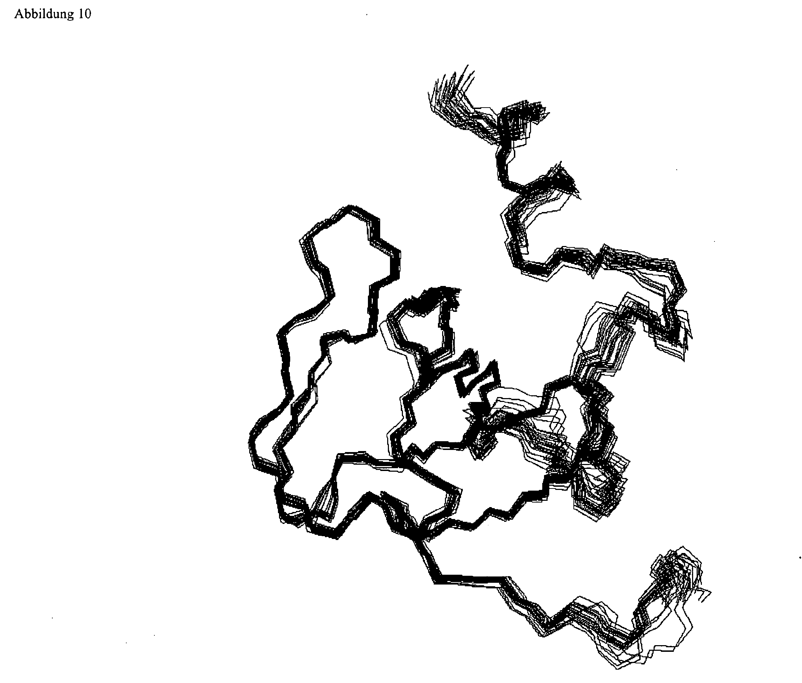

Zur Repräsentation der Lösungsstruktur der ADAP hSH3-1-Domäne wurden für beide Formen jeweils die 20 Strukturen mit der niedrigsten Gesamtenergie ausgewählt. Diese Ensembles sind in Abbildung 9 (reduziertes hSH3-1) und 10 (oxidiertes SH3-1) dargestellt.

Die beiden Ensembles können wie nachstehend näher charakterisiert werden (Tabelle 3):

Tabelle 3:

|  | Reduzierte Form | Oxidierte Form |
|---|---|---|
| Abstandsrestraints |  |  |
| Gesamt | 1119 | 999 |
| "Long-Range" (> i,i+4) | 384 | 305 |
| Diederwinkel | 97 | 110 |
| Wasserstoffbrücken | 0 | 0 |
| Ramachandran-Statistik (%) |  |  |
| Bevorzugt |  | 78.1 |
| Zusätzlich erlaubt |  | 17.5 |
| Weiterhin erlaubt |  | 2.6 |
| Verboten |  | 1.8 |
| RMS-Daten [Å] |  |  |
| Sekundärstrukturelemente |  | 0.29 |
| Geordnete Bereiche |  | 0.41 |
| AS 12- 87 |  | 0.66 |

Aus der Literatur ist bekannt, dass der Rückgratwinkel von vicinalen Cysteinen, die über eine Disulfidbrücke einen Achtringbilden, auch die cis-Konformation annehmen kann. Daher wurde im vorliegenden Fall die Strukturberechnung für die oxidierte Form der hSH3-1-Domäne sowohl mit einer vordefinierten trans- als auch cis-Bindung durchgeführt. Für die cis-Konformation ergibt sich bei gleichem Restraint-Satz und ansonsten identischen Bedingungen der Rechnung eine etwas höhere Gesamtenergie. Insbesondere sind im Bereich der Reste 33 bis 36 werden einige NOE-Restraints verletzt, die durch die trans-Konformation erfüllt werden können. Daher wird im weiteren von einer trans-Konformation dieser Bindung ausgegangen.

Mutagenisierung von Aminosäureresten in Bezug auf molekulare Bibliotheken:

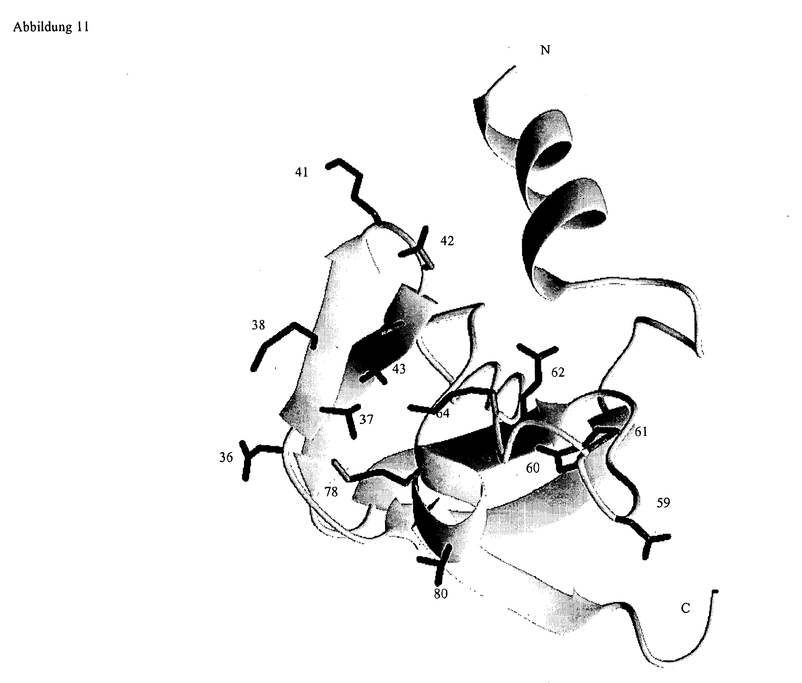

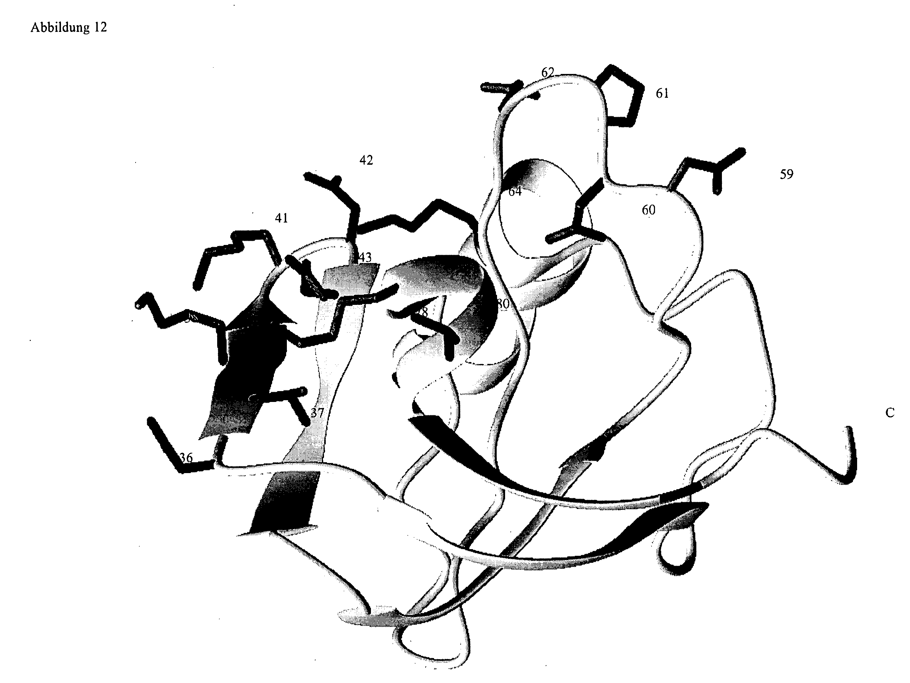

Auf der Basis des Strukturvergleichs wurden unter anderem folgende Aminosäuren ausgewählt, die einer Mutagenese unterzogen werden: D36, V37, K38, G40, K41, Q42, E43, D59, N60, P61, E62, K64, K78, T80, A81 (Abbildung 11 und 12). Die Aminosäuren an diesen Positionen erfüllen zumindest zwei der folgenden Kriterien:

1. Sie sind weitgehend Lösungsmittelexponiert, d.h. sie behindern aller Voraussicht nach nicht die Faltung des Proteins.
2. Sie sind in relativer räumlicher Nähe zu dem Cystein-Paar (< 10Å) und zeigen eine signifikante Änderung der NH-Gruppen-Resonanzen zwischen reduzierter und oxidierter Form. Dies gewährleistet die Einbindung der Cysteine in die möglichen Bindungsepitope, und damit ein differentiell unterschiedliches Verhalten der beiden Redox-Formen.
3. Zusammen genommen und unter Berücksichtigung der beiden Redox-Cysteine bilden diese Aminosäuren ein oberflächenexponiertes Epitop mit einer grossen Anzahl möglicher Bindungsmodi.

Das Protokoll für die Randomisierung wird im Moment bearbeitet und es wird nach folgender Strategie vorgegangen: Einführung der randomisierten Stellen durch eine ZweiSchritt-Gen-Assemblierung oder eine Ein-Schritt-Gen-Synthese mit Hilfe der PCR-Reaktion. Die randomisierten Oligonukleotide werden dabei durch die Triplets NNK oder durch Trinukleotide (N: jede Base möglich, K: G oder T) kodiert, was einerseits den Einbau aller 20 Aminosäuren erlaubt, andererseits aber nur das Auftreten des Amber-Stop-Kodons (im Falle des NNK Codes) erlaubt. Letzteres kann in E.coli XL1Blue supprimiert werden, da in diesem Stamm TAG als Glutamat gelesen wird. Bei Verwendung der Trinukleotide wird gezielt nur ein Satz von 19 Codons (keine Stop-Codons, kein Cystein) verwendet.

Im Falle der Verwendung des Phage Display soll die molekulare Bibliothek entweder als GenIII-oder GenVIII-Fusionsprotein exprimiert werden und die Phagen bzw. Phagemide nach Standard-Protokollen gewonnen werden. Alternative Display-Methoden sind ebenfalls möglich, z.B. das Ribosome Display, einem in vitro-Display-Verfahren, bei dem eine deutlich größere Vielfalt in der molekularen Bibliothek als mit dem Phage Display erzielt werden kann (Hanes & Plückthun, Proc. Nat. Acad. Sci. 94, 4937-4942 (1997); Roberts & Szostak 94, 12297-12302 (1997)).

Die erhaltenen molekularen Bibliotheken werden ähnlich wie Antikörper-Bibliotheken interaktionsrelevante Proteinvarianten enthalten, die eine Vielzahl unterschiedlicher Zielmolekülen erkennen können. Als Zielstrukturen werden eingesetzt: Proteine, DNA, Lipide, organische Moleküle (Fluorophore, insbesondere in der Fluoreszenzmikroskopie eingesetzte Substanzen), die an ein festes Trägermaterial gebunden werden (entweder kovalente Kopplung, oder affinitätsbasiert). Für das Panning werden wir in vitro-Bedingungen wählen, die entweder die oxidierte oder die reduzierte Form der Proteine einstellen. Konkret wird z.B. folgendes Protokoll angewendet (am Bsp. eines GST-Fusionsproteins): 100 mg des GST-Fusionsproteins, die an Glutathion-Sepharose 4B gebunden sind werden mit $10^8$-$10^{12}$ infektiösen Partikel bei 4 °C über Nacht entweder in Anwesenheit von 2 mM $H_2O_2$ oder 2mM DTT (bzw. β-Mercapto-Ethanol) in einem Volumen von 400 μl inkubiert. Anschliessend wird für den mit $H_2O_2$ inkubierten Ansatz einige Male mit PBS, 0.1 % Tween20 und 2 mM $H_2O_2$ gewaschen, und anschliessend mit PBS, 0.1 % Tween20 und 2 mM DTT (β-ME) eluiert. Bei diesem Schritt werden die Phagen freigesetzt, die nur in der oxidierten Form an das GST-Zielprotein binden. Anschliessend können die noch gebundenen Phagen mit 100 mM glycine HCl, pH 2.2 eluiert werden. Für den mit DTT inkubierten Ansatz wird mit PBS, 0.1% Tween und 2 mM DTT (β-ME) gewaschen, bevor in PBS mit 2mM $H_2O_2$ eluiert wird. In diesem Schritt werden diesmal nur die Phagen eluiert, die ausschliesslich in der reduzierten Form an das Ziel-Protein binden. Die übrigen bindenden Phagen werden im nächsten Schritt mit 100 mM glycine HCl, pH 2.2 eluiert. Alternativ kann eine "Preclearing"-Methode angewendet werden, bei der die Phagen unter oxidierenden (reduzierenden) Bedingungen vorinkubiert werden, die GST-beads mit unter diesen Bedingungen bindenden Phagen abzentrifugiert werden, um dann nach entsprechenden Wasch-Schritten unter reduzierenden (oxidierenden) Bedingungen inkubiert und bindende Phagen anschliessend (nach weiteren Wasch-Schritten) mit 100 mM glycine HC1, pH 2.2 eluiert werden. Gebundene und weiter zu charakterisierende Phagen werden in jedem der oben geschilderten Fälle zur Infektion von z.B. E.coli XL1-Blue-Zellen benutzt und die Phagen werden über Nacht in 2xYT-Medium in der Anwesenheit des Selektionsmarkers (Kanamycin, Tetracyclin, oder Ampicilin, je nach verwendetem Vektor) bei 37°C, 200 rpm amplifiziert. Nach mehreren solcher Selektionsrunden können positive Klone sequenziert und anschliessend für weitere Experimente über eine geeignete Klonierungskassette in einen Expressionsvektor (pTFT74, pET28d modified) ligiert werden. Targtetbindende Proteine der Bibliothek können anschliessend in E.coli oder Insektenzellen exprimiert und über die vorhandenen Tags (His$_6$-tag, GST-Tag, Strep-Tag), sowie einer sich anschliessenden Ionenaustausch- oder Gelfiltrations-Säule gereinigt werden.

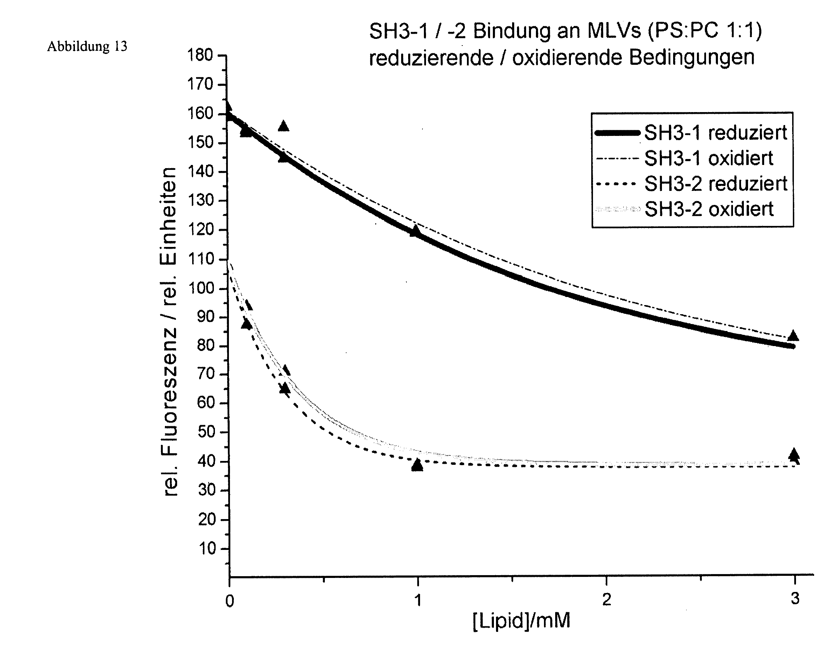

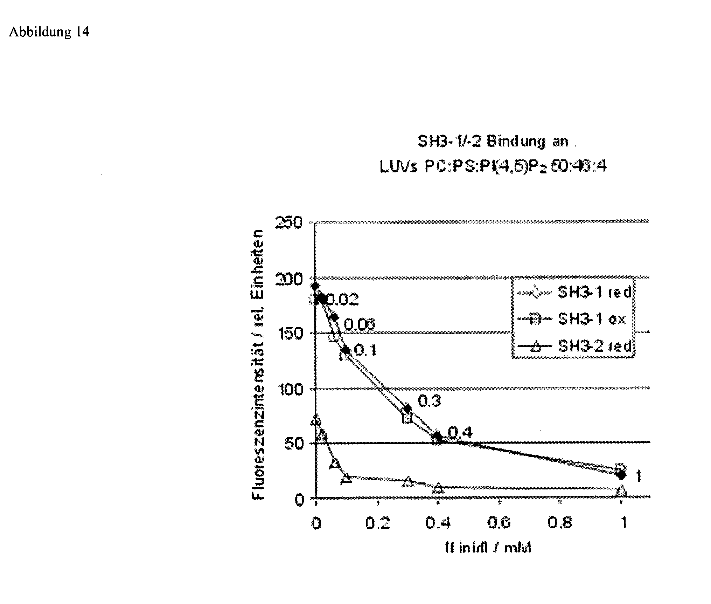

Lipid-Bindung: Die hSH3-1-Domäne bindet im Lipid-Overlay-Assay (Echelon) und im Liposom-Versuch an saure Lipide innerhalb von Lipid-Membranen (Liposomen) (Abbildung 13, 14). Basische Reste, die auf der Oberfläche der Domäne lokalisiert sind (Lys15, Lys16, Lys18, Lys20, Lys38, Lys41) sind in Anbetracht der Struktur für die Bindung zumindest mitverantwortlich. Auch aus Homolgiebetrachtungen zur hSH3-2-Domäne lassen sich die wahrscheinlichen Strukturelemente, die für die Lipidbindung verantwortlich sind, erschliessen.

Die Lipidbindungseigenschaften sind für zukünftige Experimente, welche diese Bindungsfunktion modifizieren, erfindungsrelevant. Eine weiterer möglicher Ansatz besteht in der Verknüpfung von Protein- und LipidBindungseigenschaften, die im Prinzip einen kooperativen, möglicherweise redox-regulierten Schalter darstellen können.

Für die sequenzhomologe ADAP hSH3-2-Domäne wurden die Bindungseigenschaften untersucht. Es konnte gezeigt werden, dass die ADAP hSH3-2-Domäne eine Lipid.-Interaktionsdomäne ist (Abbildung 13,14). Sie bindet saure Lipide wie z. B. Phosphatidylinositole. Positiv geladene Reste an der Oberfläche der Domäne binden an polyvalente Lipide wie PIP$_2$ oder PIP$_3$ bevorzugt gegenüber monovalenten Posphatidylserin-Phospholipiden. Die N-terminale Helix des hSH3-2-Folds ist für diese Wechselwirkung notwendig. Ausserdem tragen basische Reste des SH3-Scaffolds zur Lipid-Bindung bei.

Phosphorylierungen:

In einem in vitro-Kinase-Assay konnten für das Protein ADAP eine Reihe von Phosphorylierungsstellen identifiziert werden. Dazu gehört Tyr 86 (Seq. ID 1) in der hSH3-1-Domäne. Die $^{15}$N-HSQC-Spektren der phosphorylierten und unphosphorylierten Form der Domäne unterscheiden sich signifikant voneinander, was die Möglichkeit einer Strukturänderung impliziert.

Muteine:

Die Muteine mit den Mutationen K32E, K32Q, V37K, Q48K und A81E wurden durch Orts-spezifische Mutagenese erzeugt und konnten in E.coli analog SEQ ID Nr.1 exprimiert werden.

Einige mg des jeweils $^{15}$N-markierten Muteins wurden einer NMR-Redox-Titration analog der für SEQ ID Nr. 1 beschriebenen Methode bestimmt. Die jeweiligen Redox-Titrationen sind in Abbildung 15 und 16 gezeigt. Ein erster Fit der Werte für das Redox-Potential ergibt Werte von -201 und -209 mV für die K32E bzw. A81E-Mutante. Es konnte also gezeigt werden, dass stabile Muteine mit leicht unterschiedlichem Redox-Potential erzeugt werden können.
Weitere Proteine mit CC-Motiven, die potentiell Redoxreguliert sind:
Aus der Datenbank der humanen Proteine der Swiss-Prot-Datenbank wurden alle Protein mit CC-Motiven, die in einer über das SMART-Domänen-Annotations-Programm definierten Domäne lagen und nicht mehr als 4 weitere Cysteine besitzen ausgewählt und sind in der folgenden Tabelle 4 zusammengefasst:

Tabelle 4:

```
          PTB
  O00213   ELVQKFQVYY  LGNVPVAKPV  GVDVINGALE  SVLSSSSREQ  WTPSHVSVAP
  P98077   GLSLSVPATR  QVIANHHMPS  ISFASGGDTD  MTDYVAYVAK  DPINQRACHI
     Gadd45beta
  O75293   MTLEELVACD  NAAQKMQTVT  AAVEELLVAA  QRQDRLTVGV  YESAKLMNVD
     VavDH
  P15498   ENEEAEG  DEIYEDLMRS  EPVSMPPKMT  EYDKRCCCLR  EIQQTEEKYT  DTL
     LER
  O60304   LEEEPSVETE  DPSPETFRQL  FRLFCYQEVA  GPREALSRLW  ELCCRWLRPE
  P49910   ..........  ..XQELCRQL  FRQFCYQDSP  GPREALSRLR  ELCCQWLKPE
  O14771   ..........  ...SEACRQR  FRQFCYGDVH  GPHEAFSQLW  ELCCRWLRPE
  Q6P9G9   ..........  ...CEVFRQR  FRQFQYREAA  GPHEAFNKLW  ELCCQWLKPK
     PH
  o14939   KGLEGMIRKR  SGGHRVPGLT  CCGRDQVCYR  WSKRWLVVKD  SFLLYMCLET
  q13393   KGIEGMIMKR  SGGHRIPGLN  CCGQGRACYR  WSKRWLIVKD  SFLLYMKPDS
  q20653   ILRVGFVLRS  ARRAPCCRIM  VLCSDRILFG  HRGVNLDGNF  FTVHAEFKLK
  q20653_1 NTMCGYLKRK  LRNSSGWQDL  WVVMCCHTLY  FYRNHNEREP  LAHLSLMDYG
  q17637   GWKQCCVRYL  LPWSKRWLMV  RDSFVAYMDH  RTEQIRMVLL  MDRDFKVAAG
  q62245   FIMEGTLTRV  GAKHERHIFL  FDGLMICCKS  NHGQPRLPGA  SSAEYRLKEK
  o75135   VLKQGYMMKK  GHRRKNWTER  WFVLKPNIIS  YYVSEDLKDK  KGDILLDENC
  o75563   VLKAGYLEKR  RKDHSFLGFE  WQKRWCALSK  TVFYYYGSDK  DKQQKGEFAI
  p24135   ALELGTVMTV  FSARKSTPER  RTVQMIMETR  QVAWSKTADK  IEGFLDIMEI
  p40995   LQYFGQLLVW  DVVNVCKADI  EREYHVYLFE  KILLCCKEMS  TLKRQARSIS
  p16885   ALELGTVMTV  FSFRKSTPER  RTVQVIMETR  QVAWSKTADK  IEGFLDIMEI
  q07889   FIMEGTLTRV  GAKHERHIFL  FDGLMICCKS  NHGQPRLPGA  SNAEYRLKEK
  q9nr81   YYKERLLYLE  EGQKDSLIDS  SRVLCCHGEL  KNNRGVKLHV  FLFQEVLVIT
```

```
q96bj8       RLCEGTLFRK  ISSRRRQDKL  WFCCLSPNHK  LLQYGDMEEG  ASPPTLESLP
q96p48       DTKHGMMKFR  EDRSLLGLGL  PSGGFHDRYF  ILNSSCLRLY  KEVRSQRPWS
q13009       LSMGDLLLHT  TVIWLNPPAS  LGKWKKEPEL  AAFVFKTAVV  LVYKDGSKQK
  ILWEQ
O00291       GVKLEVNERI  LGCCTSLMQA  IQVLIVASKD  LQREIVESGR  GTASPKEFYA
  CLECT
O60449       CPSPVLNTPW  IPFQNCCYNF  IITKNRHMAT  TQDEVHTKCQ  KLNPKSHILS
  HAT
O94906       EDARIMLSRA  VECCPTSVEL  WLALARLETY  ~~~~~~~~~~  ~~~~~~~~~~
  CYCLIN
O75909       RFIFDVGTRL  GLHYDTLATG  IIYFHRFYMF  HSFKQFPRYV  TGACCLFLAG
P47829       NFLRRISKAD  DYDVQSRTLG  KYLLEITIVD  YKFIGMRPSL  CCASAMYLAR
O23215       MQIIDVASIL  GLDCDISEHA  FQLFRDCCSA  TCLRNRSVEA  LATACLVQAI
Q41731       CFMRRFLKAA  QSEKKLELLS  FFMIELSLVE  YEMLQFCPSM  LAAAAIYTAQ
P13365       DFIMYCHTRL  NLSTSTLFLT  FTILDKYSSR  FIIKSYNYQL  LSLTALWISS
O22345       IRIRNLCERI  QYMEQTERVY  NVFKQILDQQ  TTLFFNRHMH  QLILCCLYGV
  FBOX
Q9Y2K7       MQREVWMSVF  RYLSRRELCE  CMRVCKTWYK  WCCDKRLWTK  I~~~~~~~~~
  SPRY
P21817       YSKWYFEVMV  DEVTPFLTAQ  ATHLRVGWAL  TEGYTPYPGA  GEGWGGNGVG
Q92736       ..........  ..........  .......WAS  TEGYSPYPGG  GEEWGGNGVG
  RAB
P51151       MAGKSSLFKV  ILLGDGGVGK  SSLMNRYVTN  KFDTQLFHTI  GVEFLNKDLE
  SH3
P55345       EFVAIADYAA  TDETQLSFLR  GEKILILRQT  TADWWWGERA  GCCGYIPANH
  MIR
Q92736_1     HRTLLYGHAI  LLRHSYSGMY  LCCLSTSRSS  TDKLAFDVGL  QEDTTGEACW
  RRM
Q92879       KMFVGQVPRT  WSEKDLRELF  EQYGAVYEIN  VLRDRSQNPP  QSKGCCFVTF
P26599       RIIVENLFYP  VTLDVLHQIF  SKFGTVLKII  TFTKNNQFQA  LLQYADPVSA
  PDZ
Q9BXL6       SVIGGNLTGI  FIHRVTPGSA  ADQMALRPGT  QIVMVDYEAS  EPLFKAVLED
Q9C0E4       HDFGFSVSDG  LLEKGVYVHT  VRPDGPAHRG  GLQPFDRVLQ  VNHVRTRDFD
Q9Y3R0       EDFGFSVADG  LLEKGVYVKN  IRPAGPGDLG  GLKPYDRLLQ  VNHVRTRDFD
  TUDOR
Q9BXT4       ..PAIGDICC  AQFSEDDQWY  RASVLAYASE  ESVLVGYVDY  GNFEILSLMR
Q9BXT4_1     FKAEIGQPCC  AFFAGDGSWY  RALVKEILPN  GHVKVHFVDY  GNIEEVTADE
Q9BXT4_2     YRPRIGDACC  AKYTSDDFWY  RA.VVLGTSD  TDVEVLYADY  GNIETLPLCR
  GGL
P63211       KDKLKMEVDQ  LKKEVTLERM  LVSKCCEEVR  DYVEERSGED  PLVKGIPEDK
  14_3_3
P62258       MDDREDLVYQ  AKLAEQAERY  DEMVESMKKV  AGMDVELTVE  ERNLLSVAYK
  IGV
Q96KV6       NTTLRCCLSP  EENAEDMEVR  WFQSQFSPAV  FVYKGGRERT  EEQKEEYRGR
  DEP
Q8WZA2       SRAPHMIRDR  KYHLKTYRQC  CVGTELVDWM  MQQTPCVHSR  TQAVGMWQVL
             51                                                      100
  PTB
O00213       ATLTILHQQT  EAVLGECRVR  FLSFLAVGRD  VHTFAFIMAA  GPASFCCHMF
P98077       LECCEGLAQS  IISTVGQAFE  LRFKQYLHSP  PKVALPPERL  AGPEESAWGD
  Gadd45beta
O75293       PDSVVLCLLA  IDEEEEDDIA  LQIHFTLIQS  FCCDNDINIV  RVSGMQRLAQ
  VavDH
P15498       GSIQQHF  LKPLQRFLKP  QDIEIIFINI  EDLLRVHTHF  LKEMKEALGT  PGA LER
O60304       LRTKEQILEL  LVLEQFLTVL  PGEIQARVRE  QQPESGEEAV  VLVEGLQRKP
P49910       IHTKEQILEL  LVLEQFLTIL  PGDLQAWVHE  HYPESGEEAV  TILEDLERGT
O14771       LRTKEQILEL  LVLEQFLTVL  PGEIQGWVRE  QHPGSGEEAV  ALVEDLQKQP
Q6P9G9       MRSKEQILEL  LVLEQFLTIL  PTEIETWVRE  HCPENRERVV  SLIEDLQREL
  PH
o14939       GAISFVQLFD  PGFEVQVGKR  STEARHGVRI  DTSHRSLILK  CSSYRQARWW
```

```
q13393      GAIAFVLLVD  KEFKIKVGKK  ETETKYGIRI  DNLSRTLILK  CNSYRHARWW
q20653      GMMIDEGDTY  KVMDGENNVI  TLHNADISIV  FAAPDRASWI  EDITEAIKNA
q20653_1    VGLPTVADKI  DNHENCFKLF  YGSHTYFFRT  DSYYFFERWV  DSIFQAAISR
q17637      GKETEGIPTG  LIITNSQHEL  HLKCRRLQDT  ATWKYIIEQA  MGGIGNTWLQ
q62245      FFMRKVQIND  KDDTSEYKHA  FEIILKDGNS  VIFSAKSAEE  KNNWMAALIS
o75135      CVESLPDKDG  KKCLFLVKCF  DKTFEISASD  KKKKQEWIQA  IHSTIHLL~~
o75563      DGYSVRMNNT  LRKDGKKDCC  FEISAPDKRI  YQFTAASPKD  AEEWVQQLKF
p24135      KEIRPGKNSK  DFERAKAVRH  KADCCFTIFY  GTQFVLSTLS  LATDSKEDAV
p40995      MNKKTKRLDS  LQLKGRILTS  NITTVVPNHH  MGSYAIQIFW  RGDPQHESFI
p16885      KEIRPGKNSK  DFERAKAVRQ  KEDCCFTILY  GTQFVLSTLS  LAADSKEDAV
q07889      FFMRKVQIND  KDDTNEYKHA  FEIILKDENS  VIFSAKSAEE  KNNWMAALIS
q9nr81      RAVTHNEQLC  YQLYRQPIPV  KDLLLEDLQD  GEVRLGGSLR  GAFSNNERIK
q96bj8      EQLPVADMRA  LLTGKDCPHV  REKGSGKQNK  DLYELAFSIS  YDRGEEEAYL
q96p48      GAPETSHRPE  KEWPIKSLKV  YLGVKKKLRP  PTCWGFTVVH  ETEKHEKQQW
q13009      KKLVGSHRLS  IYEDWDPFRF  RHMIPTEALQ  VRALASADAE  ANAVCEIVHV
ILWEQ
O00291      KNSRWTEGLI  SASKAVGWGA  TVMVDAADLV  VQGRGKFEEL  MVCSHEIAAS
CLECT
O60449      IRDEKENNFV  LEQLLYFNYM  ASWVMLGITY  RNNSLMWFDK  TPLSYTHWRA
HAT
O94906      ~~~~~~~~~~  ~~~~~~~~~~  ~~~~~~~~~~  ~~~~~~~~~~  ~~~~~~~~~~
CYCLIN
O75909      KVEETPKKCK  DIIKTARSLL  NDVQFGQFGD  DPKEEVMVLE  RILLQ~~~~~
P47829      LILGKLPVWN  GNLIHYSGGY  RISDMRECIE  LMFQ~~~~~~  ~~~~~~~~~~
O23215      REAQEPRTLQ  EISIAANVQQ  KEIGKYIKIL  GE~~~~~~~~  ~~~~~~~~~~
Q41731      CTINGFKSWN  KCCELHTRYS  EEHLMVCSRM  MVE~~~~~~~  ~~~~~~~~~~
P13365      KFWDSKNRMA  TLKVLQNLCC  NQYSIKQFTT  MEMHLFK~~~  ~~~~~~~~~~
O22345      AKVCQLELSF  REILNNYKKE  AQCKPEVFLS  IYIGSRNHNG  VLISRHVDII
FBOX
Q9Y2K7      ~~~~~~~~~~  ~~~~~~~~~~  ~~~~~~~~~~  ~~~~~~~~~~  ~~~~~~~~~~
SPRY
P21817      DDLYSYGFDG  LHLWTGHVAR  PVTSPGQHLL  APEDVISCCL  DLSVPSISFR
Q92736      DDLFSYGFDG  LHLWSGCIAR  TVSSPNQHLL  RTDDVISCCL  DLSAPSISFR
RAB
P51151      VDGHFVTMQI  WDTAGQERFR  SLRTPFYRGS  DCCLLTFSVD  DSQSFQNLSN
SH3
P55345      VGKHV~~~~~  ~~~~~~~~~~  ~~~~~~~~~~  ~~~~~~~~~~  ~~~~~~~~~~
MIR
Q92736_1    WTIHPA~~~~  ~~~~~~~~~~  ~~~~~~~~~~  ~~~~~~~~~~  ~~~~~~~~~~
RRM
Q92879      YTRKAALEAQ  NALHNMKVLP  GMHHPIQMK~  ~~~~~~~~~~  ~~~~~~~~~~
P26599      QHAKLSLDGQ  NIYNACCTLR  ~~~~~~~~~~  ~~~~~~~~~~  ~~~~~~~~~~
PDZ
Q9BXL6      TTLEEAVGLL  RRVDGFCCLS  VKVNT~~~~~  ~~~~~~~~~~  ~~~~~~~~~~
Q9C0E4      CCLAVPLLAE  AGDVLELIIS  RKP~~~~~~~  ~~~~~~~~~~  ~~~~~~~~~~
Q9Y3R0      CCLVVPLIAE  SGNKLDLVIS  RNP~~~~~~~  ~~~~~~~~~~  ~~~~~~~~~~
TUDOR
Q9BXT4      LCPIIPKLL~  ~~~~~~~~~~  ~~~~~~~~~~  ~~~~~~~~~~  ~~~~~~~~~~
Q9BXT4_1    LRMISSTFL~  ~~~~~~~~~~  ~~~~~~~~~~  ~~~~~~~~~~  ~~~~~~~~~~
Q9BXT4_2    VQPITSSHL~  ~~~~~~~~~~  ~~~~~~~~~~  ~~~~~~~~~~  ~~~~~~~~~~
GGL
P63211      NPFKELKGGC  VIS~~~~~~~  ~~~~~~~~~~  ~~~~~~~~~~  ~~~~~~~~~~
14_3_3
P62258      NVIGARRASW  RIISSIEQKE  ENKGGEDKLK  MIREYRQMVE  TELKLICCDI
IGV
Q96KV6      TTFVSKDSRG  SVALIIHNVT  AEDNGIYQCY  F~~~~~~~~~  ~~~~~~~~~~
DEP
Q8WZA2      LEDGVLNHVD  QEHHFQDKYL  FYRFLD~~~~  ~~~~~~~~~~  ~~~~~~~~~~
            101                                                    150
PTB
```

```
O00213      WCEPNAASLS EAVQAACMLR YQKCLDARSQ ~~~~~~~~~~ ~~~~~~~~~~
P98077      EEDSLEHNYY NSIPGKEPPL GGLVDSRLAL TQPCALTALD QGPSPSLRDA
  Gadd45beta
O75293      LLGEPAETQG TTEARDLHCL LVTNPHTDAW KSHGLVEVAS YCEESRGNNQ
  VavDH
P15498      ANLYQVF IKYKERFLVY GRYCSQVESA SKHLDRVAAA REDVQMKLEE CSQ
  LER
O60304      RKHRQRG~~~ ~~~~~~~~~~ ~~~~~~~~~~ ~~~~~~~~~~ ~~~~~~~~~~
P49910      DEAVLQVQAH EHGQEIFQKK VSPPGP~~~~ ~~~~~~~~~~ ~~~~~~~~~~
O14771      VKAWRQDVPS EEAEPEAA~~ ~~~~~~~~~~ ~~~~~~~~~~ ~~~~~~~~~~
Q6P9G9      EIPEQQVDMH DILLEELAPV GT~~~~~~~~ ~~~~~~~~~~ ~~~~~~~~~~
  PH
o14939      AQEITELAQG P~~~~~~~~~ ~~~~~~~~~~ ~~~~~~~~~~ ~~~~~~~~~~
q13393      GGAIEEFIQK H~~~~~~~~~ ~~~~~~~~~~ ~~~~~~~~~~ ~~~~~~~~~~
q20653      ~~~~~~~~~~ ~~~~~~~~~~ ~~~~~~~~~~ ~~~~~~~~~~ ~~~~~~~~~~
q20653_1    ~~~~~~~~~~ ~~~~~~~~~~ ~~~~~~~~~~ ~~~~~~~~~~ ~~~~~~~~~~
q17637      PHRFSSSFPV R~~~~~~~~~ ~~~~~~~~~~ ~~~~~~~~~~ ~~~~~~~~~~
q62245      LQYRS~~~~~ ~~~~~~~~~~ ~~~~~~~~~~ ~~~~~~~~~~ ~~~~~~~~~~
o75135      ~~~~~~~~~~ ~~~~~~~~~~ ~~~~~~~~~~ ~~~~~~~~~~ ~~~~~~~~~~
o75563      VLQDM~~~~~ ~~~~~~~~~~ ~~~~~~~~~~ ~~~~~~~~~~ ~~~~~~~~~~
p24135      KWLSGLKILH QEA~~~~~~~ ~~~~~~~~~~ ~~~~~~~~~~ ~~~~~~~~~~
p40995      LKLRNEESHK LWMSVLNRLL WKN~~~~~~~ ~~~~~~~~~~ ~~~~~~~~~~
p16885      NWLSGLKILH QEA~~~~~~~ ~~~~~~~~~~ ~~~~~~~~~~ ~~~~~~~~~~
q07889      LQYRS~~~~~ ~~~~~~~~~~ ~~~~~~~~~~ ~~~~~~~~~~ ~~~~~~~~~~
q9nr81      NFFRVSFKNG SQSQTHSLQA NDTFNKQQWL NCIRQAKETV ~~~~~~~~~~
q96bj8      NFIAPSKREF YLWTDGLSAL LGSP~~~~~~ ~~~~~~~~~~ ~~~~~~~~~~
q96p48      YLCCDTQMEL REWFATFLFV QHDG~~~~~~ ~~~~~~~~~~ ~~~~~~~~~~
q13009      KSESEGRPER VFHLCCSSPE SRKDFLKAVH SILRDK~~~~ ~~~~~~~~~~
  ILWEQ
O00291      TAQLVAASKV KADKDSPNLA QLQQASRGVN QATAGVVAST ISGKSQIEET
  CLECT
O60449      GRPTIKNEKF LAGLSTDGFW DIQTFKVIEE AVYFHQHSIL ACK~~~~~~~
  HAT
O94906      ~~~~~~~~~~ ~~~~~~~~~~ ~~~~~~~~~~ ~~~~~~~~~~ ~~~~~~~~~~
  CYCLIN
O75909      ~~~~~~~~~~ ~~~~~~~~~~ ~~~~~~~~~~ ~~~~~~~~~~ ~~~~~~~~~~
P47829      ~~~~~~~~~~ ~~~~~~~~~~ ~~~~~~~~~~ ~~~~~~~~~~ ~~~~~~~~~~
O23215      ~~~~~~~~~~ ~~~~~~~~~~ ~~~~~~~~~~ ~~~~~~~~~~ ~~~~~~~~~~
Q41731      ~~~~~~~~~~ ~~~~~~~~~~ ~~~~~~~~~~ ~~~~~~~~~~ ~~~~~~~~~~
P13365      ~~~~~~~~~~ ~~~~~~~~~~ ~~~~~~~~~~ ~~~~~~~~~~ ~~~~~~~~~~
O22345      TFYNEVFV~~ ~~~~~~~~~~ ~~~~~~~~~~ ~~~~~~~~~~ ~~~~~~~~~~
  FBOX
Q9Y2K7      ~~~~~~~~~~ ~~~~~~~~~~ ~~~~~~~~~~ ~~~~~~~~~~ ~~~~~~~~~~
  SPRY
P21817      INGCPVQGVF ESFNLDGLFF PVVSFSAGVK VRFLLGGRH~ ~~~~~~~~~~
Q92736      INGQPVQGMF ENFNIDGLFF PVVSFSAGIK VRFLLGGRH~ ~~~~~~~~~~
  RAB
P51151      WKKEFIYYAD VKEPESFPFV ILGNKIDISE RQVSTEEAQA WCRDNGDYPY
  SH3
P55345      ~~~~~~~~~~ ~~~~~~~~~~ ~~~~~~~~~~ ~~~~~~~~~~ ~~~~~~~~~~
  MIR
Q92736_1    ~~1.00"~~~~~~~ ~~~~~~~~~~ ~~~~~~~~~~ ~~~~~~~~~~ ~~~~~~~~~~~
  RRM
Q92879      ~~~~~~~~~~ ~~~~~~~~~~ ~~~~~~~~~~ ~~~~~~~~~~ ~~~~~~~~~~
P26599      ~~~~~~~~~~ ~~~~~~~~~~ ~~~~~~~~~~ ~~~~~~~~~~ ~~~~~~~~~~
  PDZ
Q9BXL6      ~~~~~~~~~~ ~~~~~~~~~~ ~~~~~~~~~~ ~~~~~~~~~~ ~~~~~~~~~~
Q9C0E4      ~~~~~~~~~~ ~~~~~~~~~~ ~~~~~~~~~~ ~~~~~~~~~~ ~~~~~~~~~~
Q9Y3R0      ~~~~~~~~~~ ~~~~~~~~~~ ~~~~~~~~~~ ~~~~~~~~~~ ~~~~~~~~~~
  TUDOR
```

```
Q9BXT4    ~~~~~~~~~  ~~~~~~~~~  ~~~~~~~~~  ~~~~~~~~~  ~~~~~~~~~
Q9BXT4_1  ~~~~~~~~~  ~~~~~~~~~  ~~~~~~~~~  ~~~~~~~~~  ~~~~~~~~~
Q9BXT4_2  ~~~~~~~~~  ~~~~~~~~~  ~~~~~~~~~  ~~~~~~~~~  ~~~~~~~~~
  GGL
P63211    ~~~~~~~~~  ~~~~~~~~~  ~~~~~~~~~  ~~~~~~~~~  ~~~~~~~~~
 14_3_3
P62258    LDVLDKHLIP AANTGESKVF YYKMKGDYHR YLAEFATGND RKEAAENSLV
  IGV
Q96KV6    ~~~~~~~~~  ~~~~~~~~~  ~~~~~~~~~  ~~~~~~~~~  ~~~~~~~~~
  DEP
Q8WZA2    ~~~~~~~~~  ~~~~~~~~~  ~~~~~~~~~  ~~~~~~~~~  ~~~~~~~~~
          151                                             200


  Gadd45beta
O75293    WVPYISLQER
  VavDH
P15498    RANNGRF TLRDLLMVPM QRVLKYHLLL QELVKHTQEA MEKENLRLAL DAM
  PTB
O00213    ~~~~~~~~~  ~~~~~~~~~  ~~~~~~~~~  ~~~~~~~~~  ~~~~~~~~~
P98077    CSLPWDVGST GTAP~~~~~~  ~~~~~~~~~  ~~~~~~~~~  ~~~~~~~~~
  LER
O60304    ~~~~~~~~~  ~~~~~~~~~  ~~~~~~~~~  ~~~~~~~~~  ~~~~~~~~~
P49910    ~~~~~~~~~  ~~~~~~~~~  ~~~~~~~~~  ~~~~~~~~~  ~~~~~~~~~
O14771    ~~~~~~~~~  ~~~~~~~~~  ~~~~~~~~~  ~~~~~~~~~  ~~~~~~~~~
Q6P9G9    ~~~~~~~~~  ~~~~~~~~~  ~~~~~~~~~  ~~~~~~~~~  ~~~~~~~~~
  PH
o14939    ~~~~~~~~~  ~~~~~~~~~  ~~~~~~~~~  ~~~~~~~~~  ~~~~~~~~~
q13393    ~~~~~~~~~  ~~~~~~~~~  ~~~~~~~~~  ~~~~~~~~~  ~~~~~~~~~
q20653    ~~~~~~~~~  ~~~~~~~~~  ~~~~~~~~~  ~~~~~~~~~  ~~~~~~~~~
q20653_1  ~~~~~~~~~  ~~~~~~~~~  ~~~~~~~~~  ~~~~~~~~~  ~~~~~~~~~
q17637    ~~~~~~~~~  ~~~~~~~~~  ~~~~~~~~~  ~~~~~~~~~  ~~~~~~~~~
q62245    ~~~~~~~~~  ~~~~~~~~~  ~~~~~~~~~  ~~~~~~~~~  ~~~~~~~~~
o75135    ~~~~~~~~~  ~~~~~~~~~  ~~~~~~~~~  ~~~~~~~~~  ~~~~~~~~~
o75563    ~~~~~~~~~  ~~~~~~~~~  ~~~~~~~~~  ~~~~~~~~~  ~~~~~~~~~
p24135    ~~~~~~~~~  ~~~~~~~~~  ~~~~~~~~~  ~~~~~~~~~  ~~~~~~~~~
p40995    ~~~~~~~~~  ~~~~~~~~~  ~~~~~~~~~  ~~~~~~~~~  ~~~~~~~~~
p16885    ~~~~~~~~~  ~~~~~~~~~  ~~~~~~~~~  ~~~~~~~~~  ~~~~~~~~~
q07889    ~~~~~~~~~  ~~~~~~~~~  ~~~~~~~~~  ~~~~~~~~~  ~~~~~~~~~
q9nr81    ~~~~~~~~~  ~~~~~~~~~  ~~~~~~~~~  ~~~~~~~~~  ~~~~~~~~~
q96bj8    ~~~~~~~~~  ~~~~~~~~~  ~~~~~~~~~  ~~~~~~~~~  ~~~~~~~~~
q96p48    ~~~~~~~~~  ~~~~~~~~~  ~~~~~~~~~  ~~~~~~~~~  ~~~~~~~~~
q13009    ~~~~~~~~~  ~~~~~~~~~  ~~~~~~~~~  ~~~~~~~~~  ~~~~~~~~~
  ILWEQ
O00291    DNMDFSSMTL TQIKRQEMDS QVRVLELENE LQKERQKLGE LRKKHYELA~
  CLECT
O60449    ~~~~~~~~~  ~~~~~~~~~  ~~~~~~~~~  ~~~~~~~~~  ~~~~~~~~~
  HAT
O94906    ~~~~~~~~~  ~~~~~~~~~  ~~~~~~~~~  ~~~~~~~~~  ~~~~~~~~~
  CYCLIN
O75909    ~~~~~~~~~  ~~~~~~~~~  ~~~~~~~~~  ~~~~~~~~~  ~~~~~~~~~
P47829    ~~~~~~~~~  ~~~~~~~~~  ~~~~~~~~~  ~~~~~~~~~  ~~~~~~~~~
O23215    ~~~~~~~~~  ~~~~~~~~~  ~~~~~~~~~  ~~~~~~~~~  ~~~~~~~~~
Q41731    ~~~~~~~~~  ~~~~~~~~~  ~~~~~~~~~  ~~~~~~~~~  ~~~~~~~~~
P13365    ~~~~~~~~~  ~~~~~~~~~  ~~~~~~~~~  ~~~~~~~~~  ~~~~~~~~~
O22345    ~~~~~~~~~  ~~~~~~~~~  ~~~~~~~~~  ~~~~~~~~~  ~~~~~~~~~
  FBOX
Q9Y2K7    ~~~~~~~~~  ~~~~~~~~~  ~~~~~~~~~  ~~~~~~~~~  ~~~~~~~~~
  SPRY
P21817    ~~~~~~~~~  ~~~~~~~~~  ~~~~~~~~~  ~~~~~~~~~  ~~~~~~~~~
Q92736    ~~~~~~~~~  ~~~~~~~~~  ~~~~~~~~~  ~~~~~~~~~  ~~~~~~~~~
```

```
       RAB
       P51151    FETSAKDATN VAAAFEEAVR RVLAT~~~~~ ~~~~~~~~~~ ~~~~~~~~~~
       SH3
       P55345    ~~~~~~~~~~ ~~~~~~~~~~ ~~~~~~~~~~ ~~~~~~~~~~ ~~~~~~~~~~
       MIR
       Q92736_1  ~~~~~~~~~~ ~~~~~~~~~~ ~~~~~~~~~~ ~~~~~~~~~~ ~~~~~~~~~~
       RRM
       Q92879    ~~~~~~~~~~ ~~~~~~~~~~ ~~~~~~~~~~ ~~~~~~~~~~ ~~~~~~~~~~
       P26599    ~~~~~~~~~~ ~~~~~~~~~~ ~~~~~~~~~~ ~~~~~~~~~~ ~~~~~~~~~~
       PDZ
       Q9BXL6    ~~~~~~~~~~ ~~~~~~~~~~ ~~~~~~~~~~ ~~~~~~~~~~ ~~~~~~~~~~
       Q9C0E4    ~~~~~~~~~~ ~~~~~~~~~~ ~~~~~~~~~~ ~~~~~~~~~~ ~~~~~~~~~~
       Q9Y3R0    ~~~~~~~~~~ ~~~~~~~~~~ ~~~~~~~~~~ ~~~~~~~~~~ ~~~~~~~~~~
       TUDOR
       Q9BXT4    ~~~~~~~~~~ ~~~~~~~~~~ ~~~~~~~~~~ ~~~~~~~~~~ ~~~~~~~~~~
     Q9BXT4_1    ~~~~~~~~~~ ~~~~~~~~~~ ~~~~~~~~~~ ~~~~~~~~~~ ~~~~~~~~~~
     Q9BXT4_2    ~~~~~~~~~~ ~~~~~~~~~~ ~~~~~~~~~~ ~~~~~~~~~~ ~~~~~~~~~~
       GGL
       P63211    ~~~~~~~~~~ ~~~~~~~~~~ ~~~~~~~~~~ ~~~~~~~~~~ ~~~~~~~~~~
       14_3_3
       P62258    AYKAASDIAM TELPPTHPIR LGLALNFSVF YYEILNSPDR ACRLAKAAFD
       IGV
       Q96KV6    ~~~~~~~~~~ ~~~~~~~~~~ ~~~~~~~~~~ ~~~~~~~~~~ ~~~~~~~~~~
       DEP
       Q8WZA2    ~~~~~~~~~~ ~~~~~~~~~~ ~~~~~~~~~~ ~~~~~~~~~~ ~~~~~~~~~~
                 201                                              245
       VavDH
       P15498    RDLAQCV NEVKRDNETL
       PTB
       O00213    ~~~~~~~~~~ ~~~~~~~~~~ ~~~~~~~~~~ ~~~~~~~~~~ ~~~~~
       P98077    ~~~~~~~~~~ ~~~~~~~~~~ ~~~~~~~~~~ ~~~~~~~~~~ ~~~~~
       LER
       O60304    ~~~~~~~~~~ ~~~~~~~~~~ ~~~~~~~~~~ ~~~~~~~~~~ ~~~~~
       P49910    ~~~~~~~~~~ ~~~~~~~~~~ ~~~~~~~~~~ ~~~~~~~~~~ ~~~~~
       O14771    ~~~~~~~~~~ ~~~~~~~~~~ ~~~~~~~~~~ ~~~~~~~~~~ ~~~~~
       Q6P9G9    ~~~~~~~~~~ ~~~~~~~~~~ ~~~~~~~~~~ ~~~~~~~~~~ ~~~~~
       PH
       o14939    ~~~~~~~~~~ ~~~~~~~~~~ ~~~~~~~~~~ ~~~~~~~~~~ ~~~~~
       q13393    ~~~~~~~~~~ ~~~~~~~~~~ ~~~~~~~~~~ ~~~~~~~~~~ ~~~~~
       q20653    ~~~~~~~~~~ ~~~~~~~~~~ ~~~~~~~~~~ ~~~~~~~~~~ ~~~~~
     q20653_1    ~~~~~~~~~~ ~~~~~~~~~~ ~~~~~~~~~~ ~~~~~~~~~~ ~~~~~
       q17637    ~~~~~~~~~~ ~~~~~~~~~~ ~~~~~~~~~~ ~~~~~~~~~~ ~~~~~
       q62245    ~~~~~~~~~~ ~~~~~~~~~~ ~~~~~~~~~~ ~~~~~~~~~~ ~~~~~
       o75135    ~~~~~~~~~~ ~~~~~~~~~~ ~~~~~~~~~~ ~~~~~~~~~~ ~~~~~
       o75563    ~~~~~~~~~~ ~~~~~~~~~~ ~~~~~~~~~~ ~~~~~~~~~~ ~~~~~
       p24135    ~~~~~~~~~~ ~~~~~~~~~~ ~~~~~~~~~~ ~~~~~~~~~~ ~~~~~
       p40995    ~~~~~~~~~~ ~~~~~~~~~~ ~~~~~~~~~~ ~~~~~~~~~~ ~~~~~
       p16885    ~~~~~~~~~~ ~~~~~~~~~~ ~~~~~~~~~~ ~~~~~~~~~~ ~~~~~
       q07889    ~~~~~~~~~~ ~~~~~~~~~~ ~~~~~~~~~~ ~~~~~~~~~~ ~~~~~
       q9nr81    ~~~~~~~~~~ ~~~~~~~~~~ ~~~~~~~~~~ ~~~~~~~~~~ ~~~~~
       q96bj8    ~~~~~~~~~~ ~~~~~~~~~~ ~~~~~~~~~~ ~~~~~~~~~~ ~~~~~
       q96p48    ~~~~~~~~~~ ~~~~~~~~~~ ~~~~~~~~~~ ~~~~~~~~~~ ~~~~~
       q13009    ~~~~~~~~~~ ~~~~~~~~~~ ~~~~~~~~~~ ~~~~~~~~~~ ~~~~~
       ILWEQ
       O00291    ~~~~~~~~~~ ~~~~~~~~~~ ~~~~~~~~~~ ~~~~~~~~~~ ~~~~~
       CLECT
       O60449    ~~~~~~~~~~ ~~~~~~~~~~ ~~~~~~~~~~ ~~~~~~~~~~ ~~~~~
       HAT
       O94906    ~~~~~~~~~~ ~~~~~~~~~~ ~~~~~~~~~~ ~~~~~~~~~~ ~~~~~
       CYCLIN
```

```
O75909    ~~~~~~~~~~ ~~~~~~~~~~ ~~~~~~~~~~ ~~~~~~~~~~ ~~~~~
P47829    ~~~~~~~~~~ ~~~~~~~~~~ ~~~~~~~~~~ ~~~~~~~~~~ ~~~~~
O23215    ~~~~~~~~~~ ~~~~~~~~~~ ~~~~~~~~~~ ~~~~~~~~~~ ~~~~~
Q41731    ~~~~~~~~~~ ~~~~~~~~~~ ~~~~~~~~~~ ~~~~~~~~~~ ~~~~~
P13365    ~~~~~~~~~~ ~~~~~~~~~~ ~~~~~~~~~~ ~~~~~~~~~~ ~~~~~
O22345    ~~~~~~~~~~ ~~~~~~~~~~ ~~~~~~~~~~ ~~~~~~~~~~ ~~~~~
  FBOX
Q9Y2K7    ~~~~~~~~~~ ~~~~~~~~~~ ~~~~~~~~~~ ~~~~~~~~~~ ~~~~~
  SPRY
P21817    ~~~~~~~~~~ ~~~~~~~~~~ ~~~~~~~~~~ ~~~~~~~~~~ ~~~~~
Q92736    ~~~~~~~~~~ ~~~~~~~~~~ ~~~~~~~~~~ ~~~~~~~~~~ ~~~~~
  RAB
P51151    ~~~~~~~~~~ ~~~~~~~~~~ ~~~~~~~~~~ ~~~~~~~~~~ ~~~~~
  SH3
P55345    ~~~~~~~~~~ ~~~~~~~~~~ ~~~~~~~~~~ ~~~~~~~~~~ ~~~~~
  MIR
Q92736_1  ~~~~~~~~~~ ~~~~~~~~~~ ~~~~~~~~~~ ~~~~~~~~~~ ~~~~~
  RRM
Q92879    ~~~~~~~~~~ ~~~~~~~~~~ ~~~~~~~~~~ ~~~~~~~~~~ ~~~~~
P26599    ~~~~~~~~~~ ~~~~~~~~~~ ~~~~~~~~~~ ~~~~~~~~~~ ~~~~~
  PDZ
Q9BXL6    ~~~~~~~~~~ ~~~~~~~~~~ ~~~~~~~~~~ ~~~~~~~~~~ ~~~~~
Q9C0E4    ~~~~~~~~~~ ~~~~~~~~~~ ~~~~~~~~~~ ~~~~~~~~~~ ~~~~~
Q9Y3R0    ~~~~~~~~~~ ~~~~~~~~~~ ~~~~~~~~~~ ~~~~~~~~~~ ~~~~~
  TUDOR
Q9BXT4    ~~~~~~~~~~ ~~~~~~~~~~ ~~~~~~~~~~ ~~~~~~~~~~ ~~~~~
Q9BXT4_1  ~~~~~~~~~~ ~~~~~~~~~~ ~~~~~~~~~~ ~~~~~~~~~~ ~~~~~
Q9BXT4_2  ~~~~~~~~~~ ~~~~~~~~~~ ~~~~~~~~~~ ~~~~~~~~~~ ~~~~~
  GGL
P63211    ~~~~~~~~~~ ~~~~~~~~~~ ~~~~~~~~~~ ~~~~~~~~~~ ~~~~~
14_3_3
P62258    DAIAELDTLS EESYKDSTLI MQLLRDNLTL WTSDMQGDGE EQNKE
  IGV
Q96KV6    ~~~~~~~~~~ ~~~~~~~~~~ ~~~~~~~~~~ ~~~~~~~~~~ ~~~~~
  DEP
Q8WZA2    ~~~~~~~~~~ ~~~~~~~~~~ ~~~~~~~~~~ ~~~~~~~~~~ ~~~~~
          1                                          50
```

Tabelle 5: Atomkoordinaten der dreidimensionalen Strukturen der reduzierten und oxidierten ADAP-Sequenzen gemäß SEQ ID Nr. 1

Atomkoordinaten der Struktur der ADAP hSH3-1-Domäne, reduzierte Form

| | | | | | | | | | |
|------|----|-----|-----|---|---------|---------|---------|------|------|
| ATOM | 1  | CA  | GLU | 1 | -11.757 | -27.572 | -16.559 | 1.00 | 0.00 |
| ATOM | 2  | HA  | GLU | 1 | -10.713 | -27.334 | -16.421 | 1.00 | 0.00 |
| ATOM | 3  | CB  | GLU | 1 | -12.053 | -28.929 | -15.919 | 1.00 | 0.00 |
| ATOM | 4  | HB1 | GLU | 1 | -12.948 | -29.337 | -16.365 | 1.00 | 0.00 |
| ATOM | 5  | HB2 | GLU | 1 | -12.222 | -28.786 | -14.861 | 1.00 | 0.00 |
| ATOM | 6  | CG  | GLU | 1 | -10.932 | -29.940 | -16.090 | 1.00 | 0.00 |
| ATOM | 7  | HG1 | GLU | 1 | -9.985  | -29.423 | -16.029 | 1.00 | 0.00 |
| ATOM | 8  | HG2 | GLU | 1 | -11.026 | -30.403 | -17.061 | 1.00 | 0.00 |
| ATOM | 9  | CD  | GLU | 1 | -10.960 | -31.027 | -15.033 | 1.00 | 0.00 |
| ATOM | 10 | OE1 | GLU | 1 | -10.496 | -32.150 | -15.323 | 1.00 | 0.00 |
| ATOM | 11 | OE2 | GLU | 1 | -11.445 | -30.755 | -13.915 | 1.00 | 0.00 |
| ATOM | 12 | C   | GLU | 1 | -12.064 | -27.615 | -18.052 | 1.00 | 0.00 |
| ATOM | 13 | O   | GLU | 1 | -11.156 | -27.666 | -18.882 | 1.00 | 0.00 |
| ATOM | 14 | N   | GLU | 1 | -12.573 | -26.536 | -15.872 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 15 | HT1 | GLU | 1 | -12.548 | -25.675 | -16.454 | 1.00 | 0.00 |
| ATOM | 16 | HT2 | GLU | 1 | -12.149 | -26.369 | -14.936 | 1.00 | 0.00 |
| ATOM | 17 | HT3 | GLU | 1 | -13.542 | -26.902 | -15.786 | 1.00 | 0.00 |
| ATOM | 18 | N | LYS | 2 | -13.350 | -27.596 | -18.387 | 1.00 | 0.00 |
| ATOM | 19 | HN | LYS | 2 | -14.028 | -27.555 | -17.680 | 1.00 | 0.00 |
| ATOM | 20 | CA | LYS | 2 | -13.778 | -27.633 | -19.780 | 1.00 | 0.00 |
| ATOM | 21 | HA | LYS | 2 | -12.950 | -27.994 | -20.371 | 1.00 | 0.00 |
| ATOM | 22 | CB | LYS | 2 | -14.962 | -28.587 | -19.947 | 1.00 | 0.00 |
| ATOM | 23 | HB1 | LYS | 2 | -15.857 | -28.096 | -19.597 | 1.00 | 0.00 |
| ATOM | 24 | HB2 | LYS | 2 | -14.787 | -29.468 | -19.347 | 1.00 | 0.00 |
| ATOM | 25 | CG | LYS | 2 | -15.190 | -29.027 | -21.384 | 1.00 | 0.00 |
| ATOM | 26 | HG1 | LYS | 2 | -14.604 | -29.914 | -21.576 | 1.00 | 0.00 |
| ATOM | 27 | HG2 | LYS | 2 | -14.878 | -28.235 | -22.047 | 1.00 | 0.00 |
| ATOM | 28 | CD | LYS | 2 | -16.655 | -29.338 | -21.645 | 1.00 | 0.00 |
| ATOM | 29 | HD1 | LYS | 2 | -17.037 | -29.936 | -20.830 | 1.00 | 0.00 |
| ATOM | 30 | HD2 | LYS | 2 | -16.737 | -29.890 | -22.569 | 1.00 | 0.00 |
| ATOM | 31 | CE | LYS | 2 | -17.483 | -28.068 | -21.756 | 1.00 | 0.00 |
| ATOM | 32 | HE1 | LYS | 2 | -17.344 | -27.483 | -20.858 | 1.00 | 0.00 |
| ATOM | 33 | HE2 | LYS | 2 | -18.524 | -28.338 | -21.850 | 1.00 | 0.00 |
| ATOM | 34 | NZ | LYS | 2 | -17.087 | -27.248 | -22.934 | 1.00 | 0.00 |
| ATOM | 35 | HZ1 | LYS | 2 | -17.760 | -26.466 | -23.068 | 1.00 | 0.00 |
| ATOM | 36 | HZ2 | LYS | 2 | -17.081 | -27.835 | -23.792 | 1.00 | 0.00 |
| ATOM | 37 | HZ3 | LYS | 2 | -16.136 | -26.852 | -22.791 | 1.00 | 0.00 |
| ATOM | 38 | C | LYS | 2 | -14.160 | -26.240 | -20.270 | 1.00 | 0.00 |
| ATOM | 39 | O | LYS | 2 | -13.993 | -25.916 | -21.996 | 1.00 | 0.00 |
| ATOM | 40 | N | LYS | 3 | -14.674 | -25.419 | -19.359 | 1.00 | 0.00 |
| ATOM | 41 | HN | LYS | 3 | -14.783 | -25.734 | -18.438 | 1.00 | 0.00 |
| ATOM | 42 | CA | LYS | 3 | -15.080 | -24.060 | -19.698 | 1.00 | 0.00 |
| ATOM | 43 | HA | LYS | 3 | -14.710 | -23.840 | -20.688 | 1.00 | 0.00 |
| ATOM | 44 | CB | LYS | 3 | -16.605 | -23.946 | -19.701 | 1.00 | 0.00 |
| ATOM | 45 | HB1 | LYS | 3 | -16.934 | -23.636 | -18.720 | 1.00 | 0.00 |
| ATOM | 46 | HB2 | LYS | 3 | -17.027 | -24.916 | -19.922 | 1.00 | 0.00 |
| ATOM | 47 | CG | LYS | 3 | -17.190 | -22.952 | -20.719 | 1.00 | 0.00 |
| ATOM | 48 | HG1 | LYS | 3 | -16.423 | -22.852 | -21.521 | 1.00 | 0.00 |
| ATOM | 49 | HG2 | LYS | 3 | -17.277 | -21.996 | -20.236 | 1.00 | 0.00 |
| ATOM | 50 | CD | LYS | 3 | -18.470 | -23.406 | -21.297 | 1.00 | 0.00 |
| ATOM | 51 | HD1 | LYS | 3 | -18.353 | -24.400 | -21.705 | 1.00 | 0.00 |
| ATOM | 52 | HD2 | LYS | 3 | -18.762 | -22.725 | -22.082 | 1.00 | 0.00 |
| ATOM | 53 | CE | LYS | 3 | -19.559 | -23.432 | -20.237 | 1.00 | 0.00 |
| ATOM | 54 | HE1 | LYS | 3 | -19.531 | -22.501 | -19.689 | 1.00 | 0.00 |
| ATOM | 55 | HE2 | LYS | 3 | -19.367 | -24.252 | -19.562 | 1.00 | 0.00 |
| ATOM | 56 | NZ | LYS | 3 | -20.912 | -23.603 | -20.835 | 1.00 | 0.00 |
| ATOM | 57 | HZ1 | LYS | 3 | -20.966 | -23.104 | -21.746 | 1.00 | 0.00 |
| ATOM | 58 | HZ2 | LYS | 3 | -21.636 | -23.217 | -20.197 | 1.00 | 0.00 |
| ATOM | 59 | HZ3 | LYS | 3 | -21.108 | -24.612 | -20.992 | 1.00 | 0.00 |
| ATOM | 60 | C | LYS | 3 | -14.486 | -23.054 | -18.717 | 1.00 | 0.00 |
| ATOM | 61 | 0 | LYS | 3 | -14.477 | -23.282 | -17.508 | 1.00 | 0.00 |
| ATOM | 62 | N | GLU | 4 | -13.990 | -21.941 | -19.247 | 1.00 | 0.00 |
| ATOM | 63 | HN | GLU | 4 | -14.026 | -21.816 | -20.219 | 1.00 | 0.00 |
| ATOM | 64 | CA | GLU | 4 | -13.394 | -20.899 | -18.418 | 1.00 | 0.00 |

(fortgesetzt)

Atomkoordinaten der Struktur der ADAP hSH3-1-Domäne, reduzierte Form

| ATOM | 65 | HA | GLU | 4 | -12.876 | -21.382 | -17.603 | 1.00 | 0.00 |
|------|-----|------|-----|---|---------|---------|---------|------|------|
| ATOM | 66 | CB | GLU | 4 | -12.388 | -20.084 | -19.233 | 1.00 | 0.00 |
| ATOM | 67 | HB1 | GLU | 4 | -12.918 | -19.547 | -20.005 | 1.00 | 0.00 |
| ATOM | 68 | HB2 | GLU | 4 | -11.684 | -20.760 | -19.695 | 1.00 | 0.00 |
| ATOM | 69 | CG | GLU | 4 | -11.607 | -19.078 | -18.403 | 1.00 | 0.00 |
| ATOM | 70 | HG1 | GLU | 4 | -11.143 | -19.595 | -17.576 | 1.00 | 0.00 |
| ATOM | 71 | HG2 | GLU | 4 | -12.292 | -18.335 | -18.023 | 1.00 | 0.00 |
| ATOM | 72 | CD | GLU | 4 | -10.527 | -18.375 | -19.203 | 1.00 | 0.00 |
| ATOM | 73 | OE1 | GLU | 4 | -9.541 | -19.042 | -19.583 | 1.00 | 0.00 |
| ATOM | 74 | OE2 | GLU | 4 | -10.668 | -17.159 | -19.451 | 1.00 | 0.00 |
| ATOM | 75 | C | GLU | 4 | -14.466 | -19.979 | -17.845 | 1.00 | 0.00 |
| ATOM | 76 | O | GLU | 4 | -15.323 | -19.478 | -18.573 | 1.00 | 0.00 |
| ATOM | 77 | N | GLN | 5 | -14.413 | -19.761 | -16.535 | 1.00 | 0.00 |
| ATOM | 78 | HN | GLN | 5 | -13.706 | -20.189 | -16.008 | 1.00 | 0.00 |
| ATOM | 79 | CA | GLN | 5 | -15.380 | -18.900 | -15.863 | 1.00 | 0.00 |
| ATOM | 80 | HA | GLN | 5 | -15.711 | -18.157 | -16.573 | 1.00 | 0.00 |
| ATOM | 81 | CB | GLN | 5 | -16.586 | -19.719 | -15.400 | 1.00 | 0.00 |
| ATOM | 82 | HB1 | GLN | 5 | -16.997 | -19.262 | -14.511 | 1.00 | 0.00 |
| ATOM | 83 | HB2 | GLN | 5 | -16.258 | -20.719 | -15.161 | 1.00 | 0.00 |
| ATOM | 84 | CG | GLN | 5 | -17.691 | -19.815 | -16.439 | 1.00 | 0.00 |
| ATOM | 85 | HG1 | GLN | 5 | -17.262 | -19.658 | -17.418 | 1.00 | 0.00 |
| ATOM | 86 | HG2 | GLN | 5 | -18.423 | -19.047 | -16.241 | 1.00 | 0.00 |
| ATOM | 87 | CD | GLN | 5 | -18.388 | -21.162 | -16.426 | 1.00 | 0.00 |
| ATOM | 88 | OE1 | GLN | 5 | -19.392 | -21.347 | -15.738 | 1.00 | 0.00 |
| ATOM | 89 | NE2 | GLN | 5 | -17.857 | -22.110 | -17.188 | 1.00 | 0.00 |
| ATOM | 90 | HE21 | GLN | 5 | -17.056 | -21.891 | -17.709 | 1.00 | 0.00 |
| ATOM | 91 | HE22 | GLN | 5 | -18.287 | -22.991 | -17.199 | 1.00 | 0.00 |
| ATOM | 92 | C | GLN | 5 | -14.743 | -18.193 | -14.672 | 1.00 | 0.00 |
| ATOM | 93 | O | GLN | 5 | -14.897 | -16.984 | -14.501 | 1.00 | 0.00 |
| ATOM | 94 | N | LYS | 6 | -14.027 | -18.955 | -13.851 | 1.00 | 0.00 |
| ATOM | 95 | HN | LYS | 6 | -13.941 | -19.912 | -14.041 | 1.00 | 0.00 |
| ATOM | 96 | CA | LYS | 6 | -13.367 | -18.400 | -12.675 | 1.00 | 0.00 |
| ATOM | 97 | HA | LYS | 6 | -13.240 | -17.340 | -12.835 | 1.00 | 0.00 |
| ATOM | 98 | CB | LYS | 6 | -14.229 | -18.616 | -11.430 | 1.00 | 0.00 |
| ATOM | 99 | HB1 | LYS | 6 | -13.723 | -18.189 | -10.577 | 1.00 | 0.00 |
| ATOM | 100 | HB2 | LYS | 6 | -14.352 | -19.677 | -11.271 | 1.00 | 0.00 |
| ATOM | 101 | CG | LYS | 6 | -15.609 | -17.986 | -11.530 | 1.00 | 0.00 |
| ATOM | 102 | HG1 | LYS | 6 | -16.296 | -18.711 | -11.942 | 1.00 | 0.00 |
| ATOM | 103 | HG2 | LYS | 6 | -15.556 | -17.127 | -12.181 | 1.00 | 0.00 |
| ATOM | 104 | CD | LYS | 6 | -16.117 | -17.541 | -10.168 | 1.00 | 0.00 |
| ATOM | 105 | HD1 | LYS | 6 | -15.319 | -17.037 | -9.644 | 1.00 | 0.00 |
| ATOM | 106 | HD2 | LYS | 6 | -16.426 | -18.411 | -9.608 | 1.00 | 0.00 |
| ATOM | 107 | CE | LYS | 6 | -17.300 | -16.594 | -10.297 | 1.00 | 0.00 |
| ATOM | 108 | HE1 | LYS | 6 | -17.878 | -16.635 | -9.386 | 1.00 | 0.00 |
| ATOM | 109 | HE2 | LYS | 6 | -17.913 | -16.915 | -11.126 | 1.00 | 0.00 |
| ATOM | 110 | NZ | LYS | 6 | -16.864 | -15.190 | -10.533 | 1.00 | 0.00 |
| ATOM | 111 | HZ1 | LYS | 6 | -15.903 | -15.047 | -10.160 | 1.00 | 0.00 |
| ATOM | 112 | HZ2 | LYS | 6 | -17.510 | -14.530 | -10.056 | 1.00 | 0.00 |
| ATOM | 113 | HZ3 | LYS | 6 | -16.863 | -14.982 | -11.552 | 1.00 | 0.00 |
| ATOM | 114 | C | LYS | 6 | -11.994 | -19.034 | -12.473 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 115 | 0 | LYS | 6 | -11.842 | -19.974 | -11.692 | 1.00 | 0.00 |
| ATOM | 116 | N | GLU | 7 | -10.997 | -18.513 | -13.181 | 1.00 | 0.00 |
| ATOM | 117 | HN | GLU | 7 | -11.181 | -17.764 | -13.786 | 1.00 | 0.00 |
| ATOM | 118 | CA | GLU | 7 | -9.637 | -19.028 | -13.078 | 1.00 | 0.00 |
| ATOM | 119 | HA | GLU | 7 | -9.683 | -19.991 | -12.594 | 1.00 | 0.00 |
| ATOM | 120 | CB | GLU | 7 | -9.028 | -19.200 | -14.471 | 1.00 | 0.00 |
| ATOM | 121 | HB1 | GLU | 7 | -7.957 | -19.084 | -14.400 | 1.00 | 0.00 |
| ATOM | 122 | HB2 | GLU | 7 | -9.423 | -18.433 | -15.121 | 1.00 | 0.00 |
| ATOM | 123 | CG | GLU | 7 | -9.320 | -20.553 | -15.099 | 1.00 | 0.00 |
| ATOM | 124 | HG1 | GLU | 7 | -10.295 | -20.517 | -15.563 | 1.00 | 0.00 |
| ATOM | 125 | HG2 | GLU | 7 | -9.319 | -21.304 | -14.323 | 1.00 | 0.00 |
| ATOM | 126 | CD | GLU | 7 | -8.297 | -20.941 | -16.149 | 1.00 | 0.00 |
| ATOM | 127 | OE1 | GLU | 7 | -7.426 | -21.783 | -15.846 | 1.00 | 0.00 |
| ATOM | 128 | OE2 | GLU | 7 | -8.368 | -20.404 | -17.274 | 1.00 | 0.00 |
| ATOM | 129 | C | GLU | 7 | -8.764 | -18.098 | -12.241 | 1.00 | 0.00 |
| ATOM | 130 | O | GLU | 7 | -8.055 | -18.542 | -11.339 | 1.00 | 0.00 |
| ATOM | 131 | N | LYS | 8 | -8.823 | -16.806 | -12.547 | 1.00 | 0.00 |
| ATOM | 132 | HN | LYS | 8 | -9.408 | -16.513 | -13.277 | 1.00 | 0.00 |
| ATOM | 133 | CA | LYS | 8 | -8.038 | -15.813 | -11.822 | 1.00 | 0.00 |
| ATOM | 134 | HA | LYS | 8 | -7.091 | -16.263 | -11.565 | 1.00 | 0.00 |
| ATOM | 135 | CB | LYS | 8 | -7.784 | -14.590 | -12.705 | 1.00 | 0.00 |
| ATOM | 136 | HB1 | LYS | 8 | -8.664 | -13.963 | -12.694 | 1.00 | 0.00 |
| ATOM | 137 | HB2 | LYS | 8 | -7.602 | -14.922 | -13.717 | 1.00 | 0.00 |
| ATOM | 138 | CG | LYS | 8 | -6.597 | -13.752 | -12.257 | 1.00 | 0.00 |
| ATOM | 139 | HG1 | LYS | 8 | -6.373 | -13.986 | -11.227 | 1.00 | 0.00 |
| ATOM | 140 | HG2 | LYS | 8 | -6.855 | -12.707 | -12.342 | 1.00 | 0.00 |
| ATOM | 141 | CD | LYS | 8 | -5.367 | -14.027 | -13.106 | 1.00 | 0.00 |
| ATOM | 142 | HD1 | LYS | 8 | -4.704 | -13.175 | -13.049 | 1.00 | 0.00 |
| ATOM | 143 | HD2 | LYS | 8 | -5.675 | -14.177 | -14.130 | 1.00 | 0.00 |
| ATOM | 144 | CE | LYS | 8 | -4.625 | -15.264 | -12.628 | 1.00 | 0.00 |
| ATOM | 145 | HE1 | LYS | 8 | -3.899 | -15.545 | -13.377 | 1.00 | 0.00 |
| ATOM | 146 | HE2 | LYS | 8 | -5.336 | -16.067 | -12.499 | 1.00 | 0.00 |
| ATOM | 147 | NZ | LYS | 8 | -3.923 | -15.027 | -11.337 | 1.00 | 0.00 |
| ATOM | 148 | HZ1 | LYS | 8 | -4.463 | -14.356 | -10.754 | 1.00 | 0.00 |
| ATOM | 149 | HZ2 | LYS | 8 | -2.976 | -19.633 | -11.512 | 1.00 | 0.00 |
| ATOM | 150 | HZ3 | LYS | 8 | -3.823 | -15.921 | -10.815 | 1.00 | 0.00 |
| ATOM | 151 | C | LYS | 8 | -8.746 | -15.390 | -10.539 | 1.00 | 0.00 |
| ATOM | 152 | O | LYS | 8 | -9.907 | -14.979 | -10.566 | 1.00 | 0.00 |
| ATOM | 153 | N | GLU | 9 | -8.041 | -15.493 | -9.418 | 1.00 | 0.00 |
| ATOM | 154 | HN | GLU | 9 | -7.120 | -15.827 | -9.962 | 1.00 | 0.00 |
| ATOM | 155 | CA | GLU | 9 | -8.602 | -15.121 | -8.125 | 1.00 | 0.00 |
| ATOM | 156 | HA | GLU | 9 | -9.666 | -14.990 | -8.251 | 1.00 | 0.00 |
| ATOM | 157 | CB | GLU | 9 | -8.355 | -16.230 | -7.100 | 1.00 | 0.00 |
| ATOM | 158 | HB1 | GLU | 9 | -8.780 | -17.150 | -7.473 | 1.00 | 0.00 |
| ATOM | 159 | HB2 | GLU | 9 | -8.846 | -15.966 | -6.175 | 1.00 | 0.00 |
| ATOM | 160 | CG | GLU | 9 | -6.883 | -16.471 | -6.807 | 1.00 | 0.00 |
| ATOM | 161 | HG1 | GLU | 9 | -6.595 | -15.865 | -5.960 | 1.00 | 0.00 |
| ATOM | 162 | HG2 | GLU | 9 | -6.304 | -16.179 | -7.670 | 1.00 | 0.00 |
| ATOM | 163 | CD | GLU | 9 | -6.581 | -17.922 | -6.490 | 1.00 | 0.00 |
| ATOM | 164 | OE1 | GLU | 9 | -6.656 | -18.299 | -5.302 | 1.00 | 0.00 |

(fortgesetzt)

Atomkoordinaten der Struktur der ADAP hSH3-1-Domäne, reduzierte Form

| ATOM | 165 | OE2 | GLU | 9 | -6.271 | -18.683 | -7.431 | 1.00 | 0.00 |
|------|-----|-----|-----|---|--------|---------|--------|------|------|
| ATOM | 166 | C | GLU | 9 | -8.000 | -13.811 | -7.627 | 1.00 | 0.00 |
| ATOM | 167 | O | GLU | 9 | -6.884 | -13.448 | -7.999 | 1.00 | 0.00 |
| ATOM | 168 | N | LYS | 10 | -8.748 | -13.105 | -6.785 | 1.00 | 0.00 |
| ATOM | 169 | HN | LYS | 10 | -9.629 | -13.447 | -6.526 | 1.00 | 0.00 |
| ATOM | 170 | CA | LYS | 10 | -8.288 | -11.834 | -6.236 | 1.00 | 0.00 |
| ATOM | 171 | HA | LYS | 10 | -7.355 | -12.013 | -5.724 | 1.00 | 0.00 |
| ATOM | 172 | CB | LYS | 10 | -8.053 | -10.824 | -7.361 | 1.00 | 0.00 |
| ATOM | 173 | HB1 | LYS | 10 | -8.922 | -10.188 | -7.446 | 1.00 | 0.00 |
| ATOM | 174 | HB2 | LYS | 10 | -7.919 | -11.361 | -8.289 | 1.00 | 0.00 |
| ATOM | 175 | CG | LYS | 10 | -6.836 | -9.941 | -7.141 | 1.00 | 0.00 |
| ATOM | 176 | HG1 | LYS | 10 | -6.140 | -10.458 | -6.496 | 1.00 | 0.00 |
| ATOM | 177 | HG2 | LYS | 10 | -7.150 | -9.022 | -6.671 | 1.00 | 0.00 |
| ATOM | 178 | CD | LYS | 10 | -6.142 | -9.612 | -8.453 | 1.00 | 0.00 |
| ATOM | 179 | HD1 | LYS | 10 | -6.062 | -10.513 | -9.043 | 1.00 | 0.00 |
| ATOM | 180 | HD2 | LYS | 10 | -5.155 | -9.229 | -8.241 | 1.00 | 0.00 |
| ATOM | 181 | CE | LYS | 10 | -6.915 | -8.569 | -9.244 | 1.00 | 0.00 |
| ATOM | 182 | HE1 | LYS | 10 | -6.973 | -7.663 | -8.660 | 1.00 | 0.00 |
| ATOM | 183 | HE2 | LYS | 10 | -7.911 | -8.942 | -9.430 | 1.00 | 0.00 |
| ATOM | 184 | NZ | LYS | 10 | -6.262 | -8.265 | -10.548 | 1.00 | 0.00 |
| ATOM | 185 | HZ1 | LYS | 10 | -5.355 | -7.781 | -10.389 | 1.00 | 0.00 |
| ATOM | 186 | HZ2 | LYS | 10 | -6.875 | -7.650 | -11.120 | 1.00 | 0.00 |
| ATOM | 187 | HZ3 | LYS | 10 | -6.086 | -9.145 | -11.073 | 1.00 | 0.00 |
| ATOM | 188 | C | LYS | 10 | -9.297 | -11.276 | -5.237 | 1.00 | 0.00 |
| ATOM | 189 | O | LYS | 10 | -8.922 | -10.740 | -4.195 | 1.00 | 0.00 |
| ATOM | 190 | N | LYS | 11 | -10.579 | -11.407 | -5.563 | 1.00 | 0.00 |
| ATOM | 191 | HN | LYS | 11 | -10.815 | -11.844 | -6.408 | 1.00 | 0.00 |
| ATOM | 192 | CA | LYS | 11 | -11.642 | -10.916 | -4.694 | 1.00 | 0.00 |
| ATOM | 193 | HA | LYS | 11 | -12.586 | -11.135 | -5.171 | 1.00 | 0.00 |
| ATOM | 194 | CB | LYS | 11 | -11.592 | -11.626 | -3.340 | 1.00 | 0.00 |
| ATOM | 195 | HB1 | LYS | 11 | -12.503 | -11.413 | -2.801 | 1.00 | 0.00 |
| ATOM | 196 | HB2 | LYS | 11 | -10.753 | -11.246 | -2.778 | 1.00 | 0.00 |
| ATOM | 197 | CG | LYS | 11 | -11.447 | -13.135 | -3.450 | 1.00 | 0.00 |
| ATOM | 198 | HG1 | LYS | 11 | -10.518 | -13.362 | -3.954 | 1.00 | 0.00 |
| ATOM | 199 | HG2 | LYS | 11 | -12.274 | -13.526 | -4.024 | 1.00 | 0.00 |
| ATOM | 200 | CD | LYS | 11 | -11.441 | -13.796 | -2.081 | 1.00 | 0.00 |
| ATOM | 201 | HD1 | LYS | 11 | -10.692 | -13.319 | -1.466 | 1.00 | 0.00 |
| ATOM | 202 | HD2 | LYS | 11 | -11.201 | -14.842 | -2.198 | 1.00 | 0.00 |
| ATOM | 203 | CE | LYS | 11 | -12.794 | -13.674 | -1.398 | 1.00 | 0.00 |
| ATOM | 204 | HE1 | LYS | 11 | -13.003 | -14.596 | -0.876 | 1.00 | 0.00 |
| ATOM | 205 | HE2 | LYS | 11 | -13.550 | -13.508 | -2.152 | 1.00 | 0.00 |
| ATOM | 206 | NZ | LYS | 11 | -12.826 | -12.547 | -0.426 | 1.00 | 0.00 |
| ATOM | 207 | HZ1 | LYS | 11 | -12.322 | -11.724 | -0.815 | 1.00 | 0.00 |
| ATOM | 208 | HZ2 | LYS | 11 | -12.368 | -12.830 | 0.464 | 1.00 | 0.00 |
| ATOM | 209 | HZ3 | LYS | 11 | -13.809 | -12.275 | -0.225 | 1.00 | 0.00 |
| ATOM | 210 | C | LYS | 11 | -11.530 | -9.408 | -4.495 | 1.00 | 0.00 |
| ATOM | 211 | O | LYS | 11 | -11.786 | -8.894 | -3.406 | 1.00 | 0.00 |
| ATOM | 212 | N | GLU | 12 | -11.146 | -8.703 | -5.555 | 1.00 | 0.00 |
| ATOM | 213 | HN | GLU | 12 | -10.957 | -9.169 | -6.396 | 1.00 | 0.00 |
| ATOM | 214 | CA | GLU | 12 | -11.000 | -7.253 | -5.497 | 1.00 | 0.00 |

(fortgesetzt)

Atomkoordinaten der Struktur der ADAP hSH3-1-Domäne, reduzierte Form

| ATOM | 215 | HA | GLU | 12 | -11.934 | -6.840 | -5.144 | 1.00 | 0.00 |
|------|-----|------|-----|----|---------|--------|--------|------|------|
| ATOM | 216 | CB | GLU | 12 | -9.887 | -6.871 | -4.521 | 1.00 | 0.00 |
| ATOM | 217 | HB1 | GLU | 12 | -10.250 | -6.994 | -3.511 | 1.00 | 0.00 |
| ATOM | 218 | HB2 | GLU | 12 | -9.628 | -5.833 | -4.676 | 1.00 | 0.00 |
| ATOM | 219 | CG | GLU | 12 | -8.626 | -7.705 | -4.679 | 1.00 | 0.00 |
| ATOM | 220 | HG1 | GLU | 12 | -8.647 | -8.187 | -5.645 | 1.00 | 0.00 |
| ATOM | 221 | HG2 | GLU | 12 | -8.607 | -8.456 | -3.903 | 1.00 | 0.00 |
| ATOM | 222 | CD | GLU | 12 | -7.361 | -6.875 | -4.578 | 1.00 | 0.00 |
| ATOM | 223 | OE1 | GLU | 12 | -6.952 | -6.551 | -3.443 | 1.00 | 0.00 |
| ATOM | 224 | OE2 | GLU | 12 | -6.779 | -6.549 | -5.634 | 1.00 | 0.00 |
| ATOM | 225 | C | GLU | 12 | -10.701 | -6.681 | -6.878 | 1.00 | 0.00 |
| ATOM | 226 | O | GLU | 12 | -9.802 | -7.153 | -7.575 | 1.00 | 0.00 |
| ATOM | 227 | N | GLN | 13 | -11.459 | -5.661 | -7.268 | 1.00 | 0.00 |
| ATOM | 228 | HN | GLN | 13 | -12.159 | -5.329 | -6.668 | 1.00 | 0.00 |
| ATOM | 229 | CA | GLN | 13 | -11.274 | -5.024 | -8.567 | 1.00 | 0.00 |
| ATOM | 230 | HA | GLN | 13 | -11.396 | -5.780 | -9.327 | 1.00 | 0.00 |
| ATOM | 231 | CB | GLN | 13 | -12.326 | -3.933 | -8.777 | 1.00 | 0.00 |
| ATOM | 232 | HB1 | GLN | 13 | -11.916 | -3.175 | -9.427 | 1.00 | 0.00 |
| ATOM | 233 | HB2 | GLN | 13 | -12.561 | -3.487 | -7.822 | 1.00 | 0.00 |
| ATOM | 234 | CG | GLN | 13 | -13.617 | -4.444 | -9.395 | 1.00 | 0.00 |
| ATOM | 235 | HG1 | GLN | 13 | -13.410 | -5.367 | -9.917 | 1.00 | 0.00 |
| ATOM | 236 | HG2 | GLN | 13 | -13.981 | -3.708 | -10.097 | 1.00 | 0.00 |
| ATOM | 237 | CD | GLN | 13 | -14.696 | -4.702 | -8.362 | 1.00 | 0.00 |
| ATOM | 238 | OE1 | GLN | 13 | -14.882 | -3.916 | -7.433 | 1.00 | 0.00 |
| ATOM | 239 | NE2 | GLN | 13 | -15.413 | -5.808 | -8.519 | 1.00 | 0.00 |
| ATOM | 240 | HE21 | GLN | 13 | -15.209 | -6.388 | -9.283 | 1.00 | 0.00 |
| ATOM | 241 | HE22 | GLN | 13 | -16.118 | -6.000 | -7.866 | 1.00 | 0.00 |
| ATOM | 242 | C | GLN | 13 | -9.875 | -4.428 | -8.686 | 1.00 | 0.00 |
| ATOM | 243 | O | GLN | 13 | -9.183 | -4.638 | -9.682 | 1.00 | 0.00 |
| ATOM | 244 | N | GLU | 14 | -9.466 | -3.683 | -7.665 | 1.00 | 0.00 |
| ATOM | 245 | HN | GLU | 14 | -10.063 | -3.551 | -6.899 | 1.00 | 0.00 |
| ATOM | 246 | CA | GLU | 14 | -8.149 | -3.056 | -7.656 | 1.00 | 0.00 |
| ATOM | 247 | HA | GLU | 14 | -7.419 | -3.815 | -7.891 | 1.00 | 0.00 |
| ATOM | 248 | CB | GLU | 14 | -8.079 | -1.953 | -8.713 | 1.00 | 0.00 |
| ATOM | 249 | HB1 | GLU | 14 | -7.998 | -2.410 | -9.689 | 1.00 | 0.00 |
| ATOM | 250 | HB2 | GLU | 14 | -7.200 | -1.352 | -8.533 | 1.00 | 0.00 |
| ATOM | 251 | CG | GLU | 14 | -9.291 | -1.035 | -8.717 | 1.00 | 0.00 |
| ATOM | 252 | HG1 | GLU | 14 | -9.189 | -0.322 | -7.912 | 1.00 | 0.00 |
| ATOM | 253 | HG2 | GLU | 14 | -10.178 | -1.630 | -8.559 | 1.00 | 0.00 |
| ATOM | 254 | CD | GLU | 14 | -9.443 | -0.277 | -10.021 | 1.00 | 0.00 |
| ATOM | 255 | OE1 | GLU | 14 | -8.410 | 0.075 | -10.628 | 1.00 | 0.00 |
| ATOM | 256 | OE2 | GLU | 14 | -10.596 | -0.035 | -10.436 | 1.00 | 0.00 |
| ATOM | 257 | C | GLU | 14 | -7.832 | -2.478 | -6.280 | 1.00 | 0.00 |
| ATOM | 258 | O | GLU | 14 | -8.625 | -2.600 | -5.346 | 1.00 | 0.00 |
| ATOM | 259 | N | ILE | 15 | -6.668 | -1.846 | -6.163 | 1.00 | 0.00 |
| ATOM | 260 | HN | ILE | 15 | -6.083 | -1.779 | -6.944 | 1.00 | 0.00 |
| ATOM | 261 | CA | ILE | 15 | -6.250 | -1.247 | -4.898 | 1.00 | 0.00 |
| ATOM | 262 | HA | ILE | 15 | -6.701 | -1.809 | -4.100 | 1.00 | 0.00 |
| ATOM | 263 | CB | ILE | 15 | -4.718 | -1.266 | -4.707 | 1.00 | 0.00 |
| ATOM | 264 | HB | ILE | 15 | -4.467 | -0.494 | -3.997 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 265 | CG1 | ILE | 15 | -3.988 | -0.980 | -6.014 | 1.00 | 0.00 |
| ATOM | 266 | HG11 | ILE | 15 | -4.282 | -1.702 | -6.760 | 1.00 | 0.00 |
| ATOM | 267 | HG12 | ILE | 15 | -2.934 | -1.050 | -5.846 | 1.00 | 0.00 |
| ATOM | 268 | CG2 | ILE | 15 | -4.255 | -2.576 | -4.128 | 1.00 | 0.00 |
| ATOM | 269 | HG21 | ILE | 15 | -4.972 | -3.345 | -4.362 | 1.00 | 0.00 |
| ATOM | 270 | HG22 | ILE | 15 | -4.172 | -2.471 | -3.055 | 1.00 | 0.00 |
| ATOM | 271 | HG23 | ILE | 15 | -3.287 | -2.831 | -4.553 | 1.00 | 0.00 |
| ATOM | 272 | CD1 | ILE | 15 | -4.249 | 0.387 | -6.555 | 1.00 | 0.00 |
| ATOM | 273 | HD11 | ILE | 15 | -4.576 | 1.033 | -5.745 | 1.00 | 0.00 |
| ATOM | 274 | HD12 | ILE | 15 | -5.012 | 0.334 | -7.313 | 1.00 | 0.00 |
| ATOM | 275 | HD13 | ILE | 15 | -3.344 | 0.773 | -6.985 | 1.00 | 0.00 |
| ATOM | 276 | C | ILE | 15 | -6.699 | 0.197 | -4.795 | 1.00 | 0.00 |
| ATOM | 277 | O | ILE | 15 | -7.009 | 0.695 | -3.713 | 1.00 | 0.00 |
| ATOM | 278 | N | LYS | 16 | -6.721 | 0.872 | -5.922 | 1.00 | 0.00 |
| ATOM | 279 | HN | LYS | 16 | -6.471 | 0.431 | -6.750 | 1.00 | 0.00 |
| ATOM | 280 | CA | LYS | 16 | -7.106 | 2.251 | -5.960 | 1.00 | 0.00 |
| ATOM | 281 | HA | LYS | 16 | -6.362 | 2.825 | -5.378 | 1.00 | 0.00 |
| ATOM | 282 | CB | LYS | 16 | -7.074 | 2.715 | -7.423 | 1.00 | 0.00 |
| ATOM | 283 | HB1 | LYS | 16 | -6.179 | 2.313 | -7.876 | 1.00 | 0.00 |
| ATOM | 284 | HB2 | LYS | 16 | -7.025 | 3.790 | -7.457 | 1.00 | 0.00 |
| ATOM | 285 | CG | LYS | 16 | -8.266 | 2.260 | -8.256 | 1.00 | 0.00 |
| ATOM | 286 | HG1 | LYS | 16 | -8.918 | 1.659 | -7.642 | 1.00 | 0.00 |
| ATOM | 287 | HG2 | LYS | 16 | -7.907 | 1.669 | -9.085 | 1.00 | 0.00 |
| ATOM | 288 | CD | LYS | 16 | -9.052 | 3.442 | -8.803 | 1.00 | 0.00 |
| ATOM | 289 | HD1 | LYS | 16 | -8.743 | 3.626 | -9.821 | 1.00 | 0.00 |
| ATOM | 290 | HD2 | LYS | 16 | -8.842 | 4.312 | -8.198 | 1.00 | 0.00 |
| ATOM | 291 | CE | LYS | 16 | -10.549 | 3.176 | -8.780 | 1.00 | 0.00 |
| ATOM | 292 | HE1 | LYS | 16 | -10.977 | 3.672 | -7.922 | 1.00 | 0.00 |
| ATOM | 293 | HE2 | LYS | 16 | -10.711 | 2.111 | -8.698 | 1.00 | 0.00 |
| ATOM | 294 | NZ | LYS | 16 | -11.220 | 3.673 | -10.013 | 1.00 | 0.00 |
| ATOM | 295 | HZ1 | LYS | 16 | -12.023 | 3.058 | -10.255 | 1.00 | 0.00 |
| ATOM | 296 | HZ2 | LYS | 16 | -10.550 | 3.678 | -10.808 | 1.00 | 0.00 |
| ATOM | 297 | HZ3 | LYS | 16 | -11.570 | 4.641 | -9.863 | 1.00 | 0.00 |
| ATOM | 298 | C | LYS | 16 | -8.505 | 2.383 | -5.339 | 1.00 | 0.00 |
| ATOM | 299 | O | LYS | 16 | -8.874 | 3.400 | -4.766 | 1.00 | 0.00 |
| ATOM | 300 | N | LYS | 17 | -9.273 | 1.312 | -5.433 | 1.00 | 0.00 |
| ATOM | 301 | HN | LYS | 17 | -8.920 | 0.514 | -5.879 | 1.00 | 0.00 |
| ATOM | 302 | CA | LYS | 17 | -10.615 | 1.282 | -4.865 | 1.00 | 0.00 |
| ATOM | 303 | HA | LYS | 17 | -11.262 | 1.885 | -5.484 | 1.00 | 0.00 |
| ATOM | 304 | CB | LYS | 17 | -11.132 | -0.152 | -4.835 | 1.00 | 0.00 |
| ATOM | 305 | HB1 | LYS | 17 | -10.580 | -0.697 | -4.082 | 1.00 | 0.00 |
| ATOM | 306 | HB2 | LYS | 17 | -10.957 | -0.607 | -5.799 | 1.00 | 0.00 |
| ATOM | 307 | CG | LYS | 17 | -12.614 | -0.262 | -4.515 | 1.00 | 0.00 |
| ATOM | 308 | HG1 | LYS | 17 | -13.169 | -0.312 | -5.440 | 1.00 | 0.00 |
| ATOM | 309 | HG2 | LYS | 17 | -12.918 | 0.611 | -3.957 | 1.00 | 0.00 |
| ATOM | 310 | CD | LYS | 17 | -12.915 | -1.503 | -3.690 | 1.00 | 0.00 |
| ATOM | 311 | HD1 | LYS | 17 | -12.361 | -1.450 | -2.764 | 1.00 | 0.00 |
| ATOM | 312 | HD2 | LYS | 17 | -12.608 | -2.376 | -4.246 | 1.00 | 0.00 |
| ATOM | 313 | CE | LYS | 17 | -14.397 | -1.616 | -3.375 | 1.00 | 0.00 |
| ATOM | 314 | HE1 | LYS | 17 | -14.534 | -2.369 | -2.613 | 1.00 | 0.00 |

(fortgesetzt)

Atomkoordinaten der Struktur der ADAP hSH3-1-Domäne, reduzierte Form

| ATOM | 315 | HE2 | LYS | 17 | -14.921 | -1.914 | -4.271 | 1.00 | 0.00 |
|------|-----|-----|-----|----|---------|--------|--------|------|------|
| ATOM | 316 | NZ | LYS | 17 | -14.962 | -0.327 | -2.890 | 1.00 | 0.00 |
| ATOM | 317 | HZ1 | LYS | 17 | -14.214 | 0.250 | -2.456 | 1.00 | 0.00 |
| ATOM | 318 | HZ2 | LYS | 17 | -15.703 | -0.505 | -2.182 | 1.00 | 0.00 |
| ATOM | 319 | HZ3 | LYS | 17 | -15.378 | 0.202 | -3.684 | 1.00 | 0.00 |
| ATOM | 320 | C | LYS | 17 | -10.605 | 1.842 | -3.447 | 1.00 | 0.00 |
| ATOM | 321 | O | LYS | 17 | -11.556 | 2.487 | -3.006 | 1.00 | 0.00 |
| ATOM | 322 | N | LYS | 18 | -9.518 | 1.564 | -2.739 | 1.00 | 0.00 |
| ATOM | 323 | HN | LYS | 18 | -8.808 | 1.023 | -3.154 | 1.00 | 0.00 |
| ATOM | 324 | CA | LYS | 18 | -9.355 | 2.005 | -1.357 | 1.00 | 0.00 |
| ATOM | 325 | HA | LYS | 18 | -10.340 | 2.125 | -0.933 | 1.00 | 0.00 |
| ATOM | 326 | CB | LYS | 18 | -8.619 | 0.902 | -0.582 | 1.00 | 0.00 |
| ATOM | 327 | HB1 | LYS | 18 | -7.598 | 1.205 | -0.395 | 1.00 | 0.00 |
| ATOM | 328 | HB2 | LYS | 18 | -8.605 | 0.010 | -1.194 | 1.00 | 0.00 |
| ATOM | 329 | CG | LYS | 18 | -9.286 | 0.554 | 0.740 | 1.00 | 0.00 |
| ATOM | 330 | HG1 | LYS | 18 | -9.719 | -0.432 | 0.662 | 1.00 | 0.00 |
| ATOM | 331 | HG2 | LYS | 18 | -10.065 | 1.273 | 0.934 | 1.00 | 0.00 |
| ATOM | 332 | CD | LYS | 18 | -8.308 | 0.571 | 1.901 | 1.00 | 0.00 |
| ATOM | 333 | HD1 | LYS | 18 | -7.413 | 0.050 | 1.611 | 1.00 | 0.00 |
| ATOM | 334 | HD2 | LYS | 18 | -8.759 | 0.072 | 2.745 | 1.00 | 0.00 |
| ATOM | 335 | CE | LYS | 18 | -7.948 | 1.989 | 2.306 | 1.00 | 0.00 |
| ATOM | 336 | HE1 | LYS | 18 | -8.798 | 2.629 | 2.121 | 1.00 | 0.00 |
| ATOM | 337 | HE2 | LYS | 18 | -7.112 | 2.320 | 1.709 | 1.00 | 0.00 |
| ATOM | 338 | NZ | LYS | 18 | -7.581 | 2.076 | 3.747 | 1.00 | 0.00 |
| ATOM | 339 | HZ1 | LYS | 18 | -8.006 | 1.286 | 4.273 | 1.00 | 0.00 |
| ATOM | 340 | HZ2 | LYS | 18 | -7.924 | 2.970 | 4.151 | 1.00 | 0.00 |
| ATOM | 341 | HZ3 | LYS | 18 | -6.547 | 2.034 | 3.855 | 1.00 | 0.00 |
| ATOM | 342 | C | LYS | 18 | -8.610 | 3.351 | -1.257 | 1.00 | 0.00 |
| ATOM | 343 | O | LYS | 18 | -9.074 | 4.286 | -0.604 | 1.00 | 0.00 |
| ATOM | 344 | N | PHE | 19 | -7.457 | 3.422 | -1.906 | 1.00 | 0.00 |
| ATOM | 345 | HN | PHE | 19 | -7.157 | 2.629 | -2.398 | 1.00 | 0.00 |
| ATOM | 346 | CA | PHE | 19 | -6.595 | 4.614 | -1.927 | 1.00 | 0.00 |
| ATOM | 347 | HA | PHE | 19 | -7.106 | 5.386 | -1.371 | 1.00 | 0.00 |
| ATOM | 348 | CB | PHE | 19 | -5.244 | 4.329 | -1.250 | 1.00 | 0.00 |
| ATOM | 349 | HB1 | PHE | 19 | -4.700 | 3.649 | -1.875 | 1.00 | 0.00 |
| ATOM | 350 | HB2 | PHE | 19 | -5.429 | 3.849 | -0.305 | 1.00 | 0.00 |
| ATOM | 351 | CG | PHE | 19 | -4.339 | 5.535 | -0.979 | 1.00 | 0.00 |
| ATOM | 352 | CD1 | PHE | 19 | -4.302 | 6.179 | 0.265 | 1.00 | 0.00 |
| ATOM | 353 | HD1 | PHE | 19 | -4.967 | 5.858 | 1.050 | 1.00 | 0.00 |
| ATOM | 354 | CD2 | PHE | 19 | -3.468 | 5.980 | -1.959 | 1.00 | 0.00 |
| ATOM | 355 | HD2 | PHE | 19 | -3.474 | 5.500 | -2.915 | 1.00 | 0.00 |
| ATOM | 356 | CE1 | PHE | 19 | -3.416 | 7.238 | 0.497 | 1.00 | 0.00 |
| ATOM | 357 | HE1 | PHE | 19 | -3.368 | 7.730 | 1.474 | 1.00 | 0.00 |
| ATOM | 358 | CE2 | PHE | 19 | -2.602 | 7.030 | -1.729 | 1.00 | 0.00 |
| ATOM | 359 | HE2 | PHE | 19 | -1.936 | 7.361 | -2.510 | 1.00 | 0.00 |
| ATOM | 360 | CZ | PHE | 19 | -2.577 | 7.655 | -0.506 | 1.00 | 0.00 |
| ATOM | 361 | HZ | PHE | 19 | -1.893 | 8.467 | -0.333 | 1.00 | 0.00 |
| ATOM | 362 | C | PHE | 19 | -6.429 | 5.097 | -3.376 | 1.00 | 0.00 |
| ATOM | 363 | O | PHE | 19 | -5.351 | 5.510 | -3.770 | 1.00 | 0.00 |
| ATOM | 364 | N | LYS | 20 | -7.516 | 4.955 | -4.163 | 1.00 | 0.00 |

(fortgesetzt)

Atomkoordinaten der Struktur der ADAP hSH3-1-Domäne, reduzierte Form

| ATOM | 365 | HN | LYS | 20 | -8.285 | 4.554 | -3.753 | 1.00 | 0.00 |
|------|-----|------|-----|----|--------|-------|---------|------|------|
| ATOM | 366 | CA | LYS | 20 | -7.609 | 5.247 | -5.631 | 1.00 | 0.00 |
| ATOM | 367 | HA | LYS | 20 | -7.806 | 4.325 | -6.134 | 1.00 | 0.00 |
| ATOM | 368 | CB | LYS | 20 | -8.788 | 6.189 | -5.887 | 1.00 | 0.00 |
| ATOM | 369 | HB1 | LYS | 20 | -8.508 | 6.904 | -6.647 | 1.00 | 0.00 |
| ATOM | 370 | HB2 | LYS | 20 | -9.013 | 6.719 | -4.973 | 1.00 | 0.00 |
| ATOM | 371 | CG | LYS | 20 | -10.049 | 5.475 | -6.347 | 1.00 | 0.00 |
| ATOM | 372 | HG1 | LYS | 20 | -9.826 | 4.428 | -6.489 | 1.00 | 0.00 |
| ATOM | 373 | HG2 | LYS | 20 | -10.373 | 5.905 | -7.283 | 1.00 | 0.00 |
| ATOM | 374 | CD | LYS | 20 | -11.172 | 5.608 | -5.331 | 1.00 | 0.00 |
| ATOM | 375 | HD1 | LYS | 20 | -11.154 | 6.605 | -4.917 | 1.00 | 0.00 |
| ATOM | 376 | HD2 | LYS | 20 | -11.019 | 4.886 | -4.543 | 1.00 | 0.00 |
| ATOM | 377 | CE | LYS | 20 | -12.531 | 5.361 | -5.967 | 1.00 | 0.00 |
| ATOM | 378 | HE1 | LYS | 20 | -13.181 | 4.908 | -5.232 | 1.00 | 0.00 |
| ATOM | 379 | HE2 | LYS | 20 | -12.407 | 4.687 | -6.801 | 1.00 | 0.00 |
| ATOM | 380 | NZ | LYS | 20 | -13.154 | 6.623 | -6.453 | 1.00 | 0.00 |
| ATOM | 381 | HZ1 | LYS | 20 | -13.767 | 6.427 | -7.270 | 1.00 | 0.00 |
| ATOM | 382 | HZ2 | LYS | 20 | -12.417 | 7.298 | -6.740 | 1.00 | 0.00 |
| ATOM | 383 | HZ3 | LYS | 20 | -13.727 | 7.053 | -5.699 | 1.00 | 0.00 |
| ATOM | 384 | C | LYS | 20 | -6.350 | 5.843 | -6.221 | 1.00 | 0.00 |
| ATOM | 385 | O | LYS | 20 | -6.373 | 6.691 | -7.112 | 1.00 | 0.00 |
| ATOM | 386 | N | LEU | 21 | -5.263 | 5.370 | -5.671 | 1.00 | 0.00 |
| ATOM | 387 | HN | LEU | 21 | -5.363 | 4.683 | -4.967 | 1.00 | 0.00 |
| ATOM | 388 | CA | LEU | 21 | -3.929 | 5.769 | -6.026 | 1.00 | 0.00 |
| ATOM | 389 | HA | LEU | 21 | -3.854 | 6.815 | -5.819 | 1.00 | 0.00 |
| ATOM | 390 | CB | LEU | 21 | -3.002 | 5.008 | -5.073 | 1.00 | 0.00 |
| ATOM | 391 | HB1 | LEU | 21 | -3.653 | 4.458 | -4.392 | 1.00 | 0.00 |
| ATOM | 392 | HB2 | LEU | 21 | -2.424 | 5.718 | -4.507 | 1.00 | 0.00 |
| ATOM | 393 | CG | LEU | 21 | -2.048 | 3.985 | -5.692 | 1.00 | 0.00 |
| ATOM | 394 | HG | LEU | 21 | -1.762 | 4.302 | -6.682 | 1.00 | 0.00 |
| ATOM | 395 | CD1 | LEU | 21 | -0.785 | 3.840 | -4.855 | 1.00 | 0.00 |
| ATOM | 396 | HD11 | LEU | 21 | -0.598 | 4.759 | -4.319 | 1.00 | 0.00 |
| ATOM | 397 | HD12 | LEU | 21 | 0.051 | 3.622 | -5.501 | 1.00 | 0.00 |
| ATOM | 398 | HD13 | LEU | 21 | -0.913 | 3.033 | -4.150 | 1.00 | 0.00 |
| ATOM | 399 | CD2 | LEU | 21 | -2.758 | 2.653 | -5.803 | 1.00 | 0.00 |
| ATOM | 400 | HD21 | LEU | 21 | -2.610 | 2.252 | -6.791 | 1.00 | 0.00 |
| ATOM | 401 | HD22 | LEU | 21 | -3.821 | 2.788 | -5.620 | 1.00 | 0.00 |
| ATOM | 402 | HD23 | LEU | 21 | -2.360 | 1.966 | -5.070 | 1.00 | 0.00 |
| ATOM | 403 | C | LEU | 21 | -3.550 | 5.490 | -7.499 | 1.00 | 0.00 |
| ATOM | 404 | O | LEU | 21 | -2.521 | 5.975 | -7.969 | 1.00 | 0.00 |
| ATOM | 405 | N | THR | 22 | -4.335 | 4.673 | -8.207 | 1.00 | 0.00 |
| ATOM | 406 | HN | THR | 22 | -5.125 | 4.278 | -7.786 | 1.00 | 0.00 |
| ATOM | 407 | CA | THR | 22 | -4.004 | 4.311 | -9.597 | 1.00 | 0.00 |
| ATOM | 408 | HA | THR | 22 | -2.936 | 4.160 | -9.633 | 1.00 | 0.00 |
| ATOM | 409 | CB | THR | 22 | -4.674 | 2.996 | -9.991 | 1.00 | 0.00 |
| ATOM | 410 | HB | THR | 22 | -4.429 | 2.779 | -11.021 | 1.00 | 0.00 |
| ATOM | 411 | OG1 | THR | 22 | -6.082 | 3.095 | -9.886 | 1.00 | 0.00 |
| ATOM | 412 | HG1 | THR | 22 | -6.491 | 2.386 | -10.387 | 1.00 | 0.00 |
| ATOM | 413 | CG2 | THR | 22 | -4.222 | 1.824 | -9.156 | 1.00 | 0.00 |
| ATOM | 414 | HG21 | THR | 22 | -3.992 | 2.166 | -8.160 | 1.00 | 0.00 |

(fortgesetzt)

Atomkoordinaten der Struktur der ADAP hSH3-1-Domäne, reduzierte Form

| ATOM | 415 | HG22 | THR | 22 | -3.341 | 1.383 | -9.599 | 1.00 | 0.00 |
|------|-----|------|-----|----|--------|-------|--------|------|------|
| ATOM | 416 | HG23 | THR | 22 | -5.011 | 1.088 | -9.112 | 1.00 | 0.00 |
| ATOM | 417 | C | THR | 22 | -4.361 | 5.367 | -10.640 | 1.00 | 0.00 |
| ATOM | 418 | O | THR | 22 | -3.659 | 5.497 | -11.644 | 1.00 | 0.00 |
| ATOM | 419 | N | GLY | 23 | -5.465 | 6.080 | -10.455 | 1.00 | 0.00 |
| ATOM | 420 | HN | GLY | 23 | -6.026 | 5.922 | -9.667 | 1.00 | 0.00 |
| ATOM | 421 | CA | GLY | 23 | -5.864 | 7.045 | -11.466 | 1.00 | 0.00 |
| ATOM | 422 | HA1 | GLY | 23 | -6.902 | 7.294 | -11.303 | 1.00 | 0.00 |
| ATOM | 423 | HA2 | GLY | 23 | -5.771 | 6.581 | -12.435 | 1.00 | 0.00 |
| ATOM | 424 | C | GLY | 23 | -5.050 | 8.329 | -11.467 | 1.00 | 0.00 |
| ATOM | 425 | O | GLY | 23 | -4.862 | 8.934 | -12.522 | 1.00 | 0.00 |
| ATOM | 426 | N | PRO | 24 | -4.558 | 8.787 | -10.309 | 1.00 | 0.00 |
| ATOM | 427 | CA | PRO | 24 | -3.781 | 10.004 | -10.197 | 1.00 | 0.00 |
| ATOM | 428 | HA | PRO | 24 | -3.914 | 10.667 | -11.037 | 1.00 | 0.00 |
| ATOM | 429 | CB | PRO | 24 | -4.409 | 10.595 | -8.953 | 1.00 | 0.00 |
| ATOM | 430 | HB1 | PRO | 24 | -5.359 | 11.044 | -9.198 | 1.00 | 0.00 |
| ATOM | 431 | HB2 | PRO | 24 | -3.747 | 11.332 | -8.521 | 1.00 | 0.00 |
| ATOM | 432 | CG | PRO | 24 | -4.576 | 9.400 | -8.066 | 1.00 | 0.00 |
| ATOM | 433 | HG1 | PRO | 24 | -5.448 | 9.524 | -7.442 | 1.00 | 0.00 |
| ATOM | 434 | HG2 | PRO | 24 | -3.692 | 9.279 | -7.459 | 1.00 | 0.00 |
| ATOM | 435 | CD | PRO | 24 | -4.745 | 8.200 | -8.980 | 1.00 | 0.00 |
| ATOM | 436 | HD1 | PRO | 24 | -5.734 | 7.780 | -8.879 | 1.00 | 0.00 |
| ATOM | 437 | HD2 | PRO | 24 | -3.991 | 7.458 | -8.776 | 1.00 | 0.00 |
| ATOM | 438 | C | PRO | 24 | -2.300 | 9.735 | -9.943 | 1.00 | 0.00 |
| ATOM | 439 | O | PRO | 24 | -1.864 | 9.714 | -8.793 | 1.00 | 0.00 |
| ATOM | 440 | N | ILE | 25 | -1.526 | 9.538 | -11.003 | 1.00 | 0.00 |
| ATOM | 441 | HN | ILE | 25 | -1.917 | 9.568 | -11.897 | 1.00 | 0.00 |
| ATOM | 442 | CA | ILE | 25 | -0.098 | 9.288 | -10.840 | 1.00 | 0.00 |
| ATOM | 443 | HA | ILE | 25 | 0.018 | 8.489 | -10.123 | 1.00 | 0.00 |
| ATOM | 444 | CB | ILE | 25 | 0.573 | 8.862 | -12.160 | 1.00 | 0.00 |
| ATOM | 445 | HB | ILE | 25 | 0.056 | 7.992 | -12.534 | 1.00 | 0.00 |
| ATOM | 446 | CG1 | ILE | 25 | 2.036 | 8.497 | -11.902 | 1.00 | 0.00 |
| ATOM | 447 | HG11 | ILE | 25 | 2.185 | 8.376 | -10.837 | 1.00 | 0.00 |
| ATOM | 448 | HG12 | ILE | 25 | 2.669 | 9.293 | -12.263 | 1.00 | 0.00 |
| ATOM | 449 | CG2 | ILE | 25 | 0.462 | 9.962 | -13.206 | 1.00 | 0.00 |
| ATOM | 450 | HG21 | ILE | 25 | 1.361 | 10.561 | -13.196 | 1.00 | 0.00 |
| ATOM | 451 | HG22 | ILE | 25 | -0.390 | 10.587 | -12.982 | 1.00 | 0.00 |
| ATOM | 452 | HG23 | ILE | 25 | 0.336 | 9.518 | -14.183 | 1.00 | 0.00 |
| ATOM | 453 | CD1 | ILE | 25 | 2.467 | 7.216 | -12.581 | 1.00 | 0.00 |
| ATOM | 454 | HD11 | ILE | 25 | 3.545 | 7.170 | -12.610 | 1.00 | 0.00 |
| ATOM | 455 | HD12 | ILE | 25 | 2.077 | 7.193 | -13.587 | 1.00 | 0.00 |
| ATOM | 456 | HD13 | ILE | 25 | 2.086 | 6.371 | -12.027 | 1.00 | 0.00 |
| ATOM | 457 | C | ILE | 25 | 0.577 | 10.544 | -10.296 | 1.00 | 0.00 |
| ATOM | 458 | O | ILE | 25 | 1.226 | 11.294 | -11.024 | 1.00 | 0.00 |
| ATOM | 459 | N | GLN | 26 | 0.365 | 10.776 | -9.010 | 1.00 | 0.00 |
| ATOM | 460 | HN | GLN | 26 | -0.193 | 10.147 | -8.506 | 1.00 | 0.00 |
| ATOM | 461 | CA | GLN | 26 | 0.882 | 11.950 | -8.324 | 1.00 | 0.00 |
| ATOM | 462 | HA | GLN | 26 | 1.391 | 12.541 | -9.060 | 1.00 | 0.00 |
| ATOM | 463 | CB | GLN | 26 | -0.265 | 12.775 | -7.738 | 1.00 | 0.00 |
| ATOM | 464 | HB1 | GLN | 26 | -0.430 | 12.465 | -6.716 | 1.00 | 0.00 |

(fortgesetzt)

Atomkoordinaten der Struktur der ADAP hSH3-1-Domäne, reduzierte Form

| ATOM | 465 | HB2 | GLN | 26 | -1.160 | 12.584 | -8.311 | 1.00 | 0.00 |
|------|-----|------|-----|----|--------|--------|--------|------|------|
| ATOM | 466 | CG | GLN | 26 | -0.004 | 14.272 | -7.746 | 1.00 | 0.00 |
| ATOM | 467 | HG1 | GLN | 26 | -0.289 | 14.669 | -8.709 | 1.00 | 0.00 |
| ATOM | 468 | HG2 | GLN | 26 | 1.050 | 14.442 | -7.584 | 1.00 | 0.00 |
| ATOM | 469 | CD | GLN | 26 | -0.783 | 15.005 | -6.671 | 1.00 | 0.00 |
| ATOM | 470 | OE1 | GLN | 26 | -2.014 | 15.012 | -6.675 | 1.00 | 0.00 |
| ATOM | 471 | NE2 | GLN | 26 | -0.066 | 15.627 | -5.742 | 1.00 | 0.00 |
| ATOM | 472 | HE21 | GLN | 26 | 0.911 | 15.579 | -5.802 | 1.00 | 0.00 |
| ATOM | 473 | HE22 | GLN | 26 | -0.543 | 16.109 | -5.035 | 1.00 | 0.00 |
| ATOM | 474 | C | GLN | 26 | 1.891 | 11.610 | -7.241 | 1.00 | 0.00 |
| ATOM | 475 | O | GLN | 26 | 1.896 | 12.248 | -6.188 | 1.00 | 0.00 |
| ATOM | 476 | N | VAL | 27 | 2.721 | 10.594 | -7.461 | 1.00 | 0.00 |
| ATOM | 477 | HN | VAL | 27 | 2.667 | 10.095 | -8.302 | 1.00 | 0.00 |
| ATOM | 478 | CA | VAL | 27 | 3.684 | 10.201 | -6.435 | 1.00 | 0.00 |
| ATOM | 479 | HA | VAL | 27 | 3.138 | 9.708 | -5.651 | 1.00 | 0.00 |
| ATOM | 480 | CB | VAL | 27 | 4.763 | 9.234 | -6.959 | 1.00 | 0.00 |
| ATOM | 481 | HB | VAL | 27 | 5.661 | 9.382 | -6.377 | 1.00 | 0.00 |
| ATOM | 482 | CG1 | VAL | 27 | 4.314 | 7.796 | -6.775 | 1.00 | 0.00 |
| ATOM | 483 | HG11 | VAL | 27 | 5.019 | 7.273 | -6.141 | 1.00 | 0.00 |
| ATOM | 484 | HG12 | VAL | 27 | 4.259 | 7.311 | -7.737 | 1.00 | 0.00 |
| ATOM | 485 | HG13 | VAL | 27 | 3.341 | 7.785 | -6.313 | 1.00 | 0.00 |
| ATOM | 486 | CG2 | VAL | 27 | 5.088 | 9.516 | -8.414 | 1.00 | 0.00 |
| ATOM | 487 | HG21 | VAL | 27 | 5.997 | 8.999 | -8.683 | 1.00 | 0.00 |
| ATOM | 488 | HG22 | VAL | 27 | 5.221 | 10.578 | -8.553 | 1.00 | 0.00 |
| ATOM | 489 | HG23 | VAL | 27 | 4.278 | 9.168 | -9.036 | 1.00 | 0.00 |
| ATOM | 490 | C | VAL | 27 | 4.354 | 11.430 | -5.856 | 1.00 | 0.00 |
| ATOM | 491 | O | VAL | 27 | 5.104 | 12.139 | -6.526 | 1.00 | 0.00 |
| ATOM | 492 | N | ILE | 28 | 4.012 | 11.688 | -4.604 | 1.00 | 0.00 |
| ATOM | 493 | HN | ILE | 28 | 3.381 | 11.087 | -4.158 | 1.00 | 0.00 |
| ATOM | 494 | CA | ILE | 28 | 4.498 | 12.850 | -3.887 | 1.00 | 0.00 |
| ATOM | 495 | HA | ILE | 28 | 4.929 | 13.529 | -4.608 | 1.00 | 0.00 |
| ATOM | 496 | CB | ILE | 28 | 3.339 | 13.586 | -3.166 | 1.00 | 0.00 |
| ATOM | 497 | HB | ILE | 28 | 3.687 | 13.882 | -2.186 | 1.00 | 0.00 |
| ATOM | 498 | CG1 | ILE | 28 | 2.105 | 12.685 | -3.001 | 1.00 | 0.00 |
| ATOM | 499 | HG11 | ILE | 28 | 1.853 | 12.251 | -3.958 | 1.00 | 0.00 |
| ATOM | 500 | HG12 | ILE | 28 | 1.276 | 13.285 | -2.657 | 1.00 | 0.00 |
| ATOM | 501 | CG2 | ILE | 28 | 2.958 | 14.839 | -3.935 | 1.00 | 0.00 |
| ATOM | 502 | HG21 | ILE | 28 | 3.848 | 15.308 | -4.326 | 1.00 | 0.00 |
| ATOM | 503 | HG22 | ILE | 28 | 2.446 | 15.525 | -3.277 | 1.00 | 0.00 |
| ATOM | 504 | HG23 | ILE | 28 | 2.304 | 14.568 | -4.754 | 1.00 | 0.00 |
| ATOM | 505 | CD1 | ILE | 28 | 2.294 | 11.553 | -2.011 | 1.00 | 0.00 |
| ATOM | 506 | HD11 | ILE | 28 | 2.239 | 11.992 | -1.005 | 1.00 | 0.00 |
| ATOM | 507 | HD12 | ILE | 28 | 3.259 | 11.095 | -2.167 | 1.00 | 0.00 |
| ATOM | 508 | HD13 | ILE | 28 | 1.516 | 10.814 | -2.154 | 1.00 | 0.00 |
| ATOM | 509 | C | ILE | 28 | 5.555 | 12.495 | -2.856 | 1.00 | 0.00 |
| ATOM | 510 | O | ILE | 28 | 6.278 | 13.372 | -2.383 | 1.00 | 0.00 |
| ATOM | 511 | N | HIS | 29 | 5.632 | 11.226 | -2.479 | 1.00 | 0.00 |
| ATOM | 512 | HN | HIS | 29 | 5.022 | 10.563 | -2.865 | 1.00 | 0.00 |
| ATOM | 513 | CA | HIS | 29 | 6.594 | 10.822 | -1.969 | 1.00 | 0.00 |
| ATOM | 514 | HA | HIS | 29 | 7.498 | 11.390 | -1.616 | 1.00 | 0.00 |

(fortgesetzt)

Atomkoordinaten der Struktur der ADAP hSH3-1-Domäne, reduzierte Form

| ATOM | 515 | CB | HIS | 29 | 6.031 | 11.143 | -0.088 | 1.00 | 0.00 |
|------|-----|------|-----|----|--------|--------|--------|------|------|
| ATOM | 516 | HB1 | HIS | 29 | 6.710 | 10.771 | 0.660 | 1.00 | 0.00 |
| ATOM | 517 | HB2 | HIS | 29 | 5.079 | 10.647 | 0.024 | 1.00 | 0.00 |
| ATOM | 518 | CG | HIS | 29 | 5.814 | 12.603 | 0.157 | 1.00 | 0.00 |
| ATOM | 519 | ND1 | HIS | 29 | 4.560 | 13.163 | 0.295 | 1.00 | 0.00 |
| ATOM | 520 | HD1 | HIS | 29 | 3.706 | 12.679 | 0.248 | 1.00 | 0.00 |
| ATOM | 521 | CD2 | HIS | 29 | 6.696 | 13.622 | 0.293 | 1.00 | 0.00 |
| ATOM | 522 | HD2 | HIS | 29 | 7.773 | 13.548 | 0.243 | 1.00 | 0.00 |
| ATOM | 523 | CE1 | HIS | 29 | 4.681 | 14.462 | 0.504 | 1.00 | 0.00 |
| ATOM | 524 | HE1 | HIS | 29 | 3.868 | 15.158 | 0.649 | 1.00 | 0.00 |
| ATOM | 525 | NE2 | HIS | 29 | 5.966 | 14.765 | 0.509 | 1.00 | 0.00 |
| ATOM | 526 | HE2 | HIS | 29 | 6.338 | 15.645 | 0.728 | 1.00 | 0.00 |
| ATOM | 527 | C | HIS | 29 | 6.922 | 9.339 | -1.516 | 1.00 | 0.00 |
| ATOM | 528 | O | HIS | 29 | 6.165 | 8.529 | -2.052 | 1.00 | 0.00 |
| ATOM | 529 | N | LEU | 30 | 8.040 | 8.996 | -0.886 | 1.00 | 0.00 |
| ATOM | 530 | HN | LEU | 30 | 8.568 | 9.694 | -0.445 | 1.00 | 0.00 |
| ATOM | 531 | CA | LEU | 30 | 8.474 | 7.614 | -0.774 | 1.00 | 0.00 |
| ATOM | 532 | HA | LEU | 30 | 7.886 | 7.017 | -1.454 | 1.00 | 0.00 |
| ATOM | 533 | CB | LEU | 30 | 9.961 | 7.472 | -1.111 | 1.00 | 0.00 |
| ATOM | 534 | HB1 | LEU | 30 | 10.112 | 6.506 | -1.571 | 1.00 | 0.00 |
| ATOM | 535 | HB2 | LEU | 30 | 10.520 | 7.503 | -0.188 | 1.00 | 0.00 |
| ATOM | 536 | CG | LEU | 30 | 10.525 | 8.540 | -2.051 | 1.00 | 0.00 |
| ATOM | 537 | HG | LEU | 30 | 10.315 | 9.518 | -1.642 | 1.00 | 0.00 |
| ATOM | 538 | CD1 | LEU | 30 | 12.034 | 8.399 | -2.177 | 1.00 | 0.00 |
| ATOM | 539 | HD11 | LEU | 30 | 12.447 | 9.305 | -2.592 | 1.00 | 0.00 |
| ATOM | 540 | HD12 | LEU | 30 | 12.264 | 7.567 | -2.826 | 1.00 | 0.00 |
| ATOM | 541 | HD13 | LEU | 30 | 12.461 | 8.222 | -1.201 | 1.00 | 0.00 |
| ATOM | 542 | CD2 | LEU | 30 | 9.863 | 8.449 | -3.418 | 1.00 | 0.00 |
| ATOM | 543 | HD21 | LEU | 30 | 10.378 | 9.097 | -4.111 | 1.00 | 0.00 |
| ATOM | 544 | HD22 | LEU | 30 | 8.830 | 8.753 | -3.339 | 1.00 | 0.00 |
| ATOM | 545 | HD23 | LEU | 30 | 9.912 | 7.930 | -3.774 | 1.00 | 0.00 |
| ATOM | 546 | C | LEU | 30 | 8.209 | 7.163 | 0.654 | 1.00 | 0.00 |
| ATOM | 547 | O | LEU | 30 | 8.783 | 7.705 | 1.598 | 1.00 | 0.00 |
| ATOM | 548 | N | ALA | 31 | 7.311 | 6.206 | 0.817 | 1.00 | 0.00 |
| ATOM | 549 | HN | ALA | 31 | 6.862 | 5.828 | 0.037 | 1.00 | 0.00 |
| ATOM | 550 | CA | ALA | 31 | 6.947 | 5.735 | 2.138 | 1.00 | 0.00 |
| ATOM | 551 | HA | ALA | 31 | 7.459 | 6.346 | 2.865 | 1.00 | 0.00 |
| ATOM | 552 | CB | ALA | 31 | 5.451 | 5.918 | 2.340 | 1.00 | 0.00 |
| ATOM | 553 | HB1 | ALA | 31 | 5.032 | 6.417 | 1.476 | 1.00 | 0.00 |
| ATOM | 554 | HB2 | ALA | 31 | 5.277 | 6.518 | 3.233 | 1.00 | 0.00 |
| ATOM | 555 | HB3 | ALA | 31 | 4.982 | 4.951 | 2.449 | 1.00 | 0.00 |
| ATOM | 556 | C | ALA | 31 | 7.339 | 4.284 | 2.350 | 1.00 | 0.00 |
| ATOM | 557 | O | ALA | 31 | 7.036 | 3.425 | 1.531 | 1.00 | 0.00 |
| ATOM | 558 | N | LYS | 32 | 8.004 | 4.018 | 3.465 | 1.00 | 0.00 |
| ATOM | 559 | HN | LYS | 32 | 8.207 | 4.748 | 4.086 | 1.00 | 0.00 |
| ATOM | 560 | CA | LYS | 32 | 8.423 | 2.665 | 3.800 | 1.00 | 0.00 |
| ATOM | 561 | HA | LYS | 32 | 8.320 | 2.055 | 2.915 | 1.00 | 0.00 |
| ATOM | 562 | CB | LYS | 32 | 9.888 | 2.658 | 4.243 | 1.00 | 0.00 |
| ATOM | 563 | HB1 | LYS | 32 | 10.002 | 1.966 | 5.064 | 1.00 | 0.00 |
| ATOM | 564 | HB2 | LYS | 32 | 10.153 | 3.649 | 4.581 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 565 | CG | LYS | 32 | 10.854 | 2.256 | 3.142 | 1.00 | 0.00 |
| ATOM | 566 | HG1 | LYS | 32 | 10.575 | 2.762 | 2.229 | 1.00 | 0.00 |
| ATOM | 567 | HG2 | LYS | 32 | 10.795 | 1.188 | 2.996 | 1.00 | 0.00 |
| ATOM | 568 | CD | LYS | 32 | 12.286 | 2.626 | 3.494 | 1.00 | 0.00 |
| ATOM | 569 | HD1 | LYS | 32 | 12.314 | 3.660 | 3.806 | 1.00 | 0.00 |
| ATOM | 570 | HD2 | LYS | 32 | 12.907 | 2.494 | 2.621 | 1.00 | 0.00 |
| ATOM | 571 | CE | LYS | 32 | 12.825 | 1.756 | 4.618 | 1.00 | 0.00 |
| ATOM | 572 | HE1 | LYS | 32 | 13.059 | 0.780 | 4.220 | 1.00 | 0.00 |
| ATOM | 573 | HE2 | LYS | 32 | 12.065 | 1.661 | 5.379 | 1.00 | 0.00 |
| ATOM | 574 | NZ | LYS | 32 | 14.052 | 2.337 | 5.230 | 1.00 | 0.00 |
| ATOM | 575 | HZ1 | LYS | 32 | 13.804 | 2.878 | 6.084 | 1.00 | 0.00 |
| ATOM | 576 | HZ2 | LYS | 32 | 14.522 | 2.974 | 4.555 | 1.00 | 0.00 |
| ATOM | 577 | HZ3 | LYS | 32 | 14.714 | 1.579 | 5.493 | 1.00 | 0.00 |
| ATOM | 578 | C | LYS | 32 | 7.540 | 2.087 | 4.903 | 1.00 | 0.00 |
| ATOM | 579 | O | LYS | 32 | 7.231 | 2.768 | 5.875 | 1.00 | 0.00 |
| ATOM | 580 | N | ALA | 33 | 7.138 | 0.832 | 4.754 | 1.00 | 0.00 |
| ATOM | 581 | HN | ALA | 33 | 7.397 | 0.343 | 3.958 | 1.00 | 0.00 |
| ATOM | 582 | CA | ALA | 33 | 6.291 | 0.184 | 5.741 | 1.00 | 0.00 |
| ATOM | 583 | HA | ALA | 33 | 5.414 | 0.796 | 5.893 | 1.00 | 0.00 |
| ATOM | 584 | CB | ALA | 33 | 5.849 | -1.174 | 5.233 | 1.00 | 0.00 |
| ATOM | 585 | HB1 | ALA | 33 | 5.461 | -1.757 | 6.054 | 1.00 | 0.00 |
| ATOM | 586 | HB2 | ALA | 33 | 6.693 | -1.686 | 4.795 | 1.00 | 0.00 |
| ATOM | 587 | HB3 | ALA | 33 | 5.080 | -1.044 | 4.486 | 1.00 | 0.00 |
| ATOM | 588 | C | ALA | 33 | 7.037 | 0.038 | 7.059 | 1.00 | 0.00 |
| ATOM | 589 | O | ALA | 33 | 8.242 | -0.199 | 7.088 | 1.00 | 0.00 |
| ATOM | 590 | N | CYS | 34 | 6.317 | 0.258 | 8.145 | 1.00 | 0.00 |
| ATOM | 591 | HN | CYS | 34 | 5.369 | 0.484 | 8.040 | 1.00 | 0.00 |
| ATOM | 592 | CA | CYS | 34 | 6.903 | 0.217 | 9.484 | 1.00 | 0.00 |
| ATOM | 593 | HA | CYS | 34 | 7.969 | 0.328 | 9.366 | 1.00 | 0.00 |
| ATOM | 594 | CB | CYS | 34 | 6.396 | 1.907 | 10.299 | 1.00 | 0.00 |
| ATOM | 595 | HB1 | CYS | 34 | 5.376 | 1.218 | 10.602 | 1.00 | 0.00 |
| ATOM | 596 | HB2 | CYS | 34 | 6.424 | 2.294 | 9.684 | 1.00 | 0.00 |
| ATOM | 597 | SG | CYS | 34 | 7.361 | 1.745 | 11.791 | 1.00 | 0.00 |
| ATOM | 598 | HG | CYS | 34 | 7.434 | 2.697 | 11.887 | 1.00 | 0.00 |
| ATOM | 599 | C | CYS | 34 | 6.646 | -1.075 | 10.269 | 1.00 | 0.00 |
| ATOM | 600 | O | CYS | 34 | 7.377 | -1.373 | 11.215 | 1.00 | 0.00 |
| ATOM | 601 | N | CYS | 35 | 5.565 | -1.788 | 9.965 | 1.00 | 0.00 |
| ATOM | 602 | HN | CYS | 35 | 4.962 | -1.481 | 9.249 | 1.00 | 0.00 |
| ATOM | 603 | CA | CYS | 35 | 5.211 | -2.972 | 10.767 | 1.00 | 0.00 |
| ATOM | 604 | HA | CYS | 35 | 5.810 | -2.979 | 11.660 | 1.00 | 0.00 |
| ATOM | 605 | CB | CYS | 35 | 3.763 | -2.826 | 11.155 | 1.00 | 0.00 |
| ATOM | 606 | HB1 | CYS | 35 | 3.176 | -3.049 | 10.272 | 1.00 | 0.00 |
| ATOM | 607 | HB2 | CYS | 35 | 3.577 | -1.810 | 11.463 | 1.00 | 0.00 |
| ATOM | 608 | SG | CYS | 35 | 3.223 | -3.927 | 12.483 | 1.00 | 0.00 |
| ATOM | 609 | HG | CYS | 35 | 3.294 | -3.449 | 13.312 | 1.00 | 0.00 |
| ATOM | 610 | C | CYS | 35 | 5.322 | -4.313 | 10.060 | 1.00 | 0.00 |
| ATOM | 611 | O | CYS | 35 | 5.256 | -5.359 | 10.706 | 1.00 | 0.00 |
| ATOM | 612 | N | ASP | 36 | 5.409 | -4.292 | 8.755 | 1.00 | 0.00 |
| ATOM | 613 | HN | ASP | 36 | 5.376 | -3.427 | 8.303 | 1.00 | 0.00 |
| ATOM | 614 | CA | ASP | 36 | 5.441 | -5.534 | 7.972 | 1.00 | 0.00 |

(fortgesetzt)

Atomkoordinaten der Struktur der ADAP hSH3-1-Domäne, reduzierte Form

| ATOM | 615 | HA | ASP | 36 | 5.818 | -5.303 | 6.985 | 1.00 | 0.00 |
|------|-----|------|-----|-----|--------|---------|--------|------|------|
| ATOM | 616 | CB | ASP | 36 | 6.320 | -6.614 | 8.627 | 1.00 | 0.00 |
| ATOM | 617 | HB1 | ASP | 36 | 6.555 | -7.368 | 7.890 | 1.00 | 0.00 |
| ATOM | 618 | HB2 | ASP | 36 | 5.771 | -7.073 | 9.435 | 1.00 | 0.00 |
| ATOM | 619 | CG | ASP | 36 | 7.623 | -6.071 | 9.187 | 1.00 | 0.00 |
| ATOM | 620 | OD1 | ASP | 36 | 7.643 | -5.664 | 10.367 | 1.00 | 0.00 |
| ATOM | 621 | OD2 | ASP | 36 | 8.628 | -6.062 | 8.445 | 1.00 | 0.00 |
| ATOM | 622 | C | ASP | 36 | 4.006 | -6.029 | 7.857 | 1.00 | 0.00 |
| ATOM | 623 | O | ASP | 36 | 3.660 | -7.131 | 8.284 | 1.00 | 0.00 |
| ATOM | 624 | N | VAL | 37 | 3.178 | -5.150 | 7.319 | 1.00 | 0.00 |
| ATOM | 625 | HN | VAL | 37 | 3.544 | -4.285 | 7.047 | 1.00 | 0.00 |
| ATOM | 626 | CA | VAL | 37 | 1.746 | -5.372 | 7.162 | 1.00 | 0.00 |
| ATOM | 627 | HA | VAL | 37 | 1.288 | -5.286 | 8.135 | 1.00 | 0.00 |
| ATOM | 628 | CB | VAL | 37 | 1.165 | -4.266 | 6.272 | 1.00 | 0.00 |
| ATOM | 629 | HB | VAL | 37 | 1.619 | -4.356 | 5.302 | 1.00 | 0.00 |
| ATOM | 630 | CG1 | VAL | 37 | -0.332 | -4.425 | 6.098 | 1.00 | 0.00 |
| ATOM | 631 | HG11 | VAL | 37 | -0.540 | -4.690 | 5.071 | 1.00 | 0.00 |
| ATOM | 632 | HG12 | VAL | 37 | -0.826 | -3.495 | 6.338 | 1.00 | 0.00 |
| ATOM | 633 | HG13 | VAL | 37 | -0.691 | -5.206 | 6.751 | 1.00 | 0.00 |
| ATOM | 634 | CG2 | VAL | 37 | 1.513 | -2.894 | 6.833 | 1.00 | 0.00 |
| ATOM | 635 | HG21 | VAL | 37 | 1.050 | -2.129 | 6.229 | 1.00 | 0.00 |
| ATOM | 636 | HG22 | VAL | 37 | 2.586 | -2.761 | 6.823 | 1.00 | 0.00 |
| ATOM | 637 | HG23 | VAL | 37 | 1.152 | -2.819 | 7.848 | 1.00 | 0.00 |
| ATOM | 638 | C | VAL | 37 | 1.380 | -6.729 | 6.561 | 1.00 | 0.00 |
| ATOM | 639 | O | VAL | 37 | 1.780 | -7.066 | 5.446 | 1.00 | 0.00 |
| ATOM | 640 | N | LYS | 38 | 0.574 | -7.479 | 7.312 | 1.00 | 0.00 |
| ATOM | 641 | HN | LYS | 38 | 0.274 | -7.126 | 8.176 | 1.00 | 0.00 |
| ATOM | 642 | CA | LYS | 38 | 0.081 | -8.785 | 6.882 | 1.00 | 0.00 |
| ATOM | 643 | HA | LYS | 38 | 0.290 | -8.891 | 5.828 | 1.00 | 0.00 |
| ATOM | 644 | CB | LYS | 38 | 0.788 | -9.903 | 7.651 | 1.00 | 0.00 |
| ATOM | 645 | HB1 | LYS | 38 | 0.219 | -10.129 | 8.540 | 1.00 | 0.00 |
| ATOM | 646 | HB2 | LYS | 38 | 1.770 | -9.559 | 7.941 | 1.00 | 0.00 |
| ATOM | 647 | CG | LYS | 38 | 0.950 | -11.185 | 6.850 | 1.00 | 0.00 |
| ATOM | 648 | HG1 | LYS | 38 | 1.879 | -11.659 | 7.131 | 1.00 | 0.00 |
| ATOM | 649 | HG2 | LYS | 38 | 0.971 | -10.940 | 5.798 | 1.00 | 0.00 |
| ATOM | 650 | CD | LYS | 38 | -0.196 | -12.150 | 7.107 | 1.00 | 0.00 |
| ATOM | 651 | HD1 | LYS | 38 | -1.058 | -11.590 | 7.438 | 1.00 | 0.00 |
| ATOM | 652 | HD2 | LYS | 38 | 0.099 | -12.848 | 7.876 | 1.00 | 0.00 |
| ATOM | 653 | CE | LYS | 38 | -0.562 | -12.927 | 5.853 | 1.00 | 0.00 |
| ATOM | 654 | HE1 | LYS | 38 | 0.293 | -12.945 | 5.193 | 1.00 | 0.00 |
| ATOM | 655 | HE2 | LYS | 38 | -1.383 | -12.426 | 5.361 | 1.00 | 0.00 |
| ATOM | 656 | NZ | LYS | 38 | -0.966 | -14.326 | 6.163 | 1.00 | 0.00 |
| ATOM | 657 | HZ1 | LYS | 38 | -0.700 | -14.957 | 5.380 | 1.00 | 0.00 |
| ATOM | 658 | HZ2 | LYS | 38 | -1.995 | -14.379 | 6.303 | 1.00 | 0.00 |
| ATOM | 659 | HZ3 | LYS | 38 | -0.492 | -14.650 | 7.031 | 1.00 | 0.00 |
| ATOM | 660 | C | LYS | 38 | -1.433 | -8.873 | 7.101 | 1.00 | 0.00 |
| ATOM | 661 | O | LYS | 38 | -1.881 | -9.232 | 8.190 | 1.00 | 0.00 |
| ATOM | 662 | N | GLY | 39 | -2.223 | -8.532 | 6.081 | 1.00 | 0.00 |
| ATOM | 663 | HN | GLY | 39 | -1.822 | -8.240 | 5.233 | 1.00 | 0.00 |
| ATOM | 664 | CA | GLY | 39 | -3.676 | -8.576 | 6.232 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 665 | HA1 | GLY | 39 | -4.079 | -7.597 | 6.056 | 1.00 | 0.00 |
| ATOM | 666 | HA2 | GLY | 39 | -3.903 | -8.858 | 7.249 | 1.00 | 0.00 |
| ATOM | 667 | C | GLY | 39 | -4.357 | -9.566 | 5.302 | 1.00 | 0.00 |
| ATOM | 668 | O | GLY | 39 | -3.745 | -10.073 | 4.362 | 1.00 | 0.00 |
| ATOM | 669 | N | GLY | 40 | -5.630 | -9.852 | 5.586 | 1.00 | 0.00 |
| ATOM | 670 | HN | GLY | 40 | -6.052 | -9.408 | 6.345 | 1.00 | 0.00 |
| ATOM | 671 | CA | GLY | 40 | -6.382 | -10.807 | 4.775 | 1.00 | 0.00 |
| ATOM | 672 | HA1 | GLY | 40 | -6.587 | -11.677 | 5.383 | 1.00 | 0.00 |
| ATOM | 673 | HA2 | GLY | 40 | -5.760 | -11.112 | 3.949 | 1.00 | 0.00 |
| ATOM | 674 | C | GLY | 40 | -7.705 | -10.293 | 4.208 | 1.00 | 0.00 |
| ATOM | 675 | O | GLY | 40 | -8.294 | -10.946 | 3.346 | 1.00 | 0.00 |
| ATOM | 676 | N | LYS | 41 | -8.181 | -9.136 | 4.666 | 1.00 | 0.00 |
| ATOM | 677 | HN | LYS | 41 | -7.680 | -8.644 | 5.349 | 1.00 | 0.00 |
| ATOM | 678 | CA | LYS | 41 | -9.444 | -8.584 | 4.156 | 1.00 | 0.00 |
| ATOM | 679 | HA | LYS | 41 | -9.964 | -9.367 | 3.622 | 1.00 | 0.00 |
| ATOM | 680 | CB | LYS | 41 | -10.318 | -8.086 | 5.309 | 1.00 | 0.00 |
| ATOM | 681 | HB1 | LYS | 41 | -10.081 | -7.050 | 5.503 | 1.00 | 0.00 |
| ATOM | 682 | HB2 | LYS | 41 | -10.097 | -8.668 | 6.191 | 1.00 | 0.00 |
| ATOM | 683 | CG | LYS | 41 | -11.809 | -8.189 | 5.031 | 1.00 | 0.00 |
| ATOM | 684 | HG1 | LYS | 41 | -12.318 | -8.458 | 5.945 | 1.00 | 0.00 |
| ATOM | 685 | HG2 | LYS | 41 | -11.974 | -8.953 | 4.286 | 1.00 | 0.00 |
| ATOM | 686 | CD | LYS | 41 | -12.373 | -6.874 | 4.520 | 1.00 | 0.00 |
| ATOM | 687 | HD1 | LYS | 41 | -13.239 | -7.078 | 3.908 | 1.00 | 0.00 |
| ATOM | 688 | HD2 | LYS | 41 | -11.620 | -6.376 | 3.927 | 1.00 | 0.00 |
| ATOM | 689 | CE | LYS | 41 | -12.781 | -5.960 | 5.665 | 1.00 | 0.00 |
| ATOM | 690 | HE1 | LYS | 41 | -13.004 | -6.567 | 6.530 | 1.00 | 0.00 |
| ATOM | 691 | HE2 | LYS | 41 | -13.664 | -5.412 | 5.373 | 1.00 | 0.00 |
| ATOM | 692 | NZ | LYS | 41 | -11.704 | -4.993 | 6.014 | 1.00 | 0.00 |
| ATOM | 693 | HZ1 | LYS | 41 | -11.122 | -4.788 | 5.177 | 1.00 | 0.00 |
| ATOM | 694 | HZ2 | LYS | 41 | -11.094 | -5.390 | 6.757 | 1.00 | 0.00 |
| ATOM | 695 | HZ3 | LYS | 41 | -12.120 | -4.105 | 6.361 | 1.00 | 0.00 |
| ATOM | 696 | C | LYS | 41 | -9.138 | -7.448 | 3.199 | 1.00 | 0.00 |
| ATOM | 697 | O | LYS | 41 | -9.653 | -7.382 | 2.083 | 1.00 | 0.00 |
| ATOM | 698 | N | ASN | 42 | -8.230 | -6.605 | 3.643 | 1.00 | 0.00 |
| ATOM | 699 | HN | ASN | 42 | -7.837 | -6.768 | 4.526 | 1.00 | 0.00 |
| ATOM | 700 | CA | ASN | 42 | -7.730 | -5.492 | 2.864 | 1.00 | 0.00 |
| ATOM | 701 | HA | ASN | 42 | -7.683 | -5.784 | 1.825 | 1.00 | 0.00 |
| ATOM | 702 | CB | ASN | 42 | -8.617 | -4.256 | 3.029 | 1.00 | 0.00 |
| ATOM | 703 | HB1 | ASN | 42 | -7.997 | -3.371 | 3.017 | 1.00 | 0.00 |
| ATOM | 704 | HB2 | ASN | 42 | -9.134 | -4.315 | 3.976 | 1.00 | 0.00 |
| ATOM | 705 | CG | ASN | 42 | -9.651 | -4.134 | 1.927 | 1.00 | 0.00 |
| ATOM | 706 | OD1 | ASN | 42 | -9.315 | -4.133 | 0.742 | 1.00 | 0.00 |
| ATOM | 707 | ND2 | ASN | 42 | -10.917 | -4.029 | 2.312 | 1.00 | 0.00 |
| ATOM | 708 | HD21 | ASN | 42 | -11.111 | -4.037 | 3.272 | 1.00 | 0.00 |
| ATOM | 709 | HD22 | ASN | 42 | -11.607 | -3.949 | 1.620 | 1.00 | 0.00 |
| ATOM | 710 | C | ASN | 42 | -6.339 | -5.227 | 3.376 | 1.00 | 0.00 |
| ATOM | 711 | O | ASN | 42 | -6.156 | -4.687 | 4.467 | 1.00 | 0.00 |
| ATOM | 712 | N | GLU | 43 | -5.358 | -5.661 | 2.611 | 1.00 | 0.00 |
| ATOM | 713 | HN | GLU | 43 | -5.566 | -6.117 | 1.772 | 1.00 | 0.00 |
| ATOM | 714 | CA | GLU | 43 | -3.984 | -5.522 | 3.025 | 1.00 | 0.00 |

(fortgesetzt)

Atomkoordinaten der Struktur der ADAP hSH3-1-Domäne, reduzierte Form

| ATOM | 715 | HA | GLU | 43 | -3.804 | -4.485 | 3.223 | 1.00 | 0.00 |
|------|-----|------|-----|----|--------|---------|--------|------|------|
| ATOM | 716 | CB | GLU | 43 | -3.767 | -6.304 | 4.315 | 1.00 | 0.00 |
| ATOM | 717 | HB1 | GLU | 43 | -3.377 | -7.283 | 4.079 | 1.00 | 0.00 |
| ATOM | 718 | HB2 | GLU | 43 | -4.721 | -6.419 | 4.808 | 1.00 | 0.00 |
| ATOM | 719 | CG | GLU | 43 | -2.815 | -5.624 | 5.280 | 1.00 | 0.00 |
| ATOM | 720 | HG1 | GLU | 43 | -2.457 | -4.712 | 4.825 | 1.00 | 0.00 |
| ATOM | 721 | HG2 | GLU | 43 | -1.981 | -6.282 | 5.469 | 1.00 | 0.00 |
| ATOM | 722 | CD | GLU | 43 | -3.469 | -5.279 | 6.605 | 1.00 | 0.00 |
| ATOM | 723 | OE1 | GLU | 43 | -3.147 | -5.938 | 7.615 | 1.00 | 0.00 |
| ATOM | 724 | OE2 | GLU | 43 | -4.302 | -4.349 | 6.631 | 1.00 | 0.00 |
| ATOM | 725 | C | GLU | 43 | -3.028 | -6.004 | 1.945 | 1.00 | 0.00 |
| ATOM | 726 | O | GLU | 43 | -3.442 | -6.595 | 0.947 | 1.00 | 0.00 |
| ATOM | 727 | N | LEU | 44 | -1.746 | -5.774 | 2.170 | 1.00 | 0.00 |
| ATOM | 728 | HN | LEU | 44 | -1.483 | -5.319 | 2.996 | 1.00 | 0.00 |
| ATOM | 729 | CA | LEU | 44 | -0.713 | -6.207 | 1.244 | 1.00 | 0.00 |
| ATOM | 730 | HA | LEU | 44 | -1.137 | -6.949 | 0.584 | 1.00 | 0.00 |
| ATOM | 731 | CB | LEU | 44 | -0.202 | -5.028 | 0.423 | 1.00 | 0.00 |
| ATOM | 732 | HB1 | LEU | 44 | 0.756 | -4.727 | 0.815 | 1.00 | 0.00 |
| ATOM | 733 | HB2 | LEU | 44 | -0.893 | -4.219 | 0.549 | 1.00 | 0.00 |
| ATOM | 734 | CG | LEU | 44 | -0.044 | -5.292 | -1.068 | 1.00 | 0.00 |
| ATOM | 735 | HG | LEU | 44 | 0.189 | -4.365 | -1.569 | 1.00 | 0.00 |
| ATOM | 736 | CD1 | LEU | 44 | 1.105 | -6.254 | -1.314 | 1.00 | 0.00 |
| ATOM | 737 | HD11 | LEU | 44 | 1.585 | -6.006 | -2.249 | 1.00 | 0.00 |
| ATOM | 738 | HD12 | LEU | 44 | 0.727 | -7.264 | -1.361 | 1.00 | 0.00 |
| ATOM | 739 | HD13 | LEU | 44 | 1.821 | -6.172 | -0.510 | 1.00 | 0.00 |
| ATOM | 740 | CD2 | LEU | 44 | -1.346 | -5.829 | -1.644 | 1.00 | 0.00 |
| ATOM | 741 | HD21 | LEU | 44 | -1.413 | -6.890 | -1.456 | 1.00 | 0.00 |
| ATOM | 742 | HD22 | LEU | 44 | -1.371 | -5.649 | -2.708 | 1.00 | 0.00 |
| ATOM | 743 | HD23 | LEU | 44 | -2.179 | -5.325 | -1.174 | 1.00 | 0.00 |
| ATOM | 744 | C | LEU | 44 | 0.430 | -6.825 | 2.023 | 1.00 | 0.00 |
| ATOM | 745 | O | LEU | 44 | 0.634 | -6.495 | 3.191 | 1.00 | 0.00 |
| ATOM | 746 | N | SER | 45 | 1.183 | -7.713 | 1.391 | 1.00 | 0.00 |
| ATOM | 747 | HN | SER | 45 | 0.990 | -7.947 | 0.461 | 1.00 | 0.00 |
| ATOM | 748 | CA | SER | 45 | 2.294 | -8.334 | 2.080 | 1.00 | 0.00 |
| ATOM | 749 | HA | SER | 45 | 2.113 | -8.236 | 3.141 | 1.00 | 0.00 |
| ATOM | 750 | CB | SER | 45 | 2.419 | -9.810 | 1.745 | 1.00 | 0.00 |
| ATOM | 751 | HB1 | SER | 45 | 3.250 | -10.214 | 2.307 | 1.00 | 0.00 |
| ATOM | 752 | HB2 | SER | 45 | 2.603 | -9.927 | 0.687 | 1.00 | 0.00 |
| ATOM | 753 | OG | SER | 45 | 1.242 | -10.520 | 2.090 | 1.00 | 0.00 |
| ATOM | 754 | HG | SER | 45 | 0.524 | -10.248 | 1.514 | 1.00 | 0.00 |
| ATOM | 755 | C | SER | 45 | 3.594 | -7.632 | 1.754 | 1.00 | 0.00 |
| ATOM | 756 | O | SER | 45 | 3.918 | -7.370 | 0.595 | 1.00 | 0.00 |
| ATOM | 757 | N | PHE | 46 | 4.330 | -7.350 | 2.804 | 1.00 | 0.00 |
| ATOM | 758 | HN | PHE | 46 | 3.995 | -7.616 | 3.688 | 1.00 | 0.00 |
| ATOM | 759 | CA | PHE | 46 | 5.620 | -6.691 | 2.706 | 1.00 | 0.00 |
| ATOM | 760 | HA | PHE | 46 | 6.226 | -7.271 | 2.023 | 1.00 | 0.00 |
| ATOM | 761 | CB | PHE | 46 | 5.484 | -5.264 | 2.154 | 1.00 | 0.00 |
| ATOM | 762 | HB1 | PHE | 46 | 5.283 | -5.324 | 1.094 | 1.00 | 0.00 |
| ATOM | 763 | HB2 | PHE | 46 | 6.409 | -4.732 | 2.299 | 1.00 | 0.00 |
| ATOM | 764 | CG | PHE | 46 | 4.386 | -4.454 | 2.778 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 765 | CD1 | PHE | 46 | 4.609 | -3.692 | 3.919 | 1.00 | 0.00 |
| ATOM | 766 | HD1 | PHE | 46 | 5.587 | -3.682 | 4.381 | 1.00 | 0.00 |
| ATOM | 767 | CD2 | PHE | 46 | 3.125 | -4.443 | 2.207 | 1.00 | 0.00 |
| ATOM | 768 | HD2 | PHE | 46 | 2.938 | -5.032 | 1.324 | 1.00 | 0.00 |
| ATOM | 769 | CE1 | PHE | 46 | 3.594 | -2.944 | 4.469 | 1.00 | 0.00 |
| ATOM | 770 | HE1 | PHE | 46 | 3.781 | -2.356 | 5.353 | 1.00 | 0.00 |
| ATOM | 771 | CE2 | PHE | 46 | 2.109 | -3.695 | 2.756 | 1.00 | 0.00 |
| ATOM | 772 | HE2 | PHE | 46 | 1.134 | -3.692 | 2.296 | 1.00 | 0.00 |
| ATOM | 773 | CZ | PHE | 46 | 2.343 | -2.943 | 3.887 | 1.00 | 0.00 |
| ATOM | 774 | HZ | PHE | 46 | 1.545 | -2.361 | 4.321 | 1.00 | 0.00 |
| ATOM | 775 | C | PHE | 46 | 6.294 | -6.707 | 4.068 | 1.00 | 0.00 |
| ATOM | 776 | O | PHE | 46 | 5.738 | -7.237 | 5.031 | 1.00 | 0.00 |
| ATOM | 777 | N | LYS | 47 | 7.486 | -6.146 | 4.157 | 1.00 | 0.00 |
| ATOM | 778 | HN | LYS | 47 | 7.887 | -5.737 | 3.365 | 1.00 | 0.00 |
| ATOM | 779 | CA | LYS | 47 | 8.206 | -6.130 | 5.422 | 1.00 | 0.00 |
| ATOM | 780 | HA | LYS | 47 | 7.500 | -6.366 | 6.204 | 1.00 | 0.00 |
| ATOM | 781 | CB | LYS | 47 | 9.305 | -7.194 | 5.418 | 1.00 | 0.00 |
| ATOM | 782 | HB1 | LYS | 47 | 10.144 | -6.831 | 5.993 | 1.00 | 0.00 |
| ATOM | 783 | HB2 | LYS | 47 | 9.623 | -7.363 | 4.400 | 1.00 | 0.00 |
| ATOM | 784 | CG | LYS | 47 | 8.860 | -8.523 | 6.007 | 1.00 | 0.00 |
| ATOM | 785 | HG1 | LYS | 47 | 8.746 | -9.240 | 5.206 | 1.00 | 0.00 |
| ATOM | 786 | HG2 | LYS | 47 | 7.913 | -8.385 | 6.506 | 1.00 | 0.00 |
| ATOM | 787 | CD | LYS | 47 | 9.871 | -9.058 | 7.009 | 1.00 | 0.00 |
| ATOM | 788 | HD1 | LYS | 47 | 10.146 | -8.263 | 7.687 | 1.00 | 0.00 |
| ATOM | 789 | HD2 | LYS | 47 | 10.746 | -9.399 | 6.476 | 1.00 | 0.00 |
| ATOM | 790 | CE | LYS | 47 | 9.301 | -10.217 | 7.811 | 1.00 | 0.00 |
| ATOM | 791 | HE1 | LYS | 47 | 9.323 | -11.106 | 7.198 | 1.00 | 0.00 |
| ATOM | 792 | HE2 | LYS | 47 | 8.279 | -9.987 | 8.075 | 1.00 | 0.00 |
| ATOM | 793 | NZ | LYS | 47 | 10.078 | -10.466 | 9.056 | 1.00 | 0.00 |
| ATOM | 794 | HZ1 | LYS | 47 | 9.709 | -11.302 | 9.550 | 1.00 | 0.00 |
| ATOM | 795 | HZ2 | LYS | 47 | 10.011 | -9.645 | 9.690 | 1.00 | 0.00 |
| ATOM | 796 | HZ3 | LYS | 47 | 11.079 | -10.632 | 8.826 | 1.00 | 0.00 |
| ATOM | 797 | C | LYS | 47 | 8.806 | -4.764 | 5.709 | 1.00 | 0.00 |
| ATOM | 798 | O | LYS | 47 | 9.668 | -4.290 | 4.970 | 1.00 | 0.00 |
| ATOM | 799 | N | GLN | 48 | 8.340 | -4.149 | 6.798 | 1.00 | 0.00 |
| ATOM | 800 | HN | GLN | 48 | 7.649 | -4.597 | 7.329 | 1.00 | 0.00 |
| ATOM | 801 | CA | GLN | 48 | 8.809 | -2.833 | 7.236 | 1.00 | 0.00 |
| ATOM | 802 | HA | GLN | 48 | 8.006 | -2.139 | 7.070 | 1.00 | 0.00 |
| ATOM | 803 | CB | GLN | 48 | 9.130 | -2.864 | 8.734 | 1.00 | 0.00 |
| ATOM | 804 | HB1 | GLN | 48 | 9.780 | -3.703 | 8.932 | 1.00 | 0.00 |
| ATOM | 805 | HB2 | GLN | 48 | 8.210 | -2.997 | 9.283 | 1.00 | 0.00 |
| ATOM | 806 | CG | GLN | 48 | 9.811 | -1.601 | 9.246 | 1.00 | 0.00 |
| ATOM | 807 | HG1 | GLN | 48 | 9.212 | -1.181 | 10.040 | 1.00 | 0.00 |
| ATOM | 808 | HG2 | GLN | 48 | 9.882 | -0.891 | 8.436 | 1.00 | 0.00 |
| ATOM | 809 | CD | GLN | 48 | 11.206 | -1.865 | 9.780 | 1.00 | 0.00 |
| ATOM | 810 | OE1 | GLN | 48 | 11.604 | -1.315 | 10.807 | 1.00 | 0.00 |
| ATOM | 811 | NE2 | GLN | 48 | 11.957 | -2.710 | 9.083 | 1.00 | 0.00 |
| ATOM | 812 | HE21 | GLN | 48 | 11.574 | -3.110 | 8.274 | 1.00 | 0.00 |
| ATOM | 813 | HE22 | GLN | 48 | 12.862 | -2.899 | 9.406 | 1.00 | 0.00 |
| ATOM | 814 | C | GLN | 48 | 10.034 | -2.364 | 6.451 | 1.00 | 0.00 |

(fortgesetzt)

Atomkoordinaten der Struktur der ADAP hSH3-1-Domäne, reduzierte Form

| ATOM | 815 | O | GLN | 48 | 11.136 | -2.884 | 6.629 | 1.00 | 0.00 |
|------|-----|------|-----|----|--------|--------|-------|------|------|
| ATOM | 816 | N | GLY | 49 | 9.829 | -1.384 | 5.581 | 1.00 | 0.00 |
| ATOM | 817 | HN | GLY | 49 | 8.928 | -1.011 | 5.480 | 1.00 | 0.00 |
| ATOM | 818 | CA | GLY | 49 | 10.917 | -0.865 | 4.781 | 1.00 | 0.00 |
| ATOM | 819 | HA1 | GLY | 49 | 11.854 | -1.127 | 5.251 | 1.00 | 0.00 |
| ATOM | 820 | HA2 | GLY | 49 | 10.837 | 0.211 | 4.735 | 1.00 | 0.00 |
| ATOM | 821 | C | GLY | 49 | 10.905 | -1.418 | 3.371 | 1.00 | 0.00 |
| ATOM | 822 | O | GLY | 49 | 11.927 | -1.405 | 2.685 | 1.00 | 0.00 |
| ATOM | 823 | N | GLU | 50 | 9.746 | -1.907 | 2.936 | 1.00 | 0.00 |
| ATOM | 824 | HN | GLU | 50 | 8.960 | -1.891 | 3.528 | 1.00 | 0.00 |
| ATOM | 825 | CA | GLU | 50 | 9.617 | -2.466 | 1.596 | 1.00 | 0.00 |
| ATOM | 826 | HA | GLU | 50 | 10.550 | -2.920 | 1.360 | 1.00 | 0.00 |
| ATOM | 827 | CB | GLU | 50 | 8.512 | -3.523 | 1.552 | 1.00 | 0.00 |
| ATOM | 828 | HB1 | GLU | 50 | 7.651 | -3.109 | 1.049 | 1.00 | 0.00 |
| ATOM | 829 | HB2 | GLU | 50 | 8.236 | -3.781 | 2.564 | 1.00 | 0.00 |
| ATOM | 830 | CG | GLU | 50 | 8.918 | -4.798 | 0.830 | 1.00 | 0.00 |
| ATOM | 831 | HG1 | GLU | 50 | 8.057 | -5.447 | 0.761 | 1.00 | 0.00 |
| ATOM | 832 | HG2 | GLU | 50 | 9.692 | -5.289 | 1.401 | 1.00 | 0.00 |
| ATOM | 833 | CD | GLU | 50 | 9.442 | -4.534 | -0.568 | 1.00 | 0.00 |
| ATOM | 834 | OE1 | GLU | 50 | 10.463 | -3.826 | -0.696 | 1.00 | 0.00 |
| ATOM | 835 | OE2 | GLU | 50 | 8.831 | -5.036 | -1.535 | 1.00 | 0.00 |
| ATOM | 836 | C | GLU | 50 | 9.358 | -1.382 | 0.563 | 1.00 | 0.00 |
| ATOM | 837 | O | GLU | 50 | 9.240 | -1.656 | -0.631 | 1.00 | 0.00 |
| ATOM | 838 | N | GLN | 51 | 9.270 | -0.157 | 1.038 | 1.00 | 0.00 |
| ATOM | 839 | HN | GLN | 51 | 9.381 | -0.025 | 1.996 | 1.00 | 0.00 |
| ATOM | 840 | CA | GLN | 51 | 9.019 | 0.992 | 0.175 | 1.00 | 0.00 |
| ATOM | 841 | HA | GLN | 51 | 8.991 | 1.875 | 0.795 | 1.00 | 0.00 |
| ATOM | 842 | CB | GLN | 51 | 10.140 | 1.135 | -0.852 | 1.00 | 0.00 |
| ATOM | 843 | HB1 | GLN | 51 | 9.709 | 1.403 | -1.805 | 1.00 | 0.00 |
| ATOM | 844 | HB2 | GLN | 51 | 10.644 | 0.186 | -0.947 | 1.00 | 0.00 |
| ATOM | 845 | CG | GLN | 51 | 11.172 | 2.188 | -0.482 | 1.00 | 0.00 |
| ATOM | 846 | HG1 | GLN | 51 | 12.058 | 2.030 | -1.079 | 1.00 | 0.00 |
| ATOM | 847 | HG2 | GLN | 51 | 11.419 | 2.079 | 0.563 | 1.00 | 0.00 |
| ATOM | 848 | CD | GLN | 51 | 10.674 | 3.600 | -0.717 | 1.00 | 0.00 |
| ATOM | 849 | OE1 | GLN | 51 | 10.091 | 3.900 | -1.759 | 1.00 | 0.00 |
| ATOM | 850 | NE2 | GLN | 51 | 10.902 | 4.477 | 0.254 | 1.00 | 0.00 |
| ATOM | 851 | HE21 | GLN | 51 | 11.372 | 4.168 | 1.056 | 1.00 | 0.00 |
| ATOM | 852 | HE22 | GLN | 51 | 10.590 | 5.398 | 0.128 | 1.00 | 0.00 |
| ATOM | 853 | C | GLN | 51 | 7.676 | 0.839 | -0.528 | 1.00 | 0.00 |
| ATOM | 854 | O | GLN | 51 | 7.451 | -0.131 | -1.252 | 1.00 | 0.00 |
| ATOM | 855 | N | ILE | 52 | 6.774 | 1.784 | -0.295 | 1.00 | 0.00 |
| ATOM | 856 | HN | ILE | 52 | 6.997 | 2.526 | 0.303 | 1.00 | 0.00 |
| ATOM | 857 | CA | ILE | 52 | 5.448 | 1.721 | -0.896 | 1.00 | 0.00 |
| ATOM | 858 | HA | ILE | 52 | 5.497 | 1.018 | -1.683 | 1.00 | 0.00 |
| ATOM | 859 | CB | ILE | 52 | 4.384 | 1.243 | 0.126 | 1.00 | 0.00 |
| ATOM | 860 | HB | ILE | 52 | 3.572 | 1.956 | 0.118 | 1.00 | 0.00 |
| ATOM | 861 | CG1 | ILE | 52 | 4.966 | 1.173 | 1.543 | i.oo | 0.00 |
| ATOM | 862 | HG11 | ILE | 52 | 4.159 | 1.050 | 2.250 | 1.00 | 0.00 |
| ATOM | 863 | HG12 | ILE | 52 | 5.484 | 2.093 | 1.756 | 1.00 | 0.00 |
| ATOM | 864 | CG2 | ILE | 52 | 3.826 | -0.112 | -0.276 | 1.00 | 0.00 |

(fortgesetzt)

Atomkoordinaten der Struktur der ADAP hSH3-1-Domäne, reduzierte Form

| ATOM | 865 | HG21 | ILE | 52 | 4.488 | -0.575 | -0.991 | 1.00 | 0.00 |
|------|-----|------|-----|----|----|----|----|------|------|
| ATOM | 866 | HG22 | ILE | 52 | 2.851 | 0.017 | -0.716 | 1.00 | 0.00 |
| ATOM | 867 | HG23 | ILE | 52 | 3.744 | -0.742 | 0.597 | 1.00 | 0.00 |
| ATOM | 868 | CD1 | ILE | 52 | 5.942 | 0.034 | 1.749 | 1.00 | 0.00 |
| ATOM | 869 | HD11 | ILE | 52 | 5.634 | -0.553 | 2.601 | 1.00 | 0.00 |
| ATOM | 870 | HD12 | ILE | 52 | 6.929 | 0.436 | 1.926 | 1.00 | 0.00 |
| ATOM | 871 | HD13 | ILE | 52 | 5.959 | -0.591 | 0.868 | 1.00 | 0.00 |
| ATOM | 872 | C | ILE | 52 | 5.001 | 3.046 | -1.489 | 1.00 | 0.00 |
| ATOM | 873 | O | ILE | 52 | 3.869 | 3.175 | -1.935 | 1.00 | 0.00 |
| ATOM | 874 | N | GLU | 53 | 5.894 | 4.015 | -1.482 | 1.00 | 0.00 |
| ATOM | 875 | HN | GLU | 53 | 6.770 | 3.818 | -1.114 | 1.00 | 0.00 |
| ATOM | 876 | CA | GLU | 53 | 5.616 | 5.362 | -2.004 | 1.00 | 0.00 |
| ATOM | 877 | HA | GLU | 53 | 6.200 | 6.065 | -1.433 | 1.00 | 0.00 |
| ATOM | 878 | CB | GLU | 53 | 6.047 | 5.441 | -3.474 | 1.00 | 0.00 |
| ATOM | 879 | HB1 | GLU | 53 | 5.525 | 6.252 | -3.962 | 1.00 | 0.00 |
| ATOM | 880 | HB2 | GLU | 53 | 5.791 | 4.513 | -3.963 | 1.00 | 0.00 |
| ATOM | 881 | CG | GLU | 53 | 7.538 | 5.671 | -3.635 | 1.00 | 0.00 |
| ATOM | 882 | HG1 | GLU | 53 | 8.019 | 5.474 | -2.686 | 1.00 | 0.00 |
| ATOM | 883 | HG2 | GLU | 53 | 7.702 | 6.700 | -3.913 | 1.00 | 0.00 |
| ATOM | 884 | CD | GLU | 53 | 8.159 | 4.777 | -4.690 | 1.00 | 0.00 |
| ATOM | 885 | OE1 | GLU | 53 | 9.047 | 5.255 | -5.426 | 1.00 | 0.00 |
| ATOM | 886 | OE2 | GLU | 53 | 7.757 | 3.598 | -4.781 | 1.00 | 0.00 |
| ATOM | 887 | C | GLU | 53 | 4.134 | 5.712 | -1.836 | 1.00 | 0.00 |
| ATOM | 888 | O | GLU | 53 | 3.482 | 5.175 | -0.947 | 1.00 | 0.00 |
| ATOM | 889 | N | ILE | 54 | 3.596 | 6.568 | -2.708 | 1.00 | 0.00 |
| ATOM | 890 | HN | ILE | 54 | 4.161 | 6.950 | -3.414 | 1.00 | 0.00 |
| ATOM | 891 | CA | ILE | 54 | 2.177 | 6.946 | -2.666 | 1.00 | 0.00 |
| ATOM | 892 | HA | ILE | 54 | 1.590 | 6.040 | -2.723 | 1.00 | 0.00 |
| ATOM | 893 | CB | ILE | 54 | 1.754 | 7.728 | -1.390 | 1.00 | 0.00 |
| ATOM | 894 | HB | ILE | 54 | 2.019 | 8.766 | -1.526 | 1.00 | 0.00 |
| ATOM | 895 | CG1 | ILE | 54 | 2.401 | 7.210 | -0.115 | 1.00 | 0.00 |
| ATOM | 896 | HG11 | ILE | 54 | 2.542 | 6.149 | -0.186 | 1.00 | 0.00 |
| ATOM | 897 | HG12 | ILE | 54 | 1.738 | 7.412 | 0.714 | 1.00 | 0.00 |
| ATOM | 898 | CG2 | ILE | 54 | 0.260 | 7.646 | -1.213 | 1.00 | 0.00 |
| ATOM | 899 | HG21 | ILE | 54 | -0.190 | 8.585 | -1.491 | 1.00 | 0.00 |
| ATOM | 900 | HG22 | ILE | 54 | 0.046 | 7.432 | -0.173 | 1.00 | 0.00 |
| ATOM | 901 | HG23 | ILE | 54 | -0.131 | 6.853 | -1.831 | 1.00 | 0.00 |
| ATOM | 902 | CD1 | ILE | 54 | 3.728 | 7.851 | 0.188 | 1.00 | 0.00 |
| ATOM | 903 | HD11 | ILE | 54 | 3.962 | 7.694 | 1.226 | 1.00 | 0.00 |
| ATOM | 904 | HD12 | ILE | 54 | 3.672 | 8.912 | -0.018 | 1.00 | 0.00 |
| ATOM | 905 | HD13 | ILE | 54 | 4.492 | 7.402 | -0.425 | 1.00 | 0.00 |
| ATOM | 906 | C | ILE | 54 | 1.844 | 7.822 | -3.867 | 1.00 | 0.00 |
| ATOM | 907 | O | ILE | 54 | 2.708 | 8.527 | -4.374 | 1.00 | 0.00 |
| ATOM | 908 | N | ILE | 55 | 0.593 | 7.779 | -4.315 | 1.00 | 0.00 |
| ATOM | 909 | HN | ILE | 55 | -0.054 | 7.197 | -3.865 | 1.00 | 0.00 |
| ATOM | 910 | CA | ILE | 55 | 0.155 | 8.580 | -5.463 | 1.00 | 0.00 |
| ATOM | 911 | HA | ILE | 55 | 0.876 | 9.376 | -5.608 | 1.00 | 0.00 |
| ATOM | 912 | CB | ILE | 55 | 0.097 | 7.727 | -6.762 | 1.00 | 0.00 |
| ATOM | 913 | HB | ILE | 55 | -0.796 | 7.999 | -7.305 | 1.00 | 0.00 |
| ATOM | 914 | CG1 | ILE | 55 | 0.045 | 6.226 | -6.434 | 1.00 | 0.00 |

(fortgesetzt)

Atomkoordinaten der Struktur der ADAP hSH3-1-Domäne, reduzierte Form

| ATOM | 915 | HG11 | ILE | 55 | -0.204 | 5.675 | -7.327 | 1.00 | 0.00 |
|------|-----|------|-----|----|--------|-------|--------|------|------|
| ATOM | 916 | HG12 | ILE | 55 | -0.717 | 6.057 | -5.693 | 1.00 | 0.00 |
| ATOM | 917 | CG2 | ILE | 55 | 1.287 | 8.033 | -7.655 | 1.00 | 0.00 |
| ATOM | 918 | HG21 | ILE | 55 | 0.958 | 8.592 | -8.516 | 1.00 | 0.00 |
| ATOM | 919 | HG22 | ILE | 55 | 1.743 | 7.109 | -7.980 | 1.00 | 0.00 |
| ATOM | 920 | HG23 | ILE | 55 | 2.004 | 8.611 | -7.104 | 1.00 | 0.00 |
| ATOM | 921 | CD1 | ILE | 55 | 1.346 | 5.665 | -5.894 | 1.00 | 0.00 |
| ATOM | 922 | HD11 | ILE | 55 | 1.519 | 4.687 | -6.320 | 1.00 | 0.00 |
| ATOM | 923 | HD12 | ILE | 55 | 1.283 | 5.582 | -4.819 | 1.00 | 0.00 |
| ATOM | 924 | HD13 | ILE | 55 | 2.160 | 6.320 | -6.157 | 1.00 | 0.00 |
| ATOM | 925 | C | ILE | 55 | -1.221 | 9.207 | -5.203 | 1.00 | 0.00 |
| ATOM | 926 | O | ILE | 55 | -1.868 | 9.720 | -6.116 | 1.00 | 0.00 |
| ATOM | 927 | N | ARG | 56 | -1.653 | 9.152 | -3.945 | 1.00 | 0.00 |
| ATOM | 928 | HN | ARG | 56 | -1.083 | 8.731 | -3.273 | 1.00 | 0.00 |
| ATOM | 929 | CA | ARG | 56 | -2.941 | 9.697 | -3.520 | 1.00 | 0.00 |
| ATOM | 930 | HA | ARG | 56 | -3.145 | 10.526 | -4.178 | 1.00 | 0.00 |
| ATOM | 931 | CB | ARG | 56 | -4.098 | 8.695 | -3.645 | 1.00 | 0.00 |
| ATOM | 932 | HB1 | ARG | 56 | -4.668 | 8.700 | -2.728 | 1.00 | 0.00 |
| ATOM | 933 | HB2 | ARG | 56 | -3.685 | 7.709 | -3.793 | 1.00 | 0.00 |
| ATOM | 934 | CG | ARG | 56 | -5.056 | 8.987 | -4.810 | 1.00 | 0.00 |
| ATOM | 935 | HG1 | ARG | 56 | -5.813 | 8.215 | -4.827 | 1.00 | 0.00 |
| ATOM | 936 | HG2 | ARG | 56 | -4.494 | 8.945 | -5.726 | 1.00 | 0.00 |
| ATOM | 937 | CD | ARG | 56 | -5.757 | 10.362 | -4.735 | 1.00 | 0.00 |
| ATOM | 938 | HD1 | ARG | 56 | -5.985 | 10.612 | -3.707 | 1.00 | 0.00 |
| ATOM | 939 | HD2 | ARG | 56 | -6.685 | 10.297 | -5.282 | 1.00 | 0.00 |
| ATOM | 940 | NE | ARG | 56 | -4.952 | 11.435 | -5.319 | 1.00 | 0.00 |
| ATOM | 941 | HE | ARG | 56 | -4.159 | 11.725 | -4.825 | 1.00 | 0.00 |
| ATOM | 942 | CZ | ARG | 56 | -5.240 | 12.042 | -6.471 | 1.00 | 0.00 |
| ATOM | 943 | NH1 | ARG | 56 | -6.311 | 11.693 | -7.173 | 1.00 | 0.00 |
| ATOM | 944 | HH11 | ARG | 56 | -6.914 | 10.968 | -6.845 | 1.00 | 0.00 |
| ATOM | 945 | HH12 | ARG | 56 | -6.516 | 12.158 | -8.034 | 1.00 | 0.00 |
| ATOM | 946 | NH2 | ARG | 56 | -4.452 | 13.010 | -6.920 | 1.00 | 0.00 |
| ATOM | 947 | HH21 | ARG | 56 | -3.645 | 13.282 | -6.395 | 1.00 | 0.00 |
| ATOM | 948 | HH22 | ARG | 56 | -4.664 | 13.467 | -7.783 | 1.00 | 0.00 |
| ATOM | 949 | C | ARG | 56 | -2.847 | 10.259 | -2.102 | 1.00 | 0.00 |
| ATOM | 950 | O | ARG | 56 | -1.761 | 10.399 | -1.558 | 1.00 | 0.00 |
| ATOM | 951 | N | ILE | 57 | -4.013 | 10.628 | -1.582 | 1.00 | 0.00 |
| ATOM | 952 | HN | ILE | 57 | -4.798 | 10.503 | -2.147 | 1.00 | 0.00 |
| ATOM | 953 | CA | ILE | 57 | -4.234 | 11.266 | -0.264 | 1.00 | 0.00 |
| ATOM | 954 | HA | ILE | 57 | -4.777 | 10.569 | 0.362 | 1.00 | 0.00 |
| ATOM | 955 | CB | ILE | 57 | -2.958 | 11.720 | 0.475 | 1.00 | 0.00 |
| ATOM | 956 | HB | ILE | 57 | -3.271 | 12.245 | 1.366 | 1.00 | 0.00 |
| ATOM | 957 | CG1 | ILE | 57 | -2.120 | 12.676 | -0.383 | 1.00 | 0.00 |
| ATOM | 958 | HG11 | ILE | 57 | -2.369 | 13.694 | -0.121 | 1.00 | 0.00 |
| ATOM | 959 | HG12 | ILE | 57 | -2.346 | 12.515 | -1.425 | 1.00 | 0.00 |
| ATOM | 960 | CG2 | ILE | 57 | -2.147 | 10.527 | 0.920 | 1.00 | 0.00 |
| ATOM | 961 | HG21 | ILE | 57 | -1.528 | 10.195 | 0.105 | 1.00 | 0.00 |
| ATOM | 962 | HG22 | ILE | 57 | -2.809 | 9.731 | 1.221 | 1.00 | 0.00 |
| ATOM | 963 | HG23 | ILE | 57 | -1.521 | 10.817 | 1.746 | 1.00 | 0.00 |
| ATOM | 964 | CD1 | ILE | 57 | -0.626 | 12.503 | -0.196 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 965 | HD11 | ILE | 57 | -0.243 | 11.844 | -0.961 | 1.00 | 0.00 |
| ATOM | 966 | HD12 | ILE | 57 | -0.434 | 12.070 | 0.778 | 1.00 | 0.00 |
| ATOM | 967 | HD13 | ILE | 57 | -0.139 | 13.462 | -0.268 | 1.00 | 0.00 |
| ATOM | 968 | C | ILE | 57 | -5.090 | 12.485 | -0.501 | 1.00 | 0.00 |
| ATOM | 969 | O | ILE | 57 | -4.998 | 13.509 | 0.177 | 1.00 | 0.00 |
| ATOM | 970 | N | THR | 58 | -5.916 | 12.320 | -1.512 | 1.00 | 0.00 |
| ATOM | 971 | HN | THR | 58 | -5.899 | 11.467 | -1.983 | 1.00 | 0.00 |
| ATOM | 972 | CA | THR | 58 | -6.837 | 13.304 | -1.981 | 1.00 | 0.00 |
| ATOM | 973 | HA | THR | 58 | -6.756 | 14.171 | -1.343 | 1.00 | 0.00 |
| ATOM | 974 | CB | THR | 58 | -6.476 | 13.697 | -3.412 | 1.00 | 0.00 |
| ATOM | 975 | HB | THR | 58 | -6.886 | 14.674 | -3.623 | 1.00 | 0.00 |
| ATOM | 976 | OG1 | THR | 58 | -7.015 | 12.773 | -4.341 | 1.00 | 0.00 |
| ATOM | 977 | HG1 | THR | 58 | -7.106 | 13.196 | -5.197 | 1.00 | 0.00 |
| ATOM | 978 | CG2 | THR | 58 | -4.976 | 13.762 | -3.638 | 1.00 | 0.00 |
| ATOM | 979 | HG21 | THR | 58 | -4.491 | 12.944 | -3.112 | 1.00 | 0.00 |
| ATOM | 980 | HG22 | THR | 58 | -4.598 | 14.701 | -3.263 | 1.00 | 0.00 |
| ATOM | 981 | HG23 | THR | 58 | -4.767 | 13.685 | -4.693 | 1.00 | 0.00 |
| ATOM | 982 | C | THR | 58 | -8.253 | 12.754 | -1.894 | 1.00 | 0.00 |
| ATOM | 983 | O | THR | 58 | -8.509 | 11.745 | -1.239 | 1.00 | 0.00 |
| ATOM | 984 | N | ASP | 59 | -9.156 | 13.447 | -2.537 | 1.00 | 0.00 |
| ATOM | 985 | HN | ASP | 59 | -8.869 | 14.251 | -3.018 | 1.00 | 0.00 |
| ATOM | 986 | CA | ASP | 59 | -10.566 | 13.093 | -2.556 | 1.00 | 0.00 |
| ATOM | 987 | HA | ASP | 59 | -10.986 | 13.278 | -1.582 | 1.00 | 0.00 |
| ATOM | 988 | CB | ASP | 59 | -11.233 | 14.004 | -3.566 | 1.00 | 0.00 |
| ATOM | 989 | HB1 | ASP | 59 | -11.496 | 13.418 | -4.434 | 1.00 | 0.00 |
| ATOM | 990 | HB2 | ASP | 59 | -10.522 | 14.763 | -3.861 | 1.00 | 0.00 |
| ATOM | 991 | CG | ASP | 59 | -12.480 | 14.677 | -3.027 | 1.00 | 0.00 |
| ATOM | 992 | OD1 | ASP | 59 | -13.373 | 15.009 | -3.835 | 1.00 | 0.00 |
| ATOM | 993 | OD2 | ASP | 59 | -12.564 | 14.871 | -1.796 | 1.00 | 0.00 |
| ATOM | 994 | C | ASP | 59 | -10.844 | 11.641 | -2.979 | 1.00 | 0.00 |
| ATOM | 995 | O | ASP | 59 | -11.927 | 11.120 | -2.713 | 1.00 | 0.00 |
| ATOM | 996 | N | ASN | 60 | -9.912 | 11.010 | -3.686 | 1.00 | 0.00 |
| ATOM | 997 | HN | ASN | 60 | -9.083 | 11.477 | -3.919 | 1.00 | 0.00 |
| ATOM | 998 | CA | ASN | 60 | -10.140 | 9.648 | -4.186 | 1.00 | 0.00 |
| ATOM | 999 | HA | ASN | 60 | -11.153 | 9.612 | -4.555 | 1.00 | 0.00 |
| ATOM | 1000 | CB | ASN | 60 | -9.198 | 9.373 | -5.361 | 1.00 | 0.00 |
| ATOM | 1001 | HB1 | ASN | 60 | -8.496 | 8.601 | -5.082 | 1.00 | 0.00 |
| ATOM | 1002 | HB2 | ASN | 60 | -8.656 | 10.277 | -5.597 | 1.00 | 0.00 |
| ATOM | 1003 | CG | ASN | 60 | -9.940 | 8.923 | -6.604 | 1.00 | 0.00 |
| ATOM | 1004 | OD1 | ASN | 60 | -10.892 | 8.146 | -6.526 | 1.00 | 0.00 |
| ATOM | 1005 | ND2 | ASN | 60 | -9.507 | 9.410 | -7.761 | 1.00 | 0.00 |
| ATOM | 1006 | HD21 | ASN | 60 | -8.743 | 10.024 | -7.748 | 1.00 | 0.00 |
| ATOM | 1007 | HD22 | ASN | 60 | -9.968 | 9.136 | -8.581 | 1.00 | 0.00 |
| ATOM | 1008 | C | ASN | 60 | -9.979 | 8.542 | -3.129 | 1.00 | 0.00 |
| ATOM | 1009 | O | ASN | 60 | -10.864 | 7.699 | -2.983 | 1.00 | 0.00 |
| ATOM | 1010 | N | PRO | 61 | -8.848 | 8.494 | -2.405 | 1.00 | 0.00 |
| ATOM | 1011 | CA | PRO | 61 | -8.588 | 7.447 | -1.403 | 1.00 | 0.00 |
| ATOM | 1012 | HA | PRO | 61 | -8.515 | 6.479 | -1.875 | 1.00 | 0.00 |
| ATOM | 1013 | CB | PRO | 61 | -7.216 | 7.828 | -0.828 | 1.00 | 0.00 |
| ATOM | 1014 | HB1 | PRO | 61 | -6.451 | 7.212 | -1.276 | 1.00 | 0.00 |

(fortgesetzt)

Atomkoordinaten der Struktur der ADAP hSH3-1-Domäne, reduzierte Form

| ATOM | 1015 | HB2 | PRO | 61 | -7.218 | 7.689 | 0.243 | 1.00 | 0.00 |
|------|------|-----|-----|----|--------|-------|-------|------|------|
| ATOM | 1016 | CG | PRO | 61 | -7.042 | 9.258 | -1.194 | 1.00 | 0.00 |
| ATOM | 1017 | HG1 | PRO | 61 | -5.994 | 9.493 | -1.267 | 1.00 | 0.00 |
| ATOM | 1018 | HG2 | PRO | 61 | -7.522 | 9.889 | -0.460 | 1.00 | 0.00 |
| ATOM | 1019 | CD | PRO | 61 | -7.706 | 9.403 | -2.528 | 1.00 | 0.00 |
| ATOM | 1020 | HD1 | PRO | 61 | -7.046 | 9.077 | -3.314 | 1.00 | 0.00 |
| ATOM | 1021 | HD2 | PRO | 61 | -8.024 | 10.417 | -2.692 | 1.00 | 0.00 |
| ATOM | 1022 | C | PRO | 61 | -9.632 | 7.368 | -0.290 | 1.00 | 0.00 |
| ATOM | 1023 | O | PRO | 61 | -10.543 | 6.541 | -0.346 | 1.00 | 0.00 |
| ATOM | 1024 | N | GLU | 62 | -9.478 | 8.204 | 0.736 | 1.00 | 0.00 |
| ATOM | 1025 | HN | GLU | 62 | -8.721 | 8.824 | 0.739 | 1.00 | 0.00 |
| ATOM | 1026 | CA | GLU | 62 | -10.390 | 8.197 | 1.878 | 1.00 | 0.00 |
| ATOM | 1027 | HA | GLU | 62 | -10.242 | 9.119 | 2.421 | 1.00 | 0.00 |
| ATOM | 1028 | CB | GLU | 62 | -11.848 | 8.122 | 1.414 | 1.00 | 0.00 |
| ATOM | 1029 | HB1 | GLU | 62 | -12.170 | 7.091 | 1.439 | 1.00 | 0.00 |
| ATOM | 1030 | HB2 | GLU | 62 | -11.910 | 8.484 | 0.398 | 1.00 | 0.00 |
| ATOM | 1031 | CG | GLU | 62 | -12.802 | 8.940 | 2.270 | 1.00 | 0.00 |
| ATOM | 1032 | HG1 | GLU | 62 | -12.319 | 9.868 | 2.537 | 1.00 | 0.00 |
| ATOM | 1033 | HG2 | GLU | 62 | -13.029 | 8.382 | 3.166 | 1.00 | 0.00 |
| ATOM | 1034 | CD | GLU | 62 | -14.101 | 9.256 | 1.555 | 1.00 | 0.00 |
| ATOM | 1035 | OE1 | GLU | 62 | -15.104 | 9.540 | 2.244 | 1.00 | 0.00 |
| ATOM | 1036 | OE2 | GLU | 62 | -14.116 | 9.221 | 0.307 | 1.00 | 0.00 |
| ATOM | 1037 | C | GLU | 62 | -10.073 | 7.023 | 2.809 | 1.00 | 0.00 |
| ATOM | 1038 | O | GLU | 62 | -10.867 | 6.682 | 3.686 | 1.00 | 0.00 |
| ATOM | 1039 | N | GLY | 63 | -8.902 | 6.412 | 2.612 | 1.00 | 0.00 |
| ATOM | 1040 | HN | GLY | 63 | -8.308 | 6.730 | 1.901 | 1.00 | 0.00 |
| ATOM | 1041 | CA | GLY | 63 | -8.491 | 5.288 | 3.439 | 1.00 | 0.00 |
| ATOM | 1042 | HA1 | GLY | 63 | -8.369 | 4.418 | 2.812 | 1.00 | 0.00 |
| ATOM | 1043 | HA2 | GLY | 63 | -9.261 | 5.090 | 4.169 | 1.00 | 0.00 |
| ATOM | 1044 | C | GLY | 63 | -7.184 | 5.561 | 4.162 | 1.00 | 0.00 |
| ATOM | 1045 | O | GLY | 63 | -7.073 | 6.544 | 4.893 | 1.00 | 0.00 |
| ATOM | 1046 | N | LYS | 64 | -6.181 | 4.708 | 3.945 | 1.00 | 0.00 |
| ATOM | 1047 | HN | LYS | 64 | -6.307 | 3.945 | 3.326 | 1.00 | 0.00 |
| ATOM | 1048 | CA | LYS | 64 | -4.880 | 4.901 | 4.573 | 1.00 | 0.00 |
| ATOM | 1049 | HA | LYS | 64 | -4.991 | 5.649 | 5.344 | 1.00 | 0.00 |
| ATOM | 1050 | CB | LYS | 64 | -4.401 | 3.592 | 5.199 | 1.00 | 0.00 |
| ATOM | 1051 | HB1 | LYS | 64 | -3.359 | 3.687 | 5.463 | 1.00 | 0.00 |
| ATOM | 1052 | HB2 | LYS | 64 | -4.511 | 2.806 | 4.472 | 1.00 | 0.00 |
| ATOM | 1053 | CG | LYS | 64 | -5.174 | 3.195 | 6.446 | 1.00 | 0.00 |
| ATOM | 1054 | HG1 | LYS | 64 | -6.124 | 2.775 | 6.150 | 1.00 | 0.00 |
| ATOM | 1055 | HG2 | LYS | 64 | -5.341 | 4.075 | 7.049 | 1.00 | 0.00 |
| ATOM | 1056 | CD | LYS | 64 | -4.415 | 2.169 | 7.271 | 1.00 | 0.00 |
| ATOM | 1057 | HD1 | LYS | 64 | -3.369 | 2.208 | 7.002 | 1.00 | 0.00 |
| ATOM | 1058 | HD2 | LYS | 64 | -4.807 | 1.186 | 7.055 | 1.00 | 0.00 |
| ATOM | 1059 | CE | LYS | 64 | -4.553 | 2.438 | 8.760 | 1.00 | 0.00 |
| ATOM | 1060 | HE1 | LYS | 64 | -4.430 | 3.497 | 8.935 | 1.00 | 0.00 |
| ATOM | 1061 | HE2 | LYS | 64 | -3.780 | 1.896 | 9.285 | 1.00 | 0.00 |
| ATOM | 1062 | NZ | LYS | 64 | -5.881 | 2.011 | 9.281 | 1.00 | 0.00 |
| ATOM | 1063 | HZ1 | LYS | 64 | -5.884 | 0.986 | 9.454 | 1.00 | 0.00 |
| ATOM | 1064 | HZ2 | LYS | 64 | -6.625 | 2.237 | 8.591 | 1.00 | 0.00 |

(fortgesetzt)

Atomkoordinaten der Struktur der ADAP hSH3-1-Domäne, reduzierte Form

| ATOM | 1065 | HZ3 | LYS | 64 | -6.090 | 2.504 | 10.173 | 1.00 | 0.00 |
|------|------|------|-----|----|--------|--------|--------|------|------|
| ATOM | 1066 | C | LYS | 64 | -3.909 | 5.419 | 3.517 | 1.00 | 0.00 |
| ATOM | 1067 | O | LYS | 64 | -4.193 | 6.430 | 2.890 | 1.00 | 0.00 |
| ATOM | 1068 | N | TRP | 65 | -2.800 | 4.712 | 3.277 | 1.00 | 0.00 |
| ATOM | 1069 | HN | TRP | 65 | -2.634 | 3.889 | 3.775 | 1.00 | 0.00 |
| ATOM | 1070 | CA | TRP | 65 | -1.858 | 5.113 | 2.236 | 1.00 | 0.00 |
| ATOM | 1071 | HA | TRP | 65 | -2.331 | 5.892 | 1.669 | 1.00 | 0.00 |
| ATOM | 1072 | CB | TRP | 65 | -0.539 | 5.654 | 2.796 | 1.00 | 0.00 |
| ATOM | 1073 | HB1 | TRP | 65 | 0.266 | 5.381 | 2.129 | 1.00 | 0.00 |
| ATOM | 1074 | HB2 | TRP | 65 | -0.347 | 5.226 | 3.762 | 1.00 | 0.00 |
| ATOM | 1075 | CG | TRP | 65 | -0.567 | 7.146 | 2.933 | 1.00 | 0.00 |
| ATOM | 1076 | CD1 | TRP | 65 | -1.675 | 7.919 | 3.122 | 1.00 | 0.00 |
| ATOM | 1077 | HD1 | TRP | 65 | -2.669 | 7.539 | 3.200 | 1.00 | 0.00 |
| ATOM | 1078 | CD2 | TRP | 65 | 0.536 | 8.050 | 2.882 | 1.00 | 0.00 |
| ATOM | 1079 | NE1 | TRP | 65 | -1.336 | 9.233 | 3.199 | 1.00 | 0.00 |
| ATOM | 1080 | HE1 | TRP | 65 | -1.966 | 9.969 | 3.333 | 1.00 | 0.00 |
| ATOM | 1081 | CE2 | TRP | 65 | 0.015 | 9.350 | 3.051 | 1.00 | 0.00 |
| ATOM | 1082 | CE3 | TRP | 65 | 1.910 | 7.896 | 2.714 | 1.00 | 0.00 |
| ATOM | 1083 | HE3 | TRP | 65 | 2.352 | 6.919 | 2.583 | 1.00 | 0.00 |
| ATOM | 1084 | CZ2 | TRP | 65 | 0.821 | 10.483 | 3.055 | 1.00 | 0.00 |
| ATOM | 1085 | HZ2 | TRP | 65 | 0.414 | 11.475 | 3.185 | 1.00 | 0.00 |
| ATOM | 1086 | CZ3 | TRP | 65 | 2.707 | 9.021 | 2.718 | 1.00 | 0.00 |
| ATOM | 1087 | HZ3 | TRP | 65 | 3.769 | 8.917 | 2.586 | 1.00 | 0.00 |
| ATOM | 1088 | CH2 | TRP | 65 | 2.162 | 10.300 | 2.887 | 1.00 | 0.00 |
| ATOM | 1089 | HH2 | TRP | 65 | 2.826 | 11.151 | 2.881 | 1.00 | 0.00 |
| ATOM | 1090 | C | TRP | 65 | -1.613 | 3.944 | 1.307 | 1.00 | 0.00 |
| ATOM | 1091 | O | TRP | 65 | -1.628 | 2.792 | 1.734 | 1.00 | 0.00 |
| ATOM | 1092 | N | LEU | 66 | -1.432 | 4.227 | 0.029 | 1.00 | 0.00 |
| ATOM | 1093 | HN | LEU | 66 | -1.460 | 5.159 | -0.274 | 1.00 | 0.00 |
| ATOM | 1094 | CA | LEU | 66 | -1.235 | 3.162 | -0.933 | 1.00 | 0.00 |
| ATOM | 1095 | HA | LEU | 66 | -1.311 | 2.231 | -0.395 | 1.00 | 0.00 |
| ATOM | 1096 | CB | LEU | 66 | -2.334 | 3.195 | -1.989 | 1.00 | 0.00 |
| ATOM | 1097 | HB1 | LEU | 66 | -2.007 | 3.814 | -2.807 | 1.00 | 0.00 |
| ATOM | 1098 | HB2 | LEU | 66 | -3.191 | 3.646 | -1.543 | 1.00 | 0.00 |
| ATOM | 1099 | CG | LEU | 66 | -2.754 | 1.834 | -2.548 | 1.00 | 0.00 |
| ATOM | 1100 | HG | LEU | 66 | -2.003 | 1.486 | -3.241 | 1.00 | 0.00 |
| ATOM | 1101 | CD1 | LEU | 66 | -2.864 | 0.822 | -1.429 | 1.00 | 0.00 |
| ATOM | 1102 | HD11 | LEU | 66 | -3.363 | 1.278 | -0.577 | 1.00 | 0.00 |
| ATOM | 1103 | HD12 | LEU | 66 | -1.878 | 0.504 | -1.145 | 1.00 | 0.00 |
| ATOM | 1104 | HD13 | LEU | 66 | -3.431 | -0.029 | -1.768 | 1.00 | 0.00 |
| ATOM | 1105 | CD2 | LEU | 66 | -4.074 | 1.934 | -3.293 | 1.00 | 0.00 |
| ATOM | 1106 | HD21 | LEU | 66 | -4.878 | 1.611 | -2.650 | 1.00 | 0.00 |
| ATOM | 1107 | HD22 | LEU | 66 | -4.038 | 1.306 | -4.165 | 1.00 | 0.00 |
| ATOM | 1108 | HD23 | LEU | 66 | -4.243 | 2.955 | -3.594 | 1.00 | 0.00 |
| ATOM | 1109 | C | LEU | 66 | 0.123 | 3.209 | -1.594 | 1.00 | 0.00 |
| ATOM | 1110 | O | LEU | 66 | 0.923 | 4.119 | -1.376 | 1.00 | 0.00 |
| ATOM | 1111 | N | GLY | 67 | 0.365 | 2.185 | -2.387 | 1.00 | 0.00 |
| ATOM | 1112 | HN | GLY | 67 | -0.319 | 1.493 | -2.488 | 1.00 | 0.00 |
| ATOM | 1113 | CA | GLY | 67 | 1.611 | 2.040 | -3.082 | 1.00 | 0.00 |
| ATOM | 1114 | HA1 | GLY | 67 | 2.337 | 2.715 | -2.656 | 1.00 | 0.00 |

(fortgesetzt)

Atomkoordinaten der Struktur der ADAP hSH3-1-Domäne, reduzierte Form

| ATOM | 1115 | HA2 | GLY | 67 | 1.464 | 2.284 | -4.119 | 1.00 | 0.00 |
|------|------|------|------|------|--------|--------|--------|------|------|
| ATOM | 1116 | C | GLY | 67 | 2.101 | 0.614 | -2.957 | 1.00 | 0.00 |
| ATOM | 1117 | O | GLY | 67 | 1.463 | -0.198 | -2.287 | 1.00 | 0.00 |
| ATOM | 1118 | N | ARG | 68 | 3.222 | 0.295 | -3.575 | 1.00 | 0.00 |
| ATOM | 1119 | HN | ARG | 68 | 3.707 | 0.980 | -4.084 | 1.00 | 0.00 |
| ATOM | 1120 | CA | ARG | 68 | 3.765 | -1.053 | -3.484 | 1.00 | 0.00 |
| ATOM | 1121 | HA | ARG | 68 | 3.741 | -1.304 | -2.433 | 1.00 | 0.00 |
| ATOM | 1122 | CB | ARG | 68 | 2.944 | -2.124 | -4.185 | 1.00 | 0.00 |
| ATOM | 1123 | HB1 | ARG | 68 | 3.306 | -2.252 | -5.193 | 1.00 | 0.00 |
| ATOM | 1124 | HB2 | ARG | 68 | 1.900 | -1.835 | -4.206 | 1.00 | 0.00 |
| ATOM | 1125 | CG | ARG | 68 | 3.099 | -3.455 | -3.437 | 1.00 | 0.00 |
| ATOM | 1126 | HG1 | ARG | 68 | 2.176 | -3.686 | -2.933 | 1.00 | 0.00 |
| ATOM | 1127 | HG2 | ARG | 68 | 3.882 | -3.345 | -2.691 | 1.00 | 0.00 |
| ATOM | 1128 | CD | ARG | 68 | 3.478 | -4.611 | -4.365 | 1.00 | 0.00 |
| ATOM | 1129 | HD1 | ARG | 68 | 3.473 | -4.253 | -5.384 | 1.00 | 0.00 |
| ATOM | 1130 | HD2 | ARG | 68 | 2.749 | -5.400 | -4.259 | 1.00 | 0.00 |
| ATOM | 1131 | NE | ARG | 68 | 4.803 | -5.155 | -4.059 | 1.00 | 0.00 |
| ATOM | 1132 | HE | ARG | 68 | 5.488 | -5.087 | -4.755 | 1.00 | 0.00 |
| ATOM | 1133 | CZ | ARG | 68 | 5.123 | -5.742 | -2.906 | 1.00 | 0.00 |
| ATOM | 1134 | NH1 | ARG | 68 | 4.237 | -5.821 | -1.923 | 1.00 | 0.00 |
| ATOM | 1135 | HH11 | ARG | 68 | 3.324 | -5.438 | -2.041 | 1.00 | 0.00 |
| ATOM | 1136 | HH12 | ARG | 68 | 4.485 | -6.265 | -1.062 | 1.00 | 0.00 |
| ATOM | 1137 | NH2 | ARG | 68 | 6.339 | -6.242 | -2.732 | 1.00 | 0.00 |
| ATOM | 1138 | HH21 | ARG | 68 | 7.015 | -6.180 | -3.465 | 1.00 | 0.00 |
| ATOM | 1139 | HH22 | ARG | 68 | 6.579 | -6.682 | -1.866 | 1.00 | 0.00 |
| ATOM | 1140 | C | ARG | 68 | 5.210 | -1.116 | -3.976 | 1.00 | 0.00 |
| ATOM | 1141 | O | ARG | 68 | 5.911 | -0.105 | -4.031 | 1.00 | 0.00 |
| ATOM | 1142 | N | THR | 69 | 5.627 | -2.319 | -4.359 | 1.00 | 0.00 |
| ATOM | 1143 | HN | THR | 69 | 4.987 | -3.074 | -4.336 | 1.00 | 0.00 |
| ATOM | 1144 | CA | THR | 69 | 6.958 | -2.576 | -4.886 | 1.00 | 0.00 |
| ATOM | 1145 | HA | THR | 69 | 7.477 | -1.633 | -4.992 | 1.00 | 0.00 |
| ATOM | 1146 | CB | THR | 69 | 7.748 | -3.514 | -3.971 | 1.00 | 0.00 |
| ATOM | 1147 | HB | THR | 69 | 7.584 | -4.533 | -4.291 | 1.00 | 0.00 |
| ATOM | 1148 | OG1 | THR | 69 | 7.311 | -3.393 | -2.629 | 1.00 | 0.00 |
| ATOM | 1149 | HG1 | THR | 69 | 6.381 | -3.623 | -2.572 | 1.00 | 0.00 |
| ATOM | 1150 | CG2 | THR | 69 | 9.239 | -3.256 | -3.993 | 1.00 | 0.00 |
| ATOM | 1151 | HG21 | THR | 69 | 9.423 | -2.233 | -4.285 | 1.00 | 0.00 |
| ATOM | 1152 | HG22 | THR | 69 | 9.709 | -3.923 | -4.700 | 1.00 | 0.00 |
| ATOM | 1153 | HG23 | THR | 69 | 9.649 | -3.429 | -3.009 | 1.00 | 0.00 |
| ATOM | 1154 | C | THR | 69 | 6.751 | -3.197 | -6.242 | 1.00 | 0.00 |
| ATOM | 1155 | O | THR | 69 | 5.628 | -3.605 | -6.530 | 1.00 | 0.00 |
| ATOM | 1156 | N | ALA | 70 | 7.755 | -3.250 | -7.113 | 1.00 | 0.00 |
| ATOM | 1157 | HN | ALA | 70 | 8.639 | -2.890 | -6.894 | 1.00 | 0.00 |
| ATOM | 1158 | CA | ALA | 70 | 7.474 | -3.821 | -8.418 | 1.00 | 0.00 |
| ATOM | 1159 | HA | ALA | 70 | 6.665 | -3.260 | -8.865 | 1.00 | 0.00 |
| ATOM | 1160 | CB | ALA | 70 | 8.694 | -3.712 | -9.321 | 1.00 | 0.00 |
| ATOM | 1161 | HB1 | ALA | 70 | 8.726 | -4.562 | -9.987 | 1.00 | 0.00 |
| ATOM | 1162 | HB2 | ALA | 70 | 9.589 | -3.693 | -8.717 | 1.00 | 0.00 |
| ATOM | 1163 | HB3 | ALA | 70 | 8.633 | -2.803 | -9.901 | 1.00 | 0.00 |
| ATOM | 1164 | C | ALA | 70 | 7.049 | -5.278 | -8.262 | 1.00 | 0.00 |

(fortgesetzt)

Atomkoordinaten der Struktur der ADAP hSH3-1-Domäne, reduzierte Form

| | | | | | | | | | | |
|------|------|------|------|-----|--------|---------|--------|------|------|
| ATOM | 1165 | O | ALA | 70 | 7.771 | -6.211 | -8.612 | 1.00 | 0.00 |
| ATOM | 1166 | N | ARG | 71 | 5.814 | -5.422 | -7.790 | 1.00 | 0.00 |
| ATOM | 1167 | HN | ARG | 71 | 5.304 | -4.612 | -7.592 | 1.00 | 0.00 |
| ATOM | 1168 | CA | ARG | 71 | 5.148 | -6.699 | -7.620 | 1.00 | 0.00 |
| ATOM | 1169 | HA | ARG | 71 | 5.717 | -7.452 | -8.147 | 1.00 | 0.00 |
| ATOM | 1170 | CB | ARG | 71 | 5.033 | -7.077 | -6.143 | 1.00 | 0.00 |
| ATOM | 1171 | HB1 | ARG | 71 | 4.168 | -6.585 | -5.725 | 1.00 | 0.00 |
| ATOM | 1172 | HB2 | ARG | 71 | 5.918 | -6.735 | -5.627 | 1.00 | 0.00 |
| ATOM | 1173 | CG | ARG | 71 | 4.891 | -8.572 | -5.908 | 1.00 | 0.00 |
| ATOM | 1174 | HG1 | ARG | 71 | 5.199 | -9.097 | -6.801 | 1.00 | 0.00 |
| ATOM | 1175 | HG2 | ARG | 71 | 3.857 | -8.796 | -5.695 | 1.00 | 0.00 |
| ATOM | 1176 | CD | ARG | 71 | 5.746 | -9.038 | -4.740 | 1.00 | 0.00 |
| ATOM | 1177 | HD1 | ARG | 71 | 5.129 | -9.084 | -3.855 | 1.00 | 0.00 |
| ATOM | 1178 | HD2 | ARG | 71 | 6.542 | -8.326 | -4.587 | 1.00 | 0.00 |
| ATOM | 1179 | NE | ARG | 71 | 6.330 | -10.356 | -4.981 | 1.00 | 0.00 |
| ATOM | 1180 | HE | ARG | 71 | 5.921 | -10.911 | -5.678 | 1.00 | 0.00 |
| ATOM | 1181 | CZ | ARG | 71 | 7.375 | -10.837 | -4.312 | 1.00 | 0.00 |
| ATOM | 1182 | NH1 | ARG | 71 | 7.954 | -10.114 | -3.362 | 1.00 | 0.00 |
| ATOM | 1183 | HH11 | ARG | 71 | 7.606 | -9.202 | -3.144 | 1.00 | 0.00 |
| ATOM | 1184 | HH12 | ARG | 71 | 8.739 | -10.481 | -2.863 | 1.00 | 0.00 |
| ATOM | 1185 | NH2 | ARG | 71 | 7.843 | -12.045 | -4.594 | 1.00 | 0.00 |
| ATOM | 1186 | HH21 | ARG | 71 | 7.411 | -12.595 | -5.309 | 1.00 | 0.00 |
| ATOM | 1187 | HH22 | ARG | 71 | 8.629 | -12.407 | -4.092 | 1.00 | 0.00 |
| ATOM | 1188 | C | ARG | 71 | 3.774 | -6.582 | -8.249 | 1.00 | 0.00 |
| ATOM | 1189 | O | ARG | 71 | 3.335 | -7.442 | -9.013 | 1.00 | 0.00 |
| ATOM | 1190 | N | GLY | 72 | 3.112 | -5.459 | -7.922 | 1.00 | 0.00 |
| ATOM | 1191 | HN | GLY | 72 | 3.534 | -4.811 | -7.322 | 1.00 | 0.00 |
| ATOM | 1192 | CA | GLY | 72 | 1.803 | -5.181 | -8.463 | 1.00 | 0.00 |
| ATOM | 1193 | HA1 | GLY | 72 | 1.596 | -5.926 | -9.186 | 1.00 | 0.00 |
| ATOM | 1194 | HA2 | GLY | 72 | 1.831 | -4.222 | -8.956 | 1.00 | 0.00 |
| ATOM | 1195 | C | GLY | 72 | 0.673 | -5.165 | -7.442 | 1.00 | 0.00 |
| ATOM | 1196 | O | GLY | 72 | -0.456 | -4.811 | -7.781 | 1.00 | 0.00 |
| ATOM | 1197 | N | SER | 73 | 0.956 | -5.545 | -6.203 | 1.00 | 0.00 |
| ATOM | 1198 | HN | SER | 73 | 1.870 | -5.820 | -5.979 | 1.00 | 0.00 |
| ATOM | 1199 | CA | SER | 73 | -0.074 | -5.563 | -5.164 | 1.00 | 0.00 |
| ATOM | 1200 | HA | SER | 73 | -1.090 | -5.459 | -5.635 | 1.00 | 0.00 |
| ATOM | 1201 | CB | SER | 73 | -0.010 | -6.892 | -4.421 | 1.00 | 0.00 |
| ATOM | 1202 | HB1 | SER | 73 | 0.167 | -6.706 | -3.376 | 1.00 | 0.00 |
| ATOM | 1203 | HB2 | SER | 73 | 0.805 | -7.479 | -4.820 | 1.00 | 0.00 |
| ATOM | 1204 | OG | SER | 73 | -1.216 | -7.622 | -4.562 | 1.00 | 0.00 |
| ATOM | 1205 | HG | SER | 73 | -1.023 | -8.497 | -4.908 | 1.00 | 0.00 |
| ATOM | 1206 | C | SER | 73 | 0.141 | -4.414 | -4.187 | 1.00 | 0.00 |
| ATOM | 1207 | O | SER | 73 | 1.180 | -4.319 | -3.585 | 1.00 | 0.00 |
| ATOM | 1208 | N | TYR | 74 | -0.835 | -3.537 | -4.035 | 1.00 | 0.00 |
| ATOM | 1209 | HN | TYR | 74 | -1.651 | -3.638 | -4.543 | 1.00 | 0.00 |
| ATOM | 1210 | CA | TYR | 74 | -0.677 | -2.391 | -3.147 | 1.00 | 0.00 |
| ATOM | 1211 | HA | TYR | 74 | 0.378 | -2.251 | -2.976 | 1.00 | 0.00 |
| ATOM | 1212 | CB | TYR | 74 | -1.228 | -1.133 | -3.801 | 1.00 | 0.00 |
| ATOM | 1213 | HB1 | TYR | 74 | -1.194 | -0.314 | -3.099 | 1.00 | 0.00 |
| ATOM | 1214 | HB2 | TYR | 74 | -2.243 | -1.308 | -4.079 | 1.00 | 0.00 |

(fortgesetzt)

Atomkoordinaten der Struktur der ADAP hSH3-1-Domäne, reduzierte Form

| ATOM | 1215 | CG | TYR | 74 | -0.483 | -0.733 | -5.034 | 1.00 | 0.00 |
|------|------|------|-----|----|--------|--------|--------|------|------|
| ATOM | 1216 | CD1 | TYR | 74 | -0.033 | 0.562 | -5.215 | 1.00 | 0.00 |
| ATOM | 1217 | HD1 | TYR | 74 | -0.232 | 1.297 | -4.453 | 1.00 | 0.00 |
| ATOM | 1218 | CD2 | TYR | 74 | -0.225 | -1.663 | -6.017 | 1.00 | 0.00 |
| ATOM | 1219 | HD2 | TYR | 74 | -0.576 | -2.673 | -5.890 | 1.00 | 0.00 |
| ATOM | 1220 | CE1 | TYR | 74 | 0.660 | 0.918 | -6.351 | 1.00 | 0.00 |
| ATOM | 1221 | HE1 | TYR | 74 | 1.004 | 1.931 | -6.474 | 1.00 | 0.00 |
| ATOM | 1222 | CE2 | TYR | 74 | 0.458 | -1.327 | -7.147 | 1.00 | 0.00 |
| ATOM | 1223 | HE2 | TYR | 74 | 0.640 | -2.079 | -7.885 | 1.00 | 0.00 |
| ATOM | 1224 | CZ | TYR | 74 | 0.906 | -0.033 | -7.320 | 1.00 | 0.00 |
| ATOM | 1225 | OH | TYR | 74 | 1.597 | 0.309 | -8.460 | 1.00 | 0.00 |
| ATOM | 1226 | HH | TYR | 74 | 2.505 | 0.521 | -8.232 | 1.00 | 0.00 |
| ATOM | 1227 | C | TYR | 74 | -1.364 | -2.554 | -1.818 | 1.00 | 0.00 |
| ATOM | 1228 | O | TYR | 74 | -2.511 | -2.988 | -1.728 | 1.00 | 0.00 |
| ATOM | 1229 | N | GLY | 75 | -0.657 | -2.126 | -0.793 | 1.00 | 0.00 |
| ATOM | 1230 | HN | GLY | 75 | 0.232 | -1.745 | -0.949 | 1.00 | 0.00 |
| ATOM | 1231 | CA | GLY | 75 | -1.199 | -2.140 | 0.529 | 1.00 | 0.00 |
| ATOM | 1232 | HA1 | GLY | 75 | -0.442 | -2.417 | 1.229 | 1.00 | 0.00 |
| ATOM | 1233 | HA2 | GLY | 75 | -2.004 | -2.852 | 0.584 | 1.00 | 0.00 |
| ATOM | 1234 | C | GLY | 75 | -1.716 | -0.781 | 0.873 | 1.00 | 0.00 |
| ATOM | 1235 | O | GLY | 75 | -1.092 | 0.230 | 0.556 | 1.00 | 0.00 |
| ATOM | 1236 | N | TYR | 76 | -2.843 | -0.749 | 1.524 | 1.00 | 0.00 |
| ATOM | 1237 | HN | TYR | 76 | -3.290 | -1.597 | 1.750 | 1.00 | 0.00 |
| ATOM | 1238 | CA | TYR | 76 | -3.459 | 0.499 | 1.913 | 1.00 | 0.00 |
| ATOM | 1239 | HA | TYR | 76 | -3.142 | 1.247 | 1.201 | 1.00 | 0.00 |
| ATOM | 1240 | CB | TYR | 76 | -4.971 | 0.370 | 1.849 | 1.00 | 0.00 |
| ATOM | 1241 | HB1 | TYR | 76 | -5.420 | 1.345 | 1.748 | 1.00 | 0.00 |
| ATOM | 1242 | HB2 | TYR | 76 | -5.333 | -0.118 | 2.745 | 1.00 | 0.00 |
| ATOM | 1243 | CG | TYR | 76 | -5.367 | -0.467 | 0.657 | 1.00 | 0.00 |
| ATOM | 1244 | CD1 | TYR | 76 | -5.476 | 0.067 | -0.638 | 1.00 | 0.00 |
| ATOM | 1245 | HD1 | TYR | 76 | -5.304 | 1.128 | -0.821 | 1.00 | 0.00 |
| ATOM | 1246 | CD2 | TYR | 76 | -5.581 | -1.822 | 0.824 | 1.00 | 0.00 |
| ATOM | 1247 | HD2 | TYR | 76 | -5.499 | -2.246 | 1.813 | 1.00 | 0.00 |
| ATOM | 1248 | CE1 | TYR | 76 | -5.790 | -0.737 | -1.701 | 1.00 | 0.00 |
| ATOM | 1249 | HE1 | TYR | 76 | -5.855 | -0.302 | -2.684 | 1.00 | 0.00 |
| ATOM | 1250 | CE2 | TYR | 76 | -5.897 | -2.630 | -0.238 | 1.00 | 0.00 |
| ATOM | 1251 | HE2 | TYR | 76 | -6.044 | -3.683 | -0.080 | 1.00 | 0.00 |
| ATOM | 1252 | CZ | TYR | 76 | -6.001 | -2.091 | -1.499 | 1.00 | 0.00 |
| ATOM | 1253 | OH | TYR | 76 | -6.323 | -2.908 | -2.555 | 1.00 | 0.00 |
| ATOM | 1254 | HH | TYR | 76 | -7.098 | -3.429 | -2.333 | 1.00 | 0.00 |
| ATOM | 1255 | C | TYR | 76 | -2.907 | 0.883 | 3.263 | 1.00 | 0.00 |
| ATOM | 1256 | O | TYR | 76 | -3.616 | 1.180 | 4.220 | 1.00 | 0.00 |
| ATOM | 1257 | N | ILE | 77 | -1.580 | 0.827 | 3.279 | 1.00 | 0.00 |
| ATOM | 1258 | HN | ILE | 77 | -1.130 | 0.568 | 2.457 | 1.00 | 0.00 |
| ATOM | 1259 | CA | ILE | 77 | -0.757 | 1.122 | 4.428 | 1.00 | 0.00 |
| ATOM | 1260 | HA | ILE | 77 | -1.198 | 0.677 | 5.307 | 1.00 | 0.00 |
| ATOM | 1261 | CB | ILE | 77 | 0.678 | 0.540 | 4.214 | 1.00 | 0.00 |
| ATOM | 1262 | HB | ILE | 77 | 1.020 | 0.131 | 5.149 | 1.00 | 0.00 |
| ATOM | 1263 | CG1 | ILE | 77 | 1.669 | 1.605 | 3.745 | 1.00 | 0.00 |
| ATOM | 1264 | HG11 | ILE | 77 | 2.675 | 1.238 | 3.885 | 1.00 | 0.00 |

(fortgesetzt)

Atomkoordinaten der Struktur der ADAP hSH3-1-Domäne, reduzierte Form

| ATOM | 1265 | HG12 | ILE | 77 | 1.531 | 2.483 | 4.336 | 1.00 | 0.00 |
|------|------|------|-----|----|-------|-------|-------|------|------|
| ATOM | 1266 | CG2 | ILE | 77 | 0.663 | -0.569 | 3.182 | 1.00 | 0.00 |
| ATOM | 1267 | HG21 | ILE | 77 | 0.038 | -1.376 | 3.524 | 1.00 | 0.00 |
| ATOM | 1268 | HG22 | ILE | 77 | 1.670 | -0.920 | 3.025 | 1.00 | 0.00 |
| ATOM | 1269 | HG23 | ILE | 77 | 0.279 | -0.176 | 2.252 | 1.00 | 0.00 |
| ATOM | 1270 | CD1 | ILE | 77 | 1.515 | 1.996 | 2.280 | 1.00 | 0.00 |
| ATOM | 1271 | HD11 | ILE | 77 | 2.379 | 1.664 | 1.723 | 1.00 | 0.00 |
| ATOM | 1272 | HD12 | ILE | 77 | 1.426 | 3.068 | 2.198 | 1.00 | 0.00 |
| ATOM | 1273 | HD13 | ILE | 77 | 0.628 | 1.532 | 1.873 | 1.00 | 0.00 |
| ATOM | 1274 | C | ILE | 77 | -0.679 | 2.626 | 4.584 | 1.00 | 0.00 |
| ATOM | 1275 | O | ILE | 77 | -0.341 | 3.327 | 3.642 | 1.00 | 0.00 |
| ATOM | 1276 | N | LYS | 78 | -1.033 | 3.121 | 5.752 | 1.00 | 0.00 |
| ATOM | 1277 | HN | LYS | 78 | -1.328 | 2.515 | 6.464 | 1.00 | 0.00 |
| ATOM | 1278 | CA | LYS | 78 | -1.040 | 4.554 | 5.986 | 1.00 | 0.00 |
| ATOM | 1279 | HA | LYS | 78 | -1.399 | 5.030 | 5.083 | 1.00 | 0.00 |
| ATOM | 1280 | CB | LYS | 78 | -2.002 | 4.893 | 7.128 | 1.00 | 0.00 |
| ATOM | 1281 | HB1 | LYS | 78 | -1.427 | 5.087 | 8.022 | 1.00 | 0.00 |
| ATOM | 1282 | HB2 | LYS | 78 | -2.645 | 4.043 | 7.303 | 1.00 | 0.00 |
| ATOM | 1283 | CG | LYS | 78 | -2.879 | 6.104 | 6.850 | 1.00 | 0.00 |
| ATOM | 1284 | HG1 | LYS | 78 | -3.914 | 5.815 | 6.953 | 1.00 | 0.00 |
| ATOM | 1285 | HG2 | LYS | 78 | -2.697 | 6.445 | 5.842 | 1.00 | 0.00 |
| ATOM | 1286 | CD | LYS | 78 | -2.586 | 7.242 | 7.815 | 1.00 | 0.00 |
| ATOM | 1287 | HD1 | LYS | 78 | -1.547 | 7.196 | 8.106 | 1.00 | 0.00 |
| ATOM | 1288 | HD2 | LYS | 78 | -3.211 | 7.130 | 8.689 | 1.00 | 0.00 |
| ATOM | 1289 | CE | LYS | 78 | -2.863 | 8.595 | 7.182 | 1.00 | 0.00 |
| ATOM | 1290 | HE1 | LYS | 78 | -2.716 | 8.515 | 6.115 | 1.00 | 0.00 |
| ATOM | 1291 | HE2 | LYS | 78 | -2.170 | 9.317 | 7.587 | 1.00 | 0.00 |
| ATOM | 1292 | NZ | LYS | 78 | -4.254 | 9.058 | 7.446 | 1.00 | 0.00 |
| ATOM | 1293 | HZ1 | LYS | 78 | -4.929 | 8.293 | 7.244 | 1.00 | 0.00 |
| ATOM | 1294 | HZ2 | LYS | 78 | -4.480 | 9.873 | 6.842 | 1.00 | 0.00 |
| ATOM | 1295 | HZ3 | LYS | 78 | -4.353 | 9.341 | 8.442 | 1.00 | 0.00 |
| ATOM | 1296 | C | LYS | 78 | 0.340 | 5.119 | 6.297 | 1.00 | 0.00 |
| ATOM | 1297 | O | LYS | 78 | 1.189 | 4.460 | 6.879 | 1.00 | 0.00 |
| ATOM | 1298 | N | THR | 79 | 0.501 | 6.375 | 5.911 | 1.00 | 0.00 |
| ATOM | 1299 | HN | THR | 79 | -0.233 | 6.796 | 5.471 | 1.00 | 0.00 |
| ATOM | 1300 | CA | THR | 79 | 1.702 | 7.172 | 6.117 | 1.00 | 0.00 |
| ATOM | 1301 | HA | THR | 79 | 2.469 | 6.833 | 5.440 | 1.00 | 0.00 |
| ATOM | 1302 | CB | THR | 79 | 1.343 | 8.630 | 5.793 | 1.00 | 0.00 |
| ATOM | 1303 | HB | THR | 79 | 1.388 | 8.767 | 4.726 | 1.00 | 0.00 |
| ATOM | 1304 | 0G1 | THR | 79 | 2.245 | 9.531 | 6.406 | 1.00 | 0.00 |
| ATOM | 1305 | HG1 | THR | 79 | 2.100 | 9.534 | 7.355 | 1.00 | 0.00 |
| ATOM | 1306 | CG2 | THR | 79 | -0.060 | 9.017 | 6.225 | 1.00 | 0.00 |
| ATOM | 1307 | HG21 | THR | 79 | -0.327 | 8.470 | 7.116 | 1.00 | 0.00 |
| ATOM | 1308 | HG22 | THR | 79 | -0.760 | 8.775 | 5.430 | 1.00 | 0.00 |
| ATOM | 1309 | HG23 | THR | 79 | -0.097 | 10.077 | 6.425 | 1.00 | 0.00 |
| ATOM | 1310 | C | THR | 79 | 2.194 | 7.049 | 7.560 | 1.00 | 0.00 |
| ATOM | 1311 | O | THR | 79 | 3.381 | 7.163 | 7.846 | 1.00 | 0.00 |
| ATOM | 1312 | N | THR | 80 | 1.260 | 6.787 | 8.451 | 1.00 | 0.00 |
| ATOM | 1313 | HN | THR | 80 | 0.355 | 6.684 | 8.137 | 1.00 | 0.00 |
| ATOM | 1314 | CA | THR | 80 | 1.555 | 6.605 | 9.872 | 1.00 | 0.00 |

(fortgesetzt)

Atomkoordinaten der Struktur der ADAP hSH3-1-Domäne, reduzierte Form

| ATOM | 1315 | HA | THR | 80 | 2.268 | 7.360 | 10.168 | 1.00 | 0.00 |
|------|------|------|------|------|------|------|------|------|------|
| ATOM | 1316 | CB | THR | 80 | 0.282 | 6.751 | 10.708 | 1.00 | 0.00 |
| ATOM | 1317 | HB | THR | 80 | 0.527 | 6.595 | 11.749 | 1.00 | 0.00 |
| ATOM | 1318 | 0G1 | THR | 80 | -0.681 | 5.784 | 10.328 | 1.00 | 0.00 |
| ATOM | 1319 | HG1 | THR | 80 | -0.385 | 4.912 | 10.601 | 1.00 | 0.00 |
| ATOM | 1320 | CG2 | THR | 80 | -0.363 | 8.114 | 10.583 | 1.00 | 0.00 |
| ATOM | 1321 | HG21 | THR | 80 | -1.314 | 8.111 | 11.094 | 1.00 | 0.00 |
| ATOM | 1322 | HG22 | THR | 80 | -0.516 | 8.346 | 9.539 | 1.00 | 0.00 |
| ATOM | 1323 | HG23 | THR | 80 | 0.281 | 8.859 | 11.027 | 1.00 | 0.00 |
| ATOM | 1324 | C | THR | 80 | 2.171 | 5.227 | 10.086 | 1.00 | 0.00 |
| ATOM | 1325 | O | THR | 80 | 2.861 | 4.975 | 11.074 | 1.00 | 0.00 |
| ATOM | 1326 | N | ALA | 81 | 1.947 | 4.363 | 9.105 | 1.00 | 0.00 |
| ATOM | 1327 | HN | ALA | 81 | 1.433 | 4.660 | 8.334 | 1.00 | 0.00 |
| ATOM | 1328 | CA | ALA | 81 | 2.491 | 3.022 | 9.085 | 1.00 | 0.00 |
| ATOM | 1329 | HA | ALA | 81 | 2.822 | 2.764 | 10.081 | 1.00 | 0.00 |
| ATOM | 1330 | CB | ALA | 81 | 1.451 | 2.005 | 8.618 | 1.00 | 0.00 |
| ATOM | 1331 | HB1 | ALA | 81 | 0.627 | 2.522 | 8.150 | 1.00 | 0.00 |
| ATOM | 1332 | HB2 | ALA | 81 | 1.089 | 1.445 | 9.467 | i.oo | 0.00 |
| ATOM | 1333 | HB3 | ALA | 81 | 1.903 | 1.325 | 7.902 | 1.00 | 0.00 |
| ATOM | 1334 | C | ALA | 81 | 3.669 | 3.034 | 8.141 | 1.00 | 0.00 |
| ATOM | 1335 | O | ALA | 81 | 4.628 | 2.280 | 8.301 | 1.00 | 0.00 |
| ATOM | 1336 | N | VAL | 82 | 3.590 | 3.931 | 7.153 | 1.00 | 0.00 |
| ATOM | 1337 | HN | VAL | 82 | 2.807 | 4.513 | 7.090 | 1.00 | 0.00 |
| ATOM | 1338 | CA | VAL | 82 | 4.649 | 4.073 | 6.193 | 1.00 | 0.00 |
| ATOM | 1339 | HA | VAL | 82 | 5.221 | 3.200 | 6.266 | 1.00 | 0.00 |
| ATOM | 1340 | CB | VAL | 82 | 4.171 | 4.203 | 4.739 | 1.00 | 0.00 |
| ATOM | 1341 | HB | VAL | 82 | 4.519 | 5.144 | 4.352 | 1.00 | 0.00 |
| ATOM | 1342 | CG1 | VAL | 82 | 4.751 | 3.087 | 3.895 | 1.00 | 0.00 |
| ATOM | 1343 | HG11 | VAL | 82 | 4.581 | 2.140 | 4.387 | 1.00 | 0.00 |
| ATOM | 1344 | HG12 | VAL | 82 | 5.807 | 3.244 | 3.770 | 1.00 | 0.00 |
| ATOM | 1345 | HG13 | VAL | 82 | 4.269 | 3.082 | 2.930 | 1.00 | 0.00 |
| ATOM | 1346 | CG2 | VAL | 82 | 2.678 | 4.179 | 4.643 | 1.00 | 0.00 |
| ATOM | 1347 | HG21 | VAL | 82 | 2.284 | 5.144 | 4.895 | 1.00 | 0.00 |
| ATOM | 1348 | HG22 | VAL | 82 | 2.298 | 3.435 | 5.323 | 1.00 | 0.00 |
| ATOM | 1349 | HG23 | VAL | 82 | 2.401 | 3.924 | 3.633 | 1.00 | 0.00 |
| ATOM | 1350 | C | VAL | 82 | 5.519 | 5.262 | 6.526 | 1.00 | 0.00 |
| ATOM | 1351 | O | VAL | 82 | 5.053 | 6.397 | 6.525 | 1.00 | 0.00 |
| ATOM | 1352 | N | GLU | 83 | 6.780 | 5.000 | 6.814 | 1.00 | 0.00 |
| ATOM | 1353 | HN | GLU | 83 | 7.095 | 4.073 | 6.803 | 1.00 | 0.00 |
| ATOM | 1354 | CA | GLU | 83 | 7.701 | 6.061 | 7.154 | 1.00 | 0.00 |
| ATOM | 1355 | HA | GLU | 83 | 7.259 | 6.614 | 7.970 | 1.00 | 0.00 |
| ATOM | 1356 | CB | GLU | 83 | 9.040 | 5.481 | 7.614 | 1.00 | 0.00 |
| ATOM | 1357 | HB1 | GLU | 83 | 9.643 | 6.278 | 8.023 | 1.00 | 0.00 |
| ATOM | 1358 | HB2 | GLU | 83 | 9.549 | 5.059 | 6.759 | 1.00 | 0.00 |
| ATOM | 1359 | CG | GLU | 83 | 8.903 | 4.396 | 8.669 | 1.00 | 0.00 |
| ATOM | 1360 | HG1 | GLU | 83 | 8.794 | 3.442 | 8.174 | 1.00 | 0.00 |
| ATOM | 1361 | HG2 | GLU | 83 | 8.022 | 4.596 | 9.261 | 1.00 | 0.00 |
| ATOM | 1362 | CD | GLU | 83 | 10.102 | 4.328 | 9.595 | 1.00 | 0.00 |
| ATOM | 1363 | OE1 | GLU | 83 | 10.358 | 3.242 | 10.156 | 1.00 | 0.00 |
| ATOM | 1364 | OE2 | GLU | 83 | 10.785 | 5.361 | 9.758 | 1.00 | 0.00 |

(fortgesetzt)

Atomkoordinaten der Struktur der ADAP hSH3-1-Domäne, reduzierte Form

| | | | | | | | | | | |
|---|---|---|---|---|---|---|---|---|---|---|
| ATOM | 1365 | C | GLU | 83 | 7.912 | 7.005 | 5.971 | 1.00 | 0.00 |
| ATOM | 1366 | O | GLU | 83 | 9.010 | 7.092 | 5.421 | 1.00 | 0.00 |
| ATOM | 1367 | N | ILE | 84 | 6.854 | 7.729 | 5.601 | 1.00 | 0.00 |
| ATOM | 1368 | HN | ILE | 84 | 6.012 | 7.627 | 6.091 | 1.00 | 0.00 |
| ATOM | 1369 | CA | ILE | 84 | 6.922 | 8.686 | 4.507 | 1.00 | 0.00 |
| ATOM | 1370 | HA | ILE | 84 | 7.189 | 8.148 | 3.609 | 1.00 | 0.00 |
| ATOM | 1371 | CB | ILE | 84 | 5.553 | 9.381 | 4.275 | 1.00 | 0.00 |
| ATOM | 1372 | HB | ILE | 84 | 4.801 | 8.609 | 4.138 | 1.00 | 0.00 |
| ATOM | 1373 | CG1 | ILE | 84 | 5.614 | 10.262 | 3.015 | 1.00 | 0.00 |
| ATOM | 1374 | HG11 | ILE | 84 | 4.612 | 10.522 | 2.717 | 1.00 | 0.00 |
| ATOM | 1375 | HG12 | ILE | 84 | 6.085 | 9.706 | 2.219 | 1.00 | 0.00 |
| ATOM | 1376 | CG2 | ILE | 84 | 5.134 | 10.199 | 5.488 | 1.00 | 0.00 |
| ATOM | 1377 | HG21 | ILE | 84 | 4.961 | 11.223 | 5.193 | 1.00 | 0.00 |
| ATOM | 1378 | HG22 | ILE | 84 | 5.911 | 10.166 | 6.233 | 1.00 | 0.00 |
| ATOM | 1379 | HG23 | ILE | 84 | 4.225 | 9.786 | 5.897 | 1.00 | 0.00 |
| ATOM | 1380 | CD1 | ILE | 84 | 6.388 | 11.553 | 3.193 | 1.00 | 0.00 |
| ATOM | 1381 | HD11 | ILE | 84 | 6.270 | 11.910 | 4.204 | 1.00 | 0.00 |
| ATOM | 1382 | HD12 | ILE | 84 | 6.012 | 12.295 | 2.508 | 1.00 | 0.00 |
| ATOM | 1383 | HD13 | ILE | 84 | 7.435 | 11.376 | 2.994 | 1.00 | 0.00 |
| ATOM | 1384 | C | ILE | 84 | 7.995 | 9.735 | 4.793 | 1.00 | 0.00 |
| ATOM | 1385 | O | ILE | 84 | 7.717 | 10.787 | 5.368 | 1.00 | 0.00 |
| ATOM | 1386 | N | ASP | 85 | 9.234 | 9.434 | 4.414 | 1.00 | 0.00 |
| ATOM | 1387 | HN | ASP | 85 | 9.407 | 8.574 | 3.978 | 1.00 | 0.00 |
| ATOM | 1388 | CA | ASP | 85 | 10.346 | 10.350 | 4.657 | 1.00 | 0.00 |
| ATOM | 1389 | HA | ASP | 85 | 11.265 | 9.807 | 4.496 | 1.00 | 0.00 |
| ATOM | 1390 | CB | ASP | 85 | 10.284 | 11.532 | 3.688 | 1.00 | 0.00 |
| ATOM | 1391 | HB1 | ASP | 85 | 11.184 | 12.120 | 3.789 | 1.00 | 0.00 |
| ATOM | 1392 | HB2 | ASP | 85 | 9.429 | 12.145 | 3.932 | 1.00 | 0.00 |
| ATOM | 1393 | CG | ASP | 85 | 10.159 | 11.089 | 2.244 | 1.00 | 0.00 |
| ATOM | 1394 | OD1 | ASP | 85 | 11.035 | 10.332 | 1.777 | 1.00 | 0.00 |
| ATOM | 1395 | OD2 | ASP | 85 | 9.184 | 11.499 | 1.579 | 1.00 | 0.00 |
| ATOM | 1396 | C | ASP | 85 | 10.315 | 10.854 | 6.100 | 1.00 | 0.00 |
| ATOM | 1397 | O | ASP | 85 | 10.779 | 11.955 | 6.397 | 1.00 | 0.00 |
| ATOM | 1398 | N | TYR | 86 | 9.759 | 10.035 | 6.988 | 1.00 | 0.00 |
| ATOM | 1399 | HN | TYR | 86 | 9.400 | 9.174 | 6.685 | 1.00 | 0.00 |
| ATOM | 1400 | CA | TYR | 86 | 9.643 | 10.382 | 8.400 | 1.00 | 0.00 |
| ATOM | 1401 | HA | TYR | 86 | 8.994 | 11.241 | 8.481 | 1.00 | 0.00 |
| ATOM | 1402 | CB | TYR | 86 | 9.028 | 9.216 | 9.177 | 1.00 | 0.00 |
| ATOM | 1403 | HB1 | TYR | 86 | 9.425 | 9.216 | 10.181 | 1.00 | 0.00 |
| ATOM | 1404 | HB2 | TYR | 86 | 9.296 | 8.290 | 8.691 | 1.00 | 0.00 |
| ATOM | 1405 | CG | TYR | 86 | 7.516 | 9.265 | 9.275 | 1.00 | 0.00 |
| ATOM | 1406 | CD1 | TYR | 86 | 6.893 | 9.600 | 10.471 | 1.00 | 0.00 |
| ATOM | 1407 | HD1 | TYR | 86 | 7.502 | 9.835 | 11.331 | 1.00 | 0.00 |
| ATOM | 1408 | CD2 | TYR | 86 | 6.709 | 8.965 | 8.179 | 1.00 | 0.00 |
| ATOM | 1409 | HD2 | TYR | 86 | 7.168 | 8.704 | 7.237 | 1.00 | 0.00 |
| ATOM | 1410 | CE1 | TYR | 86 | 5.515 | 9.636 | 10.574 | 1.00 | 0.00 |
| ATOM | 1411 | HE1 | TYR | 86 | 5.051 | 9.899 | 11.513 | 1.00 | 0.00 |
| ATOM | 1412 | CE2 | TYR | 86 | 5.334 | 9.000 | 8.276 | 1.00 | 0.00 |
| ATOM | 1413 | HE2 | TYR | 86 | 4.729 | 8.760 | 7.413 | 1.00 | 0.00 |
| ATOM | 1414 | CZ | TYR | 86 | 4.740 | 9.334 | 9.474 | 1.00 | 0.00 |

(fortgesetzt)

Atomkoordinaten der Struktur der ADAP hSH3-1-Domäne, reduzierte Form

| | | | | | | | | | |
|---|---|---|---|---|---|---|---|---|---|
| ATOM | 1415 | OH | TYR | 86 | 3.368 | 9.369 | 9.573 | 1.00 | 0.00 |
| ATOM | 1416 | HH | TYR | 86 | 3.079 | 10.276 | 9.698 | 1.00 | 0.00 |
| ATOM | 1417 | C | TYR | 86 | 11.007 | 10.737 | 8.986 | 1.00 | 0.00 |
| ATOM | 1418 | O | TYR | 86 | 12.009 | 10.776 | 8.272 | 1.00 | 0.00 |
| ATOM | 1419 | N | ASP | 87 | 11.036 | 10.995 | 10.289 | 1.00 | 0.00 |
| ATOM | 1420 | HN | ASP | 87 | 10.204 | 10.948 | 10.805 | 1.00 | 0.00 |
| ATOM | 1421 | CA | ASP | 87 | 12.276 | 11.346 | 10.971 | 1.00 | 0.00 |
| ATOM | 1422 | HA | ASP | 87 | 12.924 | 11.827 | 10.254 | 1.00 | 0.00 |
| ATOM | 1423 | CB | ASP | 87 | 11.994 | 12.318 | 12.118 | 1.00 | 0.00 |
| ATOM | 1424 | HB1 | ASP | 87 | 12.931 | 12.629 | 12.556 | 1.00 | 0.00 |
| ATOM | 1425 | HB2 | ASP | 87 | 11.399 | 11.819 | 12.868 | 1.00 | 0.00 |
| ATOM | 1426 | CG | ASP | 87 | 11.246 | 13.554 | 11.657 | 1.00 | 0.00 |
| ATOM | 1427 | OD1 | ASP | 87 | 11.844 | 14.373 | 10.928 | 1.00 | 0.00 |
| ATOM | 1428 | OD2 | ASP | 87 | 10.062 | 13.703 | 12.026 | 1.00 | 0.00 |
| ATOM | 1429 | C | ASP | 87 | 12.974 | 10.100 | 11.506 | 1.00 | 0.00 |
| ATOM | 1430 | O | ASP | 87 | 12.434 | 9.389 | 12.354 | 1.00 | 0.00 |
| ATOM | 1431 | N | SER | 88 | 14.177 | 9.840 | 11.004 | 1.00 | 0.00 |
| ATOM | 1432 | HN | SER | 88 | 14.554 | 10.443 | 10.330 | 1.00 | 0.00 |
| ATOM | 1433 | CA | SER | 88 | 14.949 | 8.679 | 11.431 | 1.00 | 0.00 |
| ATOM | 1434 | HA | SER | 88 | 14.309 | 7.812 | 11.360 | 1.00 | 0.00 |
| ATOM | 1435 | CB | SER | 88 | 16.159 | 8.480 | 10.517 | 1.00 | 0.00 |
| ATOM | 1436 | HB1 | SER | 88 | 15.831 | 8.463 | 9.488 | 1.00 | 0.00 |
| ATOM | 1437 | HB2 | SER | 88 | 16.639 | 7.543 | 10.757 | 1.00 | 0.00 |
| ATOM | 1438 | OG | SER | 88 | 17.099 | 9.528 | 10.679 | 1.00 | 0.00 |
| ATOM | 1439 | HG | SER | 88 | 17.165 | 10.029 | 9.863 | 1.00 | 0.00 |
| ATOM | 1440 | C | SER | 88 | 15.408 | 8.833 | 12.878 | 1.00 | 0.00 |
| ATOM | 1441 | O | SER | 88 | 16.579 | 9.105 | 13.193 | 1.00 | 0.00 |
| ATOM | 1442 | N | LEU | 89 | 14.477 | 8.658 | 13.810 | 1.00 | 0.00 |
| ATOM | 1443 | HN | LEU | 89 | 13.560 | 8.443 | 13.536 | 1.00 | 0.00 |
| ATOM | 1444 | CA | LEU | 89 | 14.785 | 8.778 | 15.230 | 1.00 | 0.00 |
| ATOM | 1445 | HA | LEU | 89 | 15.603 | 9.475 | 15.334 | 1.00 | 0.00 |
| ATOM | 1446 | CB | LEU | 89 | 13.573 | 9.316 | 15.993 | 1.00 | 0.00 |
| ATOM | 1447 | HB1 | LEU | 89 | 13.601 | 8.920 | 16.997 | 1.00 | 0.00 |
| ATOM | 1448 | HB2 | LEU | 89 | 12.679 | 8.955 | 15.507 | 1.00 | 0.00 |
| ATOM | 1449 | CG | LEU | 89 | 13.489 | 10.841 | 16.084 | 1.00 | 0.00 |
| ATOM | 1450 | HG | LEU | 89 | 12.544 | 11.117 | 16.527 | 1.00 | 0.00 |
| ATOM | 1451 | CD1 | LEU | 89 | 14.600 | 11.385 | 16.968 | 1.00 | 0.00 |
| ATOM | 1452 | HD11 | LEU | 89 | 14.797 | 12.414 | 16.707 | 1.00 | 0.00 |
| ATOM | 1453 | HD12 | LEU | 89 | 15.496 | 10.799 | 16.823 | 1.00 | 0.00 |
| ATOM | 1454 | HD13 | LEU | 89 | 14.296 | 11.328 | 18.003 | 1.00 | 0.00 |
| ATOM | 1455 | CD2 | LEU | 89 | 13.559 | 11.460 | 14.696 | 1.00 | 0.00 |
| ATOM | 1456 | HD21 | LEU | 89 | 12.990 | 12.378 | 14.683 | 1.00 | 0.00 |
| ATOM | 1457 | HD22 | LEU | 89 | 13.147 | 10.771 | 13.973 | 1.00 | 0.00 |
| ATOM | 1458 | HD23 | LEU | 89 | 14.588 | 11.671 | 14.447 | 1.00 | 0.00 |
| ATOM | 1459 | C | LEU | 89 | 15.210 | 7.432 | 15.810 | 1.00 | 0.00 |
| ATOM | 1460 | O | LEU | 89 | 14.406 | 6.505 | 15.906 | 1.00 | 0.00 |
| ATOM | 1461 | N | LYS | 90 | 16.478 | 7.333 | 16.195 | 1.00 | 0.00 |
| ATOM | 1462 | HN | LYS | 90 | 17.070 | 8.107 | 16.093 | 1.00 | 0.00 |
| ATOM | 1463 | CA | LYS | 90 | 17.009 | 6.101 | 16.765 | 1.00 | 0.00 |
| ATOM | 1464 | HA | LYS | 90 | 16.174 | 5.502 | 17.097 | 1.00 | 0.00 |

(fortgesetzt)

Atomkoordinaten der Struktur der ADAP hSH3-1-Domäne, reduzierte Form

| ATOM | 1465 | CB | LYS | 90 | 17.792 | 5.321 | 15.708 | 1.00 | 0.00 |
|------|------|------|-----|-----|--------|--------|--------|------|------|
| ATOM | 1466 | HB1 | LYS | 90 | 18.042 | 4.348 | 16.106 | 1.00 | 0.00 |
| ATOM | 1467 | HB2 | LYS | 90 | 18.705 | 5.854 | 15.486 | 1.00 | 0.00 |
| ATOM | 1468 | CG | LYS | 90 | 17.028 | 5.122 | 14.409 | 1.00 | 0.00 |
| ATOM | 1469 | HG1 | LYS | 90 | 17.732 | 4.907 | 13.618 | 1.00 | 0.00 |
| ATOM | 1470 | HG2 | LYS | 90 | 16.489 | 6.029 | 14.177 | 1.00 | 0.00 |
| ATOM | 1471 | CD | LYS | 90 | 16.037 | 3.974 | 14.516 | 1.00 | 0.00 |
| ATOM | 1472 | HD1 | LYS | 90 | 15.157 | 4.319 | 15.038 | 1.00 | 0.00 |
| ATOM | 1473 | HD2 | LYS | 90 | 16.493 | 3.168 | 15.069 | 1.00 | 0.00 |
| ATOM | 1474 | CE | LYS | 90 | 15.630 | 3.461 | 13.144 | 1.00 | 0.00 |
| ATOM | 1475 | HE1 | LYS | 90 | 16.521 | 3.200 | 12.592 | 1.00 | 0.00 |
| ATOM | 1476 | HE2 | LYS | 90 | 15.102 | 4.246 | 12.622 | 1.00 | 0.00 |
| ATOM | 1477 | NZ | LYS | 90 | 14.748 | 2.265 | 13.238 | 1.00 | 0.00 |
| ATOM | 1478 | HZ1 | LYS | 90 | 15.289 | 1.449 | 13.591 | 1.00 | 0.00 |
| ATOM | 1479 | HZ2 | LYS | 90 | 14.362 | 2.030 | 12.301 | 1.00 | 0.00 |
| ATOM | 1480 | HZ3 | LYS | 90 | 13.959 | 2.453 | 13.888 | 1.00 | 0.00 |
| ATOM | 1481 | C | LYS | 90 | 17.907 | 6.398 | 17.962 | 1.00 | 0.00 |
| ATOM | 1482 | O | LYS | 90 | 19.131 | 6.429 | 17.840 | 1.00 | 0.00 |
| ATOM | 1483 | N | LEU | 91 | 17.289 | 6.617 | 19.118 | 1.00 | 0.00 |
| ATOM | 1484 | HN | LEU | 91 | 16.310 | 6.579 | 19.152 | 1.00 | 0.00 |
| ATOM | 1485 | CA | LEU | 91 | 18.032 | 6.913 | 20.338 | 1.00 | 0.00 |
| ATOM | 1486 | HA | LEU | 91 | 18.935 | 7.434 | 20.058 | 1.00 | 0.00 |
| ATOM | 1487 | CB | LEU | 91 | 17.204 | 7.813 | 21.258 | 1.00 | 0.00 |
| ATOM | 1488 | HB1 | LEU | 91 | 16.754 | 7.197 | 22.022 | 1.00 | 0.00 |
| ATOM | 1489 | HB2 | LEU | 91 | 16.415 | 8.263 | 20.672 | 1.00 | 0.00 |
| ATOM | 1490 | CG | LEU | 91 | 17.988 | 8.931 | 21.947 | 1.00 | 0.00 |
| ATOM | 1491 | HG | LEU | 91 | 18.824 | 9.212 | 21.323 | 1.00 | 0.00 |
| ATOM | 1492 | CD1 | LEU | 91 | 17.111 | 10.159 | 22.138 | 1.00 | 0.00 |
| ATOM | 1493 | HD11 | LEU | 91 | 17.416 | 10.683 | 23.032 | 1.00 | 0.00 |
| ATOM | 1494 | HD12 | LEU | 91 | 16.080 | 9.853 | 22.234 | 1.00 | 0.00 |
| ATOM | 1495 | HD13 | LEU | 91 | 17.216 | 10.812 | 21.285 | 1.00 | 0.00 |
| ATOM | 1496 | CD2 | LEU | 91 | 18.536 | 8.452 | 23.283 | 1.00 | 0.00 |
| ATOM | 1497 | HD21 | LEU | 91 | 17.716 | 8.204 | 23.940 | 1.00 | 0.00 |
| ATOM | 1498 | HD22 | LEU | 91 | 19.133 | 9.234 | 23.729 | 1.00 | 0.00 |
| ATOM | 1499 | HD23 | LEU | 91 | 19.149 | 7.576 | 23.127 | 1.00 | 0.00 |
| ATOM | 1500 | C | LEU | 91 | 18.412 | 5.628 | 21.068 | 1.00 | 0.00 |
| ATOM | 1501 | O | LEU | 91 | 17.547 | 4.843 | 21.456 | 1.00 | 0.00 |
| ATOM | 1502 | N | LYS | 92 | 19.712 | 5.420 | 21.251 | 1.00 | 0.00 |
| ATOM | 1503 | HN | LYS | 92 | 20.353 | 6.083 | 20.919 | 1.00 | 0.00 |
| ATOM | 1504 | CA | LYS | 92 | 20.207 | 4.231 | 21.935 | 1.00 | 0.00 |
| ATOM | 1505 | HA | LYS | 92 | 19.526 | 3.420 | 21.724 | 1.00 | 0.00 |
| ATOM | 1506 | CB | LYS | 92 | 21.598 | 3.861 | 21.416 | 1.00 | 0.00 |
| ATOM | 1507 | HB1 | LYS | 92 | 22.032 | 3.126 | 22.077 | 1.00 | 0.00 |
| ATOM | 1508 | HB2 | LYS | 92 | 22.217 | 4.745 | 21.418 | 1.00 | 0.00 |
| ATOM | 1509 | CG | LYS | 92 | 21.589 | 3.289 | 20.008 | 1.00 | 0.00 |
| ATOM | 1510 | HG1 | LYS | 92 | 22.551 | 3.470 | 19.550 | 1.00 | 0.00 |
| ATOM | 1511 | HG2 | LYS | 92 | 20.817 | 3.780 | 19.434 | 1.00 | 0.00 |
| ATOM | 1512 | CD | LYS | 92 | 21.318 | 1.793 | 20.016 | 1.00 | 0.00 |
| ATOM | 1513 | HD1 | LYS | 92 | 20.272 | 1.625 | 19.806 | 1.00 | 0.00 |
| ATOM | 1514 | HD2 | LYS | 92 | 21.560 | 1.398 | 20.991 | 1.00 | 0.00 |

(fortgesetzt)

Atomkoordinaten der Struktur der ADAP hSH3-1-Domäne, reduzierte Form

| ATOM | 1515 | CE | LYS | 92 | 22.154 | 1.070 | 18.972 | 1.00 | 0.00 |
|------|------|------|-----|----|--------|-------|--------|------|------|
| ATOM | 1516 | HE1 | LYS | 92 | 22.327 | 1.739 | 18.142 | 1.00 | 0.00 |
| ATOM | 1517 | HE2 | LYS | 92 | 21.608 | 0.205 | 18.628 | 1.00 | 0.00 |
| ATOM | 1518 | NZ | LYS | 92 | 23.466 | 0.628 | 19.520 | 1.00 | 0.00 |
| ATOM | 1519 | HZ1 | LYS | 92 | 24.198 | 0.681 | 18.782 | 1.00 | 0.00 |
| ATOM | 1520 | HZ2 | LYS | 92 | 23.400 | -0.354 | 19.857 | 1.00 | 0.00 |
| ATOM | 1521 | HZ3 | LYS | 92 | 23.745 | 1.237 | 20.315 | 1.00 | 0.00 |
| ATOM | 1522 | C | LYS | 92 | 20.257 | 4.451 | 23.443 | 1.00 | 0.00 |
| ATOM | 1523 | O | LYS | 92 | 20.021 | 3.529 | 24.223 | 1.00 | 0.00 |
| ATOM | 1524 | N | LYS | 93 | 20.565 | 5.679 | 23.847 | 1.00 | 0.00 |
| ATOM | 1525 | HN | LYS | 93 | 20.743 | 6.372 | 23.177 | 1.00 | 0.00 |
| ATOM | 1526 | CA | LYS | 93 | 20.646 | 6.021 | 25.262 | 1.00 | 0.00 |
| ATOM | 1527 | HA | LYS | 93 | 21.401 | 5.394 | 25.712 | 1.00 | 0.00 |
| ATOM | 1528 | CB | LYS | 93 | 21.051 | 7.487 | 25.432 | 1.00 | 0.00 |
| ATOM | 1529 | HB1 | LYS | 93 | 20.158 | 8.086 | 25.536 | 1.00 | 0.00 |
| ATOM | 1530 | HB2 | LYS | 93 | 21.586 | 7.805 | 24.549 | 1.00 | 0.00 |
| ATOM | 1531 | CG | LYS | 93 | 21.936 | 7.737 | 26.642 | 1.00 | 0.00 |
| ATOM | 1532 | HG1 | LYS | 93 | 22.963 | 7.534 | 26.373 | 1.00 | 0.00 |
| ATOM | 1533 | HG2 | LYS | 93 | 21.634 | 7.077 | 27.440 | 1.00 | 0.00 |
| ATOM | 1534 | CD | LYS | 93 | 21.827 | 9.175 | 27.129 | 1.00 | 0.00 |
| ATOM | 1535 | HD1 | LYS | 93 | 21.467 | 9.790 | 26.312 | 1.00 | 0.00 |
| ATOM | 1536 | HD2 | LYS | 93 | 22.804 | 9.517 | 27.432 | 1.00 | 0.00 |
| ATOM | 1537 | CE | LYS | 93 | 20.870 | 9.297 | 28.299 | 1.00 | 0.00 |
| ATOM | 1538 | HE1 | LYS | 93 | 20.785 | 8.333 | 28.779 | 1.00 | 0.00 |
| ATOM | 1539 | HE2 | LYS | 93 | 19.902 | 9.600 | 27.929 | 1.00 | 0.00 |
| ATOM | 1540 | NZ | LYS | 93 | 21.341 | 10.296 | 29.297 | 1.00 | 0.00 |
| ATOM | 1541 | HZ1 | LYS | 93 | 20.529 | 10.715 | 29.794 | 1.00 | 0.00 |
| ATOM | 1542 | HZ2 | LYS | 93 | 21.871 | 11.054 | 28.822 | 1.00 | 0.00 |
| ATOM | 1543 | HZ3 | LYS | 93 | 21.962 | 9.839 | 29.995 | 1.00 | 0.00 |
| ATOM | 1544 | C | LYS | 93 | 19.315 | 5.766 | 25.962 | 1.00 | 0.00 |
| ATOM | 1545 | O | LYS | 93 | 19.278 | 5.444 | 27.150 | 1.00 | 0.00 |
| ATOM | 1546 | N | ASP | 94 | 18.223 | 5.912 | 25.218 | 1.00 | 0.00 |
| ATOM | 1547 | HN | ASP | 94 | 18.316 | 6.170 | 24.278 | 1.00 | 0.00 |
| ATOM | 1548 | CA | ASP | 94 | 16.889 | 5.696 | 25.768 | 1.00 | 0.00 |
| ATOM | 1549 | HA | ASP | 94 | 16.758 | 6.378 | 26.595 | 1.00 | 0.00 |
| ATOM | 1550 | CB | ASP | 94 | 15.825 | 5.988 | 24.708 | 1.00 | 0.00 |
| ATOM | 1551 | HB1 | ASP | 94 | 15.499 | 5.057 | 24.268 | 1.00 | 0.00 |
| ATOM | 1552 | HB2 | ASP | 94 | 16.254 | 6.614 | 23.940 | 1.00 | 0.00 |
| ATOM | 1553 | CG | ASP | 94 | 14.616 | 6.698 | 25.284 | 1.00 | 0.00 |
| ATOM | 1554 | OD1 | ASP | 94 | 14.791 | 7.505 | 26.221 | 1.00 | 0.00 |
| ATOM | 1555 | OD2 | ASP | 94 | 13.493 | 6.448 | 24.797 | 1.00 | 0.00 |
| ATOM | 1556 | C | ASP | 94 | 16.736 | 4.268 | 26.280 | 1.00 | 0.00 |
| ATOM | 1557 | O | ASP | 94 | 17.712 | 3.522 | 26.371 | 1.00 | 0.00 |
| ATOM | 1558 | N | LEU | 95 | 15.506 | 3.893 | 26.614 | 1.00 | 0.00 |
| ATOM | 1559 | HN | LEU | 95 | 14.769 | 4.532 | 26.520 | 1.00 | 0.00 |
| ATOM | 1560 | CA | LEU | 95 | 15.225 | 2.553 | 27.117 | 1.00 | 0.00 |
| ATOM | 1561 | HA | LEU | 95 | 16.150 | 2.137 | 27.485 | 1.00 | 0.00 |
| ATOM | 1562 | CB | LEU | 95 | 14.217 | 2.619 | 28.267 | 1.00 | 0.00 |
| ATOM | 1563 | HB1 | LEU | 95 | 14.644 | 3.218 | 29.057 | 1.00 | 0.00 |
| ATOM | 1564 | HB2 | LEU | 95 | 14.064 | 1.616 | 28.640 | 1.00 | 0.00 |

(fortgesetzt)

Atomkoordinaten der Struktur der ADAP hSH3-1-Domäne, reduzierte Form

| ATOM | 1565 | CG | LEU | 95 | 12.854 | 3.209 | 27.901 | 1.00 | 0.00 |
|------|------|------|-----|-----|--------|--------|--------|------|------|
| ATOM | 1566 | HG | LEU | 95 | 12.968 | 3.859 | 27.046 | 1.00 | 0.00 |
| ATOM | 1567 | CD1 | LEU | 95 | 11.878 | 2.105 | 27.524 | 1.00 | 0.00 |
| ATOM | 1568 | HD11 | LEU | 95 | 12.105 | 1.215 | 28.091 | 1.00 | 0.00 |
| ATOM | 1569 | HD12 | LEU | 95 | 11.967 | 1.892 | 26.469 | 1.00 | 0.00 |
| ATOM | 1570 | HD13 | LEU | 95 | 10.870 | 2.425 | 27.743 | 1.00 | 0.00 |
| ATOM | 1571 | CD2 | LEU | 95 | 12.304 | 4.037 | 29.054 | 1.00 | 0.00 |
| ATOM | 1572 | HD21 | LEU | 95 | 12.635 | 3.613 | 29.991 | 1.00 | 0.00 |
| ATOM | 1573 | HD22 | LEU | 95 | 11.225 | 4.032 | 29.018 | 1.00 | 0.00 |
| ATOM | 1574 | HD23 | LEU | 95 | 12.663 | 5.052 | 28.971 | 1.00 | 0.00 |
| ATOM | 1575 | C | LEU | 95 | 14.689 | 1.655 | 26.006 | 1.00 | 0.00 |
| ATOM | 1576 | O | LEU | 95 | 13.897 | 0.747 | 26.257 | 1.00 | 0.00 |
| ATOM | 1577 | N | GLU | 96 | 15.128 | 1.915 | 24.779 | 1.00 | 0.00 |
| ATOM | 1578 | HN | GLU | 96 | 15.760 | 2.652 | 24.643 | 1.00 | 0.00 |
| ATOM | 1579 | CA | GLU | 96 | 14.693 | 1.130 | 23.630 | 1.00 | 0.00 |
| ATOM | 1580 | HA | GLU | 96 | 14.933 | 0.095 | 23.825 | 1.00 | 0.00 |
| ATOM | 1581 | CB | GLU | 96 | 13.181 | 1.261 | 23.439 | 1.00 | 0.00 |
| ATOM | 1582 | HB1 | GLU | 96 | 12.682 | 0.719 | 24.228 | 1.00 | 0.00 |
| ATOM | 1583 | HB2 | GLU | 96 | 12.910 | 0.826 | 22.488 | 1.00 | 0.00 |
| ATOM | 1584 | CG | GLU | 96 | 12.687 | 2.698 | 23.463 | 1.00 | 0.00 |
| ATOM | 1585 | HG1 | GLU | 96 | 13.520 | 3.355 | 23.255 | 1.00 | 0.00 |
| ATOM | 1586 | HG2 | GLU | 96 | 12.295 | 2.915 | 24.445 | 1.00 | 0.00 |
| ATOM | 1587 | CD | GLU | 96 | 11.599 | 2.958 | 22.440 | 1.00 | 0.00 |
| ATOM | 1588 | OE1 | GLU | 96 | 10.434 | 2.594 | 22.707 | 1.00 | 0.00 |
| ATOM | 1589 | OE2 | GLU | 96 | 11.911 | 3.525 | 21.372 | 1.00 | 0.00 |
| ATOM | 1590 | C | GLU | 96 | 15.417 | 1.572 | 22.363 | 1.00 | 0.00 |
| ATOM | 1591 | 0T1 | GLU | 96 | 14.902 | 2.476 | 21.672 | 1.00 | 0.00 |
| ATOM | 1592 | OT2 | GLU | 96 | 16.495 | 1.011 | 22.072 | 1.00 | 0.00 |
| END | | | | | | | | | |

Atomkooerdinaten der Struktur der ADAP hSH3-1-Domäne, oxidierte Form

| ATOM | 1 | N | GLU | 1 | -11.999 | -6.551 | -26.903 | 1.00 | 0.00 |
|------|---|------|-----|---|---------|--------|---------|------|------|
| ATOM | 2 | CA | GLU | 1 | -13.033 | -6.450 | -27.966 | 1.00 | 0.00 |
| ATOM | 3 | HA | GLU | 1 | -12.573 | -6.676 | -28.916 | 1.00 | 0.00 |
| ATOM | 4 | CB | GLU | 1 | -14.130 | -7.479 | -27.680 | 1.00 | 0.00 |
| ATOM | 5 | 1HB | GLU | 1 | -14.974 | -7.276 | -28.322 | 1.00 | 0.00 |
| ATOM | 6 | 2HB | GLU | 1 | -19.940 | -7.379 | -26.650 | 1.00 | 0.00 |
| ATOM | 7 | QB | GLU | 1 | -14.707 | -7.327 | -27.486 | 1.00 | 0.00 |
| ATOM | 8 | CG | GLU | 1 | -13.689 | -8.915 | -27.909 | 1.00 | 0.00 |
| ATOM | 9 | 1HG | GLU | 1 | -13.007 | -9.200 | -27.121 | 1.00 | 0.00 |
| ATOM | 10 | 2HG | GLU | 1 | -13.185 | -8.976 | -28.861 | 1.00 | 0.00 |
| ATOM | 11 | QG | GLU | 1 | -13.096 | -9.088 | -27.991 | 1.00 | 0.00 |
| ATOM | 12 | CD | GLU | 1 | -14.854 | -9.887 | -27.917 | 1.00 | 0.00 |
| ATOM | 13 | OE1 | GLU | 1 | -15.640 | -9.880 | -26.945 | 1.00 | 0.00 |
| ATOM | 14 | OE2 | GLU | 1 | -14.980 | -10.655 | -28.893 | 1.00 | 0.00 |
| ATOM | 15 | C | GLU | 1 | -13.631 | -5.047 | -28.023 | 1.00 | 0.00 |
| ATOM | 16 | O | GLU | 1 | -14.812 | -4.877 | -28.321 | 1.00 | 0.00 |
| ATOM | 17 | 1HT | GLU | 1 | -11.828 | -7.560 | -26.723 | 1.00 | 0.00 |
| ATOM | 18 | 2HT | GLU | 1 | -12.370 | -6.076 | -26.054 | 1.00 | 0.00 |

(fortgesetzt)

Atomkooerdinaten der Struktur der ADAP hSH3-1-Domäne, oxidierte Form

| ATOM | 19 | 3HT | GLU | 1 | -11.140 | -6.083 | -27.251 | 1.00 | 0.00 |
|------|----|-----|-----|---|---------|--------|---------|------|------|
| ATOM | 20 | N | LYS+ | 2 | -12.805 | -4.046 | -27.735 | 1.00 | 0.00 |
| ATOM | 21 | H | LYS+ | 2 | -11.874 | -4.244 | -27.505 | 1.00 | 0.00 |
| ATOM | 22 | CA | LYS+ | 2 | -13.253 | -2.658 | -27.755 | 1.00 | 0.00 |
| ATOM | 23 | HA | LYS+ | 2 | -13.815 | -2.504 | -28.663 | 1.00 | 0.00 |
| ATOM | 24 | CB | LYS+ | 2 | -14.158 | -2.375 | -26.554 | 1.00 | 0.00 |
| ATOM | 25 | 1HB | LYS+ | 2 | -14.986 | -3.069 | -26.571 | 1.00 | 0.00 |
| ATOM | 26 | 2HB | LYS+ | 2 | -14.542 | -1.369 | -26.637 | 1.00 | 0.00 |
| ATOM | 27 | QB | LYS+ | 2 | -14.764 | -2.219 | -26.604 | 1.00 | 0.00 |
| ATOM | 28 | CG | LYS+ | 2 | -13.451 | -2.507 | -25.213 | 1.00 | 0.00 |
| ATOM | 29 | 1HG | LYS+ | 2 | -13.688 | -1.645 | -24.607 | 1.00 | 0.00 |
| ATOM | 30 | 2HG | LYS+ | 2 | -12.385 | -2.547 | -25.382 | 1.00 | 0.00 |
| ATOM | 31 | QG | LYS+ | 2 | -13.037 | -2.096 | -24.995 | 1.00 | 0.00 |
| ATOM | 32 | CD | LYS+ | 2 | -13.880 | -3.764 | -24.475 | 1.00 | 0.00 |
| ATOM | 33 | 1HD | LYS+ | 2 | -13.188 | -4.561 | -24.709 | 1.00 | 0.00 |
| ATOM | 34 | 2HD | LYS+ | 2 | -14.873 | -4.040 | -24.801 | 1.00 | 0.00 |
| ATOM | 35 | QD | LYS+ | 2 | -14.030 | -4.301 | -24.755 | 1.00 | 0.00 |
| ATOM | 36 | CE | LYS+ | 2 | -13.897 | -3.548 | -22.971 | 1.00 | 0.00 |
| ATOM | 37 | 1HE | LYS+ | 2 | -14.830 | -3.077 | -22.699 | 1.00 | 0.00 |
| ATOM | 38 | 2HE | LYS+ | 2 | -13.076 | -2.900 | -22.703 | 1.00 | 0.00 |
| ATOM | 39 | QE | LYS+ | 2 | -13.953 | -2.989 | -22.701 | 1.00 | 0.00 |
| ATOM | 40 | NZ | LYS+ | 2 | -13.766 | -4.829 | -22.224 | 1.00 | 0.00 |
| ATOM | 41 | 1HZ | LYS+ | 2 | -14.702 | -5.263 | -22.091 | 1.00 | 0.00 |
| ATOM | 42 | 2HZ | LYS+ | 2 | -13.341 | -4.657 | -21.290 | 1.00 | 0.00 |
| ATOM | 43 | 3HZ | LYS+ | 2 | -13.159 | -5.490 | -22.752 | 1.00 | 0.00 |
| ATOM | 44 | QZ | LYS+ | 2 | -13.734 | -5.137 | -22.044 | 1.00 | 0.00 |
| ATOM | 45 | C | LYS+ | 2 | -12.064 | -1.703 | -27.746 | 1.00 | 0.00 |
| ATOM | 46 | O | LYS+ | 2 | -10.913 | -2.129 | -27.643 | 1.00 | 0.00 |
| ATOM | 47 | N | LYS+ | 3 | -12.349 | -0.410 | -27.853 | 1.00 | 0.00 |
| ATOM | 48 | H | LYS+ | 3 | -13.286 | -0.132 | -27.932 | 1.00 | 0.00 |
| ATOM | 49 | CA | LYS+ | 3 | -11.304 | 0.607 | -27.858 | 1.00 | 0.00 |
| ATOM | 50 | HA | LYS+ | 3 | -10.365 | 0.116 | -28.069 | 1.00 | 0.00 |
| ATOM | 51 | CB | LYS+ | 3 | -11.578 | 1.647 | -28.946 | 1.00 | 0.00 |
| ATOM | 52 | 1HB | LYS+ | 3 | -11.558 | 1.157 | -29.908 | 1.00 | 0.00 |
| ATOM | 53 | 2HB | LYS+ | 3 | -10.800 | 2.395 | -28.916 | 1.00 | 0.00 |
| ATOM | 54 | QB | LYS+ | 3 | -11.179 | 1.776 | -29.412 | 1.00 | 0.00 |
| ATOM | 55 | CG | LYS+ | 3 | -12.920 | 2.345 | -28.794 | 1.00 | 0.00 |
| ATOM | 56 | 1HG | LYS+ | 3 | -13.381 | 2.023 | -27.871 | 1.00 | 0.00 |
| ATOM | 57 | 2HG | LYS+ | 3 | -13.553 | 2.074 | -29.626 | 1.00 | 0.00 |
| ATOM | 58 | QG | LYS+ | 3 | -13.467 | 2.048 | -28.748 | 1.00 | 0.00 |
| ATOM | 59 | CD | LYS+ | 3 | -12.766 | 3.858 | -28.767 | 1.00 | 0.00 |
| ATOM | 60 | 1HD | LYS+ | 3 | -11.862 | 4.108 | -28.229 | 1.00 | 0.00 |
| ATOM | 61 | 2HD | LYS+ | 3 | -13.618 | 4.288 | -28.263 | 1.00 | 0.00 |
| ATOM | 62 | QD | LYS+ | 3 | -12.740 | 4.198 | -28.246 | 1.00 | 0.00 |
| ATOM | 63 | CE | LYS+ | 3 | -12.682 | 4.436 | -30.170 | 1.00 | 0.00 |
| ATOM | 64 | 1HE | LYS+ | 3 | -13.325 | 5.302 | -30.229 | 1.00 | 0.00 |
| ATOM | 65 | 2HE | LYS+ | 3 | -13.018 | 3.691 | -30.875 | 1.00 | 0.00 |
| ATOM | 66 | QE | LYS+ | 3 | -13.172 | 4.496 | -30.552 | 1.00 | 0.00 |
| ATOM | 67 | NZ | LYS+ | 3 | -11.292 | 4.841 | -30.522 | 1.00 | 0.00 |
| ATOM | 68 | 1HZ | LYS+ | 3 | -10.624 | 4.088 | -30.261 | 1.00 | 0.00 |

(fortgesetzt)

Atomkooerdinaten der Struktur der ADAP hSH3-1-Domäne, oxidierte Form

| ATOM | 69 | 2HZ | LYS+ | 3 | -11.034 | 5.710 | -30.014 | 1.00 | 0.00 |
|------|-----|------|------|---|---------|--------|---------|------|------|
| ATOM | 70 | 3HZ | LYS+ | 3 | -11.219 | 5.016 | -31.545 | 1.00 | 0.00 |
| ATOM | 71 | QZ | LYS+ | 3 | -10.959 | 4.938 | -30.607 | 1.00 | 0.00 |
| ATOM | 72 | C | LYS+ | 3 | -11.204 | 1.291 | -26.498 | 1.00 | 0.00 |
| ATOM | 73 | O | LYS+ | 3 | -12.138 | 1.241 | -25.697 | 1.00 | 0.00 |
| ATOM | 74 | N | GLU | 4 | -10.067 | 1.930 | -26.244 | 1.00 | 0.00 |
| ATOM | 75 | H | GLU | 4 | -9.359 | 1.934 | -26.922 | 1.00 | 0.00 |
| ATOM | 76 | CA | GLU | 4 | -9.846 | 2.624 | -24.981 | 1.00 | 0.00 |
| ATOM | 77 | HA | GLU | 4 | -10.639 | 2.343 | -24.305 | 1.00 | 0.00 |
| ATOM | 78 | CB | GLU | 4 | -8.504 | 2.209 | -24.374 | 1.00 | 0.00 |
| ATOM | 79 | 1HB | GLU | 4 | -8.306 | 2.827 | -23.511 | 1.00 | 0.00 |
| ATOM | 80 | 2HB | GLU | 4 | -7.727 | 2.368 | -25.107 | 1.00 | 0.00 |
| ATOM | 81 | QB | GLU | 4 | -8.016 | 2.598 | -24.309 | 1.00 | 0.00 |
| ATOM | 82 | CG | GLU | 4 | -8.457 | 0.753 | -23.939 | 1.00 | 0.00 |
| ATOM | 83 | 1HG | GLU | 4 | -8.627 | 0.128 | -24.803 | 1.00 | 0.00 |
| ATOM | 84 | 2HG | GLU | 4 | -9.239 | 0.582 | -23.213 | 1.00 | 0.00 |
| ATOM | 85 | QG | GLU | 4 | -8.933 | 0.355 | -24.008 | 1.00 | 0.00 |
| ATOM | 86 | CD | GLU | 4 | -7.129 | 0.373 | -23.317 | 1.00 | 0.00 |
| ATOM | 87 | OE1 | GLU | 4 | -6.508 | 1.240 | -22.666 | 1.00 | 0.00 |
| ATOM | 88 | OE2 | GLU | 4 | -6.708 | -0.791 | -23.480 | 1.00 | 0.00 |
| ATOM | 89 | C | GLU | 4 | -9.881 | 4.136 | -25.178 | 1.00 | 0.00 |
| ATOM | 90 | O | GLU | 4 | -9.079 | 4.693 | -25.927 | 1.00 | 0.00 |
| ATOM | 91 | N | GLN | 5 | -10.817 | 4.794 | -24.502 | 1.00 | 0.00 |
| ATOM | 92 | H | GLN | 5 | -11.427 | 4.294 | -23.920 | 1.00 | 0.00 |
| ATOM | 93 | CA | GLN | 5 | -10.957 | 6.242 | -24.602 | 1.00 | 0.00 |
| ATOM | 94 | HA | GLN | 5 | -10.553 | 6.549 | -25.556 | 1.00 | 0.00 |
| ATOM | 95 | CB | GLN | 5 | -12.433 | 6.638 | -24.537 | 1.00 | 0.00 |
| ATOM | 96 | 1HB | GLN | 5 | -12.532 | 7.664 | -24.860 | 1.00 | 0.00 |
| ATOM | 97 | 2HB | GLN | 5 | -12.771 | 6.557 | -23.515 | 1.00 | 0.00 |
| ATOM | 98 | QB | GLN | 5 | -12.651 | 7.111 | -24.187 | 1.00 | 0.00 |
| ATOM | 99 | CG | GLN | 5 | -13.333 | 5.777 | -25.409 | 1.00 | 0.00 |
| ATOM | 100 | 1HG | GLN | 5 | -12.726 | 5.033 | -25.904 | 1.00 | 0.00 |
| ATOM | 101 | 2HG | GLN | 5 | -13.806 | 6.404 | -26.149 | 1.00 | 0.00 |
| ATOM | 102 | QG | GLN | 5 | -13.266 | 5.719 | -26.026 | 1.00 | 0.00 |
| ATOM | 103 | CD | GLN | 5 | -14.414 | 5.069 | -24.614 | 1.00 | 0.00 |
| ATOM | 104 | OE1 | GLN | 5 | -14.372 | 3.853 | -24.432 | 1.00 | 0.00 |
| ATOM | 105 | NE2 | GLN | 5 | -15.391 | 5.831 | -24.135 | 1.00 | 0.00 |
| ATOM | 106 | 1HE2 | GLN | 5 | -15.360 | 6.793 | -24.320 | 1.00 | 0.00 |
| ATOM | 107 | 2HE2 | GLN | 5 | -16.103 | 5.400 | -23.617 | 1.00 | 0.00 |
| ATOM | 108 | QE2 | GLN | 5 | -15.731 | 6.097 | -23.968 | 1.00 | 0.00 |
| ATOM | 109 | C | GLN | 5 | -10.181 | 6.942 | -23.491 | 1.00 | 0.00 |
| ATOM | 110 | O | GLN | 5 | -9.647 | 8.033 | -23.687 | 1.00 | 0.00 |
| ATOM | 111 | N | LYS+ | 6 | -10.124 | 6.307 | -22.325 | 1.00 | 0.00 |
| ATOM | 112 | H | LYS+ | 6 | -10.571 | 5.440 | -22.231 | 1.00 | 0.00 |
| ATOM | 113 | CA | LYS+ | 6 | -9.414 | 6.869 | -21.183 | 1.00 | 0.00 |
| ATOM | 114 | HA | LYS+ | 6 | -9.237 | 7.915 | -21.384 | 1.00 | 0.00 |
| ATOM | 115 | CB | LYS+ | 6 | -10.261 | 6.744 | -19.915 | 1.00 | 0.00 |
| ATOM | 116 | 1HB | LYS+ | 6 | -9.629 | 6.908 | -19.056 | 1.00 | 0.00 |
| ATOM | 117 | 2HB | LYS+ | 6 | -10.669 | 5.745 | -19.867 | 1.00 | 0.00 |
| ATOM | 118 | QB | LYS+ | 6 | -10.149 | 6.327 | -19.461 | 1.00 | 0.00 |

(fortgesetzt)

Atomkooerdinaten der Struktur der ADAP hSH3-1-Domäne, oxidierte Form

| ATOM | 119 | CG | LYS+ | 6 | -11.414 | 7.733 | -19.855 | 1.00 | 0.00 |
|------|-----|-----|------|---|---------|--------|---------|------|------|
| ATOM | 120 | 1HG | LYS+ | 6 | -12.235 | 7.351 | -20.443 | 1.00 | 0.00 |
| ATOM | 121 | 2HG | LYS+ | 6 | -11.086 | 8.678 | -20.261 | 1.00 | 0.00 |
| ATOM | 122 | QG | LYS+ | 6 | -11.660 | 8.015 | -20.352 | 1.00 | 0.00 |
| ATOM | 123 | CD | LYS+ | 6 | -11.888 | 7.950 | -18.427 | 1.00 | 0.00 |
| ATOM | 124 | 1HD | LYS+ | 6 | -12.096 | 9.000 | -18.282 | 1.00 | 0.00 |
| ATOM | 125 | 2HD | LYS+ | 6 | -11.109 | 7.639 | -17.748 | 1.00 | 0.00 |
| ATOM | 126 | QD | LYS+ | 6 | -11.602 | 8.320 | -18.015 | 1.00 | 0.00 |
| ATOM | 127 | CE | LYS+ | 6 | -13.147 | 7.151 | -18.130 | 1.00 | 0.00 |
| ATOM | 128 | 1HE | LYS+ | 6 | -13.112 | 6.815 | -17.105 | 1.00 | 0.00 |
| ATOM | 129 | 2HE | LYS+ | 6 | -13.178 | 6.295 | -18.787 | 1.00 | 0.00 |
| ATOM | 130 | QE | LYS+ | 6 | -13.145 | 6.555 | -17.946 | 1.00 | 0.00 |
| ATOM | 131 | NZ | LYS+ | 6 | -14.380 | 7.961 | -18.333 | 1.00 | 0.00 |
| ATOM | 132 | 1HZ | LYS+ | 6 | -14.219 | 8.941 | -18.024 | 1.00 | 0.00 |
| ATOM | 133 | 2HZ | LYS+ | 6 | -15.166 | 7.561 | -17.782 | 1.00 | 0.00 |
| ATOM | 134 | 3HZ | LYS+ | 6 | -14.644 | 7.964 | -19.340 | 1.00 | 0.00 |
| ATOM | 135 | QZ | LYS+ | 6 | -14.676 | 8.155 | -18.382 | 1.00 | 0.00 |
| ATOM | 136 | C | LYS+ | 6 | -8.071 | 6.174 | -20.982 | 1.00 | 0.00 |
| ATOM | 137 | O | LYS+ | 6 | -7.769 | 5.181 | -21.645 | 1.00 | 0.00 |
| ATOM | 138 | N | GLU | 7 | -7.269 | 6.700 | -20.061 | 1.00 | 0.00 |
| ATOM | 139 | H | GLU | 7 | -7.566 | 7.491 | -19.564 | 1.00 | 0.00 |
| ATOM | 140 | CA | GLU | 7 | -5.959 | 6.129 | -19.772 | 1.00 | 0.00 |
| ATOM | 141 | HA | GLU | 7 | -6.102 | 5.099 | -19.483 | 1.00 | 0.00 |
| ATOM | 142 | CB | GLU | 7 | -5.072 | 6.177 | -21.018 | 1.00 | 0.00 |
| ATOM | 143 | 1HB | GLU | 7 | -4.832 | 7.207 | -21.236 | 1.00 | 0.00 |
| ATOM | 144 | 2HB | GLU | 7 | -5.619 | 5.760 | -21.851 | 1.00 | 0.00 |
| ATOM | 145 | QB | GLU | 7 | -5.225 | 6.484 | -21.543 | 1.00 | 0.00 |
| ATOM | 146 | CG | GLU | 7 | -3.771 | 5.405 | -20.868 | 1.00 | 0.00 |
| ATOM | 147 | 1HG | GLU | 7 | -3.346 | 5.626 | -19.900 | 1.00 | 0.00 |
| ATOM | 148 | 2HG | GLU | 7 | -3.087 | 5.723 | -21.641 | 1.00 | 0.00 |
| ATOM | 149 | QG | GLU | 7 | -3.217 | 5.675 | -20.770 | 1.00 | 0.00 |
| ATOM | 150 | CD | GLU | 7 | -3.966 | 3.906 | -20.982 | 1.00 | 0.00 |
| ATOM | 151 | OE1 | GLU | 7 | -3.366 | 3.166 | -20.175 | 1.00 | 0.00 |
| ATOM | 152 | OE2 | GLU | 7 | -4.720 | 3.472 | -21.880 | 1.00 | 0.00 |
| ATOM | 153 | C | GLU | 7 | -5.281 | 6.870 | -18.625 | 1.00 | 0.00 |
| ATOM | 154 | O | GLU | 7 | -4.625 | 6.262 | -17.779 | 1.00 | 0.00 |
| ATOM | 155 | N | LYS+ | 8 | -5.444 | 8.191 | -18.601 | 1.00 | 0.00 |
| ATOM | 156 | H | LYS+ | 8 | -5.977 | 8.620 | -19.302 | 1.00 | 0.00 |
| ATOM | 157 | CA | LYS+ | 8 | -4.846 | 9.019 | -17.557 | 1.00 | 0.00 |
| ATOM | 158 | HA | LYS+ | 8 | -3.774 | 8.967 | -17.666 | 1.00 | 0.00 |
| ATOM | 159 | CB | LYS+ | 8 | -5.293 | 10.474 | -17.716 | 1.00 | 0.00 |
| ATOM | 160 | 1HB | LYS+ | 8 | -5.016 | 10.817 | -18.702 | 1.00 | 0.00 |
| ATOM | 161 | 2HB | LYS+ | 8 | -4.784 | 11.077 | -16.979 | 1.00 | 0.00 |
| ATOM | 162 | QB | LYS+ | 8 | -4.900 | 10.947 | -17.840 | 1.00 | 0.00 |
| ATOM | 163 | CG | LYS+ | 8 | -6.789 | 10.673 | -17.544 | 1.00 | 0.00 |
| ATOM | 164 | 1HG | LYS+ | 8 | -7.043 | 10.544 | -16.501 | 1.00 | 0.00 |
| ATOM | 165 | 2HG | LYS+ | 8 | -7.311 | 9.937 | -18.137 | 1.00 | 0.00 |
| ATOM | 166 | QG | LYS+ | 8 | -7.177 | 10.240 | -17.319 | 1.00 | 0.00 |
| ATOM | 167 | CD | LYS+ | 8 | -7.222 | 12.060 | -17.989 | 1.00 | 0.00 |
| ATOM | 168 | 1HD | LYS+ | 8 | -6.848 | 12.240 | -18.987 | 1.00 | 0.00 |

(fortgesetzt)

Atomkooerdinaten der Struktur der ADAP hSH3-1-Domäne, oxidierte Form

| ATOM | 169 | 2HD | LYS+ | 8 | -6.808 | 12.791 | -17.311 | 1.00 | 0.00 |
|------|-----|-----|------|---|--------|--------|---------|------|------|
| ATOM | 170 | QD | LYS+ | 8 | -6.828 | 12.515 | -18.149 | 1.00 | 0.00 |
| ATOM | 171 | CE | LYS+ | 8 | -8.736 | 12.198 | -17.996 | 1.00 | 0.00 |
| ATOM | 172 | 1HE | LYS+ | 8 | -9.042 | 12.712 | -17.097 | 1.00 | 0.00 |
| ATOM | 173 | 2HE | LYS+ | 8 | -9.175 | 11.211 | -18.011 | 1.00 | 0.00 |
| ATOM | 174 | QE | LYS+ | 8 | -9.108 | 11.962 | -17.554 | 1.00 | 0.00 |
| ATOM | 175 | NZ | LYS+ | 8 | -9.220 | 12.959 | -19.180 | 1.00 | 0.00 |
| ATOM | 176 | 1HZ | LYS+ | 8 | -9.023 | 13.973 | -19.058 | 1.00 | 0.00 |
| ATOM | 177 | 2HZ | LYS+ | 8 | -10.245 | 12.828 | -19.296 | 1.00 | 0.00 |
| ATOM | 178 | 3HZ | LYS+ | 8 | -8.740 | 12.624 | -20.041 | 1.00 | 0.00 |
| ATOM | 179 | QZ | LYS+ | 8 | -9.336 | 13.142 | -19.465 | 1.00 | 0.00 |
| ATOM | 180 | C | LYS+ | 8 | -5.225 | 8.509 | -16.169 | 1.00 | 0.00 |
| ATOM | 181 | O | LYS+ | 8 | -6.399 | 8.496 | -15.800 | 1.00 | 0.00 |
| ATOM | 182 | N | GLU | 9 | -4.221 | 8.092 | -15.404 | 1.00 | 0.00 |
| ATOM | 183 | H | GLU | 9 | -3.306 | 8.127 | -15.754 | 1.00 | 0.00 |
| ATOM | 184 | CA | GLU | 9 | -4.447 | 7.581 | -14.057 | 1.00 | 0.00 |
| ATOM | 185 | HA | GLU | 9 | -5.345 | 6.982 | -14.077 | 1.00 | 0.00 |
| ATOM | 186 | CB | GLU | 9 | -3.273 | 6.703 | -13.618 | 1.00 | 0.00 |
| ATOM | 187 | 1HB | GLU | 9 | -3.143 | 6.803 | -12.551 | 1.00 | 0.00 |
| ATOM | 188 | 2HB | GLU | 9 | -2.377 | 7.047 | -14.115 | 1.00 | 0.00 |
| ATOM | 189 | QB | GLU | 9 | -2.760 | 6.925 | -13.333 | 1.00 | 0.00 |
| ATOM | 190 | CG | GLU | 9 | -3.961 | 5.230 | -13.941 | 1.00 | 0.00 |
| ATOM | 191 | 1HG | GLU | 9 | -4.132 | 5.142 | -14.782 | 1.00 | 0.00 |
| ATOM | 192 | 2HG | GLU | 9 | -3.894 | 4.740 | -13.082 | 1.00 | 0.00 |
| ATOM | 193 | QG | GLU | 9 | -9.013 | 4.941 | -13.932 | 1.00 | o.oo |
| ATOM | 194 | CD | GLU | 9 | -2.157 | 4.538 | -14.286 | 1.00 | 0.00 |
| ATOM | 195 | OE1 | GLU | 9 | -1.699 | 4.675 | -15.440 | 1.00 | 0.00 |
| ATOM | 196 | OE2 | GLU | 9 | -1.591 | 3.860 | -13.402 | 1.00 | 0.00 |
| ATOM | 197 | C | GLU | 9 | -4.643 | 8.727 | -13.068 | 1.00 | 0.00 |
| ATOM | 198 | O | GLU | 9 | -3.871 | 8.885 | -12.122 | 1.00 | 0.00 |
| ATOM | 199 | N | LYS+ | 10 | -5.684 | 9.523 | -13.292 | 1.00 | 0.00 |
| ATOM | 200 | H | LYS+ | 10 | -6.264 | 9.347 | -14.062 | 1.00 | 0.00 |
| ATOM | 201 | CA | LYS+ | 10 | -5.983 | 10.653 | -12.420 | 1.00 | 0.00 |
| ATOM | 202 | HA | LYS+ | 10 | -5.126 | 11.311 | -12.425 | 1.00 | 0.00 |
| ATOM | 203 | CB | LYS+ | 10 | -7.201 | 11. 419 | -12.942 | 1.00 | 0.00 |
| ATOM | 204 | 1HB | LYS+ | 10 | -7.814 | 11.714 | -12.103 | 1.00 | 0.00 |
| ATOM | 205 | 2HB | LYS+ | 10 | -7.774 | 10.766 | -13.583 | 1.00 | 0.00 |
| ATOM | 206 | QB | LYS+ | 10 | -7.794 | 11.240 | -12.843 | 1.00 | 0.00 |
| ATOM | 207 | CG | LYS+ | 10 | -6.842 | 12.666 | -13.732 | 1.00 | 0.00 |
| ATOM | 208 | 1HG | LYS+ | 10 | -7.680 | 12.938 | -14.357 | 1.00 | 0.00 |
| ATOM | 209 | 2HG | LYS+ | 10 | -5.982 | 12.455 | -14.350 | 1.00 | 0.00 |
| ATOM | 210 | QG | LYS+ | 10 | -6.831 | 12.697 | -14.353 | 1.00 | 0.00 |
| ATOM | 211 | CD | LYS+ | 10 | -6.513 | 13.832 | -12.813 | 1.00 | 0.00 |
| ATOM | 212 | 1HD | LYS+ | 10 | -5.703 | 13.543 | -12.159 | 1.00 | 0.00 |
| ATOM | 213 | 2HD | LYS+ | 10 | -7.386 | 14.072 | -12.224 | 1.00 | 0.00 |
| ATOM | 214 | QD | LYS+ | 10 | -6.544 | 13.807 | -12.192 | 1.00 | 0.00 |
| ATOM | 215 | CE | LYS+ | 10 | -6.097 | 15.063 | -13.601 | 1.00 | 0.00 |
| ATOM | 216 | 1HE | LYS+ | 10 | -6.176 | 15.929 | -12.960 | 1.00 | 0.00 |
| ATOM | 217 | 2HE | LYS+ | 10 | -6.763 | 15.178 | -14.444 | 1.00 | 0.00 |
| ATOM | 218 | QE | LYS+ | 10 | -6.469 | 15.553 | -13.702 | 1.00 | 0.00 |

(fortgesetzt)

Atomkooerdinaten der Struktur der ADAP hSH3-1-Domäne, oxidierte Form

| ATOM | 219 | NZ | LYS+ | 10 | -4.698 | 14.956 | -14.101 | 1.00 | 0.00 |
|------|-----|-----|------|----|--------|--------|---------|------|------|
| ATOM | 220 | 1HZ | LYS+ | 10 | -4.236 | 15.888 | -14.071 | 1.00 | 0.00 |
| ATOM | 221 | 2HZ | LYS+ | 10 | -4.694 | 14.612 | -15.082 | 1.00 | 0.00 |
| ATOM | 222 | 3HZ | LYS+ | 10 | -4.157 | 14.293 | -13.510 | 1.00 | 0.00 |
| ATOM | 223 | QZ | LYS+ | 10 | -4.362 | 14.931 | -14.221 | 1.00 | 0.00 |
| ATOM | 224 | C | LYS+ | 10 | -6.233 | 10.185 | -10.990 | 1.00 | 0.00 |
| ATOM | 225 | O | LYS+ | 10 | -5.868 | 10.864 | -10.030 | 1.00 | 0.00 |
| ATOM | 226 | N | LYS+ | 11 | -6.857 | 9.019 | -10.856 | 1.00 | 0.00 |
| ATOM | 227 | H | LYS+ | 11 | -7.122 | 8.524 | -11.660 | 1.00 | 0.00 |
| ATOM | 228 | CA | LYS+ | 11 | -7.157 | 8.458 | -9.545 | 1.00 | 0.00 |
| ATOM | 229 | HA | LYS+ | 11 | -7.673 | 9.213 | -8.971 | 1.00 | 0.00 |
| ATOM | 230 | CB | LYS+ | 11 | -8.063 | 7.234 | -9.686 | 1.00 | 0.00 |
| ATOM | 231 | 1HB | LYS+ | 11 | -7.554 | 6.490 | -10.281 | 1.00 | 0.00 |
| ATOM | 232 | 2HB | LYS+ | 11 | -8.970 | 7.529 | -10.192 | 1.00 | 0.00 |
| ATOM | 233 | QB | LYS+ | 11 | -8.262 | 7.010 | -10.237 | 1.00 | 0.00 |
| ATOM | 234 | CG | LYS+ | 11 | -8.444 | 6.602 | -8.357 | 1.00 | 0.00 |
| ATOM | 235 | 1HG | LYS+ | 11 | -9.078 | 7.287 | -7.813 | 1.00 | 0.00 |
| ATOM | 236 | 2HG | LYS+ | 11 | -7.546 | 6.411 | -7.789 | 1.00 | 0.00 |
| ATOM | 237 | QG | LYS+ | 11 | -8.312 | 6.849 | -7.801 | 1.00 | 0.00 |
| ATOM | 238 | CD | LYS+ | 11 | -9.189 | 5.292 | -8.557 | 1.00 | 0.00 |
| ATOM | 239 | 1HD | LYS+ | 11 | -8.469 | 4.492 | -8.650 | 1.00 | 0.00 |
| ATOM | 240 | 2HD | LYS+ | 11 | -9.776 | 5.357 | -9.460 | 1.00 | 0.00 |
| ATOM | 241 | QD | LYS+ | 11 | -9.123 | 4.924 | -9.055 | 1.00 | 0.00 |
| ATOM | 242 | CE | LYS+ | 11 | -10.112 | 4.993 | -7.388 | 1.00 | 0.00 |
| ATOM | 243 | 1HE | LYS+ | 11 | -9.700 | 5.439 | -6.495 | 1.00 | 0.00 |
| ATOM | 244 | 2HE | LYS+ | 11 | -10.174 | 3.921 | -7.259 | 1.00 | 0.00 |
| ATOM | 245 | QE | LYS+ | 11 | -9.937 | 4.680 | -6.877 | 1.00 | 0.00 |
| ATOM | 246 | NZ | LYS+ | 11 | -11.483 | 5.532 | -7.611 | 1.00 | 0.00 |
| ATOM | 247 | 1HZ | LYS+ | 11 | -12.103 | 4.787 | -7.988 | 1.00 | 0.00 |
| ATOM | 248 | 2HZ | LYS+ | 11 | -11.451 | 6.319 | -8.290 | 1.00 | 0.00 |
| ATOM | 249 | 3HZ | LYS+ | 11 | -11.881 | 5.878 | -6.714 | 1.00 | 0.00 |
| ATOM | 250 | QZ | LYS+ | 11 | -11.812 | 5.661 | -7.664 | 1.00 | 0.00 |
| ATOM | 251 | C | LYS+ | 11 | -5.875 | 8.075 | -8.812 | 1.00 | 0.00 |
| ATOM | 252 | O | LYS+ | 11 | -5.703 | 8.392 | -7.637 | 1.00 | 0.00 |
| ATOM | 253 | N | GLU | 12 | -4.978 | 7.392 | -9.518 | 1.00 | 0.00 |
| ATOM | 254 | H | GLU | 12 | -5.166 | 7.168 | -10.449 | 1.00 | 0.00 |
| ATOM | 255 | CA | GLU | 12 | -3.717 | 6.961 | -8.954 | 1.00 | 0.00 |
| ATOM | 256 | HA | GLU | 12 | -3.906 | 6.554 | -7.975 | 1.00 | 0.00 |
| ATOM | 257 | CB | GLU | 12 | -3.129 | 5.872 | -9.835 | 1.00 | 0.00 |
| ATOM | 258 | 1HB | GLU | 12 | -2.340 | 6.298 | -10.421 | 1.00 | 0.00 |
| ATOM | 259 | 2HB | GLU | 12 | -3.901 | 5.509 | -10.499 | 1.00 | 0.00 |
| ATOM | 260 | QB | GLU | 12 | -3.121 | 5.904 | -10.460 | 1.00 | 0.00 |
| ATOM | 261 | CG | GLU | 12 | -2.579 | 4.694 | -9.060 | 1.00 | 0.00 |
| ATOM | 262 | 1HG | GLU | 12 | -3.372 | 3.974 | -8.928 | 1.00 | 0.00 |
| ATOM | 263 | 2HG | GLU | 12 | -2.245 | 5.042 | -8.095 | 1.00 | 0.00 |
| ATOM | 264 | QG | GLU | 12 | -2.808 | 4.508 | -8.511 | 1.00 | 0.00 |
| ATOM | 265 | CD | GLU | 12 | -1.418 | 4.020 | -9.764 | 1.00 | 0.00 |
| ATOM | 266 | OE1 | GLU | 12 | -1.592 | 2.876 | -10.234 | 1.00 | 0.00 |
| ATOM | 267 | OE2 | GLU | 12 | -0.334 | 4.635 | -9.844 | 1.00 | 0.00 |
| ATOM | 268 | C | GLU | 12 | -2.737 | 8.124 | -8.822 | 1.00 | 0.00 |

Atomkooerdinaten der Struktur der ADAP hSH3-1-Domäne, oxidierte Form

(fortgesetzt)

Atomkooerdinaten der Struktur der ADAP hSH3-1-Domäne, oxidierte Form

| ATOM | 269 | O | GLU | 12 | -1.658 | 7.972 | -8.250 | 1.00 | 0.00 |
|------|-----|------|-----|----|--------|-------|--------|------|------|
| ATOM | 270 | N | GLN | 13 | -3.122 | 9.290 | -9.335 | 1.00 | 0.00 |
| ATOM | 271 | H | GLN | 13 | -4.001 | 9.371 | -9.759 | 1.00 | 0.00 |
| ATOM | 272 | CA | GLN | 13 | -2.288 | 10.467 | -9.251 | 1.00 | 0.00 |
| ATOM | 273 | HA | GLN | 13 | -1.255 | 10.174 | -9.345 | 1.00 | 0.00 |
| ATOM | 274 | CB | GLN | 13 | -2.668 | 11.428 | -10.368 | 1.00 | 0.00 |
| ATOM | 275 | 1HB | GLN | 13 | -3.226 | 12.243 | -9.942 | 1.00 | 0.00 |
| ATOM | 276 | 2HB | GLN | 13 | -3.302 | 10.907 | -11.064 | 1.00 | 0.00 |
| ATOM | 277 | QB | GLN | 13 | -3.264 | 11.575 | -10.503 | 1.00 | 0.00 |
| ATOM | 278 | CG | GLN | 13 | -1.479 | 11.997 | -11.125 | 1.00 | 0.00 |
| ATOM | 279 | 1HG | GLN | 13 | -1.807 | 12.303 | -12.107 | 1.00 | 0.00 |
| ATOM | 280 | 2HG | GLN | 13 | -0.728 | 11.229 | -11.221 | 1.00 | 0.00 |
| ATOM | 281 | QG | GLN | 13 | -1.268 | 11.766 | -11.664 | 1.00 | 0.00 |
| ATOM | 282 | CD | GLN | 13 | -0.861 | 13.192 | -10.426 | 1.00 | 0.00 |
| ATOM | 283 | OE1 | GLN | 13 | -1.415 | 14.291 | -10.443 | 1.00 | 0.00 |
| ATOM | 284 | NE2 | GLN | 13 | 0.295 | 12.983 | -9.806 | 1.00 | 0.00 |
| ATOM | 285 | 1HE2 | GLN | 13 | 0.678 | 12.081 | -9.834 | 1.00 | 0.00 |
| ATOM | 286 | 2HE2 | GLN | 13 | 0.717 | 13.738 | -9.345 | 1.00 | 0.00 |
| ATOM | 287 | QE2 | GLN | 13 | 0.697 | 12.910 | -9.590 | 1.00 | 0.00 |
| ATOM | 288 | C | GLN | 13 | -2.529 | 11.115 | -7.910 | 1.00 | 0.00 |
| ATOM | 289 | O | GLN | 13 | -1.612 | 11.356 | -7.124 | 1.00 | 0.00 |
| ATOM | 290 | N | GLU | 14 | -3.803 | 11.357 | -7.664 | 1.00 | 0.00 |
| ATOM | 291 | H | GLU | 14 | -4.464 | 11.110 | -8.348 | 1.00 | 0.00 |
| ATOM | 292 | CA | GLU | 14 | -4.267 | 11.944 | -6.421 | 1.00 | 0.00 |
| ATOM | 293 | HA | GLU | 14 | -3.960 | 12.976 | -6.388 | 1.00 | 0.00 |
| ATOM | 294 | CB | GLU | 14 | -5.788 | 11.867 | -6.387 | 1.00 | 0.00 |
| ATOM | 295 | 1HB | GLU | 14 | -6.091 | 11.317 | -5.510 | 1.00 | 0.00 |
| ATOM | 296 | 2HB | GLU | 14 | -6.117 | 11.331 | -7.269 | 1.00 | 0.00 |
| ATOM | 297 | QB | GLU | 14 | -6.104 | 11.324 | -6.390 | 1.00 | 0.00 |
| ATOM | 298 | CG | GLU | 14 | -6.472 | 13.225 | -6.367 | 1.00 | 0.00 |
| ATOM | 299 | 1HG | GLU | 14 | -7.541 | 13.076 | -6.391 | 1.00 | 0.00 |
| ATOM | 300 | 2HG | GLU | 14 | -6.166 | 13.781 | -7.241 | 1.00 | 0.00 |
| ATOM | 301 | QG | GLU | 14 | -6.854 | 13.428 | -6.816 | 1.00 | 0.00 |
| ATOM | 302 | CD | GLU | 14 | -6.123 | 14.035 | -5.134 | 1.00 | 0.00 |
| ATOM | 303 | OE1 | GLU | 14 | -5.093 | 14.740 | -5.158 | 1.00 | 0.00 |
| ATOM | 304 | OE2 | GLU | 14 | -6.880 | 13.964 | -4.143 | 1.00 | 0.00 |
| ATOM | 305 | C | GLU | 14 | -3.682 | 11.196 | -5.227 | 1.00 | 0.00 |
| ATOM | 306 | O | GLU | 14 | -3.202 | 11.801 | -4.268 | 1.00 | 0.00 |
| ATOM | 307 | N | ILE | 15 | -3.728 | 9.869 | -5.307 | 1.00 | 0.00 |
| ATOM | 308 | H | ILE | 15 | -4.124 | 9.458 | -6.102 | 1.00 | 0.00 |
| ATOM | 309 | CA | ILE | 15 | -3.207 | 9.009 | -4.251 | 1.00 | 0.00 |
| ATOM | 310 | HA | ILE | 15 | -3.855 | 9.093 | -3.403 | 1.00 | 0.00 |
| ATOM | 311 | CB | ILE | 15 | -3.211 | 7.536 | -4.688 | 1.00 | 0.00 |
| ATOM | 312 | HB | ILE | 15 | -2.655 | 7.457 | -5.591 | 1.00 | 0.00 |
| ATOM | 313 | QG2 | ILE | 15 | -2.403 | 6.445 | -3.389 | 1.00 | 0.00 |
| ATOM | 314 | CG2 | ILE | 15 | -2.557 | 6.653 | -3.638 | 1.00 | 0.00 |
| ATOM | 315 | 1HG2 | ILE | 15 | -3.291 | 5.982 | -3.226 | 1.00 | 0.00 |
| ATOM | 316 | 2HG2 | ILE | 15 | -2.155 | 7.271 | -2.849 | 1.00 | 0.00 |
| ATOM | 317 | 3HG2 | ILE | 15 | -1.762 | 6.082 | -4.093 | 1.00 | 0.00 |
| ATOM | 318 | CG1 | ILE | 15 | -4.634 | 7.079 | -4.945 | 1.00 | 0.00 |

(fortgesetzt)

Atomkooerdinaten der Struktur der ADAP hSH3-1-Domäne, oxidierte Form

| ATOM | 319 | 1HG1 | ILE  | 15 | -5.085 | 7.731  | -5.672 | 1.00 | 0.00 |
|------|-----|------|------|----|--------|--------|--------|------|------|
| ATOM | 320 | 2HG1 | ILE  | 15 | -5.184 | 7.136  | -4.028 | 1.00 | 0.00 |
| ATOM | 321 | QG1  | ILE  | 15 | -5.135 | 7.434  | -4.850 | 1.00 | 0.00 |
| ATOM | 322 | QD1  | ILE  | 15 | -4.754 | 5.320  | -5.579 | 1.00 | 0.00 |
| ATOM | 323 | CD1  | ILE  | 15 | -4.731 | 5.659  | -5.458 | 1.00 | 0.00 |
| ATOM | 324 | 1HD1 | ILE  | 15 | -3.801 | 5.143  | -5.266 | 1.00 | 0.00 |
| ATOM | 325 | 2HD1 | ILE  | 15 | -4.926 | 5.670  | -6.520 | 1.00 | 0.00 |
| ATOM | 326 | 3HD1 | ILE  | 15 | -5.536 | 5.147  | -4.951 | 1.00 | 0.00 |
| ATOM | 327 | C    | ILE  | 15 | -1.795 | 9.421  | -3.843 | 1.00 | 0.00 |
| ATOM | 328 | O    | ILE  | 15 | -1.433 | 9.359  | -2.667 | 1.00 | 0.00 |
| ATOM | 329 | N    | LYS+ | 16 | -1.001 | 9.844  | -4.820 | 1.00 | 0.00 |
| ATOM | 330 | H    | LYS+ | 16 | -1.347 | 9.872  | -5.737 | 1.00 | 0.00 |
| ATOM | 331 | CA   | LYS+ | 16 | 0.371  | 10.267 | -4.560 | 1.00 | 0.00 |
| ATOM | 332 | HA   | LYS+ | 16 | 0.902  | 9.429  | -4.142 | 1.00 | 0.00 |
| ATOM | 333 | CB   | LYS+ | 16 | 1.055  | 10.680 | -5.860 | 1.00 | 0.00 |
| ATOM | 334 | 1HB  | LYS+ | 16 | 1.243  | 11.741 | -5.830 | 1.00 | 0.00 |
| ATOM | 335 | 2HB  | LYS+ | 16 | 0.397  | 10.462 | -6.687 | 1.00 | 0.00 |
| ATOM | 336 | QB   | LYS+ | 16 | 0.820  | 11.102 | -6.259 | 1.00 | 0.00 |
| ATOM | 337 | CG   | LYS+ | 16 | 2.375  | 9.969  | -6.097 | 1.00 | 0.00 |
| ATOM | 338 | 1HG  | LYS+ | 16 | 2.698  | 10.154 | -7.111 | 1.00 | 0.00 |
| ATOM | 339 | 2HG  | LYS+ | 16 | 2.232  | 8.909  | -5.949 | 1.00 | 0.00 |
| ATOM | 340 | QG   | LYS+ | 16 | 2.465  | 9.531  | -6.530 | 1.00 | 0.00 |
| ATOM | 341 | CD   | LYS+ | 16 | 3.447  | 10.458 | -5.137 | 1.00 | 0.00 |
| ATOM | 342 | 1HD  | LYS+ | 16 | 3.537  | 9.751  | -4.323 | 1.00 | 0.00 |
| ATOM | 343 | 2HD  | LYS+ | 16 | 3.158  | 11.422 | -4.748 | 1.00 | 0.00 |
| ATOM | 344 | QD   | LYS+ | 16 | 3.347  | 10.586 | -4.536 | 1.00 | 0.00 |
| ATOM | 345 | CE   | LYS+ | 16 | 4.795  | 10.591 | -5.827 | 1.00 | 0.00 |
| ATOM | 346 | 1HE  | LYS+ | 16 | 4.779  | 11.472 | -6.451 | 1.00 | 0.00 |
| ATOM | 347 | 2HE  | LYS+ | 16 | 4.959  | 9.719  | -6.442 | 1.00 | 0.00 |
| ATOM | 348 | QE   | LYS+ | 16 | 4.869  | 10.595 | -6.446 | 1.00 | 0.00 |
| ATOM | 349 | NZ   | LYS+ | 16 | 5.912  | 10.709 | -4.849 | 1.00 | 0.00 |
| ATOM | 350 | 1HZ  | LYS+ | 16 | 6.372  | 9.785  | -4.718 | 1.00 | 0.00 |
| ATOM | 351 | 2HZ  | LYS+ | 16 | 5.550  | 11.036 | -3.931 | 1.00 | 0.00 |
| ATOM | 352 | 3HZ  | LYS+ | 16 | 6.618  | 11.391 | -5.193 | 1.00 | 0.00 |
| ATOM | 353 | QZ   | LYS+ | 16 | 6.180  | 10.737 | -4.614 | 1.00 | 0.00 |
| ATOM | 354 | C    | LYS+ | 16 | 0.410  | 11.417 | -3.559 | 1.00 | 0.00 |
| ATOM | 355 | O    | LYS+ | 16 | 1.373  | 11.565 | -2.807 | 1.00 | 0.00 |
| ATOM | 356 | N    | LYS+ | 17 | -0.639 | 12.231 | -3.557 | 1.00 | 0.00 |
| ATOM | 357 | H    | LYS+ | 17 | -1.377 | 12.063 | -4.181 | 1.00 | 0.00 |
| ATOM | 358 | CA   | LYS+ | 17 | -0.722 | 13.370 | -2.650 | 1.00 | 0.00 |
| ATOM | 359 | HA   | LYS+ | 17 | 0.283  | 13.619 | -2.343 | 1.00 | 0.00 |
| ATOM | 360 | CB   | LYS+ | 17 | -1.330 | 14.574 | -3.370 | 1.00 | 0.00 |
| ATOM | 361 | 1HB  | LYS+ | 17 | -1.908 | 15.148 | -2.660 | 1.00 | 0.00 |
| ATOM | 362 | 2HB  | LYS+ | 17 | -1.987 | 14.218 | -4.151 | 1.00 | 0.00 |
| ATOM | 363 | QB   | LYS+ | 17 | -1.947 | 14.683 | -3.405 | 1.00 | 0.00 |
| ATOM | 364 | CG   | LYS+ | 17 | -0.295 | 15.493 | -3.998 | 1.00 | 0.00 |
| ATOM | 365 | 1HG  | LYS+ | 17 | -0.288 | 15.333 | -5.067 | 1.00 | 0.00 |
| ATOM | 366 | 2HG  | LYS+ | 17 | 0.676  | 15.258 | -3.590 | 1.00 | 0.00 |
| ATOM | 367 | QG   | LYS+ | 17 | 0.194  | 15.296 | -4.329 | 1.00 | 0.00 |
| ATOM | 368 | CD   | LYS+ | 17 | -0.604 | 16.956 | -3.720 | 1.00 | 0.00 |

(fortgesetzt)

Atomkooerdinaten der Struktur der ADAP hSH3-1-Domäne, oxidierte Form

| ATOM | 369 | 1HD | LYS+ | 17 | -1.029 | 17.042 | -2.731 | 1.00 | 0.00 |
|------|-----|-----|------|----|--------|--------|--------|------|------|
| ATOM | 370 | 2HD | LYS+ | 17 | -1.315 | 17.310 | -4.451 | 1.00 | 0.00 |
| ATOM | 371 | QD | LYS+ | 17 | -1.172 | 17.176 | -3.591 | 1.00 | 0.00 |
| ATOM | 372 | CE | LYS+ | 17 | 0.648 | 17.814 | -3.798 | 1.00 | 0.00 |
| ATOM | 373 | 1HE | LYS+ | 17 | 0.358 | 18.836 | -3.995 | 1.00 | 0.00 |
| ATOM | 374 | 2HE | LYS+ | 17 | 1.266 | 17.453 | -4.607 | 1.00 | 0.00 |
| ATOM | 375 | QE | LYS+ | 17 | 0.812 | 18.144 | -4.301 | 1.00 | 0.00 |
| ATOM | 376 | NZ | LYS+ | 17 | 1.432 | 17.768 | -2.533 | 1.00 | 0.00 |
| ATOM | 377 | 1HZ | LYS+ | 17 | 1.101 | 18.508 | -1.880 | 1.00 | 0.00 |
| ATOM | 378 | 2HZ | LYS+ | 17 | 2.441 | 17.924 | -2.731 | 1.00 | 0.00 |
| ATOM | 379 | 3HZ | LYS+ | 17 | 1.319 | 16.842 | -2.075 | 1.00 | 0.00 |
| ATOM | 380 | QZ | LYS+ | 17 | 1.620 | 17.758 | -2.229 | 1.00 | 0.00 |
| ATOM | 381 | C | LYS+ | 17 | -1.545 | 13.039 | -1.405 | 1.00 | 0.00 |
| ATOM | 382 | O | LYS+ | 17 | -1.651 | 13.856 | -0.491 | 1.00 | 0.00 |
| ATOM | 383 | N | LYS+ | 18 | -2.132 | 11.844 | -1.370 | 1.00 | 0.00 |
| ATOM | 384 | H | LYS+ | 18 | -2.023 | 11.228 | -2.123 | 1.00 | 0.00 |
| ATOM | 385 | CA | LYS+ | 18 | -2.944 | 11.431 | -0.237 | 1.00 | 0.00 |
| ATOM | 386 | HA | LYS+ | 18 | -3.236 | 12.314 | 0.300 | 1.00 | 0.00 |
| ATOM | 387 | CB | LYS+ | 18 | -4.201 | 10.722 | -0.728 | 1.00 | 0.00 |
| ATOM | 388 | 1HB | LYS+ | 18 | -4.014 | 9.669 | -0.741 | 1.00 | 0.00 |
| ATOM | 389 | 2HB | LYS+ | 18 | -4.418 | 11.049 | -1.731 | 1.00 | 0.00 |
| ATOM | 390 | QB | LYS+ | 18 | -4.216 | 10.359 | -1.236 | 1.00 | 0.00 |
| ATOM | 391 | CG | LYS+ | 18 | -5.426 | 10.980 | 0.131 | 1.00 | 0.00 |
| ATOM | 392 | 1HG | LYS+ | 18 | -5.196 | 11.756 | 0.846 | 1.00 | 0.00 |
| ATOM | 393 | 2HG | LYS+ | 18 | -5.681 | 10.072 | 0.649 | 1.00 | 0.00 |
| ATOM | 394 | QG | LYS+ | 18 | -5.439 | 10.914 | 0.748 | 1.00 | 0.00 |
| ATOM | 395 | CD | LYS+ | 18 | -6.616 | 11.416 | -0.709 | 1.00 | 0.00 |
| ATOM | 396 | 1HD | LYS+ | 18 | -6.360 | 12.326 | -1.232 | 1.00 | 0.00 |
| ATOM | 397 | 2HD | LYS+ | 18 | -7.457 | 11.598 | -0.057 | 1.00 | 0.00 |
| ATOM | 398 | QD | LYS+ | 18 | -6.908 | 11.962 | -0.645 | 1.00 | 0.00 |
| ATOM | 399 | CE | LYS+ | 18 | -7.002 | 10.352 | -1.726 | 1.00 | 0.00 |
| ATOM | 400 | 1HE | LYS+ | 18 | -6.233 | 9.592 | -1.744 | 1.00 | 0.00 |
| ATOM | 401 | 2HE | LYS+ | 18 | -7.075 | 10.813 | -2.699 | 1.00 | 0.00 |
| ATOM | 402 | QE | LYS+ | 18 | -6.654 | 10.203 | -2.222 | 1.00 | 0.00 |
| ATOM | 403 | NZ | LYS+ | 18 | -8.306 | 9.714 | -1.394 | 1.00 | 0.00 |
| ATOM | 404 | 1HZ | LYS+ | 18 | -8.502 | 9.809 | -0.377 | 1.00 | 0.00 |
| ATOM | 405 | 2HZ | LYS+ | 18 | -8.282 | 8.704 | -1.638 | 1.00 | 0.00 |
| ATOM | 406 | 3HZ | LYS+ | 18 | -9.073 | 10.170 | -1.929 | 1.00 | 0.00 |
| ATOM | 407 | QZ | LYS+ | 18 | -8.619 | 9.561 | -1.315 | 1.00 | 0.00 |
| ATOM | 408 | C | LYS+ | 18 | -2.158 | 10.522 | 0.706 | 1.00 | 0.00 |
| ATOM | 409 | O | LYS+ | 18 | -1.692 | 10.960 | 1.759 | 1.00 | 0.00 |
| ATOM | 410 | N | PHE | 19 | -2.030 | 9.253 | 0.333 | 1.00 | 0.00 |
| ATOM | 411 | H | PHE | 19 | -2.438 | 8.955 | -0.505 | 1.00 | 0.00 |
| ATOM | 412 | CA | PHE | 19 | -1.324 | 8.278 | 1.147 | 1.00 | 0.00 |
| ATOM | 413 | HA | PHE | 19 | -1.599 | 8.449 | 2.174 | 1.00 | 0.00 |
| ATOM | 414 | CB | PHE | 19 | -1.772 | 6.881 | 0.749 | 1.00 | 0.00 |
| ATOM | 415 | 1HB | PHE | 19 | -1.356 | 6.168 | 1.438 | 1.00 | 0.00 |
| ATOM | 416 | 2HB | PHE | 19 | -1.412 | 6.666 | -0.248 | 1.00 | 0.00 |
| ATOM | 417 | QB | PHE | 19 | -1.384 | 6.417 | 0.595 | 1.00 | 0.00 |
| ATOM | 418 | QD | PHE | 19 | -3.430 | 6.709 | 0.746 | 1.00 | 0.00 |

(fortgesetzt)

Atomkooerdinaten der Struktur der ADAP hSH3-1-Domäne, oxidierte Form

| ATOM | 419 | QE | PHE | 19 | -5.875 | 6.459 | 0.731 | 1.00 | 0.00 |
|------|-----|-----|-----|----|--------|-------|-------|------|------|
| ATOM | 420 | QR | PHE | 19 | -5.143 | 6.534 | 0.736 | 1.00 | 0.00 |
| ATOM | 421 | CG | PHE | 19 | -3.265 | 6.724 | 0.745 | 1.00 | 0.00 |
| ATOM | 422 | CD1 | PHE | 19 | -3.954 | 6.588 | -0.448 | 1.00 | 0.00 |
| ATOM | 423 | 1HD | PHE | 19 | -3.409 | 6.593 | -1.378 | 1.00 | 0.00 |
| ATOM | 424 | CE1 | PHE | 19 | -5.328 | 6.449 | -0.459 | 1.00 | 0.00 |
| ATOM | 425 | 1HE | PHE | 19 | -5.852 | 6.342 | -1.399 | 1.00 | 0.00 |
| ATOM | 426 | CZ | PHE | 19 | -6.029 | 6.444 | 0.731 | 1.00 | 0.00 |
| ATOM | 427 | HZ | PHE | 19 | -7.104 | 6.335 | 0.725 | 1.00 | 0.00 |
| ATOM | 428 | CE2 | PHE | 19 | -5.353 | 6.580 | 1.929 | 1.00 | 0.00 |
| ATOM | 429 | 2HE | PHE | 19 | -5.899 | 6.577 | 2.861 | 1.00 | 0.00 |
| ATOM | 430 | CD2 | PHE | 19 | -3.979 | 6.719 | 1.933 | 1.00 | 0.00 |
| ATOM | 431 | 2HD | PHE | 19 | -3.451 | 6.826 | 2.869 | 1.00 | 0.00 |
| ATOM | 432 | C | PHE | 19 | 0.194 | 8.389 | 1.016 | 1.00 | 0.00 |
| ATOM | 433 | O | PHE | 19 | 0.930 | 7.683 | 1.706 | 1.00 | 0.00 |
| ATOM | 434 | N | LYS+ | 20 | 0.666 | 9.259 | 0.132 | 1.00 | 0.00 |
| ATOM | 435 | H | LYS+ | 20 | 0.043 | 9.793 | -0.402 | 1.00 | 0.00 |
| ATOM | 436 | CA | LYS+ | 20 | 2.100 | 9.421 | -0.066 | 1.00 | 0.00 |
| ATOM | 437 | HA | LYS+ | 20 | 2.249 | 10.201 | -0.792 | 1.00 | 0.00 |
| ATOM | 438 | CB | LYS+ | 20 | 2.779 | 9.826 | 1.292 | 1.00 | 0.00 |
| ATOM | 439 | 1HB | LYS+ | 20 | 3.522 | 9.086 | 1.494 | 1.00 | 0.00 |
| ATOM | 440 | 2HB | LYS+ | 20 | 2.035 | 9.858 | 2.022 | 1.00 | 0.00 |
| ATOM | 441 | QB | LYS+ | 20 | 2.779 | 9.472 | 1.758 | 1.00 | 0.00 |
| ATOM | 442 | CG | LYS+ | 20 | 3.462 | 11.182 | 1.178 | 1.00 | 0.00 |
| ATOM | 443 | 1HG | LYS+ | 20 | 2.729 | 11.953 | 1.369 | 1.00 | 0.00 |
| ATOM | 444 | 2HG | LYS+ | 20 | 3.881 | 11.318 | 0.193 | 1.00 | 0.00 |
| ATOM | 445 | QG | LYS+ | 20 | 3.305 | 11.636 | 0.781 | 1.00 | 0.00 |
| ATOM | 446 | CD | LYS+ | 20 | 4.576 | 11.294 | 2.207 | 1.00 | 0.00 |
| ATOM | 447 | 1HD | LYS+ | 20 | 5.522 | 11.104 | 1.720 | 1.00 | 0.00 |
| ATOM | 448 | 2HD | LYS+ | 20 | 4.413 | 10.559 | 2.982 | 1.00 | 0.00 |
| ATOM | 449 | QD | LYS+ | 20 | 4.968 | 10.831 | 2.351 | 1.00 | 0.00 |
| ATOM | 450 | CE | LYS+ | 20 | 4.614 | 12.675 | 2.839 | 1.00 | 0.00 |
| ATOM | 451 | 1HE | LYS+ | 20 | 5.338 | 12.671 | 3.641 | 1.00 | 0.00 |
| ATOM | 452 | 2HE | LYS+ | 20 | 3.637 | 12.901 | 3.240 | 1.00 | 0.00 |
| ATOM | 453 | QE | LYS+ | 20 | 4.488 | 12.786 | 3.441 | 1.00 | 0.00 |
| ATOM | 454 | NZ | LYS+ | 20 | 4.988 | 13.728 | 1.854 | 1.00 | 0.00 |
| ATOM | 455 | 1HZ | LYS+ | 20 | 4.791 | 13.399 | 0.887 | 1.00 | 0.00 |
| ATOM | 456 | 2HZ | LYS+ | 20 | 6.000 | 13.950 | 1.934 | 1.00 | 0.00 |
| ATOM | 457 | 3HZ | LYS+ | 20 | 4.440 | 14.595 | 2.032 | 1.00 | 0.00 |
| ATOM | 458 | QZ | LYS+ | 20 | 5.077 | 13.981 | 1.618 | 1.00 | 0.00 |
| ATOM | 459 | C | LYS+ | 20 | 2.711 | 8.128 | -0.599 | 1.00 | 0.00 |
| ATOM | 460 | O | LYS+ | 20 | 3.054 | 7.226 | 0.165 | 1.00 | 0.00 |
| ATOM | 461 | N | LEU | 21 | 2.825 | 8.047 | -1.918 | 1.00 | 0.00 |
| ATOM | 462 | H | LEU | 21 | 2.515 | 8.797 | -2.463 | 1.00 | 0.00 |
| ATOM | 463 | CA | LEU | 21 | 3.372 | 6.868 | -2.585 | 1.00 | 0.00 |
| ATOM | 464 | HA | LEU | 21 | 3.048 | 6.009 | -2.027 | 1.00 | 0.00 |
| ATOM | 465 | CB | LEU | 21 | 2.829 | 6.766 | -4.011 | 1.00 | 0.00 |
| ATOM | 466 | 1HB | LEU | 21 | 3.525 | 6.185 | -4.598 | 1.00 | 0.00 |
| ATOM | 467 | 2HB | LEU | 21 | 2.778 | 7.762 | -4.424 | 1.00 | 0.00 |
| ATOM | 468 | QB | LEU | 21 | 3.151 | 6.974 | -4.511 | 1.00 | 0.00 |

(fortgesetzt)

Atomkooerdinaten der Struktur der ADAP hSH3-1-Domäne, oxidierte Form

| ATOM | 469 | CG | LEU | 21 | 1.447 | 6.123 | -4.133 | 1.00 | 0.00 |
|------|-----|------|-----|----|--------|--------|--------|------|------|
| ATOM | 470 | HG | LEU | 21 | 1.448 | 5.181 | -3.604 | 1.00 | 0.00 |
| ATOM | 471 | QD1 | LEU | 21 | 0.132 | 7.224 | -3.358 | 1.00 | 0.00 |
| ATOM | 472 | QD2 | LEU | 21 | 1.043 | 5.778 | -5.938 | 1.00 | 0.00 |
| ATOM | 473 | CD1 | LEU | 21 | 0.385 | 7.014 | -3.506 | 1.00 | 0.00 |
| ATOM | 474 | 1HD1 | LEU | 21 | 0.857 | 7.835 | -2.992 | 1.00 | 0.00 |
| ATOM | 475 | 2HD1 | LEU | 21 | -0.197 | 6.437 | -2.802 | 1.00 | 0.00 |
| ATOM | 476 | 3HD1 | LEU | 21 | -0.264 | 7.399 | -4.279 | 1.00 | 0.00 |
| ATOM | 477 | CD2 | LEU | 21 | 1.120 | 5.844 | -5.592 | 1.00 | 0.00 |
| ATOM | 478 | 1HD2 | LEU | 21 | 0.524 | 6.652 | -5.989 | 1.00 | 0.00 |
| ATOM | 479 | 2HD2 | LEU | 21 | 0.568 | 4.919 | -5.666 | 1.00 | 0.00 |
| ATOM | 480 | 3HD2 | LEU | 21 | 2.037 | 5.762 | -6.158 | 1.00 | 0.00 |
| ATOM | 481 | QQD | LEU | 21 | 0.587 | 6.501 | -4.648 | 1.00 | 0.00 |
| ATOM | 482 | C | LEU | 21 | 4.897 | 6.868 | -2.608 | 1.00 | 0.00 |
| ATOM | 483 | O | LEU | 21 | 5.509 | 6.212 | -3.451 | 1.00 | 0.00 |
| ATOM | 484 | N | THR | 22 | 5.500 | 7.603 | -1.683 | 1.00 | 0.00 |
| ATOM | 485 | H | THR | 22 | 4.950 | 8.094 | -1.045 | 1.00 | 0.00 |
| ATOM | 486 | CA | THR | 22 | 6.959 | 7.698 | -1.587 | 1.00 | 0.00 |
| ATOM | 487 | HA | THR | 22 | 7.289 | 8.436 | -2.301 | 1.00 | 0.00 |
| ATOM | 488 | CB | THR | 22 | 7.367 | 8.152 | -0.185 | 1.00 | 0.00 |
| ATOM | 489 | HB | THR | 22 | 8.416 | 7.938 | -0.039 | 1.00 | 0.00 |
| ATOM | 490 | QG2 | THR | 22 | 7.109 | 9.984 | 0.105 | 1.00 | 0.00 |
| ATOM | 491 | OG1 | THR | 22 | 6.630 | 7.453 | 0.803 | 1.00 | 0.00 |
| ATOM | 492 | 1HG | THR | 22 | 5.709 | 7.723 | 0.767 | 1.00 | 0.00 |
| ATOM | 493 | CG2 | THR | 22 | 7.159 | 9.632 | 0.049 | 1.00 | 0.00 |
| ATOM | 494 | 1HG2 | THR | 22 | 7.866 | 10.193 | -0.543 | 1.00 | 0.00 |
| ATOM | 495 | 2HG2 | THR | 22 | 7.308 | 9.856 | 1.095 | 1.00 | 0.00 |
| ATOM | 496 | 3HG2 | THR | 22 | 6.153 | 9.904 | -0.237 | 1.00 | 0.00 |
| ATOM | 497 | C | THR | 22 | 7.632 | 6.363 | -1.914 | 1.00 | 0.00 |
| ATOM | 498 | O | THR | 22 | 8.712 | 6.332 | -2.506 | 1.00 | 0.00 |
| ATOM | 499 | N | GLY | 23 | 6.984 | 5.266 | -1.534 | 1.00 | 0.00 |
| ATOM | 500 | H | GLY | 23 | 6.124 | 5.350 | -1.072 | 1.00 | 0.00 |
| ATOM | 501 | CA | GLY | 23 | 7.533 | 3.950 | -1.806 | 1.00 | 0.00 |
| ATOM | 502 | 1HA | GLY | 23 | 6.929 | 3.207 | -1.305 | 1.00 | 0.00 |
| ATOM | 503 | 2HA | GLY | 23 | 8.540 | 3.903 | -1.419 | 1.00 | 0.00 |
| ATOM | 504 | QA | GLY | 23 | 7.734 | 3.555 | -1.362 | 1.00 | 0.00 |
| ATOM | 505 | C | GLY | 23 | 7.562 | 3.638 | -3.292 | 1.00 | 0.00 |
| ATOM | 506 | O | GLY | 23 | 7.454 | 4.545 | -4.117 | 1.00 | 0.00 |
| ATOM | 507 | N | PRO | 24 | 7.706 | 2.357 | -3.669 | 1.00 | 0.00 |
| ATOM | 508 | CD | PRO | 24 | 7.843 | 1.202 | -2.760 | 1.00 | 0.00 |
| ATOM | 509 | CA | PRO | 24 | 7.745 | 1.951 | -5.072 | 1.00 | 0.00 |
| ATOM | 510 | HA | PRO | 24 | 8.319 | 2.643 | -5.671 | 1.00 | 0.00 |
| ATOM | 511 | CB | PRO | 24 | 8.462 | 0.604 | -5.010 | 1.00 | 0.00 |
| ATOM | 512 | 1HB | PRO | 24 | 9.529 | 0.757 | -5.076 | 1.00 | 0.00 |
| ATOM | 513 | 2HB | PRO | 24 | 8.131 | -0.021 | -5.826 | 1.00 | 0.00 |
| ATOM | 514 | QB | PRO | 24 | 8.830 | 0.368 | -5.451 | 1.00 | 0.00 |
| ATOM | 515 | CG | PRO | 24 | 8.076 | 0.032 | -3.687 | 1.00 | 0.00 |
| ATOM | 516 | 1HG | PRO | 24 | 8.875 | -0.589 | -3.310 | 1.00 | 0.00 |
| ATOM | 517 | 2HG | PRO | 24 | 7.171 | -0.550 | -3.788 | 1.00 | 0.00 |
| ATOM | 518 | QG | PRO | 24 | 8.023 | -0.570 | -3.549 | 1.00 | 0.00 |

(fortgesetzt)

Atomkooerdinaten der Struktur der ADAP hSH3-1-Domäne, oxidierte Form

| ATOM | 519 | 1HD | PRO | 24 | 8.687 | 1.331 | -2.099 | 1.00 | 0.00 |
|------|-----|------|-----|----|-------|-------|--------|------|------|
| ATOM | 520 | 2HD | PRO | 24 | 6.938 | 1.055 | -2.189 | 1.00 | 0.00 |
| ATOM | 521 | QD | PRO | 24 | 7.813 | 1.193 | -2.144 | 1.00 | 0.00 |
| ATOM | 522 | C | PRO | 24 | 6.349 | 1.795 | -5.667 | 1.00 | 0.00 |
| ATOM | 523 | O | PRO | 24 | 5.359 | 1.722 | -4.940 | 1.00 | 0.00 |
| ATOM | 524 | N | ILE | 25 | 6.274 | 1.743 | -6.994 | 1.00 | 0.00 |
| ATOM | 525 | H | ILE | 25 | 7.091 | 1.804 | -7.521 | 1.00 | 0.00 |
| ATOM | 526 | CA | ILE | 25 | 5.002 | 1.594 | -7.682 | 1.00 | 0.00 |
| ATOM | 527 | HA | ILE | 25 | 4.240 | 1.486 | -6.930 | 1.00 | 0.00 |
| ATOM | 528 | CB | ILE | 25 | 4.668 | 2.852 | -8.524 | 1.00 | 0.00 |
| ATOM | 529 | HB | ILE | 25 | 5.228 | 3.680 | -8.117 | 1.00 | 0.00 |
| ATOM | 530 | QG2 | ILE | 25 | 5.177 | 2.641 | -10.324 | 1.00 | 0.00 |
| ATOM | 531 | CG2 | ILE | 25 | 5.081 | 2.682 | -9.980 | 1.00 | 0.00 |
| ATOM | 532 | 1HG2 | ILE | 25 | 5.983 | 2.090 | -10.033 | 1.00 | 0.00 |
| ATOM | 533 | 2HG2 | ILE | 25 | 5.260 | 3.652 | -10.418 | 1.00 | 0.00 |
| ATOM | 534 | 3HG2 | ILE | 25 | 4.290 | 2.183 | -10.520 | 1.00 | 0.00 |
| ATOM | 535 | CG1 | ILE | 25 | 3.177 | 3.173 | -8.425 | 1.00 | 0.00 |
| ATOM | 536 | 1HG1 | ILE | 25 | 2.611 | 2.329 | -8.780 | 1.00 | 0.00 |
| ATOM | 537 | 2HG1 | ILE | 25 | 2.959 | 4.031 | -9.043 | 1.00 | 0.00 |
| ATOM | 538 | QG1 | ILE | 25 | 2.785 | 3.180 | -8.911 | 1.00 | 0.00 |
| ATOM | 539 | QD1 | ILE | 25 | 2.601 | 3.558 | -6.684 | 1.00 | 0.00 |
| ATOM | 540 | CD1 | ILE | 25 | 2.712 | 3.484 | -7.019 | 1.00 | 0.00 |
| ATOM | 541 | 1HD1 | ILE | 25 | 2.664 | 2.570 | -6.445 | 1.00 | 0.00 |
| ATOM | 542 | 2HD1 | ILE | 25 | 1.732 | 3.937 | -7.055 | 1.00 | 0.00 |
| ATOM | 543 | 3HD1 | ILE | 25 | 3.407 | 4.166 | -6.552 | 1.00 | 0.00 |
| ATOM | 544 | C | ILE | 25 | 4.999 | 0.339 | -8.557 | 1.00 | 0.00 |
| ATOM | 545 | O | ILE | 25 | 5.929 | -0.466 | -8.501 | 1.00 | 0.00 |
| ATOM | 546 | N | GLN | 26 | 3.949 | 0.171 | -9.357 | 1.00 | 0.00 |
| ATOM | 547 | H | GLN | 26 | 3.236 | 0.838 | -9.355 | 1.00 | 0.00 |
| ATOM | 548 | CA | GLN | 26 | 3.829 | -0.992 | -10.229 | 1.00 | 0.00 |
| ATOM | 549 | HA | GLN | 26 | 2.909 | -0.892 | -10.786 | 1.00 | 0.00 |
| ATOM | 550 | CB | GLN | 26 | 5.003 | -1.053 | -11.210 | 1.00 | 0.00 |
| ATOM | 551 | 1HB | GLN | 26 | 5.073 | -2.054 | -11.607 | 1.00 | 0.00 |
| ATOM | 552 | 2HB | GLN | 26 | 5.914 | -0.821 | -10.678 | 1.00 | 0.00 |
| ATOM | 553 | QB | GLN | 26 | 5.493 | -1.438 | -11.142 | 1.00 | 0.00 |
| ATOM | 554 | CG | GLN | 26 | 4.873 | -0.086 | -12.376 | 1.00 | 0.00 |
| ATOM | 555 | 1HG | GLN | 26 | 5.797 | -0.094 | -12.936 | 1.00 | 0.00 |
| ATOM | 556 | 2HG | GLN | 26 | 4.697 | 0.906 | -11.987 | 1.00 | 0.00 |
| ATOM | 557 | QG | GLN | 26 | 5.247 | 0.406 | -12.462 | 1.00 | 0.00 |
| ATOM | 558 | CD | GLN | 26 | 3.736 | -0.448 | -13.310 | 1.00 | 0.00 |
| ATOM | 559 | OE1 | GLN | 26 | 2.706 | 0.226 | -13.342 | 1.00 | 0.00 |
| ATOM | 560 | NE2 | GLN | 26 | 3.917 | -1.517 | -14.076 | 1.00 | 0.00 |
| ATOM | 561 | 1HE2 | GLN | 26 | 4.763 | -2.005 | -13.997 | 1.00 | 0.00 |
| ATOM | 562 | 2HE2 | GLN | 26 | 3.198 | -1.774 | -14.690 | 1.00 | 0.00 |
| ATOM | 563 | QE2 | GLN | 26 | 3.981 | -1.890 | -14.344 | 1.00 | 0.00 |
| ATOM | 564 | C | GLN | 26 | 3.767 | -2.273 | -9.405 | 1.00 | 0.00 |
| ATOM | 565 | O | GLN | 26 | 4.409 | -3.270 | -9.734 | 1.00 | 0.00 |
| ATOM | 566 | N | VAL | 27 | 2.991 | -2.233 | -8.326 | 1.00 | 0.00 |
| ATOM | 567 | H | VAL | 27 | 2.509 | -1.405 | -8.117 | 1.00 | 0.00 |
| ATOM | 568 | CA | VAL | 27 | 2.843 | -3.381 | -7.441 | 1.00 | 0.00 |

(fortgesetzt)

Atomkooerdinaten der Struktur der ADAP hSH3-1-Domäne, oxidierte Form

| ATOM | 569 | HA | VAL | 27 | 2.136 | -3.113 | -6.669 | 1.00 | 0.00 |
|------|-----|-----|------|----|-------|--------|--------|------|------|
| ATOM | 570 | CB | VAL | 27 | 2.300 | -4.611 | -8.187 | 1.00 | 0.00 |
| ATOM | 571 | HB | VAL | 27 | 3.065 | -4.967 | -8.861 | 1.00 | 0.00 |
| ATOM | 572 | QG1 | VAL | 27 | 1.901 | -5.988 | -6.967 | 1.00 | 0.00 |
| ATOM | 573 | QG2 | VAL | 27 | 0.784 | -4.151 | -9.200 | 1.00 | 0.00 |
| ATOM | 574 | CG1 | VAL | 27 | 1.977 | -5.724 | -7.201 | 1.00 | 0.00 |
| ATOM | 575 | 1HG1 | VAL | 27 | 2.776 | -6.451 | -7.205 | 1.00 | 0.00 |
| ATOM | 576 | 2HG1 | VAL | 27 | 1.053 | -6.204 | -7.488 | 1.00 | 0.00 |
| ATOM | 577 | 3HG1 | VAL | 27 | 1.875 | -5.308 | -6.208 | 1.00 | 0.00 |
| ATOM | 578 | CG2 | VAL | 27 | 1.074 | -4.239 | -9.007 | 1.00 | 0.00 |
| ATOM | 579 | 1HG2 | VAL | 27 | 0.481 | -3.519 | -8.462 | 1.00 | 0.00 |
| ATOM | 580 | 2HG2 | VAL | 27 | 0.483 | -5.123 | -9.193 | 1.00 | 0.00 |
| ATOM | 581 | 3HG2 | VAL | 27 | 1.387 | -3.810 | -9.946 | 1.00 | 0.00 |
| ATOM | 582 | QQG | VAL | 27 | 1.343 | -5.069 | -8.084 | 1.00 | 0.00 |
| ATOM | 583 | C | VAL | 27 | 4.170 | -3.740 | -6.788 | 1.00 | 0.00 |
| ATOM | 584 | O | VAL | 27 | 5.194 | -3.863 | -7.460 | 1.00 | 0.00 |
| ATOM | 585 | N | ILE | 28 | 4.148 | -3.895 | -5.470 | 1.00 | 0.00 |
| ATOM | 586 | H | ILE | 28 | 3.303 | -3.774 | -4.989 | 1.00 | 0.00 |
| ATOM | 587 | CA | ILE | 28 | 5.353 | -4.228 | -4.717 | 1.00 | 0.00 |
| ATOM | 588 | HA | ILE | 28 | 6.166 | -4.351 | -5.415 | 1.00 | 0.00 |
| ATOM | 589 | CB | ILE | 28 | 5.713 | -3.100 | -3.733 | 1.00 | 0.00 |
| ATOM | 590 | HB | ILE | 28 | 5.280 | -3.342 | -2.773 | 1.00 | 0.00 |
| ATOM | 591 | QG2 | ILE | 28 | 7.579 | -2.987 | -3.522 | 1.00 | 0.00 |
| ATOM | 592 | CG2 | ILE | 28 | 7.223 | -3.009 | -3.563 | 1.00 | 0.00 |
| ATOM | 593 | 1HG2 | ILE | 28 | 7.449 | -2.535 | -2.620 | 1.00 | 0.00 |
| ATOM | 594 | 2HG2 | ILE | 28 | 7.641 | -2.426 | -4.369 | 1.00 | 0.00 |
| ATOM | 595 | 3HG2 | ILE | 28 | 7.647 | -4.001 | -3.578 | 1.00 | 0.00 |
| ATOM | 596 | CG1 | ILE | 28 | 5.139 | -1.766 | -4.219 | 1.00 | 0.00 |
| ATOM | 597 | 1HG1 | ILE | 28 | 5.448 | -0.983 | -3.546 | 1.00 | 0.00 |
| ATOM | 598 | 2HG1 | ILE | 28 | 4.058 | -1.825 | -4.223 | 1.00 | 0.00 |
| ATOM | 599 | QG1 | ILE | 28 | 4.753 | -1.404 | -3.884 | 1.00 | 0.00 |
| ATOM | 600 | QD1 | ILE | 28 | 5.693 | -1.302 | -5.947 | 1.00 | 0.00 |
| ATOM | 601 | CD1 | ILE | 28 | 5.587 | -1.390 | -5.614 | 1.00 | 0.00 |
| ATOM | 602 | 1HD1 | ILE | 28 | 4.738 | -1.041 | -6.183 | 1.00 | 0.00 |
| ATOM | 603 | 2HD1 | ILE | 28 | 6.016 | -2.254 | -6.100 | 1.00 | 0.00 |
| ATOM | 604 | 3HD1 | ILE | 28 | 6.326 | -0.610 | -5.556 | 1.00 | 0.00 |
| ATOM | 605 | C | ILE | 28 | 5.163 | -5.515 | -3.931 | 1.00 | 0.00 |
| ATOM | 606 | O | ILE | 28 | 6.006 | -6.411 | -3.959 | 1.00 | 0.00 |
| ATOM | 607 | N | HIS+ | 29 | 4.039 | -5.593 | -3.237 | 1.00 | 0.00 |
| ATOM | 608 | H | HIS+ | 29 | 3.412 | -4.839 | -3.269 | 1.00 | 0.00 |
| ATOM | 609 | CA | HIS+ | 29 | 3.703 | -6.766 | -2.440 | 1.00 | 0.00 |
| ATOM | 610 | HA | HIS+ | 29 | 4.394 | -7.555 | -2.692 | 1.00 | 0.00 |
| ATOM | 611 | CB | HIS+ | 29 | 3.815 | -6.449 | -0.949 | 1.00 | 0.00 |
| ATOM | 612 | 1HB | HIS+ | 29 | 3.424 | -7.279 | -0.380 | 1.00 | 0.00 |
| ATOM | 613 | 2HB | HIS+ | 29 | 3.229 | -5.569 | -0.732 | 1.00 | 0.00 |
| ATOM | 614 | QB | HIS+ | 29 | 3.327 | -6.424 | -0.556 | 1.00 | 0.00 |
| ATOM | 615 | CG | HIS+ | 29 | 5.219 | -6.196 | -0.494 | 1.00 | 0.00 |
| ATOM | 616 | ND1 | HIS+ | 29 | 6.125 | -7.208 | -0.253 | 1.00 | 0.00 |
| ATOM | 617 | CD2 | HIS+ | 29 | 5.874 | -5.039 | -0.240 | 1.00 | 0.00 |
| ATOM | 618 | 1HD | HIS+ | 29 | 5.951 | -8.167 | -0.348 | 1.00 | 0.00 |

(fortgesetzt)

Atomkooerdinaten der Struktur der ADAP hSH3-1-Domäne, oxidierte Form

| ATOM | 619 | CE1 | HIS+ | 29 | 7.275 | -6.683 | 0.132 | 1.00 | 0.00 |
|------|-----|-----|------|----|-------|--------|-------|------|------|
| ATOM | 620 | NE2 | HIS+ | 29 | 7.148 | -5.369 | 0.148 | 1.00 | 0.00 |
| ATOM | 621 | 2HD | HIS+ | 29 | 5.468 | -4.041 | -0.326 | 1.00 | 0.00 |
| ATOM | 622 | 1HE | HIS+ | 29 | 8.167 | -7.235 | 0.389 | 1.00 | 0.00 |
| ATOM | 623 | 2HE | HIS+ | 29 | 7.877 | -4.734 | 0.310 | 1.00 | 0.00 |
| ATOM | 624 | C | HIS+ | 29 | 2.291 | -7.219 | -2.759 | 1.00 | 0.00 |
| ATOM | 625 | O | HIS+ | 29 | 1.339 | -6.466 | -2.574 | 1.00 | 0.00 |
| ATOM | 626 | N | LEU | 30 | 2.155 | -8.447 | -3.239 | 1.00 | 0.00 |
| ATOM | 627 | H | LEU | 30 | 2.950 | -9.006 | -3.369 | 1.00 | 0.00 |
| ATOM | 628 | CA | LEU | 30 | 0.844 | -8.975 | -3.583 | 1.00 | 0.00 |
| ATOM | 629 | HA | LEU | 30 | 0.170 | -8.134 | -3.677 | 1.00 | 0.00 |
| ATOM | 630 | CB | LEU | 30 | 0.893 | -9.699 | -4.922 | 1.00 | 0.00 |
| ATOM | 631 | 1HB | LEU | 30 | 0.420 | -10.663 | -4.813 | 1.00 | 0.00 |
| ATOM | 632 | 2HB | LEU | 30 | 1.928 | -9.846 | -5.197 | 1.00 | 0.00 |
| ATOM | 633 | QB | LEU | 30 | 1.179 | -10.255 | -5.005 | 1.00 | 0.00 |
| ATOM | 634 | CG | LEU | 30 | 0.191 | -8.943 | -6.041 | 1.00 | 0.00 |
| ATOM | 635 | HG | LEU | 30 | 0.327 | -7.882 | -5.875 | 1.00 | 0.00 |
| ATOM | 636 | QD1 | LEU | 30 | 0.927 | -9.382 | -7.715 | 1.00 | 0.00 |
| ATOM | 637 | QD2 | LEU | 30 | -1.652 | -9.297 | -6.000 | 1.00 | 0.00 |
| ATOM | 638 | CD1 | LEU | 30 | 0.786 | -9.298 | -7.395 | 1.00 | 0.00 |
| ATOM | 639 | 1HD1 | LEU | 30 | 0.042 | -9.155 | -8.164 | 1.00 | 0.00 |
| ATOM | 640 | 2HD1 | LEU | 30 | 1.105 | -10.330 | -7.388 | 1.00 | 0.00 |
| ATOM | 641 | 3HD1 | LEU | 30 | 1.636 | -8.660 | -7.593 | 1.00 | 0.00 |
| ATOM | 642 | CD2 | LEU | 30 | -1.300 | -9.230 | -6.009 | 1.00 | 0.00 |
| ATOM | 643 | 1HD2 | LEU | 30 | -1.804 | -8.595 | -6.722 | 1.00 | 0.00 |
| ATOM | 644 | 2HD2 | LEU | 30 | -1.678 | -9.031 | -5.018 | 1.00 | 0.00 |
| ATOM | 645 | 3HD2 | LEU | 30 | -1.474 | -10.265 | -6.261 | 1.00 | 0.00 |
| ATOM | 646 | QQD | LEU | 30 | -0.362 | -9.339 | -6.858 | 1.00 | 0.00 |
| ATOM | 647 | C | LEU | 30 | 0.312 | -9.886 | -2.493 | 1.00 | 0.00 |
| ATOM | 648 | O | LEU | 30 | 0.808 | -10.993 | -2.287 | 1.00 | 0.00 |
| ATOM | 649 | N | ALA | 31 | -0.710 | -9.399 | -1.807 | 1.00 | 0.00 |
| ATOM | 650 | H | ALA | 31 | -1.055 | -8.520 | -2.032 | 1.00 | 0.00 |
| ATOM | 651 | CA | ALA | 31 | -1.343 | -10.128 | -0.737 | 1.00 | 0.00 |
| ATOM | 652 | HA | ALA | 31 | -0.830 | -11.066 | -0.624 | 1.00 | 0.00 |
| ATOM | 653 | QB | ALA | 31 | -1.198 | -9.178 | 0.884 | 1.00 | 0.00 |
| ATOM | 654 | CB | ALA | 31 | -1.226 | -9.359 | 0.573 | 1.00 | 0.00 |
| ATOM | 655 | 1HB | ALA | 31 | -0.678 | -8.445 | 0.406 | 1.00 | 0.00 |
| ATOM | 656 | 2HB | ALA | 31 | -0.704 | -9.965 | 1.301 | 1.00 | 0.00 |
| ATOM | 657 | 3HB | ALA | 31 | -2.213 | -9.124 | 0.944 | 1.00 | 0.00 |
| ATOM | 658 | C | ALA | 31 | -2.803 | -10.388 | -1.069 | 1.00 | 0.00 |
| ATOM | 659 | O | ALA | 31 | -3.262 | -10.131 | -2.181 | 1.00 | 0.00 |
| ATOM | 660 | N | LYS+ | 32 | -3.520 | -10.886 | -0.086 | 1.00 | 0.00 |
| ATOM | 661 | H | LYS+ | 32 | -3.087 | -11.046 | 0.763 | 1.00 | 0.00 |
| ATOM | 662 | CA | LYS+ | 32 | -4.935 | -11.181 | -0.229 | 1.00 | 0.00 |
| ATOM | 663 | HA | LYS+ | 32 | -5.255 | -10.785 | -1.175 | 1.00 | 0.00 |
| ATOM | 664 | CB | LYS+ | 32 | -5.184 | -12.692 | -0.204 | 1.00 | 0.00 |
| ATOM | 665 | 1HB | LYS+ | 32 | -6.115 | -12.899 | -0.714 | 1.00 | 0.00 |
| ATOM | 666 | 2HB | LYS+ | 32 | -5.270 | -13.014 | 0.822 | 1.00 | 0.00 |
| ATOM | 667 | QB | LYS+ | 32 | -5.692 | -12.957 | 0.054 | 1.00 | 0.00 |
| ATOM | 668 | CG | LYS+ | 32 | -4.086 | -13.509 | -0.869 | 1.00 | 0.00 |

(fortgesetzt)

Atomkooerdinaten der Struktur der ADAP hSH3-1-Domäne, oxidierte Form

| ATOM | 669 | 1HG | LYS+ | 32 | -4.197 | -14.544 | -0.581 | 1.00 | 0.00 |
|------|-----|-----|------|----|--------|---------|--------|------|------|
| ATOM | 670 | 2HG | LYS+ | 32 | -3.126 | -13.142 | -0.538 | 1.00 | 0.00 |
| ATOM | 671 | QG | LYS+ | 32 | -3.662 | -13.843 | -0.559 | 1.00 | 0.00 |
| ATOM | 672 | CD | LYS+ | 32 | -4.156 | -13.408 | -2.385 | 1.00 | 0.00 |
| ATOM | 673 | 1HD | LYS+ | 32 | -3.545 | -12.578 | -2.708 | 1.00 | 0.00 |
| ATOM | 674 | 2HD | LYS+ | 32 | -5.181 | -13.239 | -2.677 | 1.00 | 0.00 |
| ATOM | 675 | QD | LYS+ | 32 | -4.363 | -12.909 | -2.693 | 1.00 | 0.00 |
| ATOM | 676 | CE | LYS+ | 32 | -3.656 | -14.680 | -3.050 | 1.00 | 0.00 |
| ATOM | 677 | 1HE | LYS+ | 32 | -4.220 | -15.518 | -2.666 | 1.00 | 0.00 |
| ATOM | 678 | 2HE | LYS+ | 32 | -2.611 | -14.810 | -2.810 | 1.00 | 0.00 |
| ATOM | 679 | QE | LYS+ | 32 | -3.415 | -15.164 | -2.738 | 1.00 | 0.00 |
| ATOM | 680 | NZ | LYS+ | 32 | -3.809 | -14.630 | -4.530 | 1.00 | 0.00 |
| ATOM | 681 | 1HZ | LYS+ | 32 | -3.175 | -13.910 | -4.931 | 1.00 | 0.00 |
| ATOM | 682 | 2HZ | LYS+ | 32 | -4.789 | -14.389 | -4.780 | 1.00 | 0.00 |
| ATOM | 683 | 3HZ | LYS+ | 32 | -3.575 | -15.554 | -4.945 | 1.00 | 0.00 |
| ATOM | 684 | QZ | LYS+ | 32 | -3.846 | -14.618 | -4.886 | 1.00 | 0.00 |
| ATOM | 685 | C | LYS+ | 32 | -5.724 | -10.502 | 0.882 | 1.00 | 0.00 |
| ATOM | 686 | O | LYS+ | 32 | -5.660 | -10.913 | 2.041 | 1.00 | 0.00 |
| ATOM | 687 | N | ALA | 33 | -6.454 | -9.450 | 0.520 | 1.00 | 0.00 |
| ATOM | 688 | H | ALA | 33 | -6.449 | -9.171 | -0.418 | 1.00 | 0.00 |
| ATOM | 689 | CA | ALA | 33 | -7.253 | -8.690 | 1.481 | 1.00 | 0.00 |
| ATOM | 690 | HA | ALA | 33 | -6.587 | -8.004 | 1.988 | 1.00 | 0.00 |
| ATOM | 691 | QB | ALA | 33 | -8.553 | -7.676 | 0.579 | 1.00 | 0.00 |
| ATOM | 692 | CB | ALA | 33 | -8.305 | -7.871 | 0.753 | 1.00 | 0.00 |
| ATOM | 693 | 1HB | ALA | 33 | -9.195 | -7.804 | 1.360 | 1.00 | 0.00 |
| ATOM | 694 | 2HB | ALA | 33 | -8.544 | -8.346 | -0.187 | 1.00 | 0.00 |
| ATOM | 695 | 3HB | ALA | 33 | -7.921 | -6.879 | 0.564 | 1.00 | 0.00 |
| ATOM | 696 | C | ALA | 33 | -7.903 | -9.599 | 2.521 | 1.00 | 0.00 |
| ATOM | 697 | O | ALA | 33 | -8.544 | -10.594 | 2.180 | 1.00 | 0.00 |
| ATOM | 698 | N | CYS | 34 | -7.708 | -9.261 | 3.789 | 1.00 | 0.00 |
| ATOM | 699 | H | CYS | 34 | -7.170 | -8.470 | 3.993 | 1.00 | 0.00 |
| ATOM | 700 | CA | CYS | 34 | -8.246 | -10.054 | 4.890 | 1.00 | 0.00 |
| ATOM | 701 | HA | CYS | 34 | -8.000 | -11.086 | 4.704 | 1.00 | 0.00 |
| ATOM | 702 | CB | CYS | 34 | -7.625 | -9.638 | 6.241 | 1.00 | 0.00 |
| ATOM | 703 | 1HB | CYS | 34 | -6.657 | -9.196 | 6.056 | 1.00 | 0.00 |
| ATOM | 704 | 2HB | CYS | 34 | -7.496 | -10.519 | 6.850 | 1.00 | 0.00 |
| ATOM | 705 | QB | CYS | 34 | -7.076 | -9.857 | 6.453 | 1.00 | 0.00 |
| ATOM | 706 | SG | CYS | 34 | -8.613 | -8.422 | 7.236 | 1.00 | 0.00 |
| ATOM | 707 | C | CYS | 34 | -9.739 | -9.926 | 5.037 | 1.00 | 0.00 |
| ATOM | 708 | O | CYS | 34 | -10.434 | -10.914 | 5.274 | 1.00 | 0.00 |
| ATOM | 709 | N | CYS | 35 | -10.212 | -8.691 | 5.047 | 1.00 | 0.00 |
| ATOM | 710 | H | CYS | 35 | -9.600 | -7.931 | 4.957 | 1.00 | 0.00 |
| ATOM | 711 | CA | CYS | 35 | -11.600 | -8.460 | 5.345 | 1.00 | 0.00 |
| ATOM | 712 | HA | CYS | 35 | -12.178 | -9.270 | 4.935 | 1.00 | 0.00 |
| ATOM | 713 | CB | CYS | 35 | -11.727 | -8.484 | 6.876 | 1.00 | 0.00 |
| ATOM | 714 | 1HB | CYS | 35 | -12.655 | -8.965 | 7.142 | 1.00 | 0.00 |
| ATOM | 715 | 2HB | CYS | 35 | -11.744 | -7.467 | 7.239 | 1.00 | 0.00 |
| ATOM | 716 | QB | CYS | 35 | -12.199 | -8.216 | 7.191 | 1.00 | 0.00 |
| ATOM | 717 | SG | CYS | 35 | -10.361 | -9.370 | 7.758 | 1.00 | 0.00 |
| ATOM | 718 | C | CYS | 35 | -12.123 | -7.133 | 4.798 | 1.00 | 0.00 |

(fortgesetzt)

Atomkooerdinaten der Struktur der ADAP hSH3-1-Domäne, oxidierte Form

| ATOM | 719 | O | CYS | 35 | -11.379 | -6.339 | 4.221 | 1.00 | 0.00 |
|------|-----|-----|-----|----|---------|--------|-------|------|------|
| ATOM | 720 | N | ASP | 36 | -13.423 | -6.913 | 4.998 | 1.00 | 0.00 |
| ATOM | 721 | H | ASP | 36 | -13.947 | -7.595 | 5.463 | 1.00 | 0.00 |
| ATOM | 722 | CA | ASP | 36 | -14.096 | -5.697 | 4.549 | 1.00 | 0.00 |
| ATOM | 723 | HA | ASP | 36 | -14.002 | -5.638 | 3.477 | 1.00 | 0.00 |
| ATOM | 724 | CB | ASP | 36 | -15.580 | -5.755 | 4.916 | 1.00 | 0.00 |
| ATOM | 725 | 1HB | ASP | 36 | -15.685 | -5.622 | 5.983 | 1.00 | 0.00 |
| ATOM | 726 | 2HB | ASP | 36 | -15.978 | -6.719 | 4.640 | 1.00 | 0.00 |
| ATOM | 727 | QB | ASP | 36 | -15.831 | -6.171 | 5.311 | 1.00 | 0.00 |
| ATOM | 728 | CG | ASP | 36 | -16.391 | -4.681 | 4.219 | 1.00 | 0.00 |
| ATOM | 729 | OD1 | ASP | 36 | -15.929 | -4.171 | 3.176 | 1.00 | 0.00 |
| ATOM | 730 | OD2 | ASP | 36 | -17.490 | -4.351 | 4.714 | 1.00 | 0.00 |
| ATOM | 731 | C | ASP | 36 | -13.476 | -4.451 | 5.169 | 1.00 | 0.00 |
| ATOM | 732 | O | ASP | 36 | -13.561 | -4.236 | 6.379 | 1.00 | 0.00 |
| ATOM | 733 | N | VAL | 37 | -12.864 | -3.626 | 4.330 | 1.00 | 0.00 |
| ATOM | 734 | H | VAL | 37 | -12.838 | -3.857 | 3.375 | 1.00 | 0.00 |
| ATOM | 735 | CA | VAL | 37 | -12.240 | -2.386 | 4.791 | 1.00 | 0.00 |
| ATOM | 736 | HA | VAL | 37 | -12.377 | -2.333 | 5.862 | 1.00 | 0.00 |
| ATOM | 737 | CB | VAL | 37 | -10.731 | -2.367 | 4.506 | 1.00 | 0.00 |
| ATOM | 738 | HB | VAL | 37 | -10.350 | -1.385 | 4.746 | 1.00 | 0.00 |
| ATOM | 739 | QG1 | VAL | 37 | -9.843 | -3.624 | 5.589 | 1.00 | 0.00 |
| ATOM | 740 | QG2 | VAL | 37 | -10.401 | -2.700 | 2.694 | 1.00 | 0.00 |
| ATOM | 741 | CG1 | VAL | 37 | -10.012 | -3.384 | 5.381 | 1.00 | 0.00 |
| ATOM | 742 | 1HG1 | VAL | 37 | -9.011 | -3.538 | 5.007 | 1.00 | 0.00 |
| ATOM | 743 | 2HG1 | VAL | 37 | -10.552 | -4.320 | 5.364 | 1.00 | 0.00 |
| ATOM | 744 | 3HG1 | VAL | 37 | -9.965 | -3.015 | 6.395 | 1.00 | 0.00 |
| ATOM | 745 | CG2 | VAL | 37 | -10.464 | -2.637 | 3.039 | 1.00 | 0.00 |
| ATOM | 746 | 1HG2 | VAL | 37 | -11.140 | -2.051 | 2.437 | 1.00 | 0.00 |
| ATOM | 747 | 2HG2 | VAL | 37 | -10.617 | -3.683 | 2.837 | 1.00 | 0.00 |
| ATOM | 748 | 3HG2 | VAL | 37 | -9.447 | -2.366 | 2.807 | 1.00 | 0.00 |
| ATOM | 749 | QQG | VAL | 37 | -10.122 | -3.162 | 4.141 | 1.00 | 0.00 |
| ATOM | 750 | C | VAL | 37 | -12.900 | -1.157 | 4.159 | 1.00 | 0.00 |
| ATOM | 751 | O | VAL | 37 | -13.512 | -1.246 | 3.094 | 1.00 | 0.00 |
| ATOM | 752 | N | LYS+ | 38 | -12.794 | -0.021 | 4.846 | 1.00 | 0.00 |
| ATOM | 753 | H | LYS+ | 38 | -12.311 | -0.028 | 5.698 | 1.00 | 0.00 |
| ATOM | 754 | CA | LYS+ | 38 | -13.400 | 1.233 | 4.389 | 1.00 | 0.00 |
| ATOM | 755 | HA | LYS+ | 38 | -14.464 | 1.077 | 4.351 | 1.00 | 0.00 |
| ATOM | 756 | CB | LYS+ | 38 | -13.117 | 2.351 | 5.401 | 1.00 | 0.00 |
| ATOM | 757 | 1HB | LYS+ | 38 | -12.559 | 3.138 | 4.912 | 1.00 | 0.00 |
| ATOM | 758 | 2HB | LYS+ | 38 | -12.524 | 1.947 | 6.205 | 1.00 | 0.00 |
| ATOM | 759 | QB | LYS+ | 38 | -12.541 | 2.543 | 5.559 | 1.00 | 0.00 |
| ATOM | 760 | CG | LYS+ | 38 | -14.375 | 2.962 | 5.998 | 1.00 | 0.00 |
| ATOM | 761 | 1HG | LYS+ | 38 | -14.927 | 3.461 | 5.215 | 1.00 | 0.00 |
| ATOM | 762 | 2HG | LYS+ | 38 | -14.091 | 3.679 | 6.753 | 1.00 | 0.00 |
| ATOM | 763 | QG | LYS+ | 38 | -14.509 | 3.570 | 5.984 | 1.00 | 0.00 |
| ATOM | 764 | CD | LYS+ | 38 | -15.262 | 1.904 | 6.633 | 1.00 | 0.00 |
| ATOM | 765 | 1HD | LYS+ | 38 | -14.649 | 1.064 | 6.928 | 1.00 | 0.00 |
| ATOM | 766 | 2HD | LYS+ | 38 | -15.995 | 1.579 | 5.909 | 1.00 | 0.00 |
| ATOM | 767 | QD | LYS+ | 38 | -15.322 | 1.322 | 6.419 | 1.00 | 0.00 |
| ATOM | 768 | CE | LYS+ | 38 | -15.985 | 2.443 | 7.856 | 1.00 | 0.00 |

(fortgesetzt)

Atomkooerdinaten der Struktur der ADAP hSH3-1-Domäne, oxidierte Form

| ATOM | 769 | 1HE | LYS+ | 38 | -15.256 | 2.872 | 8.529 | 1.00 | 0.00 |
|------|-----|-----|------|----|---------|-------|-------|------|------|
| ATOM | 770 | 2HE | LYS+ | 38 | -16.491 | 1.627 | 8.349 | 1.00 | 0.00 |
| ATOM | 771 | QE | LYS+ | 38 | -15.874 | 2.250 | 8.439 | 1.00 | 0.00 |
| ATOM | 772 | NZ | LYS+ | 38 | -16.985 | 3.486 | 7.496 | 1.00 | 0.00 |
| ATOM | 773 | 1HZ | LYS+ | 38 | -17.463 | 3.834 | 8.352 | 1.00 | 0.00 |
| ATOM | 774 | 2HZ | LYS+ | 38 | -17.698 | 3.091 | 6.851 | 1.00 | 0.00 |
| ATOM | 775 | 3HZ | LYS+ | 38 | -16.513 | 4.286 | 7.026 | 1.00 | 0.00 |
| ATOM | 776 | QZ | LYS+ | 38 | -17.225 | 3.737 | 7.410 | 1.00 | 0.00 |
| ATOM | 777 | C | LYS+ | 38 | -12.937 | 1.660 | 2.994 | 1.00 | 0.00 |
| ATOM | 778 | O | LYS+ | 38 | -13.626 | 1.416 | 2.005 | 1.00 | 0.00 |
| ATOM | 779 | N | GLY | 39 | -11.789 | 2.329 | 2.925 | 1.00 | 0.00 |
| ATOM | 780 | H | GLY | 39 | -11.293 | 2.515 | 3.745 | 1.00 | 0.00 |
| ATOM | 781 | CA | GLY | 39 | -11.286 | 2.813 | 1.652 | 1.00 | 0.00 |
| ATOM | 782 | 1HA | GLY | 39 | -11.430 | 2.062 | 0.904 | 1.00 | 0.00 |
| ATOM | 783 | 2HA | GLY | 39 | -10.237 | 3.003 | 1.744 | 1.00 | 0.00 |
| ATOM | 784 | QA | GLY | 39 | -10.833 | 2.533 | 1.324 | 1.00 | 0.00 |
| ATOM | 785 | C | GLY | 39 | -11.976 | 4.085 | 1.215 | 1.00 | 0.00 |
| ATOM | 786 | O | GLY | 39 | -12.038 | 4.392 | 0.024 | 1.00 | 0.00 |
| ATOM | 787 | N | GLY | 40 | -12.497 | 4.827 | 2.184 | 1.00 | 0.00 |
| ATOM | 788 | H | GLY | 40 | -12.417 | 4.532 | 3.112 | 1.00 | 0.00 |
| ATOM | 789 | CA | GLY | 40 | -13.180 | 6.064 | 1.883 | 1.00 | 0.00 |
| ATOM | 790 | 1HA | GLY | 40 | -13.804 | 6.331 | 2.723 | 1.00 | 0.00 |
| ATOM | 791 | 2HA | GLY | 40 | -13.804 | 5.912 | 1.019 | 1.00 | 0.00 |
| ATOM | 792 | QA | GLY | 40 | -13.804 | 6.121 | 1.871 | 1.00 | 0.00 |
| ATOM | 793 | C | GLY | 40 | -12.221 | 7.202 | 1.604 | 1.00 | 0.00 |
| ATOM | 794 | O | GLY | 40 | -11.886 | 7.471 | 0.450 | 1.00 | 0.00 |
| ATOM | 795 | N | LYS+ | 41 | -11.789 | 7.883 | 2.661 | 1.00 | 0.00 |
| ATOM | 796 | H | LYS+ | 41 | -12.099 | 7.627 | 3.555 | 1.00 | 0.00 |
| ATOM | 797 | CA | LYS+ | 41 | -10.875 | 9.008 | 2.516 | 1.00 | 0.00 |
| ATOM | 798 | HA | LYS+ | 41 | -10.929 | 9.340 | 1.490 | 1.00 | 0.00 |
| ATOM | 799 | CB | LYS+ | 41 | -11.313 | 10.160 | 3.423 | 1.00 | 0.00 |
| ATOM | 800 | 1HB | LYS+ | 41 | -10.721 | 10.137 | 4.326 | 1.00 | 0.00 |
| ATOM | 801 | 2HB | LYS+ | 41 | -12.353 | 10.022 | 3.683 | 1.00 | 0.00 |
| ATOM | 802 | QB | LYS+ | 41 | -11.537 | 10.080 | 4.004 | 1.00 | 0.00 |
| ATOM | 803 | CG | LYS+ | 41 | -11.158 | 11.531 | 2.784 | 1.00 | 0.00 |
| ATOM | 804 | 1HG | LYS+ | 41 | -11.917 | 11.650 | 2.025 | 1.00 | 0.00 |
| ATOM | 805 | 2HG | LYS+ | 41 | -10.180 | 11.596 | 2.332 | 1.00 | 0.00 |
| ATOM | 806 | QG | LYS+ | 41 | -11.048 | 11.623 | 2.179 | 1.00 | 0.00 |
| ATOM | 807 | CD | LYS+ | 41 | -11.304 | 12.646 | 3.809 | 1.00 | 0.00 |
| ATOM | 808 | 1HD | LYS+ | 41 | -11.673 | 12.227 | 4.734 | 1.00 | 0.00 |
| ATOM | 809 | 2HD | LYS+ | 41 | -12.008 | 13.374 | 3.436 | 1.00 | 0.00 |
| ATOM | 810 | QD | LYS+ | 41 | -11.841 | 12.800 | 4.085 | 1.00 | 0.00 |
| ATOM | 811 | CE | LYS+ | 41 | -9.976 | 13.337 | 4.075 | 1.00 | 0.00 |
| ATOM | 812 | 1HE | LYS+ | 41 | -9.177 | 12.631 | 3.903 | 1.00 | 0.00 |
| ATOM | 813 | 2HE | LYS+ | 41 | -9.953 | 13.662 | 5.104 | 1.00 | 0.00 |
| ATOM | 814 | QE | LYS+ | 41 | -9.565 | 13.147 | 4.504 | 1.00 | 0.00 |
| ATOM | 815 | NZ | LYS+ | 41 | -9.778 | 14.519 | 3.190 | 1.00 | 0.00 |
| ATOM | 816 | 1HZ | LYS+ | 41 | -9.200 | 14.255 | 2.366 | 1.00 | 0.00 |
| ATOM | 817 | 2HZ | LYS+ | 41 | -9.294 | 15.278 | 3.710 | 1.00 | 0.00 |
| ATOM | 818 | 3HZ | LYS+ | 41 | -10.697 | 14.874 | 2.857 | 1.00 | 0.00 |

(fortgesetzt)

Atomkooerdinaten der Struktur der ADAP hSH3-1-Domäne, oxidierte Form

| ATOM | 819 | QZ | LYS+ | 41 | -9.730 | 14.802 | 2.978 | 1.00 | 0.00 |
|------|-----|------|------|----|--------|--------|--------|------|------|
| ATOM | 820 | C | LYS+ | 41 | -9.427 | 8.629 | 2.828 | 1.00 | 0.00 |
| ATOM | 821 | O | LYS+ | 41 | -8.530 | 8.879 | 2.024 | 1.00 | 0.00 |
| ATOM | 822 | N | ASN | 42 | -9.194 | 8.059 | 4.011 | 1.00 | 0.00 |
| ATOM | 823 | H | ASN | 42 | -9.940 | 7.907 | 4.628 | 1.00 | 0.00 |
| ATOM | 824 | CA | ASN | 42 | -7.835 | 7.698 | 4.420 | 1.00 | 0.00 |
| ATOM | 825 | HA | ASN | 42 | -7.166 | 8.039 | 3.645 | 1.00 | 0.00 |
| ATOM | 826 | CB | ASN | 42 | -7.456 | 8.412 | 5.728 | 1.00 | 0.00 |
| ATOM | 827 | 1HB | ASN | 42 | -6.554 | 8.983 | 5.566 | 1.00 | 0.00 |
| ATOM | 828 | 2HB | ASN | 42 | -7.273 | 7.673 | 6.493 | 1.00 | 0.00 |
| ATOM | 829 | QB | ASN | 42 | -6.913 | 8.328 | 6.030 | 1.00 | 0.00 |
| ATOM | 830 | CG | ASN | 42 | -8.536 | 9.355 | 6.226 | 1.00 | 0.00 |
| ATOM | 831 | OD1 | ASN | 42 | -9.610 | 8.922 | 6.647 | 1.00 | 0.00 |
| ATOM | 832 | ND2 | ASN | 42 | -8.257 | 10.652 | 6.181 | 1.00 | 0.00 |
| ATOM | 833 | 1HD2 | ASN | 42 | -7.382 | 10.925 | 5.834 | 1.00 | 0.00 |
| ATOM | 834 | 2HD2 | ASN | 42 | -8.936 | 11.284 | 6.497 | 1.00 | 0.00 |
| ATOM | 835 | QD2 | ASN | 42 | -8.159 | 11.105 | 6.166 | 1.00 | 0.00 |
| ATOM | 836 | C | ASN | 42 | -7.632 | 6.191 | 4.589 | 1.00 | 0.00 |
| ATOM | 837 | O | ASN | 42 | -6.545 | 5.761 | 4.976 | 1.00 | 0.00 |
| ATOM | 838 | N | GLU | 43 | -8.649 | 5.379 | 4.309 | 1.00 | 0.00 |
| ATOM | 839 | H | GLU | 43 | -9.505 | 5.740 | 4.001 | 1.00 | 0.00 |
| ATOM | 840 | CA | GLU | 43 | -8.506 | 3.944 | 4.453 | 1.00 | 0.00 |
| ATOM | 841 | HA | GLU | 43 | -7.550 | 3.757 | 4.905 | 1.00 | 0.00 |
| ATOM | 842 | CB | GLU | 43 | -9.605 | 3.387 | 5.361 | 1.00 | 0.00 |
| ATOM | 843 | 1HB | GLU | 43 | -9.818 | 2.370 | 5.067 | 1.00 | 0.00 |
| ATOM | 844 | 2HB | GLU | 43 | -10.497 | 3.983 | 5.234 | 1.00 | 0.00 |
| ATOM | 845 | QB | GLU | 43 | -10.157 | 3.177 | 5.150 | 1.00 | 0.00 |
| ATOM | 846 | CG | GLU | 43 | -9.235 | 3.388 | 6.835 | 1.00 | 0.00 |
| ATOM | 847 | 1HG | GLU | 43 | -9.431 | 4.370 | 7.241 | 1.00 | 0.00 |
| ATOM | 848 | 2HG | GLU | 43 | -8.184 | 3.163 | 6.930 | 1.00 | 0.00 |
| ATOM | 849 | QG | GLU | 43 | -8.807 | 3.767 | 7.085 | 1.00 | 0.00 |
| ATOM | 850 | CD | GLU | 43 | -10.023 | 2.368 | 7.633 | 1.00 | 0.00 |
| ATOM | 851 | OE1 | GLU | 43 | -10.075 | 1.194 | 7.209 | 1.00 | 0.00 |
| ATOM | 852 | OE2 | GLU | 43 | -10.586 | 2.742 | 8.684 | 1.00 | 0.00 |
| ATOM | 853 | C | GLU | 43 | -8.553 | 3.266 | 3.097 | 1.00 | 0.00 |
| ATOM | 854 | O | GLU | 43 | -8.627 | 3.933 | 2.065 | 1.00 | 0.00 |
| ATOM | 855 | N | LEU | 44 | -8.520 | 1.939 | 3.104 | 1.00 | 0.00 |
| ATOM | 856 | H | LEU | 44 | -8.462 | 1.469 | 3.953 | 1.00 | 0.00 |
| ATOM | 857 | CA | LEU | 44 | -8.581 | 1.165 | 1.878 | 1.00 | 0.00 |
| ATOM | 858 | HA | LEU | 44 | -8.539 | 1.851 | 1.049 | 1.00 | 0.00 |
| ATOM | 859 | CB | LEU | 44 | -7.405 | 0.210 | 1.788 | 1.00 | 0.00 |
| ATOM | 860 | 1HB | LEU | 44 | -7.777 | -0.791 | 1.893 | 1.00 | 0.00 |
| ATOM | 861 | 2HB | LEU | 44 | -6.736 | 0.419 | 2.599 | 1.00 | 0.00 |
| ATOM | 862 | QB | LEU | 44 | -7.256 | -0.186 | 2.246 | 1.00 | 0.00 |
| ATOM | 863 | CG | LEU | 44 | -6.634 | 0.300 | 0.485 | 1.00 | 0.00 |
| ATOM | 864 | HG | LEU | 44 | -6.412 | 1.336 | 0.294 | 1.00 | 0.00 |
| ATOM | 865 | QD1 | LEU | 44 | -5.002 | -0.646 | 0.581 | 1.00 | 0.00 |
| ATOM | 866 | QD2 | LEU | 44 | -7.702 | -0.342 | -0.927 | 1.00 | 0.00 |
| ATOM | 867 | CD1 | LEU | 44 | -5.316 | -0.465 | 0.564 | 1.00 | 0.00 |
| ATOM | 868 | 1HD1 | LEU | 44 | -4.840 | -0.278 | 1.515 | 1.00 | 0.00 |

(fortgesetzt)

Atomkooerdinaten der Struktur der ADAP hSH3-1-Domäne, oxidierte Form

| ATOM | 869 | 2HD1 | LEU | 44 | -4.663 | -0.139 | -0.231 | 1.00 | 0.00 |
|------|-----|------|-----|----|--------|--------|--------|------|------|
| ATOM | 870 | 3HD1 | LEU | 44 | -5.504 | -1.522 | 0.460 | 1.00 | 0.00 |
| ATOM | 871 | CD2 | LEU | 44 | -7.496 | -0.219 | -0.656 | 1.00 | 0.00 |
| ATOM | 872 | 1HD2 | LEU | 44 | -7.774 | 0.603 | -1.298 | 1.00 | 0.00 |
| ATOM | 873 | 2HD2 | LEU | 44 | -8.388 | -0.676 | -0.256 | 1.00 | 0.00 |
| ATOM | 874 | 3HD2 | LEU | 44 | -6.945 | -0.952 | -1.226 | 1.00 | 0.00 |
| ATOM | 875 | QQD | LEU | 44 | -6.352 | -0.494 | -0.173 | 1.00 | 0.00 |
| ATOM | 876 | C | LEU | 44 | -9.888 | 0.391 | 1.808 | 1.00 | 0.00 |
| ATOM | 877 | O | LEU | 44 | -10.472 | 0.044 | 2.834 | 1.00 | 0.00 |
| ATOM | 878 | N | SER | 45 | -10.350 | 0.136 | 0.593 | 1.00 | 0.00 |
| ATOM | 879 | H | SER | 45 | -9.841 | 0.450 | -0.182 | 1.00 | 0.00 |
| ATOM | 880 | CA | SER | 45 | -11.602 | -0.586 | 0.392 | 1.00 | 0.00 |
| ATOM | 881 | HA | SER | 45 | -12.036 | -0.758 | 1.364 | 1.00 | 0.00 |
| ATOM | 882 | CB | SER | 45 | -12.584 | 0.238 | -0.439 | 1.00 | 0.00 |
| ATOM | 883 | 1HB | SER | 45 | -12.692 | 1.211 | 0.003 | 1.00 | 0.00 |
| ATOM | 884 | 2HB | SER | 45 | -13.543 | -0.257 | -0.447 | 1.00 | 0.00 |
| ATOM | 885 | QB | SER | 45 | -13.118 | 0.477 | -0.222 | 1.00 | 0.00 |
| ATOM | 886 | OG | SER | 45 | -12.132 | 0.386 | -1.773 | 1.00 | 0.00 |
| ATOM | 887 | HG | SER | 45 | -11.399 | 1.006 | -1.796 | 1.00 | 0.00 |
| ATOM | 888 | C | SER | 45 | -11.373 | -1.932 | -0.276 | 1.00 | 0.00 |
| ATOM | 889 | O | SER | 45 | -10.633 | -2.044 | -1.254 | 1.00 | 0.00 |
| ATOM | 890 | N | PHE | 46 | -12.031 | -2.943 | 0.272 | 1.00 | 0.00 |
| ATOM | 891 | H | PHE | 46 | -12.605 | -2.754 | 1.046 | 1.00 | 0.00 |
| ATOM | 892 | CA | PHE | 46 | -11.951 | -4.318 | -0.227 | 1.00 | 0.00 |
| ATOM | 893 | HA | PHE | 46 | -12.600 | -4.402 | -1.087 | 1.00 | 0.00 |
| ATOM | 894 | CB | PHE | 46 | -10.520 | -4.687 | -0.636 | 1.00 | 0.00 |
| ATOM | 895 | 1HB | PHE | 46 | -10.239 | -4.112 | -1.504 | 1.00 | 0.00 |
| ATOM | 896 | 2HB | PHE | 46 | -10.485 | -5.738 | -0.881 | 1.00 | 0.00 |
| ATOM | 897 | QB | PHE | 46 | -10.362 | -4.925 | -1.192 | 1.00 | 0.00 |
| ATOM | 898 | QD | PHE | 46 | -9.396 | -4.408 | 0.558 | 1.00 | 0.00 |
| ATOM | 899 | QE | PHE | 46 | -7.741 | -3.995 | 2.331 | 1.00 | 0.00 |
| ATOM | 900 | QR | PHE | 46 | -8.237 | -4.117 | 1.799 | 1.00 | 0.00 |
| ATOM | 901 | CG | PHE | 46 | -9.507 | -4.435 | 0.441 | 1.00 | 0.00 |
| ATOM | 902 | CD1 | PHE | 46 | -9.452 | -5.250 | 1.560 | 1.00 | 0.00 |
| ATOM | 903 | 1HD | PHE | 46 | -10.146 | -6.073 | 1.649 | 1.00 | 0.00 |
| ATOM | 904 | CE1 | PHE | 46 | -8.524 | -5.019 | 2.558 | 1.00 | 0.00 |
| ATOM | 905 | 1HE | PHE | 46 | -8.492 | -5.660 | 3.426 | 1.00 | 0.00 |
| ATOM | 906 | CZ | PHE | 46 | -7.638 | -3.965 | 2.441 | 1.00 | 0.00 |
| ATOM | 907 | HZ | PHE | 46 | -6.910 | -3.779 | 3.219 | 1.00 | 0.00 |
| ATOM | 908 | CE2 | PHE | 46 | -7.682 | -3.149 | 1.329 | 1.00 | 0.00 |
| ATOM | 909 | 2HE | PHE | 46 | -6.990 | -2.329 | 1.235 | 1.00 | 0.00 |
| ATOM | 910 | CD2 | PHE | 46 | -8.613 | -3.384 | 0.337 | 1.00 | 0.00 |
| ATOM | 911 | 2HD | PHE | 46 | -8.646 | -2.744 | -0.532 | 1.00 | 0.00 |
| ATOM | 912 | C | PHE | 46 | -12.432 | -5.279 | 0.849 | 1.00 | 0.00 |
| ATOM | 913 | O | PHE | 46 | -12.357 | -4.969 | 2.039 | 1.00 | 0.00 |
| ATOM | 914 | N | LYS+ | 47 | -12.933 | -6.441 | 0.445 | 1.00 | 0.00 |
| ATOM | 915 | H | LYS+ | 47 | -12.983 | -6.649 | -0.504 | 1.00 | 0.00 |
| ATOM | 916 | CA | LYS+ | 47 | -13.420 | -7.408 | 1.395 | 1.00 | 0.00 |
| ATOM | 917 | HA | LYS+ | 47 | -13.316 | -6.992 | 2.378 | 1.00 | 0.00 |
| ATOM | 918 | CB | LYS+ | 47 | -14.887 | -7.703 | 1.148 | 1.00 | 0.00 |

(fortgesetzt)

Atomkooerdinaten der Struktur der ADAP hSH3-1-Domäne, oxidierte Form

| ATOM | 919 | 1HB | LYS+ | 47 | -14.957 | -8.603 | 0.570 | 1.00 | 0.00 |
|------|-----|-----|------|----|---------|--------|-------|------|------|
| ATOM | 920 | 2HB | LYS+ | 47 | -15.324 | -6.886 | 0.593 | 1.00 | 0.00 |
| ATOM | 921 | QB | LYS+ | 47 | -15.141 | -7.744 | 0.581 | 1.00 | 0.00 |
| ATOM | 922 | CG | LYS+ | 47 | -15.680 | -7.902 | 2.422 | 1.00 | 0.00 |
| ATOM | 923 | 1HG | LYS+ | 47 | -15.072 | -7.596 | 3.259 | 1.00 | 0.00 |
| ATOM | 924 | 2HG | LYS+ | 47 | -15.928 | -8.949 | 2.517 | 1.00 | 0.00 |
| ATOM | 925 | QG | LYS+ | 47 | -15.500 | -8.272 | 2.888 | 1.00 | 0.00 |
| ATOM | 926 | CD | LYS+ | 47 | -16.960 | -7.085 | 2.406 | 1.00 | 0.00 |
| ATOM | 927 | 1HD | LYS+ | 47 | -16.711 | -6.053 | 2.203 | 1.00 | 0.00 |
| ATOM | 928 | 2HD | LYS+ | 47 | -17.439 | -7.161 | 3.369 | 1.00 | 0.00 |
| ATOM | 929 | QD | LYS+ | 47 | -17.075 | -6.607 | 2.786 | 1.00 | 0.00 |
| ATOM | 930 | CE | LYS+ | 47 | -17.918 | -7.579 | 1.334 | 1.00 | 0.00 |
| ATOM | 931 | 1HE | LYS+ | 47 | -17.965 | -8.657 | 1.383 | 1.00 | 0.00 |
| ATOM | 932 | 2HE | LYS+ | 47 | -17.541 | -7.279 | 0.368 | 1.00 | 0.00 |
| ATOM | 933 | QE | LYS+ | 47 | -17.753 | -7.968 | 0.876 | 1.00 | 0.00 |
| ATOM | 934 | NZ | LYS+ | 47 | -19.288 | -7.024 | 1.515 | 1.00 | 0.00 |
| ATOM | 935 | 1HZ | LYS+ | 47 | -19.774 | -7.515 | 2.292 | 1.00 | 0.00 |
| ATOM | 936 | 2HZ | LYS+ | 47 | -19.841 | -7.146 | 0.644 | 1.00 | 0.00 |
| ATOM | 937 | 3HZ | LYS+ | 47 | -19.235 | -6.009 | 1.741 | 1.00 | 0.00 |
| ATOM | 938 | QZ | LYS+ | 47 | -19.617 | -6.890 | 1.559 | 1.00 | 0.00 |
| ATOM | 939 | C | LYS+ | 47 | -12.644 | -8.704 | 1.334 | 1.00 | 0.00 |
| ATOM | 940 | O | LYS+ | 47 | -12.085 | -9.074 | 0.302 | 1.00 | 0.00 |
| ATOM | 941 | N | GLN | 48 | -12.659 | -9.387 | 2.462 | 1.00 | 0.00 |
| ATOM | 942 | H | GLN | 48 | -13.153 | -9.003 | 3.221 | 1.00 | 0.00 |
| ATOM | 943 | CA | GLN | 48 | -12.014 | -10.686 | 2.644 | 1.00 | 0.00 |
| ATOM | 944 | HA | GLN | 48 | -11.017 | -10.525 | 3.022 | 1.00 | 0.00 |
| ATOM | 945 | CB | GLN | 48 | -12.817 | -11.470 | 3.676 | 1.00 | 0.00 |
| ATOM | 946 | 1HB | GLN | 48 | -12.521 | -11.160 | 4.666 | 1.00 | 0.00 |
| ATOM | 947 | 2HB | GLN | 48 | -12.611 | -12.523 | 3.557 | 1.00 | 0.00 |
| ATOM | 948 | QB | GLN | 48 | -12.566 | -11.841 | 4.111 | 1.00 | 0.00 |
| ATOM | 949 | CG | GLN | 48 | -14.318 | -11.249 | 3.532 | 1.00 | 0.00 |
| ATOM | 950 | 1HG | GLN | 48 | -14.485 | -10.492 | 2.764 | 1.00 | 0.00 |
| ATOM | 951 | 2HG | GLN | 48 | -14.709 | -10.897 | 4.476 | 1.00 | 0.00 |
| ATOM | 952 | QG | GLN | 48 | -14.597 | -10.695 | 3.620 | 1.00 | 0.00 |
| ATOM | 953 | CD | GLN | 48 | -15.060 | -12.511 | 3.140 | 1.00 | 0.00 |
| ATOM | 954 | OE1 | GLN | 48 | -15.087 | -12.891 | 1.969 | 1.00 | 0.00 |
| ATOM | 955 | NE2 | GLN | 48 | -15.670 | -13.168 | 4.121 | 1.00 | 0.00 |
| ATOM | 956 | 1HE2 | GLN | 48 | -15.606 | -12.806 | 5.029 | 1.00 | 0.00 |
| ATOM | 957 | 2HE2 | GLN | 48 | -16.158 | -13.988 | 3.896 | 1.00 | 0.00 |
| ATOM | 958 | QE2 | GLN | 48 | -15.882 | -13.397 | 4.462 | 1.00 | 0.00 |
| ATOM | 959 | C | GLN | 48 | -11.942 | -11.508 | 1.352 | 1.00 | 0.00 |
| ATOM | 960 | O | GLN | 48 | -12.767 | -12.394 | 1.129 | 1.00 | 0.00 |
| ATOM | 961 | N | GLY | 49 | -10.950 | -11.226 | 0.515 | 1.00 | 0.00 |
| ATOM | 962 | H | GLY | 49 | -10.316 | -10.519 | 0.747 | 1.00 | 0.00 |
| ATOM | 963 | CA | GLY | 49 | -10.801 | -11.978 | -0.723 | 1.00 | 0.00 |
| ATOM | 964 | 1HA | GLY | 49 | -11.726 | -12.500 | -0.921 | 1.00 | 0.00 |
| ATOM | 965 | 2HA | GLY | 49 | -10.018 | -12.709 | -0.588 | 1.00 | 0.00 |
| ATOM | 966 | QA | GLY | 49 | -10.872 | -12.605 | -0.755 | 1.00 | 0.00 |
| ATOM | 967 | C | GLY | 49 | -10.456 | -11.122 | -1.930 | 1.00 | 0.00 |
| ATOM | 968 | O | GLY | 49 | -10.641 | -11.553 | -3.069 | 1.00 | 0.00 |

(fortgesetzt)

Atomkooerdinaten der Struktur der ADAP hSH3-1-Domäne, oxidierte Form

| ATOM | 969 | N | GLU | 50 | -9.954 | -9.918 | -1.693 | 1.00 | 0.00 |
|---|---|---|---|---|---|---|---|---|---|
| ATOM | 970 | H | GLU | 50 | -9.819 | -9.626 | -0.779 | 1.00 | 0.00 |
| ATOM | 971 | CA | GLU | 50 | -9.584 | -9.022 | -2.771 | 1.00 | 0.00 |
| ATOM | 972 | HA | GLU | 50 | -10.034 | -9.384 | -3.676 | 1.00 | 0.00 |
| ATOM | 973 | CB | GLU | 50 | -10.106 | -7.622 | -2.490 | 1.00 | 0.00 |
| ATOM | 974 | 1HB | GLU | 50 | -9.331 | -7.072 | -1.995 | 1.00 | 0.00 |
| ATOM | 975 | 2HB | GLU | 50 | -10.963 | -7.691 | -1.836 | 1.00 | 0.00 |
| ATOM | 976 | QB | GLU | 50 | -10.147 | -7.382 | -1.916 | 1.00 | 0.00 |
| ATOM | 977 | CG | GLU | 50 | -10.515 | -6.860 | -3.739 | 1.00 | 0.00 |
| ATOM | 978 | 1HG | GLU | 50 | -9.892 | -7.179 | -4.561 | 1.00 | 0.00 |
| ATOM | 979 | 2HG | GLU | 50 | -10.368 | -5.805 | -3.565 | 1.00 | 0.00 |
| ATOM | 980 | QG | GLU | 50 | -10.130 | -6.492 | -4.063 | 1.00 | 0.00 |
| ATOM | 981 | CD | GLU | 50 | -11.966 | -7.089 | -4.112 | 1.00 | 0.00 |
| ATOM | 982 | OE1 | GLU | 50 | -12.262 | -8.135 | -4.729 | 1.00 | 0.00 |
| ATOM | 983 | OE2 | GLU | 50 | -12.807 | -6.225 | -3.789 | 1.00 | 0.00 |
| ATOM | 984 | C | GLU | 50 | -8.074 | -8.984 | -2.949 | 1.00 | 0.00 |
| ATOM | 985 | O | GLU | 50 | -7.349 | -8.460 | -2.104 | 1.00 | 0.00 |
| ATOM | 986 | N | GLN | 51 | -7.616 | -9.536 | -4.061 | 1.00 | 0.00 |
| ATOM | 987 | H | GLN | 51 | -8.252 | -9.925 | -4.675 | 1.00 | 0.00 |
| ATOM | 988 | CA | GLN | 51 | -6.193 | -9.572 | -4.387 | 1.00 | 0.00 |
| ATOM | 989 | HA | GLN | 51 | -5.721 | -10.301 | -3.745 | 1.00 | 0.00 |
| ATOM | 990 | CB | GLN | 51 | -5.990 | -9.982 | -5.847 | 1.00 | 0.00 |
| ATOM | 991 | 1HB | GLN | 51 | -5.022 | -9.636 | -6.176 | 1.00 | 0.00 |
| ATOM | 992 | 2HB | GLN | 51 | -6.754 | -9.514 | -6.450 | 1.00 | 0.00 |
| ATOM | 993 | QB | GLN | 51 | -5.888 | -9.575 | -6.313 | 1.00 | 0.00 |
| ATOM | 994 | CG | GLN | 51 | -6.061 | -11.484 | -6.073 | 1.00 | 0.00 |
| ATOM | 995 | 1HG | GLN | 51 | -6.973 | -11.858 | -5.631 | 1.00 | 0.00 |
| ATOM | 996 | 2HG | GLN | 51 | -5.213 | -11.948 | -5.591 | 1.00 | 0.00 |
| ATOM | 997 | QG | GLN | 51 | -6.093 | -11.903 | -5.611 | 1.00 | 0.00 |
| ATOM | 998 | CD | GLN | 51 | -6.045 | -11.855 | -7.542 | 1.00 | 0.00 |
| ATOM | 999 | OE1 | GLN | 51 | -5.297 | -11.279 | -8.332 | 1.00 | 0.00 |
| ATOM | 1000 | NE2 | GLN | 51 | -6.873 | -12.824 | -7.917 | 1.00 | 0.00 |
| ATOM | 1001 | 1HE2 | GLN | 51 | -7.440 | -13.238 | -7.234 | 1.00 | 0.00 |
| ATOM | 1002 | 2HE2 | GLN | 51 | -6.883 | -13.085 | -8.862 | 1.00 | 0.00 |
| ATOM | 1003 | QE2 | GLN | 51 | -7.162 | -13.162 | -8.048 | 1.00 | 0.00 |
| ATOM | 1004 | C | GLN | 51 | -5.565 | -8.210 | -4.134 | 1.00 | 0.00 |
| ATOM | 1005 | O | GLN | 51 | -5.663 | -7.299 | -4.954 | 1.00 | 0.00 |
| ATOM | 1006 | N | ILE | 52 | -4.954 | -8.082 | -2.969 | 1.00 | 0.00 |
| ATOM | 1007 | H | ILE | 52 | -4.949 | -8.848 | -2.359 | 1.00 | 0.00 |
| ATOM | 1008 | CA | ILE | 52 | -4.333 | -6.831 | -2.545 | 1.00 | 0.00 |
| ATOM | 1009 | HA | ILE | 52 | -4.918 | -6.026 | -2.957 | 1.00 | 0.00 |
| ATOM | 1010 | CB | ILE | 52 | -4.367 | -6.700 | -1.011 | 1.00 | 0.00 |
| ATOM | 1011 | HB | ILE | 52 | -5.079 | -7.411 | -0.631 | 1.00 | 0.00 |
| ATOM | 1012 | QG2 | ILE | 52 | -2.687 | -7.100 | -0.242 | 1.00 | 0.00 |
| ATOM | 1013 | CG2 | ILE | 52 | -3.009 | -7.023 | -0.389 | 1.00 | 0.00 |
| ATOM | 1014 | 1HG2 | ILE | 52 | -2.549 | -7.839 | -0.927 | 1.00 | 0.00 |
| ATOM | 1015 | 2HG2 | ILE | 52 | -3.144 | -7.308 | 0.641 | 1.00 | 0.00 |
| ATOM | 1016 | 3HG2 | ILE | 52 | -2.369 | -6.153 | -0.441 | 1.00 | 0.00 |
| ATOM | 1017 | CG1 | ILE | 52 | -4.823 | -5.301 | -0.623 | 1.00 | 0.00 |
| ATOM | 1018 | 1HG1 | ILE | 52 | -5.232 | -4.802 | -1.489 | 1.00 | 0.00 |

(fortgesetzt)

Atomkooerdinaten der Struktur der ADAP hSH3-1-Domäne, oxidierte Form

| ATOM | 1019 | 2HG1 | ILE | 52 | -3.979 | -4.751 | -0.265 | 1.00 | 0.00 |
|------|------|------|-----|----|--------|--------|--------|------|------|
| ATOM | 1020 | QG1 | ILE | 52 | -4.606 | -4.776 | -0.877 | 1.00 | 0.00 |
| ATOM | 1021 | QD1 | ILE | 52 | -6.127 | -5.297 | 0.714 | 1.00 | 0.00 |
| ATOM | 1022 | CD1 | ILE | 52 | -5.876 | -5.298 | 0.457 | 1.00 | 0.00 |
| ATOM | 1023 | 1HD1 | ILE | 52 | -5.922 | -6.273 | 0.918 | 1.00 | 0.00 |
| ATOM | 1024 | 2HD1 | ILE | 52 | -6.835 | -5.061 | 0.022 | 1.00 | 0.00 |
| ATOM | 1025 | 3HD1 | ILE | 52 | -5.623 | -4.558 | 1.201 | 1.00 | 0.00 |
| ATOM | 1026 | C | ILE | 52 | -2.895 | -6.679 | -3.033 | 1.00 | 0.00 |
| ATOM | 1027 | O | ILE | 52 | -2.232 | -7.662 | -3.362 | 1.00 | 0.00 |
| ATOM | 1028 | N | GLU | 53 | -2.419 | -5.432 | -3.056 | 1.00 | 0.00 |
| ATOM | 1029 | H | GLU | 53 | -2.995 | -4.692 | -2.769 | 1.00 | 0.00 |
| ATOM | 1030 | CA | GLU | 53 | -1.057 | -5.146 | -3.484 | 1.00 | 0.00 |
| ATOM | 1031 | HA | GLU | 53 | -0.484 | -6.051 | -3.352 | 1.00 | 0.00 |
| ATOM | 1032 | CB | GLU | 53 | -1.030 | -4.761 | -4.963 | 1.00 | 0.00 |
| ATOM | 1033 | 1HB | GLU | 53 | -0.020 | -4.489 | -5.234 | 1.00 | 0.00 |
| ATOM | 1034 | 2HB | GLU | 53 | -1.674 | -3.910 | -5.115 | 1.00 | 0.00 |
| ATOM | 1035 | QB | GLU | 53 | -0.847 | -4.199 | -5.174 | 1.00 | 0.00 |
| ATOM | 1036 | CG | GLU | 53 | -1.485 | -5.879 | -5.884 | 1.00 | 0.00 |
| ATOM | 1037 | 1HG | GLU | 53 | -1.862 | -6.689 | -5.280 | 1.00 | 0.00 |
| ATOM | 1038 | 2HG | GLU | 53 | -0.636 | -6.223 | -6.456 | 1.00 | 0.00 |
| ATOM | 1039 | QG | GLU | 53 | -1.249 | -6.456 | -5.868 | 1.00 | 0.00 |
| ATOM | 1040 | CD | GLU | 53 | -2.572 | -5.441 | -6.846 | 1.00 | 0.00 |
| ATOM | 1041 | OE1 | GLU | 53 | -3.739 | -5.836 | -6.642 | 1.00 | 0.00 |
| ATOM | 1042 | OE2 | GLU | 53 | -2.256 | -4.704 | -7.804 | 1.00 | 0.00 |
| ATOM | 1043 | C | GLU | 53 | -0.420 | -4.032 | -2.643 | 1.00 | 0.00 |
| ATOM | 1044 | O | GLU | 53 | 0.027 | -3.018 | -3.178 | 1.00 | 0.00 |
| ATOM | 1045 | N | ILE | 54 | -0.375 | -4.232 | -1.325 | 1.00 | 0.00 |
| ATOM | 1046 | H | ILE | 54 | -0.751 | -5.044 | -0.954 | 1.00 | 0.00 |
| ATOM | 1047 | CA | ILE | 54 | 0.205 | -3.265 | -0.409 | 1.00 | 0.00 |
| ATOM | 1048 | HA | ILE | 54 | -0.435 | -2.399 | -0.374 | 1.00 | 0.00 |
| ATOM | 1049 | CB | ILE | 54 | 0.302 | -3.860 | 1.002 | 1.00 | 0.00 |
| ATOM | 1050 | HB | ILE | 54 | 1.020 | -3.282 | 1.560 | 1.00 | 0.00 |
| ATOM | 1051 | QG2 | ILE | 54 | -1.363 | -3.753 | 1.869 | 1.00 | 0.00 |
| ATOM | 1052 | CG2 | ILE | 54 | -1.043 | -3.773 | 1.704 | 1.00 | 0.00 |
| ATOM | 1053 | 1HG2 | ILE | 54 | -1.359 | -2.742 | 1.750 | 1.00 | 0.00 |
| ATOM | 1054 | 2HG2 | ILE | 54 | -0.955 | -4.168 | 2.704 | 1.00 | 0.00 |
| ATOM | 1055 | 3HG2 | ILE | 54 | -1.774 | -4.348 | 1.153 | 1.00 | 0.00 |
| ATOM | 1056 | CG1 | ILE | 54 | 0.773 | -5.313 | 0.937 | 1.00 | 0.00 |
| ATOM | 1057 | 1HG1 | ILE | 54 | 1.648 | -5.375 | 0.307 | 1.00 | 0.00 |
| ATOM | 1058 | 2HG1 | ILE | 54 | -0.011 | -5.924 | 0.519 | 1.00 | 0.00 |
| ATOM | 1059 | QG1 | ILE | 54 | 0.818 | -5.650 | 0.413 | 1.00 | 0.00 |
| ATOM | 1060 | QD1 | ILE | 54 | 1.214 | -6.017 | 2.605 | 1.00 | 0.00 |
| ATOM | 1061 | CD1 | ILE | 54 | 1.130 | -5.883 | 2.284 | 1.00 | 0.00 |
| ATOM | 1062 | 1HD1 | ILE | 54 | 1.008 | -5.121 | 3.035 | 1.00 | 0.00 |
| ATOM | 1063 | 2HD1 | ILE | 54 | 2.156 | -6.215 | 2.273 | 1.00 | 0.00 |
| ATOM | 1064 | 3HD1 | ILE | 54 | 0.480 | -6.716 | 2.507 | 1.00 | 0.00 |
| ATOM | 1065 | C | ILE | 54 | 1.593 | -2.842 | -0.863 | 1.00 | 0.00 |
| ATOM | 1066 | O | ILE | 54 | 2.589 | -3.509 | -0.594 | 1.00 | 0.00 |
| ATOM | 1067 | N | ILE | 55 | 1.628 | -1.729 | -1.577 | 1.00 | 0.00 |
| ATOM | 1068 | H | ILE | 55 | 0.783 | -1.267 | -1.764 | 1.00 | 0.00 |

(fortgesetzt)

Atomkooerdinaten der Struktur der ADAP hSH3-1-Domäne, oxidierte Form

| ATOM | 1069 | CA | ILE | 55 | 2.867 | -1.188 | -2.118 | 1.00 | 0.00 |
|------|------|-----|------|----|--------|--------|--------|------|------|
| ATOM | 1070 | HA | ILE | 55 | 3.141 | -1.825 | -2.943 | 1.00 | 0.00 |
| ATOM | 1071 | CB | ILE | 55 | 2.641 | 0.241 | -2.687 | 1.00 | 0.00 |
| ATOM | 1072 | HB | ILE | 55 | 1.842 | 0.700 | -2.128 | 1.00 | 0.00 |
| ATOM | 1073 | QG2 | ILE | 55 | 4.171 | 1.334 | -2.514 | 1.00 | 0.00 |
| ATOM | 1074 | CG2 | ILE | 55 | 3.880 | 1.123 | -2.547 | 1.00 | 0.00 |
| ATOM | 1075 | 1HG2 | ILE | 55 | 4.769 | 0.522 | -2.653 | 1.00 | 0.00 |
| ATOM | 1076 | 2HG2 | ILE | 55 | 3.879 | 1.596 | -1.576 | 1.00 | 0.00 |
| ATOM | 1077 | 3HG2 | ILE | 55 | 3.867 | 1.883 | -3.314 | 1.00 | 0.00 |
| ATOM | 1078 | CG1 | ILE | 55 | 2.226 | 0.156 | -4.160 | 1.00 | 0.00 |
| ATOM | 1079 | 1HG1 | ILE | 55 | 3.085 | -0.126 | -4.752 | 1.00 | 0.00 |
| ATOM | 1080 | 2HG1 | ILE | 55 | 1.877 | 1.124 | -4.485 | 1.00 | 0.00 |
| ATOM | 1081 | QG1 | ILE | 55 | 2.481 | 0.499 | -4.619 | 1.00 | 0.00 |
| ATOM | 1082 | QD1 | ILE | 55 | 0.864 | -1.087 | -4.496 | 1.00 | 0.00 |
| ATOM | 1083 | CD1 | ILE | 55 | 1.126 | -0.847 | -4.431 | 1.00 | 0.00 |
| ATOM | 1084 | 1HD1 | ILE | 55 | 1.543 | -1.844 | -4.445 | 1.00 | 0.00 |
| ATOM | 1085 | 2HD1 | ILE | 55 | 0.671 | -0.635 | -5.387 | 1.00 | 0.00 |
| ATOM | 1086 | 3HD1 | ILE | 55 | 0.378 | -0.782 | -3.655 | 1.00 | 0.00 |
| ATOM | 1087 | C | ILE | 55 | 4.023 | -1.203 | -1.103 | 1.00 | 0.00 |
| ATOM | 1088 | O | ILE | 55 | 4.905 | -2.055 | -1.182 | 1.00 | 0.00 |
| ATOM | 1089 | N | ARG+ | 56 | 4.041 | -0.252 | -0.170 | 1.00 | 0.00 |
| ATOM | 1090 | H | ARG+ | 56 | 3.328 | 0.420 | -0.153 | 1.00 | 0.00 |
| ATOM | 1091 | CA | ARG+ | 56 | 5.125 | -0.182 | 0.812 | 1.00 | 0.00 |
| ATOM | 1092 | HA | ARG+ | 56 | 5.865 | -0.918 | 0.534 | 1.00 | 0.00 |
| ATOM | 1093 | CB | ARG+ | 56 | 5.781 | 1.201 | 0.778 | 1.00 | 0.00 |
| ATOM | 1094 | 1HB | ARG+ | 56 | 5.532 | 1.728 | 1.688 | 1.00 | 0.00 |
| ATOM | 1095 | 2HB | ARG+ | 56 | 5.389 | 1.752 | -0.064 | 1.00 | 0.00 |
| ATOM | 1096 | QB | ARG+ | 56 | 5.461 | 1.740 | 0.812 | 1.00 | 0.00 |
| ATOM | 1097 | CG | ARG+ | 56 | 7.296 | 1.155 | 0.654 | 1.00 | 0.00 |
| ATOM | 1098 | 1HG | ARG+ | 56 | 7.688 | 2.154 | 0.773 | 1.00 | 0.00 |
| ATOM | 1099 | 2HG | ARG+ | 56 | 7.555 | 0.780 | -0.324 | 1.00 | 0.00 |
| ATOM | 1100 | QG | ARG+ | 56 | 7.621 | 1.467 | 0.225 | 1.00 | 0.00 |
| ATOM | 1101 | CD | ARG+ | 56 | 7.920 | 0.250 | 1.707 | 1.00 | 0.00 |
| ATOM | 1102 | 1HD | ARG+ | 56 | 8.211 | -0.678 | 1.237 | 1.00 | 0.00 |
| ATOM | 1103 | 2HD | ARG+ | 56 | 7.187 | 0.049 | 2.474 | 1.00 | 0.00 |
| ATOM | 1104 | QD | ARG+ | 56 | 7.699 | -0.315 | 1.855 | 1.00 | 0.00 |
| ATOM | 1105 | NE | ARG+ | 56 | 9.097 | 0.858 | 2.324 | 1.00 | 0.00 |
| ATOM | 1106 | HE | ARG+ | 56 | 9.960 | 0.414 | 2.186 | 1.00 | 0.00 |
| ATOM | 1107 | CZ | ARG+ | 56 | 9.062 | 1.971 | 3.053 | 1.00 | 0.00 |
| ATOM | 1108 | NH1 | ARG+ | 56 | 7.912 | 2.600 | 3.263 | 1.00 | 0.00 |
| ATOM | 1109 | 1HH1 | ARG+ | 56 | 7.065 | 2.239 | 2.874 | 1.00 | 0.00 |
| ATOM | 1110 | 2HH1 | ARG+ | 56 | 7.893 | 3.437 | 3.811 | 1.00 | 0.00 |
| ATOM | 1111 | QH1 | ARG+ | 56 | 7.479 | 2.838 | 3.342 | 1.00 | 0.00 |
| ATOM | 1112 | NH2 | ARG+ | 56 | 10.180 | 2.457 | 3.574 | 1.00 | 0.00 |
| ATOM | 1113 | 1HH2 | ARG+ | 56 | 11.049 | 1.987 | 3.419 | 1.00 | 0.00 |
| ATOM | 1114 | 2HH2 | ARG+ | 56 | 10.154 | 3.293 | 4.121 | 1.00 | 0.00 |
| ATOM | 1115 | QH2 | ARG+ | 56 | 10.601 | 2.640 | 3.770 | 1.00 | 0.00 |
| ATOM | 1116 | C | ARG+ | 56 | 4.642 | -0.490 | 2.224 | 1.00 | 0.00 |
| ATOM | 1117 | O | ARG+ | 56 | 3.887 | 0.282 | 2.815 | 1.00 | 0.00 |
| ATOM | 1118 | N | ILE | 57 | 5.092 | -1.621 | 2.765 | 1.00 | 0.00 |

(fortgesetzt)

Atomkooerdinaten der Struktur der ADAP hSH3-1-Domäne, oxidierte Form

| ATOM | 1119 | H | ILE | 57 | 5.697 | -2.192 | 2.248 | 1.00 | 0.00 |
|------|------|------|------|------|--------|--------|--------|------|------|
| ATOM | 1120 | CA | ILE | 57 | 4.711 | -2.021 | 4.112 | 1.00 | 0.00 |
| ATOM | 1121 | HA | ILE | 57 | 4.237 | -1.175 | 4.587 | 1.00 | 0.00 |
| ATOM | 1122 | CB | ILE | 57 | 3.689 | -3.155 | 4.081 | 1.00 | 0.00 |
| ATOM | 1123 | HB | ILE | 57 | 3.477 | -3.446 | 5.098 | 1.00 | 0.00 |
| ATOM | 1124 | QG2 | ILE | 57 | 2.099 | -2.514 | 3.304 | 1.00 | 0.00 |
| ATOM | 1125 | CG2 | ILE | 57 | 2.401 | -2.638 | 3.455 | 1.00 | 0.00 |
| ATOM | 1126 | 1HG2 | ILE | 57 | 2.079 | -1.751 | 3.976 | 1.00 | 0.00 |
| ATOM | 1127 | 2HG2 | ILE | 57 | 1.638 | -3.395 | 3.518 | 1.00 | 0.00 |
| ATOM | 1128 | 3HG2 | ILE | 57 | 2.581 | -2.396 | 2.418 | 1.00 | 0.00 |
| ATOM | 1129 | CG1 | ILE | 57 | 4.236 | -4.363 | 3.313 | 1.00 | 0.00 |
| ATOM | 1130 | 1HG1 | ILE | 57 | 3.898 | -5.270 | 3.793 | 1.00 | 0.00 |
| ATOM | 1131 | 2HG1 | ILE | 57 | 5.313 | -4.335 | 3.327 | 1.00 | 0.00 |
| ATOM | 1132 | QG1 | ILE | 57 | 4.605 | -4.802 | 3.560 | 1.00 | 0.00 |
| ATOM | 1133 | QD1 | ILE | 57 | 3.695 | -4.420 | 1.529 | 1.00 | 0.00 |
| ATOM | 1134 | CD1 | ILE | 57 | 3.800 | -4.411 | 1.870 | 1.00 | 0.00 |
| ATOM | 1135 | 1HD1 | ILE | 57 | 3.927 | -5.411 | 1.488 | 1.00 | 0.00 |
| ATOM | 1136 | 2HD1 | ILE | 57 | 4.398 | -3.721 | 1.295 | 1.00 | 0.00 |
| ATOM | 1137 | 3HD1 | ILE | 57 | 2.760 | -4.128 | 1.803 | 1.00 | 0.00 |
| ATOM | 1138 | C | ILE | 57 | 5.915 | -2.420 | 4.957 | 1.00 | 0.00 |
| ATOM | 1139 | O | ILE | 57 | 6.391 | -3.552 | 4.906 | 1.00 | 0.00 |
| ATOM | 1140 | N | THR | 58 | 6.381 | -1.470 | 5.750 | 1.00 | 0.00 |
| ATOM | 1141 | H | THR | 58 | 5.940 | -0.597 | 5.746 | 1.00 | 0.00 |
| ATOM | 1142 | CA | THR | 58 | 7.513 | -1.684 | 6.641 | 1.00 | 0.00 |
| ATOM | 1143 | HA | THR | 58 | 8.080 | -2.525 | 6.277 | 1.00 | 0.00 |
| ATOM | 1144 | CB | THR | 58 | 8.409 | -0.442 | 6.665 | 1.00 | 0.00 |
| ATOM | 1145 | HB | THR | 58 | 8.157 | 0.154 | 7.530 | 1.00 | 0.00 |
| ATOM | 1146 | QG2 | THR | 58 | 10.236 | -0.849 | 6.762 | 1.00 | 0.00 |
| ATOM | 1147 | OG1 | THR | 58 | 8.206 | 0.346 | 5.506 | 1.00 | 0.00 |
| ATOM | 1148 | 1HG | THR | 58 | 8.102 | 1.266 | 5.759 | 1.00 | 0.00 |
| ATOM | 1149 | CG2 | THR | 58 | 9.884 | -0.770 | 6.743 | 1.00 | 0.00 |
| ATOM | 1150 | 1HG2 | THR | 58 | 10.017 | -1.842 | 6.717 | 1.00 | 0.00 |
| ATOM | 1151 | 2HG2 | THR | 58 | 10.292 | -0.381 | 7.664 | 1.00 | 0.00 |
| ATOM | 1152 | 3HG2 | THR | 58 | 10.398 | -0.324 | 5.905 | 1.00 | 0.00 |
| ATOM | 1153 | C | THR | 58 | 7.004 | -2.003 | 8.040 | 1.00 | 0.00 |
| ATOM | 1154 | O | THR | 58 | 5.877 | -2.469 | 8.204 | 1.00 | 0.00 |
| ATOM | 1155 | N | ASP | 59 | 7.825 | -1.744 | 9.049 | 1.00 | 0.00 |
| ATOM | 1156 | H | ASP | 59 | 8.709 | -1.368 | 8.863 | 1.00 | 0.00 |
| ATOM | 1157 | CA | ASP | 59 | 7.429 | -2.000 | 10.431 | 1.00 | 0.00 |
| ATOM | 1158 | HA | ASP | 59 | 7.347 | -3.068 | 10.559 | 1.00 | 0.00 |
| ATOM | 1159 | CB | ASP | 59 | 8.486 | -1.460 | 11.397 | 1.00 | 0.00 |
| ATOM | 1160 | 1HB | ASP | 59 | 7.997 | -1.095 | 12.289 | 1.00 | 0.00 |
| ATOM | 1161 | 2HB | ASP | 59 | 9.017 | -0.648 | 10.925 | 1.00 | 0.00 |
| ATOM | 1162 | QB | ASP | 59 | 8.507 | -0.872 | 11.607 | 1.00 | 0.00 |
| ATOM | 1163 | CG | ASP | 59 | 9.493 | -2.520 | 11.802 | 1.00 | 0.00 |
| ATOM | 1164 | OD1 | ASP | 59 | 9.916 | -3.302 | 10.925 | 1.00 | 0.00 |
| ATOM | 1165 | OD2 | ASP | 59 | 9.857 | -2.567 | 12.995 | 1.00 | 0.00 |
| ATOM | 1166 | C | ASP | 59 | 6.071 | -1.360 | 10.730 | 1.00 | 0.00 |
| ATOM | 1167 | O | ASP | 59 | 5.371 | -1.771 | 11.654 | 1.00 | 0.00 |
| ATOM | 1168 | N | ASN | 60 | 5.698 | -0.362 | 9.926 | 1.00 | 0.00 |

(fortgesetzt)

Atomkooerdinaten der Struktur der ADAP hSH3-1-Domäne, oxidierte Form

| ATOM | 1169 | H | ASN | 60 | 6.291 | -0.086 | 9.198 | 1.00 | 0.00 |
|------|------|------|-----|----|-------|--------|-------|------|------|
| ATOM | 1170 | CA | ASN | 60 | 4.423 | 0.320 | 10.086 | 1.00 | 0.00 |
| ATOM | 1171 | HA | ASN | 60 | 4.391 | 1.131 | 9.373 | 1.00 | 0.00 |
| ATOM | 1172 | CB | ASN | 60 | 3.272 | -0.642 | 9.784 | 1.00 | 0.00 |
| ATOM | 1173 | 1HB | ASN | 60 | 2.525 | -0.555 | 10.559 | 1.00 | 0.00 |
| ATOM | 1174 | 2HB | ASN | 60 | 3.652 | -1.652 | 9.768 | 1.00 | 0.00 |
| ATOM | 1175 | QB | ASN | 60 | 3.088 | -1.103 | 10.163 | 1.00 | 0.00 |
| ATOM | 1176 | CG | ASN | 60 | 2.615 | -0.355 | 8.448 | 1.00 | 0.00 |
| ATOM | 1177 | OD1 | ASN | 60 | 1.393 | -0.409 | 8.322 | 1.00 | 0.00 |
| ATOM | 1178 | ND2 | ASN | 60 | 3.427 | -0.048 | 7.443 | 1.00 | 0.00 |
| ATOM | 1179 | 1HD2 | ASN | 60 | 4.391 | -0.024 | 7.617 | 1.00 | 0.00 |
| ATOM | 1180 | 2HD2 | ASN | 60 | 3.028 | 0.142 | 6.568 | 1.00 | 0.00 |
| ATOM | 1181 | QD2 | ASN | 60 | 3.709 | 0.059 | 7.092 | 1.00 | 0.00 |
| ATOM | 1182 | C | ASN | 60 | 4.265 | 0.897 | 11.491 | 1.00 | 0.00 |
| ATOM | 1183 | O | ASN | 60 | 3.762 | 0.225 | 12.390 | 1.00 | 0.00 |
| ATOM | 1184 | N | PRO | 61 | 4.677 | 2.160 | 11.695 | 1.00 | 0.00 |
| ATOM | 1185 | CD | PRO | 61 | 5.269 | 3.047 | 10.678 | 1.00 | 0.00 |
| ATOM | 1186 | CA | PRO | 61 | 4.554 | 2.819 | 12.997 | 1.00 | 0.00 |
| ATOM | 1187 | HA | PRO | 61 | 5.064 | 2.265 | 13.773 | 1.00 | 0.00 |
| ATOM | 1188 | CB | PRO | 61 | 5.237 | 4.173 | 12.787 | 1.00 | 0.00 |
| ATOM | 1189 | 1HB | PRO | 61 | 6.256 | 4.125 | 13.142 | 1.00 | 0.00 |
| ATOM | 1190 | 2HB | PRO | 61 | 4.698 | 4.938 | 13.327 | 1.00 | 0.00 |
| ATOM | 1191 | QB | PRO | 61 | 5.477 | 4.531 | 13.235 | 1.00 | 0.00 |
| ATOM | 1192 | CG | PRO | 61 | 5.186 | 4.406 | 11.316 | 1.00 | 0.00 |
| ATOM | 1193 | 1HG | PRO | 61 | 6.024 | 5.015 | 11.011 | 1.00 | 0.00 |
| ATOM | 1194 | 2HG | PRO | 61 | 4.255 | 4.886 | 11.052 | 1.00 | 0.00 |
| ATOM | 1195 | QG | PRO | 61 | 5.139 | 4.951 | 11.031 | 1.00 | 0.00 |
| ATOM | 1196 | 1HD | PRO | 61 | 6.299 | 2.781 | 10.488 | 1.00 | 0.00 |
| ATOM | 1197 | 2HD | PRO | 61 | 4.697 | 3.024 | 9.763 | 1.00 | 0.00 |
| ATOM | 1198 | QD | PRO | 61 | 5.498 | 2.902 | 10.126 | 1.00 | 0.00 |
| ATOM | 1199 | C | PRO | 61 | 3.093 | 3.006 | 13.380 | 1.00 | 0.00 |
| ATOM | 1200 | O | PRO | 61 | 2.713 | 2.836 | 14.538 | 1.00 | 0.00 |
| ATOM | 1201 | N | GLU | 62 | 2.277 | 3.348 | 12.388 | 1.00 | 0.00 |
| ATOM | 1202 | H | GLU | 62 | 2.641 | 3.462 | 11.489 | 1.00 | 0.00 |
| ATOM | 1203 | CA | GLU | 62 | 0.855 | 3.550 | 12.599 | 1.00 | 0.00 |
| ATOM | 1204 | HA | GLU | 62 | 0.724 | 3.959 | 13.586 | 1.00 | 0.00 |
| ATOM | 1205 | CB | GLU | 62 | 0.305 | 4.539 | 11.569 | 1.00 | 0.00 |
| ATOM | 1206 | 1HB | GLU | 62 | 0.947 | 5.407 | 11.549 | 1.00 | 0.00 |
| ATOM | 1207 | 2HB | GLU | 62 | -0.686 | 4.842 | 11.871 | 1.00 | 0.00 |
| ATOM | 1208 | QB | GLU | 62 | 0.130 | 5.125 | 11.710 | 1.00 | 0.00 |
| ATOM | 1209 | CG | GLU | 62 | 0.224 | 3.976 | 10.158 | 1.00 | 0.00 |
| ATOM | 1210 | 1HG | GLU | 62 | -0.448 | 3.131 | 10.160 | 1.00 | 0.00 |
| ATOM | 1211 | 2HG | GLU | 62 | 1.209 | 3.651 | 9.857 | 1.00 | 0.00 |
| ATOM | 1212 | QG | GLU | 62 | 0.380 | 3.391 | 10.008 | 1.00 | 0.00 |
| ATOM | 1213 | CD | GLU | 62 | -0.278 | 4.994 | 9.154 | 1.00 | 0.00 |
| ATOM | 1214 | OE1 | GLU | 62 | 0.475 | 5.319 | 8.211 | 1.00 | 0.00 |
| ATOM | 1215 | OE2 | GLU | 62 | -1.423 | 5.468 | 9.308 | 1.00 | 0.00 |
| ATOM | 1216 | C | GLU | 62 | 0.109 | 2.221 | 12.508 | 1.00 | 0.00 |
| ATOM | 1217 | O | GLU | 62 | -0.892 | 2.010 | 13.193 | 1.00 | 0.00 |
| ATOM | 1218 | N | GLY | 63 | 0.612 | 1.324 | 11.662 | 1.00 | 0.00 |

(fortgesetzt)

Atomkooerdinaten der Struktur der ADAP hSH3-1-Domäne, oxidierte Form

| ATOM | 1219 | H   | GLY  | 63 | 1.413  | 1.544  | 11.149 | 1.00 | 0.00 |
|------|------|-----|------|----|--------|--------|--------|------|------|
| ATOM | 1220 | CA  | GLY  | 63 | -0.009 | 0.027  | 11.503 | 1.00 | 0.00 |
| ATOM | 1221 | 1HA | GLY  | 63 | -0.524 | -0.216 | 12.415 | 1.00 | 0.00 |
| ATOM | 1222 | 2HA | GLY  | 63 | 0.761  | -0.708 | 11.331 | 1.00 | 0.00 |
| ATOM | 1223 | QA  | GLY  | 63 | 0.119  | -0.462 | 11.873 | 1.00 | 0.00 |
| ATOM | 1224 | C   | GLY  | 63 | -0.997 | -0.023 | 10.353 | 1.00 | 0.00 |
| ATOM | 1225 | O   | GLY  | 63 | -2.004 | -0.726 | 10.430 | 1.00 | 0.00 |
| ATOM | 1226 | N   | LYS+ | 64 | -0.727 | 0.728  | 9.286  | 1.00 | 0.00 |
| ATOM | 1227 | H   | LYS+ | 64 | 0.083  | 1.279  | 9.275  | 1.00 | 0.00 |
| ATOM | 1228 | CA  | LYS+ | 64 | -1.631 | 0.748  | 8.140  | 1.00 | 0.00 |
| ATOM | 1229 | HA  | LYS+ | 64 | -2.331 | -0.056 | 8.276  | 1.00 | 0.00 |
| ATOM | 1230 | CB  | LYS+ | 64 | -2.400 | 2.063  | 8.103  | 1.00 | 0.00 |
| ATOM | 1231 | 1HB | LYS+ | 64 | -3.240 | 1.951  | 7.432  | 1.00 | 0.00 |
| ATOM | 1232 | 2HB | LYS+ | 64 | -1.749 | 2.836  | 7.724  | 1.00 | 0.00 |
| ATOM | 1233 | QB  | LYS+ | 64 | -2.494 | 2.393  | 7.578  | 1.00 | 0.00 |
| ATOM | 1234 | CG  | LYS+ | 64 | -2.930 | 2.510  | 9.459  | 1.00 | 0.00 |
| ATOM | 1235 | 1HG | LYS+ | 64 | -2.598 | 3.521  | 9.646  | 1.00 | 0.00 |
| ATOM | 1236 | 2HG | LYS+ | 64 | -2.536 | 1.856  | 10.222 | 1.00 | 0.00 |
| ATOM | 1237 | QG  | LYS+ | 64 | -2.567 | 2.689  | 9.934  | 1.00 | 0.00 |
| ATOM | 1238 | CD  | LYS+ | 64 | -4.451 | 2.468  | 9.514  | 1.00 | 0.00 |
| ATOM | 1239 | 1HD | LYS+ | 64 | -4.754 | 1.720  | 10.233 | 1.00 | 0.00 |
| ATOM | 1240 | 2HD | LYS+ | 64 | -4.832 | 2.208  | 8.539  | 1.00 | 0.00 |
| ATOM | 1241 | QD  | LYS+ | 64 | -4.793 | 1.964  | 9.386  | 1.00 | 0.00 |
| ATOM | 1242 | CE  | LYS+ | 64 | -5.031 | 3.812  | 9.926  | 1.00 | 0.00 |
| ATOM | 1243 | 1HE | LYS+ | 64 | -4.295 | 4.344  | 10.511 | 1.00 | 0.00 |
| ATOM | 1244 | 2HE | LYS+ | 64 | -5.911 | 3.640  | 10.527 | 1.00 | 0.00 |
| ATOM | 1245 | QE  | LYS+ | 64 | -5.103 | 3.992  | 10.519 | 1.00 | 0.00 |
| ATOM | 1246 | NZ  | LYS+ | 64 | -5.405 | 4.641  | 8.797  | 1.00 | 0.00 |
| ATOM | 1247 | 1HZ | LYS+ | 64 | -6.278 | 5.171  | 8.945  | 1.00 | 0.00 |
| ATOM | 1248 | 2HZ | LYS+ | 64 | -5.563 | 4.033  | 7.917  | 1.00 | 0.00 |
| ATOM | 1249 | 3HZ | LYS+ | 64 | -4.644 | 5.315  | 8.528  | 1.00 | 0.00 |
| ATOM | 1250 | QZ  | LYS+ | 64 | -5.495 | 4.840  | 8.464  | 1.00 | 0.00 |
| ATOM | 1251 | C   | LYS+ | 64 | -0.890 | 0.525  | 6.816  | 1.00 | 0.00 |
| ATOM | 1252 | O   | LYS+ | 64 | 0.238  | 0.984  | 6.641  | 1.00 | 0.00 |
| ATOM | 1253 | N   | TRP  | 65 | -1.533 | -0.207 | 5.896  | 1.00 | 0.00 |
| ATOM | 1254 | H   | TRP  | 65 | -2.421 | -0.560 | 6.108  | 1.00 | 0.00 |
| ATOM | 1255 | CA  | TRP  | 65 | -0.934 | -0.529 | 4.593  | 1.00 | 0.00 |
| ATOM | 1256 | HA  | TRP  | 65 | 0.077  | -0.143 | 4.595  | 1.00 | 0.00 |
| ATOM | 1257 | CB  | TRP  | 65 | -0.847 | -2.038 | 4.431  | 1.00 | 0.00 |
| ATOM | 1258 | 1HB | TRP  | 65 | -0.576 | -2.264 | 3.417  | 1.00 | 0.00 |
| ATOM | 1259 | 2HB | TRP  | 65 | -1.789 | -2.503 | 4.658  | 1.00 | 0.00 |
| ATOM | 1260 | QB  | TRP  | 65 | -1.182 | -2.384 | 4.037  | 1.00 | 0.00 |
| ATOM | 1261 | CG  | TRP  | 65 | 0.189  | -2.618 | 5.310  | 1.00 | 0.00 |
| ATOM | 1262 | CD1 | TRP  | 65 | 1.091  | -1.922 | 6.058  | 1.00 | 0.00 |
| ATOM | 1263 | CD2 | TRP  | 65 | 0.457  | -3.998 | 5.519  | 1.00 | 0.00 |
| ATOM | 1264 | CE3 | TRP  | 65 | -0.118 | -5.183 | 5.054  | 1.00 | 0.00 |
| ATOM | 1265 | CE2 | TRP  | 65 | 1.558  | -4.068 | 6.380  | 1.00 | 0.00 |
| ATOM | 1266 | NE1 | TRP  | 65 | 1.931  | -2.789 | 6.695  | 1.00 | 0.00 |
| ATOM | 1267 | HD  | TRP  | 65 | 1.131  | -0.839 | 6.112  | 1.00 | 0.00 |
| ATOM | 1268 | 3HE | TRP  | 65 | -0.979 | -5.170 | 4.400  | 1.00 | 0.00 |

(fortgesetzt)

Atomkooerdinaten der Struktur der ADAP hSH3-1-Domäne, oxidierte Form

| ATOM | 1269 | CZ3 | TRP | 65 | 0.425 | -6.385 | 5.460 | 1.00 | 0.00 |
|---|---|---|---|---|---|---|---|---|---|
| ATOM | 1270 | CZ2 | TRP | 65 | 2.102 | -5.265 | 6.780 | 1.00 | 0.00 |
| ATOM | 1271 | 1HE | TRP | 65 | 2.675 | -2.539 | 7.281 | 1.00 | 0.00 |
| ATOM | 1272 | 3HZ | TRP | 65 | 0.000 | -7.314 | 5.109 | 1.00 | 0.00 |
| ATOM | 1273 | CH2 | TRP | 65 | 1.531 | -6.418 | 6.314 | 1.00 | 0.00 |
| ATOM | 1274 | 2HZ | TRP | 65 | 2.951 | -5.293 | 7.423 | 1.00 | 0.00 |
| ATOM | 1275 | HH | TRP | 65 | 1.932 | -7.376 | 6.604 | 1.00 | 0.00 |
| ATOM | 1276 | C | TRP | 65 | -1.675 | 0.076 | 3.400 | 1.00 | 0.00 |
| ATOM | 1277 | O | TRP | 65 | -2.816 | 0.511 | 3.505 | 1.00 | 0.00 |
| ATOM | 1278 | N | LEU | 66 | -0.993 | 0.086 | 2.254 | 1.00 | 0.00 |
| ATOM | 1279 | H | LEU | 66 | -0.083 | -0.282 | 2.244 | 1.00 | 0.00 |
| ATOM | 1280 | CA | LEU | 66 | -1.547 | 0.622 | 1.012 | 1.00 | 0.00 |
| ATOM | 1281 | HA | LEU | 66 | -2.444 | 1.173 | 1.246 | 1.00 | 0.00 |
| ATOM | 1282 | CB | LEU | 66 | -0.541 | 1.548 | 0.350 | 1.00 | 0.00 |
| ATOM | 1283 | 1HB | LEU | 66 | -0.897 | 1.784 | -0.641 | 1.00 | 0.00 |
| ATOM | 1284 | 2HB | LEU | 66 | 0.387 | 1.005 | 0.260 | 1.00 | 0.00 |
| ATOM | 1285 | QB | LEU | 66 | -0.255 | 1.394 | -0.191 | 1.00 | 0.00 |
| ATOM | 1286 | CG | LEU | 66 | -0.281 | 2.858 | 1.089 | 1.00 | 0.00 |
| ATOM | 1287 | HG | LEU | 66 | 0.280 | 3.523 | 0.449 | 1.00 | 0.00 |
| ATOM | 1288 | QD1 | LEU | 66 | -1.913 | 3.688 | 1.508 | 1.00 | 0.00 |
| ATOM | 1289 | QD2 | LEU | 66 | 0.729 | 2.549 | 2.647 | 1.00 | 0.00 |
| ATOM | 1290 | CD1 | LEU | 66 | -1.600 | 3.532 | 1.928 | 1.00 | 0.00 |
| ATOM | 1291 | 1HD1 | LEU | 66 | -2.140 | 3.749 | 0.515 | 1.00 | 0.00 |
| ATOM | 1292 | 2HD1 | LEU | 66 | -1.411 | 4.447 | 1.964 | 1.00 | 0.00 |
| ATOM | 1293 | 3HD1 | LEU | 66 | -2.188 | 2.869 | 2.045 | 1.00 | 0.00 |
| ATOM | 1294 | CD2 | LEU | 66 | 0.535 | 2.608 | 2.349 | 1.00 | 0.00 |
| ATOM | 1295 | 1HD2 | LEU | 66 | 1.364 | 1.955 | 2.117 | 1.00 | 0.00 |
| ATOM | 1296 | 2HD2 | LEU | 66 | -0.090 | 2.146 | 3.098 | 1.00 | 0.00 |
| ATOM | 1297 | 3HD2 | LEU | 66 | 0.912 | 3.548 | 2.726 | 1.00 | 0.00 |
| ATOM | 1298 | QQD | LEU | 66 | -0.592 | 3.119 | 2.078 | 1.00 | 0.00 |
| ATOM | 1299 | C | LEU | 66 | -1.883 | -0.495 | 0.040 | 1.00 | 0.00 |
| ATOM | 1300 | O | LEU | 66 | -1.406 | -0.510 | -1.096 | 1.00 | 0.00 |
| ATOM | 1301 | N | GLY | 67 | -2.688 | -1.433 | 0.489 | 1.00 | 0.00 |
| ATOM | 1302 | H | GLY | 67 | -3.032 | -1.380 | 1.405 | 1.00 | 0.00 |
| ATOM | 1303 | CA | GLY | 67 | -3.051 | -2.544 | -0.355 | 1.00 | 0.00 |
| ATOM | 1304 | 1HA | GLY | 67 | -3.660 | -3.213 | 0.209 | 1.00 | 0.00 |
| ATOM | 1305 | 2HA | GLY | 67 | -2.157 | -3.062 | -0.646 | 1.00 | 0.00 |
| ATOM | 1306 | QA | GLY | 67 | -2.908 | -3.137 | -0.219 | 1.00 | 0.00 |
| ATOM | 1307 | C | GLY | 67 | -3.794 | -2.128 | -1.610 | 1.00 | 0.00 |
| ATOM | 1308 | O | GLY | 67 | -5.019 | -2.017 | -1.611 | 1.00 | 0.00 |
| ATOM | 1309 | N | ARG+ | 68 | -3.044 | -1.910 | -2.684 | 1.00 | 0.00 |
| ATOM | 1310 | H | ARG+ | 68 | -2.077 | -2.017 | -2.613 | 1.00 | 0.00 |
| ATOM | 1311 | CA | ARG+ | 68 | -3.623 | -1.510 | -3.961 | 1.00 | 0.00 |
| ATOM | 1312 | HA | ARG+ | 68 | -4.412 | -0.814 | -3.767 | 1.00 | 0.00 |
| ATOM | 1313 | CB | ARG+ | 68 | -2.573 | -0.827 | -4.825 | 1.00 | 0.00 |
| ATOM | 1314 | 1HB | ARG+ | 68 | -1.836 | -1.556 | -5.100 | 1.00 | 0.00 |
| ATOM | 1315 | 2HB | ARG+ | 68 | -2.099 | -0.048 | -4.250 | 1.00 | 0.00 |
| ATOM | 1316 | QB | ARG+ | 68 | -1.968 | -0.802 | -4.675 | 1.00 | 0.00 |
| ATOM | 1317 | CG | ARG+ | 68 | -3.134 | -0.213 | -6.097 | 1.00 | 0.00 |
| ATOM | 1318 | 1HG | ARG+ | 68 | -3.734 | 0.646 | -5.836 | 1.00 | 0.00 |

(fortgesetzt)

Atomkooerdinaten der Struktur der ADAP hSH3-1-Domäne, oxidierte Form

| | | | | | | | | | |
|---|---|---|---|---|---|---|---|---|---|
| ATOM | 1319 | 2HG | ARG+ | 68 | -3.750 | -0.945 | -6.597 | 1.00 | 0.00 |
| ATOM | 1320 | QG | ARG+ | 68 | -3.742 | -0.150 | -6.217 | 1.00 | 0.00 |
| ATOM | 1321 | CD | ARG+ | 68 | -2.027 | 0.225 | -7.043 | 1.00 | 0.00 |
| ATOM | 1322 | 1HD | ARG+ | 68 | -1.103 | -0.246 | -6.739 | 1.00 | 0.00 |
| ATOM | 1323 | 2HD | ARG+ | 68 | -1.920 | 1.298 | -6.979 | 1.00 | 0.00 |
| ATOM | 1324 | QD | ARG+ | 68 | -1.511 | 0.526 | -6.859 | 1.00 | 0.00 |
| ATOM | 1325 | NE | ARG+ | 68 | -2.313 | -0.141 | -8.428 | 1.00 | 0.00 |
| ATOM | 1326 | HE | ARG+ | 68 | -1.750 | -0.829 | -8.840 | 1.00 | 0.00 |
| ATOM | 1327 | CZ | ARG+ | 68 | -3.288 | 0.403 | -9.152 | 1.00 | 0.00 |
| ATOM | 1328 | NH1 | ARG+ | 68 | -4.071 | 1.338 | -8.628 | 1.00 | 0.00 |
| ATOM | 1329 | 1HH1 | ARG+ | 68 | -3.931 | 1.637 | -7.684 | 1.00 | 0.00 |
| ATOM | 1330 | 2HH1 | ARG+ | 68 | -4.802 | 1.744 | -9.176 | 1.00 | 0.00 |
| ATOM | 1331 | QH1 | ARG+ | 68 | -4.366 | 1.691 | -8.430 | 1.00 | 0.00 |
| ATOM | 1332 | NH2 | ARG+ | 68 | -3.481 | 0.012 | -10.404 | 1.00 | 0.00 |
| ATOM | 1333 | 1HH2 | ARG+ | 68 | -2.895 | -0.692 | -10.804 | 1.00 | 0.00 |
| ATOM | 1334 | 2HH2 | ARG+ | 68 | -4.214 | 0.421 | -10.948 | 1.00 | 0.00 |
| ATOM | 1335 | QH2 | ARG+ | 68 | -3.554 | -0.136 | -10.876 | 1.00 | 0.00 |
| ATOM | 1336 | C | ARG+ | 68 | -4.193 | -2.718 | -4.697 | 1.00 | 0.00 |
| ATOM | 1337 | O | ARG+ | 68 | -3.618 | -3.196 | -5.675 | 1.00 | 0.00 |
| ATOM | 1338 | N | THR | 69 | -5.331 | -3.208 | -4.206 | 1.00 | 0.00 |
| ATOM | 1339 | H | THR | 69 | -5.725 | -2.779 | -3.421 | 1.00 | 0.00 |
| ATOM | 1340 | CA | THR | 69 | -6.006 | -4.368 | -4.786 | 1.00 | 0.00 |
| ATOM | 1341 | HA | THR | 69 | -5.475 | -5.247 | -4.468 | 1.00 | 0.00 |
| ATOM | 1342 | CB | THR | 69 | -7.446 | -9.999 | -4.274 | 1.00 | 0.00 |
| ATOM | 1343 | HB | THR | 69 | -7.876 | -5.389 | -4.588 | 1.00 | 0.00 |
| ATOM | 1344 | QG2 | THR | 69 | -7.583 | -4.354 | -2.406 | 1.00 | 0.00 |
| ATOM | 1345 | OG1 | THR | 69 | -8.230 | -3.400 | -4.814 | 1.00 | 0.00 |
| ATOM | 1346 | 1HG | THR | 69 | -9.129 | -3.709 | -4.946 | 1.00 | 0.00 |
| ATOM | 1347 | CG2 | THR | 69 | -7.555 | -4.372 | -2.764 | 1.00 | 0.00 |
| ATOM | 1348 | 1HG2 | THR | 69 | -6.925 | -5.126 | -2.320 | 1.00 | 0.00 |
| ATOM | 1349 | 2HG2 | THR | 69 | -8.580 | -4.542 | -2.471 | 1.00 | 0.00 |
| ATOM | 1350 | 3HG2 | THR | 69 | -7.243 | -3.394 | -2.427 | 1.00 | 0.00 |
| ATOM | 1351 | C | THR | 69 | -6.010 | -4.327 | -6.311 | 1.00 | 0.00 |
| ATOM | 1352 | O | THR | 69 | -5.699 | -3.304 | -6.920 | 1.00 | 0.00 |
| ATOM | 1353 | N | ALA | 70 | -6.369 | -5.456 | -6.921 | 1.00 | 0.00 |
| ATOM | 1354 | H | ALA | 70 | -6.609 | -6.235 | -6.378 | 1.00 | 0.00 |
| ATOM | 1355 | CA | ALA | 70 | -6.423 | -5.560 | -8.375 | 1.00 | 0.00 |
| ATOM | 1356 | HA | ALA | 70 | -5.409 | -5.523 | -8.749 | 1.00 | 0.00 |
| ATOM | 1357 | QB | ALA | 70 | -7.175 | -7.209 | -8.883 | 1.00 | 0.00 |
| ATOM | 1358 | CB | ALA | 70 | -7.031 | -6.893 | -8.786 | 1.00 | 0.00 |
| ATOM | 1359 | 1HB | ALA | 70 | -7.576 | -6.770 | -9.710 | 1.00 | 0.00 |
| ATOM | 1360 | 2HB | ALA | 70 | -7.704 | -7.236 | -8.014 | 1.00 | 0.00 |
| ATOM | 1361 | 3HB | ALA | 70 | -6.244 | -7.620 | -8.926 | 1.00 | 0.00 |
| ATOM | 1362 | C | ALA | 70 | -7.215 | -4.406 | -8.977 | 1.00 | 0.00 |
| ATOM | 1363 | O | ALA | 70 | -6.887 | -3.911 | -10.056 | 1.00 | 0.00 |
| ATOM | 1364 | N | ARG+ | 71 | -8.253 | -3.975 | -8.267 | 1.00 | 0.00 |
| ATOM | 1365 | H | ARG+ | 71 | -8.461 | -4.403 | -7.411 | 1.00 | 0.00 |
| ATOM | 1366 | CA | ARG+ | 71 | -9.080 | -2.869 | -8.730 | 1.00 | 0.00 |
| ATOM | 1367 | HA | ARG+ | 71 | -9.363 | -3.072 | -9.753 | 1.00 | 0.00 |
| ATOM | 1368 | CB | ARG+ | 71 | -10.344 | -2.751 | -7.877 | 1.00 | 0.00 |

(fortgesetzt)

Atomkooerdinaten der Struktur der ADAP hSH3-1-Domäne, oxidierte Form

| ATOM | 1369 | 1HB | ARG+ | 71 | -10.177 | -2.012 | -7.107 | 1.00 | 0.00 |
|------|------|------|------|----|---------|--------|--------|------|------|
| ATOM | 1370 | 2HB | ARG+ | 71 | -10.540 | -3.706 | -7.410 | 1.00 | 0.00 |
| ATOM | 1371 | QB | ARG+ | 71 | -10.359 | -2.859 | -7.259 | 1.00 | 0.00 |
| ATOM | 1372 | CG | ARG+ | 71 | -11.576 | -2.346 | -8.669 | 1.00 | 0.00 |
| ATOM | 1373 | 1HG | ARG+ | 71 | -11.910 | -3.190 | -9.254 | 1.00 | 0.00 |
| ATOM | 1374 | 2HG | ARG+ | 71 | -11.315 | -1.530 | -9.327 | 1.00 | 0.00 |
| ATOM | 1375 | QG | ARG+ | 71 | -11.612 | -2.360 | -9.291 | 1.00 | 0.00 |
| ATOM | 1376 | CD | ARG+ | 71 | -12.708 | -1.898 | -7.758 | 1.00 | 0.00 |
| ATOM | 1377 | 1HD | ARG+ | 71 | -12.908 | -2.680 | -7.042 | 1.00 | 0.00 |
| ATOM | 1378 | 2HD | ARG+ | 71 | -13.589 | -1.729 | -8.358 | 1.00 | 0.00 |
| ATOM | 1379 | QD | ARG+ | 71 | -13.248 | -2.205 | -7.700 | 1.00 | 0.00 |
| ATOM | 1380 | NE | ARG+ | 71 | -12.382 | -0.666 | -7.041 | 1.00 | 0.00 |
| ATOM | 1381 | HE | ARG+ | 71 | -12.600 | 0.182 | -7.481 | 1.00 | 0.00 |
| ATOM | 1382 | CZ | ARG+ | 71 | -11.815 | -0.632 | -5.836 | 1.00 | 0.00 |
| ATOM | 1383 | NH1 | ARG+ | 71 | -11.497 | -1.757 | -5.207 | 1.00 | 0.00 |
| ATOM | 1384 | 1HH1 | ARG+ | 71 | -11.680 | -2.641 | -5.635 | 1.00 | 0.00 |
| ATOM | 1385 | 2HH1 | ARG+ | 71 | -11.071 | -1.719 | -4.302 | 1.00 | 0.00 |
| ATOM | 1386 | QH1 | ARG+ | 71 | -11.375 | -2.180 | -4.969 | 1.00 | 0.00 |
| ATOM | 1387 | NH2 | ARG+ | 71 | -11.559 | 0.535 | -5.259 | 1.00 | 0.00 |
| ATOM | 1388 | 1HH2 | ARG+ | 71 | -11.794 | 1.386 | -5.728 | 1.00 | 0.00 |
| ATOM | 1389 | 2HH2 | ARG+ | 71 | -11.134 | 0.563 | -4.355 | 1.00 | 0.00 |
| ATOM | 1390 | QH2 | ARG+ | 71 | -11.969 | 0.975 | -5.041 | 1.00 | 0.00 |
| ATOM | 1391 | C | ARG+ | 71 | -8.295 | -1.564 | -8.685 | 1.00 | 0.00 |
| ATOM | 1392 | O | ARG+ | 71 | -8.573 | -0.633 | -9.441 | 1.00 | 0.00 |
| ATOM | 1393 | N | GLY | 72 | -7.305 | -1.505 | -7.796 | 1.00 | 0.00 |
| ATOM | 1394 | H | GLY | 72 | -7.125 | -2.281 | -7.220 | 1.00 | 0.00 |
| ATOM | 1395 | CA | GLY | 72 | -6.489 | -0.316 | -7.675 | 1.00 | 0.00 |
| ATOM | 1396 | 1HA | GLY | 72 | -6.594 | 0.275 | -8.569 | 1.00 | 0.00 |
| ATOM | 1397 | 2HA | GLY | 72 | -5.458 | -0.616 | -7.571 | 1.00 | 0.00 |
| ATOM | 1398 | QA | GLY | 72 | -6.026 | -0.171 | -8.070 | 1.00 | 0.00 |
| ATOM | 1399 | C | GLY | 72 | -6.864 | 0.529 | -6.481 | 1.00 | 0.00 |
| ATOM | 1400 | O | GLY | 72 | -6.767 | 1.755 | -6.520 | 1.00 | 0.00 |
| ATOM | 1401 | N | SER | 73 | -7.284 | -0.131 | -5.412 | 1.00 | 0.00 |
| ATOM | 1402 | H | SER | 73 | -7.330 | -1.105 | -5.441 | 1.00 | 0.00 |
| ATOM | 1403 | CA | SER | 73 | -7.663 | 0.561 | -4.194 | 1.00 | 0.00 |
| ATOM | 1404 | HA | SER | 73 | -8.103 | 1.494 | -4.472 | 1.00 | 0.00 |
| ATOM | 1405 | CB | SER | 73 | -8.681 | -0.258 | -3.409 | 1.00 | 0.00 |
| ATOM | 1406 | 1HB | SER | 73 | -8.159 | -0.928 | -2.757 | 1.00 | 0.00 |
| ATOM | 1407 | 2HB | SER | 73 | -9.286 | -0.828 | -4.092 | 1.00 | 0.00 |
| ATOM | 1408 | QB | SER | 73 | -8.722 | -0.878 | -3.425 | 1.00 | 0.00 |
| ATOM | 1409 | OG | SER | 73 | -9.533 | 0.572 | -2.632 | 1.00 | 0.00 |
| ATOM | 1410 | HG | SER | 73 | -9.107 | 1.419 | -2.477 | 1.00 | 0.00 |
| ATOM | 1411 | C | SER | 73 | -6.434 | 0.837 | -3.347 | 1.00 | 0.00 |
| ATOM | 1412 | O | SER | 73 | -5.834 | -0.073 | -2.787 | 1.00 | 0.00 |
| ATOM | 1413 | N | TYR | 74 | -6.052 | 2.101 | -3.279 | 1.00 | 0.00 |
| ATOM | 1414 | H | TYR | 74 | -6.565 | 2.781 | -3.764 | 1.00 | 0.00 |
| ATOM | 1415 | CA | TYR | 74 | -4.873 | 2.503 | -2.528 | 1.00 | 0.00 |
| ATOM | 1416 | HA | TYR | 74 | -4.287 | 1.623 | -2.316 | 1.00 | 0.00 |
| ATOM | 1417 | CB | TYR | 74 | -4.046 | 3.466 | -3.372 | 1.00 | 0.00 |
| ATOM | 1418 | 1HB | TYR | 74 | -4.263 | 4.476 | -3.062 | 1.00 | 0.00 |

(fortgesetzt)

Atomkooerdinaten der Struktur der ADAP hSH3-1-Domäne, oxidierte Form

| ATOM | 1419 | 2HB | TYR | 74 | -4.322 | 3.351 | -4.403 | 1.00 | 0.00 |
|------|------|-----|-----|----|--------|-------|--------|------|------|
| ATOM | 1420 | QB | TYR | 74 | -4.293 | 3.914 | -3.733 | 1.00 | 0.00 |
| ATOM | 1421 | QD | TYR | 74 | -2.395 | 3.240 | -3.260 | 1.00 | 0.00 |
| ATOM | 1422 | QE | TYR | 74 | 0.040 | 2.916 | -3.112 | 1.00 | 0.00 |
| ATOM | 1423 | QR | TYR | 74 | -1.177 | 3.078 | -3.186 | 1.00 | 0.00 |
| ATOM | 1424 | CG | TYR | 74 | -2.558 | 3.261 | -3.271 | 1.00 | 0.00 |
| ATOM | 1425 | CD1 | TYR | 74 | -1.807 | 3.947 | -2.328 | 1.00 | 0.00 |
| ATOM | 1426 | 1HD | TYR | 74 | -2.305 | 4.629 | -1.655 | 1.00 | 0.00 |
| ATOM | 1427 | CE1 | TYR | 74 | -0.441 | 3.771 | -2.242 | 1.00 | 0.00 |
| ATOM | 1428 | 1HE | TYR | 74 | 0.128 | 4.313 | -1.501 | 1.00 | 0.00 |
| ATOM | 1429 | CZ | TYR | 74 | 0.189 | 2.898 | -3.103 | 1.00 | 0.00 |
| ATOM | 1430 | CE2 | TYR | 74 | -0.539 | 2.204 | -4.047 | 1.00 | 0.00 |
| ATOM | 1431 | 2HE | TYR | 74 | -0.048 | 1.520 | -4.722 | 1.00 | 0.00 |
| ATOM | 1432 | CD2 | TYR | 74 | -1.905 | 2.391 | -4.127 | 1.00 | 0.00 |
| ATOM | 1433 | 2HD | TYR | 74 | -2.484 | 1.850 | -4.864 | 1.00 | 0.00 |
| ATOM | 1434 | OH | TYR | 74 | 1.551 | 2.721 | -3.019 | 1.00 | 0.00 |
| ATOM | 1435 | HH | TYR | 74 | 1.975 | 3.131 | -3.776 | 1.00 | 0.00 |
| ATOM | 1436 | C | TYR | 74 | -5.237 | 3.198 | -1.227 | 1.00 | 0.00 |
| ATOM | 1437 | O | TYR | 74 | -6.165 | 4.004 | -1.185 | 1.00 | 0.00 |
| ATOM | 1438 | N | GLY | 75 | -4.469 | 2.925 | -0.177 | 1.00 | 0.00 |
| ATOM | 1439 | H | GLY | 75 | -3.714 | 2.297 | -0.273 | 1.00 | 0.00 |
| ATOM | 1440 | CA | GLY | 75 | -4.715 | 3.599 | 1.085 | 1.00 | 0.00 |
| ATOM | 1441 | 1HA | GLY | 75 | -5.717 | 3.997 | 1.076 | 1.00 | 0.00 |
| ATOM | 1442 | 2HA | GLY | 75 | -4.029 | 4.426 | 1.155 | 1.00 | 0.00 |
| ATOM | 1443 | QA | GLY | 75 | -4.873 | 4.211 | 1.115 | 1.00 | 0.00 |
| ATOM | 1444 | C | GLY | 75 | -4.532 | 2.742 | 2.326 | 1.00 | 0.00 |
| ATOM | 1445 | O | GLY | 75 | -4.849 | 1.557 | 2.349 | 1.00 | 0.00 |
| ATOM | 1446 | N | TYR | 76 | -4.011 | 3.393 | 3.358 | 1.00 | 0.00 |
| ATOM | 1447 | H | TYR | 76 | -3.791 | 4.338 | 3.236 | 1.00 | 0.00 |
| ATOM | 1448 | CA | TYR | 76 | -3.741 | 2.788 | 4.664 | 1.00 | 0.00 |
| ATOM | 1449 | HA | TYR | 76 | -2.825 | 2.207 | 4.596 | 1.00 | 0.00 |
| ATOM | 1450 | CB | TYR | 76 | -3.529 | 3.912 | 5.684 | 1.00 | 0.00 |
| ATOM | 1451 | 1HB | TYR | 76 | -3.089 | 3.505 | 6.572 | 1.00 | 0.00 |
| ATOM | 1452 | 2HB | TYR | 76 | -4.486 | 4.345 | 5.932 | 1.00 | 0.00 |
| ATOM | 1453 | QB | TYR | 76 | -3.787 | 3.925 | 6.252 | 1.00 | 0.00 |
| ATOM | 1454 | QD | TYR | 76 | -2.532 | 5.159 | 5.140 | 1.00 | 0.00 |
| ATOM | 1455 | QE | TYR | 76 | -1.077 | 6.978 | 4.354 | 1.00 | 0.00 |
| ATOM | 1456 | QR | TYR | 76 | -1.804 | 6.068 | 4.747 | 1.00 | 0.00 |
| ATOM | 1457 | CG | TYR | 76 | -2.634 | 5.030 | 5.197 | 1.00 | 0.00 |
| ATOM | 1458 | CD1 | TYR | 76 | -2.987 | 6.360 | 5.387 | 1.00 | 0.00 |
| ATOM | 1459 | 1HD | TYR | 76 | -3.916 | 6.590 | 5.889 | 1.00 | 0.00 |
| ATOM | 1460 | CE1 | TYR | 76 | -2.173 | 7.386 | 4.946 | 1.00 | 0.00 |
| ATOM | 1461 | 1HE | TYR | 76 | -2.465 | 8.413 | 5.104 | 1.00 | 0.00 |
| ATOM | 1462 | CZ | TYR | 76 | -0.989 | 7.089 | 4.306 | 1.00 | 0.00 |
| ATOM | 1463 | CE2 | TYR | 76 | -0.615 | 5.776 | 4.105 | 1.00 | 0.00 |
| ATOM | 1464 | 2HE | TYR | 76 | 0.312 | 5.543 | 3.604 | 1.00 | 0.00 |
| ATOM | 1465 | CD2 | TYR | 76 | -1.435 | 4.756 | 4.549 | 1.00 | 0.00 |
| ATOM | 1466 | 2HD | TYR | 76 | -1.147 | 3.727 | 4.392 | 1.00 | 0.00 |
| ATOM | 1467 | OH | TYR | 76 | -0.175 | 8.107 | 3.868 | 1.00 | 0.00 |
| ATOM | 1468 | HH | TYR | 76 | -0.107 | 8.779 | 4.550 | 1.00 | 0.00 |

(fortgesetzt)

Atomkooerdinaten der Struktur der ADAP hSH3-1-Domäne, oxidierte Form

| | | | | | | | | | |
|---|---|---|---|---|---|---|---|---|---|
| ATOM | 1469 | C | TYR | 76 | -4.879 | 1.878 | 5.159 | 1.00 | 0.00 |
| ATOM | 1470 | O | TYR | 76 | -5.990 | 2.340 | 5.410 | 1.00 | 0.00 |
| ATOM | 1471 | N | ILE | 77 | -4.567 | 0.595 | 5.357 | 1.00 | 0.00 |
| ATOM | 1472 | H | ILE | 77 | -3.651 | 0.300 | 5.181 | 1.00 | 0.00 |
| ATOM | 1473 | CA | ILE | 77 | -5.527 | -0.375 | 5.889 | 1.00 | 0.00 |
| ATOM | 1474 | HA | ILE | 77 | -6.419 | 0.161 | 6.180 | 1.00 | 0.00 |
| ATOM | 1475 | CB | ILE | 77 | -5.920 | -1.497 | 4.892 | 1.00 | 0.00 |
| ATOM | 1476 | HB | ILE | 77 | -5.938 | -2.428 | 5.437 | 1.00 | 0.00 |
| ATOM | 1477 | QG2 | ILE | 77 | -7.655 | -1.201 | 4.236 | 1.00 | 0.00 |
| ATOM | 1478 | CG2 | ILE | 77 | -7.323 | -1.257 | 4.361 | 1.00 | 0.00 |
| ATOM | 1479 | 1HG2 | ILE | 77 | -8.043 | -1.678 | 5.047 | 1.00 | 0.00 |
| ATOM | 1480 | 2HG2 | ILE | 77 | -7.427 | -1.728 | 3.397 | 1.00 | 0.00 |
| ATOM | 1481 | 3HG2 | ILE | 77 | -7.496 | -0.196 | 4.265 | 1.00 | 0.00 |
| ATOM | 1482 | CG1 | ILE | 77 | -4.917 | -1.623 | 3.739 | 1.00 | 0.00 |
| ATOM | 1483 | 1HG1 | ILE | 77 | -4.125 | -0.915 | 3.877 | 1.00 | 0.00 |
| ATOM | 1484 | 2HG1 | ILE | 77 | -5.416 | -1.417 | 2.807 | 1.00 | 0.00 |
| ATOM | 1485 | QG1 | ILE | 77 | -4.771 | -1.166 | 3.342 | 1.00 | 0.00 |
| ATOM | 1486 | QD1 | ILE | 77 | -4.140 | -3.324 | 3.607 | 1.00 | 0.00 |
| ATOM | 1487 | CD1 | ILE | 77 | -4.290 | -2.998 | 3.632 | 1.00 | 0.00 |
| ATOM | 1488 | 1HD1 | ILE | 77 | -5.065 | -3.748 | 3.617 | 1.00 | 0.00 |
| ATOM | 1489 | 2HD1 | ILE | 77 | -3.644 | -3.165 | 4.481 | 1.00 | 0.00 |
| ATOM | 1490 | 3HD1 | ILE | 77 | -3.712 | -3.060 | 2.722 | 1.00 | 0.00 |
| ATOM | 1491 | C | ILE | 77 | -4.917 | -1.022 | 7.120 | 1.00 | 0.00 |
| ATOM | 1492 | O | ILE | 77 | -3.780 | -0.723 | 7.464 | 1.00 | 0.00 |
| ATOM | 1493 | N | LYS+ | 78 | -5.641 | -1.916 | 7.779 | 1.00 | 0.00 |
| ATOM | 1494 | H | LYS+ | 78 | -6.540 | -2.146 | 7.463 | 1.00 | 0.00 |
| ATOM | 1495 | CA | LYS+ | 78 | -5.095 | -2.580 | 8.958 | 1.00 | 0.00 |
| ATOM | 1496 | HA | LYS+ | 78 | -4.968 | -1.839 | 9.734 | 1.00 | 0.00 |
| ATOM | 1497 | CB | LYS+ | 78 | -6.033 | -3.680 | 9.454 | 1.00 | 0.00 |
| ATOM | 1498 | 1HB | LYS+ | 78 | -5.828 | -3.868 | 10.498 | 1.00 | 0.00 |
| ATOM | 1499 | 2HB | LYS+ | 78 | -5.832 | -4.576 | 8.892 | 1.00 | 0.00 |
| ATOM | 1500 | QB | LYS+ | 78 | -5.830 | -4.222 | 9.695 | 1.00 | 0.00 |
| ATOM | 1501 | CG | LYS+ | 78 | -7.511 | -3.356 | 9.309 | 1.00 | 0.00 |
| ATOM | 1502 | 1HG | LYS+ | 78 | -7.620 | -2.310 | 9.063 | 1.00 | 0.00 |
| ATOM | 1503 | 2HG | LYS+ | 78 | -8.009 | -3.563 | 10.244 | 1.00 | 0.00 |
| ATOM | 1504 | QG | LYS+ | 78 | -7.815 | -2.936 | 9.654 | 1.00 | 0.00 |
| ATOM | 1505 | CD | LYS+ | 78 | -8.150 | -4.193 | 8.212 | 1.00 | 0.00 |
| ATOM | 1506 | 1HD | LYS+ | 78 | -7.476 | -4.997 | 7.949 | 1.00 | 0.00 |
| ATOM | 1507 | 2HD | LYS+ | 78 | -8.321 | -3.568 | 7.349 | 1.00 | 0.00 |
| ATOM | 1508 | QD | LYS+ | 78 | -7.898 | -4.283 | 7.649 | 1.00 | 0.00 |
| ATOM | 1509 | CE | LYS+ | 78 | -9.475 | -4.787 | 8.661 | 1.00 | 0.00 |
| ATOM | 1510 | 1HE | LYS+ | 78 | -9.395 | -5.064 | 9.702 | 1.00 | 0.00 |
| ATOM | 1511 | 2HE | LYS+ | 78 | -9.679 | -5.668 | 8.071 | 1.00 | 0.00 |
| ATOM | 1512 | QE | LYS+ | 78 | -9.537 | -5.366 | 8.886 | 1.00 | 0.00 |
| ATOM | 1513 | NZ | LYS+ | 78 | -10.600 | -3.826 | 8.501 | 1.00 | 0.00 |
| ATOM | 1514 | 1HZ | LYS+ | 78 | -11.139 | -4.047 | 7.639 | 1.00 | 0.00 |
| ATOM | 1515 | 2HZ | LYS+ | 78 | -10.234 | -2.855 | 8.427 | 1.00 | 0.00 |
| ATOM | 1516 | 3HZ | LYS+ | 78 | -11.238 | -3.882 | 9.321 | 1.00 | 0.00 |
| ATOM | 1517 | QZ | LYS+ | 78 | -10.871 | -3.594 | 8.462 | 1.00 | 0.00 |
| ATOM | 1518 | C | LYS+ | 78 | -3.737 | -3.185 | 8.617 | 1.00 | 0.00 |

(fortgesetzt)

Atomkooerdinaten der Struktur der ADAP hSH3-1-Domäne, oxidierte Form

| ATOM | 1519 | O | LYS+ | 78 | -3.573 | -3.773 | 7.549 | 1.00 | 0.00 |
|------|------|------|------|----|--------|--------|--------|------|------|
| ATOM | 1520 | N | THR | 79 | -2.761 | -3.041 | 9.513 | 1.00 | 0.00 |
| ATOM | 1521 | H | THR | 79 | -2.938 | -2.560 | 10.349 | 1.00 | 0.00 |
| ATOM | 1522 | CA | THR | 79 | -1.430 | -3.591 | 9.263 | 1.00 | 0.00 |
| ATOM | 1523 | HA | THR | 79 | -0.969 | -2.998 | 8.485 | 1.00 | 0.00 |
| ATOM | 1524 | CB | THR | 79 | -0.563 | -3.527 | 10.522 | 1.00 | 0.00 |
| ATOM | 1525 | HB | THR | 79 | -0.431 | -4.527 | 10.908 | 1.00 | 0.00 |
| ATOM | 1526 | QG2 | THR | 79 | 1.137 | -2.809 | 10.198 | 1.00 | 0.00 |
| ATOM | 1527 | OG1 | THR | 79 | -1.182 | -2.740 | 11.524 | 1.00 | 0.00 |
| ATOM | 1528 | 1HG | THR | 79 | -1.718 | -3.302 | 12.087 | 1.00 | 0.00 |
| ATOM | 1529 | CG2 | THR | 79 | 0.810 | -2.947 | 10.261 | 1.00 | 0.00 |
| ATOM | 1530 | 1HG2 | THR | 79 | 1.244 | -2.613 | 11.193 | 1.00 | 0.00 |
| ATOM | 1531 | 2HG2 | THR | 79 | 0.724 | -2.110 | 9.583 | 1.00 | 0.00 |
| ATOM | 1532 | 3HG2 | THR | 79 | 1.442 | -3.703 | 9.820 | 1.00 | 0.00 |
| ATOM | 1533 | C | THR | 79 | -1.543 | -5.029 | 8.778 | 1.00 | 0.00 |
| ATOM | 1534 | O | THR | 79 | -0.790 | -5.472 | 7.910 | 1.00 | 0.00 |
| ATOM | 1535 | N | THR | 80 | -2.512 | -5.744 | 9.336 | 1.00 | 0.00 |
| ATOM | 1536 | H | THR | 80 | -3.084 | -5.323 | 10.011 | 1.00 | 0.00 |
| ATOM | 1537 | CA | THR | 80 | -2.763 | -7.123 | 8.964 | 1.00 | 0.00 |
| ATOM | 1538 | HA | THR | 80 | -1.949 | -7.459 | 8.349 | 1.00 | 0.00 |
| ATOM | 1539 | CB | THR | 80 | -2.832 | -7.999 | 10.207 | 1.00 | 0.00 |
| ATOM | 1540 | HB | THR | 80 | -3.844 | -7.997 | 10.566 | 1.00 | 0.00 |
| ATOM | 1541 | QG2 | THR | 80 | -2.336 | -9.778 | 9.894 | 1.00 | 0.00 |
| ATOM | 1542 | 0G1 | THR | 80 | -1.994 | -7.491 | 11.230 | 1.00 | 0.00 |
| ATOM | 1543 | 1HG | THR | 80 | -1.077 | -7.561 | 10.957 | 1.00 | 0.00 |
| ATOM | 1544 | CG2 | THR | 80 | -2.432 | -9.436 | 9.954 | 1.00 | 0.00 |
| ATOM | 1545 | 1HG2 | THR | 80 | -1.749 | -9.478 | 9.118 | 1.00 | 0.00 |
| ATOM | 1546 | 2HG2 | THR | 80 | -3.311 | -10.021 | 9.731 | 1.00 | 0.00 |
| ATOM | 1547 | 3HG2 | THR | 80 | -1.948 | -9.835 | 10.834 | 1.00 | 0.00 |
| ATOM | 1548 | C | THR | 80 | -4.062 | -7.233 | 8.172 | 1.00 | 0.00 |
| ATOM | 1549 | O | THR | 80 | -4.767 | -8.238 | 8.257 | 1.00 | 0.00 |
| ATOM | 1550 | N | ALA | 81 | -4.377 | -6.188 | 7.402 | 1.00 | 0.00 |
| ATOM | 1551 | H | ALA | 81 | -3.780 | -5.408 | 7.373 | 1.00 | 0.00 |
| ATOM | 1552 | CA | ALA | 81 | -5.594 | -6.172 | 6.603 | 1.00 | 0.00 |
| ATOM | 1553 | HA | ALA | 81 | -6.411 | -6.491 | 7.233 | 1.00 | 0.00 |
| ATOM | 1554 | QB | ALA | 81 | -5.959 | -4.423 | 6.020 | 1.00 | 0.00 |
| ATOM | 1555 | CB | ALA | 81 | -5.889 | -4.758 | 6.131 | 1.00 | 0.00 |
| ATOM | 1556 | 1HB | ALA | 81 | -6.815 | -4.749 | 5.576 | 1.00 | 0.00 |
| ATOM | 1557 | 2HB | ALA | 81 | -5.086 | -4.416 | 5.498 | 1.00 | 0.00 |
| ATOM | 1558 | 3HB | ALA | 81 | -5.976 | -4.103 | 6.986 | 1.00 | 0.00 |
| ATOM | 1559 | C | ALA | 81 | -5.499 | -7.111 | 5.406 | 1.00 | 0.00 |
| ATOM | 1560 | O | ALA | 81 | -6.441 | -7.218 | 4.624 | 1.00 | 0.00 |
| ATOM | 1561 | N | VAL | 82 | -4.360 | -7.783 | 5.256 | 1.00 | 0.00 |
| ATOM | 1562 | H | VAL | 82 | -3.636 | -7.657 | 5.900 | 1.00 | 0.00 |
| ATOM | 1563 | CA | VAL | 82 | -4.163 | -8.699 | 4.141 | 1.00 | 0.00 |
| ATOM | 1564 | HA | VAL | 82 | -5.128 | -9.108 | 3.876 | 1.00 | 0.00 |
| ATOM | 1565 | CB | VAL | 82 | -3.600 | -7.957 | 2.909 | 1.00 | 0.00 |
| ATOM | 1566 | HB | VAL | 82 | -3.810 | -8.549 | 2.030 | 1.00 | 0.00 |
| ATOM | 1567 | QG1 | VAL | 82 | -4.444 | -6.285 | 2.708 | 1.00 | 0.00 |
| ATOM | 1568 | QG2 | VAL | 82 | -1.738 | -7.732 | 3.040 | 1.00 | 0.00 |

(fortgesetzt)

Atomkooerdinaten der Struktur der ADAP hSH3-1-Domäne, oxidierte Form

| ATOM | 1569 | CG1 | VAL | 82 | -4.283 | -6.605 | 2.745 | 1.00 | 0.00 |
|------|------|------|------|------|--------|--------|--------|------|------|
| ATOM | 1570 | 1HG1 | VAL | 82 | -3.977 | -6.155 | 1.813 | 1.00 | 0.00 |
| ATOM | 1571 | 2HG1 | VAL | 82 | -4.003 | -5.961 | 3.565 | 1.00 | 0.00 |
| ATOM | 1572 | 3HG1 | VAL | 82 | -5.354 | -6.740 | 2.745 | 1.00 | 0.00 |
| ATOM | 1573 | CG2 | VAL | 82 | -2.094 | -7.775 | 3.015 | 1.00 | 0.00 |
| ATOM | 1574 | 1HG2 | VAL | 82 | -1.612 | -8.740 | 2.982 | 1.00 | 0.00 |
| ATOM | 1575 | 2HG2 | VAL | 82 | -1.856 | -7.285 | 3.948 | 1.00 | 0.00 |
| ATOM | 1576 | 3HG2 | VAL | 82 | -1.748 | -7.170 | 2.191 | 1.00 | 0.00 |
| ATOM | 1577 | QQG | VAL | 82 | -3.091 | -7.008 | 2.874 | 1.00 | 0.00 |
| ATOM | 1578 | C | VAL | 82 | -3.232 | -9.849 | 4.530 | 1.00 | 0.00 |
| ATOM | 1579 | O | VAL | 82 | -2.795 | -9.941 | 5.677 | 1.00 | 0.00 |
| ATOM | 1580 | N | GLU | 8.3 | -2.937 | -10.724 | 3.573 | 1.00 | 0.00 |
| ATOM | 1581 | H | GLU | 83 | -3.313 | -10.602 | 2.682 | 1.00 | 0.00 |
| ATOM | 1582 | CA | GLU | 83 | -2.064 | -11.860 | 3.823 | 1.00 | 0.00 |
| ATOM | 1583 | HA | GLU | 83 | -1.562 | -11.681 | 4.756 | 1.00 | 0.00 |
| ATOM | 1584 | CB | GLU | 83 | -2.884 | -13.146 | 3.941 | 1.00 | 0.00 |
| ATOM | 1585 | 1HB | GLU | 83 | -3.601 | -13.030 | 4.741 | 1.00 | 0.00 |
| ATOM | 1586 | 2HB | GLU | 83 | -2.220 | -13.963 | 4.180 | 1.00 | 0.00 |
| ATOM | 1587 | QB | GLU | 83 | -2.910 | -13.497 | 4.461 | 1.00 | 0.00 |
| ATOM | 1588 | CG | GLU | 83 | -3.641 | -13.503 | 2.673 | 1.00 | 0.00 |
| ATOM | 1589 | 1HG | GLU | 83 | -3.008 | -14.121 | 2.053 | 1.00 | 0.00 |
| ATOM | 1590 | 2HG | GLU | 83 | -3.883 | -12.592 | 2.146 | 1.00 | 0.00 |
| ATOM | 1591 | QG | GLU | 83 | -3.445 | -13.357 | 2.099 | 1.00 | 0.00 |
| ATOM | 1592 | CD | GLU | 83 | -4.927 | -14.255 | 2.954 | 1.00 | 0.00 |
| ATOM | 1593 | OE1 | GLU | 83 | -5.128 | -15.332 | 2.356 | 1.00 | 0.00 |
| ATOM | 1594 | OE2 | GLU | 83 | -5.734 | -13.767 | 3.774 | 1.00 | 0.00 |
| ATOM | 1595 | C | GLU | 83 | -1.016 | -12.008 | 2.722 | 1.00 | 0.00 |
| ATOM | 1596 | O | GLU | 83 | -1.327 | -12.411 | 1.602 | 1.00 | 0.00 |
| ATOM | 1597 | N | ILE | 84 | 0.227 | -11.680 | 3.057 | 1.00 | 0.00 |
| ATOM | 1598 | H | ILE | 84 | 0.407 | -11.372 | 3.970 | 1.00 | 0.00 |
| ATOM | 1599 | CA | ILE | 84 | 1.336 | -11.777 | 2.109 | 1.00 | 0.00 |
| ATOM | 1600 | HA | ILE | 84 | 0.970 | -12.279 | 1.223 | 1.00 | 0.00 |
| ATOM | 1601 | CB | ILE | 84 | 1.896 | -10.393 | 1.687 | 1.00 | 0.00 |
| ATOM | 1602 | HB | ILE | 84 | 2.969 | -10.420 | 1.811 | 1.00 | 0.00 |
| ATOM | 1603 | QG2 | ILE | 84 | 1.556 | -10.067 | -0.133 | 1.00 | 0.00 |
| ATOM | 1604 | CG2 | ILE | 84 | 1.620 | -10.130 | 0.216 | 1.00 | 0.00 |
| ATOM | 1605 | 1HG2 | ILE | 84 | 2.548 | -10.164 | -0.336 | 1.00 | 0.00 |
| ATOM | 1606 | 2HG2 | ILE | 84 | 1.170 | -9.155 | 0.101 | 1.00 | 0.00 |
| ATOM | 1607 | 3HG2 | ILE | 84 | 0.949 | -10.883 | -0.162 | 1.00 | 0.00 |
| ATOM | 1608 | CG1 | ILE | 84 | 1.336 | -9.259 | 2.552 | 1.00 | 0.00 |
| ATOM | 1609 | 1HG1 | ILE | 84 | 0.728 | -9.670 | 3.339 | 1.00 | 0.00 |
| ATOM | 1610 | 2HG1 | ILE | 84 | 0.731 | -8.608 | 1.939 | 1.00 | 0.00 |
| ATOM | 1611 | QG1 | ILE | 84 | 0.729 | -9.139 | 2.639 | 1.00 | 0.00 |
| ATOM | 1612 | QD1 | ILE | 84 | 2.673 | -8.221 | 3.343 | 1.00 | 0.00 |
| ATOM | 1613 | CD1 | ILE | 84 | 2.416 | -8.420 | 3.192 | 1.00 | 0.00 |
| ATOM | 1614 | 1HD1 | ILE | 84 | 3.007 | -7.950 | 2.421 | 1.00 | 0.00 |
| ATOM | 1615 | 2HD1 | ILE | 84 | 3.049 | -9.051 | 3.797 | 1.00 | 0.00 |
| ATOM | 1616 | 3HD1 | ILE | 84 | 1.963 | -7.662 | 3.813 | 1.00 | 0.00 |
| ATOM | 1617 | C | ILE | 84 | 2.485 | -12.585 | 2.697 | 1.00 | 0.00 |
| ATOM | 1618 | O | ILE | 84 | 2.648 | -12.654 | 3.915 | 1.00 | 0.00 |

(fortgesetzt)

Atomkooerdinaten der Struktur der ADAP hSH3-1-Domäne, oxidierte Form

| ATOM | 1619 | N | ASP | 85 | 3.296 | -13.176 | 1.826 | 1.00 | 0.00 |
|---|---|---|---|---|---|---|---|---|---|
| ATOM | 1620 | H | ASP | 85 | 3.129 | -13.071 | 0.866 | 1.00 | 0.00 |
| ATOM | 1621 | CA | ASP | 85 | 4.446 | -13.949 | 2.270 | 1.00 | 0.00 |
| ATOM | 1622 | HA | ASP | 85 | 4.120 | -14.617 | 3.054 | 1.00 | 0.00 |
| ATOM | 1623 | CB | ASP | 85 | 5.019 | -14.770 | 1.113 | 1.00 | 0.00 |
| ATOM | 1624 | 1HB | ASP | 85 | 5.802 | -15.413 | 1.489 | 1.00 | 0.00 |
| ATOM | 1625 | 2HB | ASP | 85 | 5.433 | -14.099 | 0.375 | 1.00 | 0.00 |
| ATOM | 1626 | QB | ASP | 85 | 5.617 | -14.756 | 0.932 | 1.00 | 0.00 |
| ATOM | 1627 | CG | ASP | 85 | 3.970 | -15.633 | 0.441 | 1.00 | 0.00 |
| ATOM | 1628 | OD1 | ASP | 85 | 3.784 | -15.496 | -0.787 | 1.00 | 0.00 |
| ATOM | 1629 | OD2 | ASP | 85 | 3.332 | -16.447 | 1.143 | 1.00 | 0.00 |
| ATOM | 1630 | C | ASP | 85 | 5.512 | -13.010 | 2.827 | 1.00 | 0.00 |
| ATOM | 1631 | O | ASP | 85 | 6.058 | -12.180 | 2.100 | 1.00 | 0.00 |
| ATOM | 1632 | N | TYR | 86 | 5.790 | -13.129 | 4.122 | 1.00 | 0.00 |
| ATOM | 1633 | H | TYR | 86 | 5.314 | -13.795 | 4.653 | 1.00 | 0.00 |
| ATOM | 1634 | CA | TYR | 86 | 6.775 | -12.272 | 4.773 | 1.00 | 0.00 |
| ATOM | 1635 | HA | TYR | 86 | 6.697 | -11.308 | 4.315 | 1.00 | 0.00 |
| ATOM | 1636 | CB | TYR | 86 | 6.465 | -12.137 | 6.267 | 1.00 | 0.00 |
| ATOM | 1637 | 1HB | TYR | 86 | 7.184 | -11.467 | 6.715 | 1.00 | 0.00 |
| ATOM | 1638 | 2HB | TYR | 86 | 6.551 | -13.107 | 6.732 | 1.00 | 0.00 |
| ATOM | 1639 | QB | TYR | 86 | 6.867 | -12.287 | 6.723 | 1.00 | 0.00 |
| ATOM | 1640 | QD | TYR | 86 | 4.914 | -11.538 | 6.609 | 1.00 | 0.00 |
| ATOM | 1641 | QE | TYR | 86 | 2.670 | -10.676 | 7.122 | 1.00 | 0.00 |
| ATOM | 1642 | QR | TYR | 86 | 3.792 | -11.107 | 6.866 | 1.00 | 0.00 |
| ATOM | 1643 | CG | TYR | 86 | 5.077 | -11.601 | 6.573 | 1.00 | 0.00 |
| ATOM | 1644 | CD1 | TYR | 86 | 4.225 | -11.169 | 5.559 | 1.00 | 0.00 |
| ATOM | 1645 | 1HD | TYR | 86 | 4.561 | -11.215 | 4.535 | 1.00 | 0.00 |
| ATOM | 1646 | CE1 | TYR | 86 | 2.963 | -10.683 | 5.845 | 1.00 | 0.00 |
| ATOM | 1647 | 1HE | TYR | 86 | 2.316 | -10.354 | 5.044 | 1.00 | 0.00 |
| ATOM | 1648 | CZ | TYR | 86 | 2.535 | -10.624 | 7.154 | 1.00 | 0.00 |
| ATOM | 1649 | CE2 | TYR | 86 | 3.359 | -11.046 | 8.175 | 1.00 | 0.00 |
| ATOM | 1650 | 2HE | TYR | 86 | 3.024 | -10.998 | 9.201 | 1.00 | 0.00 |
| ATOM | 1651 | CD2 | TYR | 86 | 4.620 | -11.530 | 7.883 | 1.00 | 0.00 |
| ATOM | 1652 | 2HD | TYR | 86 | 5.267 | -11.860 | 8.683 | 1.00 | 0.00 |
| ATOM | 1653 | OH | TYR | 86 | 1.280 | -10.141 | 7.443 | 1.00 | 0.00 |
| ATOM | 1654 | HH | TYR | 86 | 0.658 | -10.449 | 6.779 | 1.00 | 0.00 |
| ATOM | 1655 | C | TYR | 86 | 8.201 | -12.774 | 4.574 | 1.00 | 0.00 |
| ATOM | 1656 | O | TYR | 86 | 9.107 | -12.411 | 5.324 | 1.00 | 0.00 |
| ATOM | 1657 | N | ASP | 87 | 8.392 | -13.600 | 3.561 | 1.00 | 0.00 |
| ATOM | 1658 | H | ASP | 87 | 7.637 | -13.840 | 3.002 | 1.00 | 0.00 |
| ATOM | 1659 | CA | ASP | 87 | 9.703 | -14.146 | 3.255 | 1.00 | 0.00 |
| ATOM | 1660 | HA | ASP | 87 | 10.291 | -14.100 | 4.154 | 1.00 | 0.00 |
| ATOM | 1661 | CB | ASP | 87 | 9.584 | -15.605 | 2.811 | 1.00 | 0.00 |
| ATOM | 1662 | 1HB | ASP | 87 | 10.573 | -16.011 | 2.661 | 1.00 | 0.00 |
| ATOM | 1663 | 2HB | ASP | 87 | 9.037 | -15.648 | 1.881 | 1.00 | 0.00 |
| ATOM | 1664 | QB | ASP | 87 | 9.805 | -15.830 | 2.271 | 1.00 | 0.00 |
| ATOM | 1665 | CG | ASP | 87 | 8.862 | -16.462 | 3.832 | 1.00 | 0.00 |
| ATOM | 1666 | OD1 | ASP | 87 | 9.545 | -17.132 | 4.635 | 1.00 | 0.00 |
| ATOM | 1667 | OD2 | ASP | 87 | 7.613 | -16.463 | 3.830 | 1.00 | 0.00 |
| ATOM | 1668 | C | ASP | 87 | 10.396 | -13.325 | 2.170 | 1.00 | 0.00 |

(fortgesetzt)

Atomkooerdinaten der Struktur der ADAP hSH3-1-Domäne, oxidierte Form

| ATOM | 1669 | O | ASP | 87 | 11.624 | -13.267 | 2.110 | 1.00 | 0.00 |
|------|------|------|------|----|--------|---------|-------|------|------|
| ATOM | 1670 | N | SER | 88 | 9.599 | -12.694 | 1.312 | 1.00 | 0.00 |
| ATOM | 1671 | H | SER | 88 | 8.630 | -12.781 | 1.408 | 1.00 | 0.00 |
| ATOM | 1672 | CA | SER | 88 | 10.132 | -11.880 | 0.227 | 1.00 | 0.00 |
| ATOM | 1673 | HA | SER | 88 | 10.940 | -12.430 | -0.228 | 1.00 | 0.00 |
| ATOM | 1674 | CB | SER | 88 | 9.051 | -11.628 | -0.825 | 1.00 | 0.00 |
| ATOM | 1675 | 1HB | SER | 88 | 8.615 | -12.569 | -1.125 | 1.00 | 0.00 |
| ATOM | 1676 | 2HB | SER | 88 | 9.493 | -11.146 | -1.685 | 1.00 | 0.00 |
| ATOM | 1677 | QB | SER | 88 | 9.054 | -11.857 | -1.405 | 1.00 | 0.00 |
| ATOM | 1678 | OG | SER | 88 | 8.026 | -10.792 | -0.314 | 1.00 | 0.00 |
| ATOM | 1679 | HG | SER | 88 | 8.106 | -9.916 | -0.696 | 1.00 | 0.00 |
| ATOM | 1680 | C | SER | 88 | 10.671 | -10.550 | 0.747 | 1.00 | 0.00 |
| ATOM | 1681 | O | SER | 88 | 11.803 | -10.170 | 0.449 | 1.00 | 0.00 |
| ATOM | 1682 | N | LEU | 89 | 9.852 | -9.845 | 1.522 | 1.00 | 0.00 |
| ATOM | 1683 | H | LEU | 89 | 8.960 | -10.199 | 1.723 | 1.00 | 0.00 |
| ATOM | 1684 | CA | LEU | 89 | 10.245 | -8.554 | 2.080 | 1.00 | 0.00 |
| ATOM | 1685 | HA | LEU | 89 | 10.334 | -7.853 | 1.263 | 1.00 | 0.00 |
| ATOM | 1686 | CB | LEU | 89 | 9.177 | -8.046 | 3.052 | 1.00 | 0.00 |
| ATOM | 1687 | 1HB | LEU | 89 | 9.181 | -8.682 | 3.925 | 1.00 | 0.00 |
| ATOM | 1688 | 2HB | LEU | 89 | 8.214 | -8.131 | 2.570 | 1.00 | 0.00 |
| ATOM | 1689 | QB | LEU | 89 | 8.697 | -8.407 | 3.248 | 1.00 | 0.00 |
| ATOM | 1690 | CG | LEU | 89 | 9.357 | -6. 601 | 3.515 | 1.00 | 0.00 |
| ATOM | 1691 | HG | LEU | 89 | 10.399 | -6.430 | 3.745 | 1.00 | 0.00 |
| ATOM | 1692 | QD1 | LEU | 89 | 8.861 | -5.405 | 2.150 | 1.00 | 0.00 |
| ATOM | 1693 | QD2 | LEU | 89 | 8.354 | -6.281 | 5.073 | 1.00 | 0.00 |
| ATOM | 1694 | CD1 | LEU | 89 | 8.956 | -5.634 | 2.411 | 1.00 | 0.00 |
| ATOM | 1695 | 1HD1 | LEU | 89 | 9.330 | -5.994 | 1.465 | 1.00 | 0.00 |
| ATOM | 1696 | 2HD1 | LEU | 89 | 9.374 | -4.659 | 2.616 | 1.00 | 0.00 |
| ATOM | 1697 | 3HD1 | LEU | 89 | 7.879 | -5.562 | 2.370 | 1.00 | 0.00 |
| ATOM | 1698 | CD2 | LEU | 89 | 8.546 | -6.342 | 4.776 | 1.00 | 0.00 |
| ATOM | 1699 | 1HD2 | LEU | 89 | 7.626 | -5.840 | 4.514 | 1.00 | 0.00 |
| ATOM | 1700 | 2HD2 | LEU | 89 | 9.116 | -5.720 | 5.450 | 1.00 | 0.00 |
| ATOM | 1701 | 3HD2 | LEU | 89 | 8.320 | -7.282 | 5.256 | 1.00 | 0.00 |
| ATOM | 1702 | QQD | LEU | 89 | 8.607 | -5.843 | 3.612 | 1.00 | 0.00 |
| ATOM | 1703 | C | LEU | 89 | 11.592 | -8.647 | 2.794 | 1.00 | 0.00 |
| ATOM | 1704 | O | LEU | 89 | 12.565 | -8.011 | 2.388 | 1.00 | 0.00 |
| ATOM | 1705 | N | LYS+ | 90 | 11.691 | -9.444 | 3.856 | 1.00 | 0.00 |
| ATOM | 1706 | H | LYS+ | 90 | 10.832 | -9.925 | 4.131 | 1.00 | 0.00 |
| ATOM | 1707 | CA | LYS+ | 90 | 12.870 | -9.618 | 4.622 | 1.00 | 0.00 |
| ATOM | 1708 | HA | LYS+ | 90 | 13.698 | -9.588 | 3.930 | 1.00 | 0.00 |
| ATOM | 1709 | CB | LYS+ | 90 | 13.028 | -8.484 | 5.637 | 1.00 | 0.00 |
| ATOM | 1710 | 1HB | LYS+ | 90 | 13.283 | -7.577 | 5.109 | 1.00 | 0.00 |
| ATOM | 1711 | 2HB | LYS+ | 90 | 13.831 | -8.734 | 6.314 | 1.00 | 0.00 |
| ATOM | 1712 | QB | LYS+ | 90 | 13.557 | -8.155 | 5.711 | 1.00 | 0.00 |
| ATOM | 1713 | CG | LYS+ | 90 | 11.777 | -8.222 | 6.458 | 1.00 | 0.00 |
| ATOM | 1714 | 1HG | LYS+ | 90 | 11.861 | -8.742 | 7.401 | 1.00 | 0.00 |
| ATOM | 1715 | 2HG | LYS+ | 90 | 10.919 | -8.592 | 5.917 | 1.00 | 0.00 |
| ATOM | 1716 | QG | LYS+ | 90 | 11.390 | -8.667 | 6.659 | 1.00 | 0.00 |
| ATOM | 1717 | CD | LYS+ | 90 | 11.586 | -6.738 | 6.727 | 1.00 | 0.00 |
| ATOM | 1718 | 1HD | LYS+ | 90 | 10.552 | -6.558 | 6.981 | 1.00 | 0.00 |

(fortgesetzt)

Atomkooerdinaten der Struktur der ADAP hSH3-1-Domäne, oxidierte Form

| ATOM | 1719 | 2HD | LYS+ | 90 | 11.842 | -6.185 | 5.836 | 1.00 | 0.00 |
|------|------|------|------|----|--------|--------|-------|------|------|
| ATOM | 1720 | QD | LYS+ | 90 | 11.197 | -6.372 | 6.409 | 1.00 | 0.00 |
| ATOM | 1721 | CE | LYS+ | 90 | 12.467 | -6.262 | 7.871 | 1.00 | 0.00 |
| ATOM | 1722 | 1HE | LYS+ | 90 | 13.486 | -6.190 | 7.518 | 1.00 | 0.00 |
| ATOM | 1723 | 2HE | LYS+ | 90 | 12.415 | -6.983 | 8.673 | 1.00 | 0.00 |
| ATOM | 1724 | QE | LYS+ | 90 | 12.950 | -6.587 | 8.096 | 1.00 | 0.00 |
| ATOM | 1725 | NZ | LYS+ | 90 | 12.038 | -4.933 | 8.387 | 1.00 | 0.00 |
| ATOM | 1726 | 1HZ | LYS+ | 90 | 12.747 | -4.560 | 9.051 | 1.00 | 0.00 |
| ATOM | 1727 | 2HZ | LYS+ | 90 | 11.127 | -5.019 | 8.883 | 1.00 | 0.00 |
| ATOM | 1728 | 3HZ | LYS+ | 90 | 11.929 | -4.262 | 7.600 | 1.00 | 0.00 |
| ATOM | 1729 | QZ | LYS+ | 90 | 11.935 | -4.614 | 8.511 | 1.00 | 0.00 |
| ATOM | 1730 | C | LYS+ | 90 | 12.878 | -10.963 | 5.340 | 1.00 | 0.00 |
| ATOM | 1731 | O | LYS+ | 90 | 11.827 | -11.557 | 5.580 | 1.00 | 0.00 |
| ATOM | 1732 | N | LEU | 91 | 14.072 | -11.439 | 5.680 | 1.00 | 0.00 |
| ATOM | 1733 | H | LEU | 91 | 14.873 | -10.918 | 5.460 | 1.00 | 0.00 |
| ATOM | 1734 | CA | LEU | 91 | 14.220 | -12.714 | 6.371 | 1.00 | 0.00 |
| ATOM | 1735 | HA | LEU | 91 | 13.248 | -13.003 | 6.743 | 1.00 | 0.00 |
| ATOM | 1736 | CB | LEU | 91 | 14.720 | -13.788 | 5.401 | 1.00 | 0.00 |
| ATOM | 1737 | 1HB | LEU | 91 | 15.753 | -14.002 | 5.632 | 1.00 | 0.00 |
| ATOM | 1738 | 2HB | LEU | 91 | 14.669 | -13.389 | 4.398 | 1.00 | 0.00 |
| ATOM | 1739 | QB | LEU | 91 | 15.211 | -13.695 | 5.015 | 1.00 | 0.00 |
| ATOM | 1740 | CG | LEU | 91 | 13.940 | -15.103 | 5.434 | 1.00 | 0.00 |
| ATOM | 1741 | HG | LEU | 91 | 14.419 | -15.815 | 4.776 | 1.00 | 0.00 |
| ATOM | 1742 | QD1 | LEU | 91 | 13.939 | -15.827 | 7.170 | 1.00 | 0.00 |
| ATOM | 1743 | QD2 | LEU | 91 | 12.180 | -14.840 | 4.826 | 1.00 | 0.00 |
| ATOM | 1744 | CD1 | LEU | 91 | 13.939 | -15.689 | 6.838 | 1.00 | 0.00 |
| ATOM | 1745 | 1HD1 | LEU | 91 | 14.903 | -15.523 | 7.298 | 1.00 | 0.00 |
| ATOM | 1746 | 2HD1 | LEU | 91 | 13.744 | -16.749 | 6.785 | 1.00 | 0.00 |
| ATOM | 1747 | 3HD1 | LEU | 91 | 13.172 | -15.209 | 7.427 | 1.00 | 0.00 |
| ATOM | 1748 | CD2 | LEU | 91 | 12.517 | -14.891 | 4.942 | 1.00 | 0.00 |
| ATOM | 1749 | 1HD2 | LEU | 91 | 12.193 | -15.761 | 4.390 | 1.00 | 0.00 |
| ATOM | 1750 | 2HD2 | LEU | 91 | 12.484 | -14.023 | 4.300 | 1.00 | 0.00 |
| ATOM | 1751 | 3HD2 | LEU | 91 | 11.863 | -14.737 | 5.788 | 1.00 | 0.00 |
| ATOM | 1752 | QQD | LEU | 91 | 13.060 | -15.334 | 5.998 | 1.00 | 0.00 |
| ATOM | 1753 | C | LEU | 91 | 15.179 | -12.585 | 7.549 | 1.00 | 0.00 |
| ATOM | 1754 | O | LEU | 91 | 16.153 | -11.833 | 7.490 | 1.00 | 0.00 |
| ATOM | 1755 | N | LYS+ | 92 | 14.899 | -13.322 | 8.619 | 1.00 | 0.00 |
| ATOM | 1756 | H | LYS+ | 92 | 14.110 | -13.902 | 8.607 | 1.00 | 0.00 |
| ATOM | 1757 | CA | LYS+ | 92 | 15.738 | -13.289 | 9.811 | 1.00 | 0.00 |
| ATOM | 1758 | HA | LYS+ | 92 | 16.474 | -12.510 | 9.677 | 1.00 | 0.00 |
| ATOM | 1759 | CB | LYS+ | 92 | 14.895 | -12.966 | 11.046 | 1.00 | 0.00 |
| ATOM | 1760 | 1HB | LYS+ | 92 | 14.720 | -13.878 | 11.598 | 1.00 | 0.00 |
| ATOM | 1761 | 2HB | LYS+ | 92 | 13.946 | -12.564 | 10.724 | 1.00 | 0.00 |
| ATOM | 1762 | QB | LYS+ | 92 | 14.333 | -13.221 | 11.161 | 1.00 | 0.00 |
| ATOM | 1763 | CG | LYS+ | 92 | 15.546 | -11.960 | 11.982 | 1.00 | 0.00 |
| ATOM | 1764 | 1HG | LYS+ | 92 | 16.035 | -11.200 | 11.391 | 1.00 | 0.00 |
| ATOM | 1765 | 2HG | LYS+ | 92 | 16.277 | -12.470 | 12.590 | 1.00 | 0.00 |
| ATOM | 1766 | QG | LYS+ | 92 | 16.156 | -11.835 | 11.991 | 1.00 | 0.00 |
| ATOM | 1767 | CD | LYS+ | 92 | 14.522 | -11.299 | 12.892 | 1.00 | 0.00 |
| ATOM | 1768 | 1HD | LYS+ | 92 | 14.646 | -11.683 | 13.894 | 1.00 | 0.00 |

(fortgesetzt)

Atomkooerdinaten der Struktur der ADAP hSH3-1-Domäne, oxidierte Form

| ATOM | 1769 | 2HD | LYS+ | 92 | 13.531 | -11.535 | 12.534 | 1.00 | 0.00 |
|------|------|-----|------|----|--------|---------|--------|------|------|
| ATOM | 1770 | QD | LYS+ | 92 | 14.088 | -11.609 | 13.214 | 1.00 | 0.00 |
| ATOM | 1771 | CE | LYS+ | 92 | 14.689 | -9.788 | 12.918 | 1.00 | 0.00 |
| ATOM | 1772 | 1HE | LYS+ | 92 | 13.925 | -9.343 | 12.298 | 1.00 | 0.00 |
| ATOM | 1773 | 2HE | LYS+ | 92 | 15.662 | -9.539 | 12.522 | 1.00 | 0.00 |
| ATOM | 1774 | QE | LYS+ | 92 | 14.794 | -9.441 | 12.410 | 1.00 | 0.00 |
| ATOM | 1775 | NZ | LYS+ | 92 | 14.574 | -9.240 | 14.298 | 1.00 | 0.00 |
| ATOM | 1776 | 1HZ | LYS+ | 92 | 15.068 | -8.327 | 14.362 | 1.00 | 0.00 |
| ATOM | 1777 | 2HZ | LYS+ | 92 | 14.996 | -9.899 | 14.981 | 1.00 | 0.00 |
| ATOM | 1778 | 3HZ | LYS+ | 92 | 13.572 | -9.098 | 14.544 | 1.00 | 0.00 |
| ATOM | 1779 | QZ | LYS+ | 92 | 14.545 | -9.108 | 14.629 | 1.00 | 0.00 |
| ATOM | 1780 | C | LYS+ | 92 | 16.462 | -14.618 | 10.005 | 1.00 | 0.00 |
| ATOM | 1781 | O | LYS+ | 92 | 17.612 | -14.652 | 10.442 | 1.00 | 0.00 |
| ATOM | 1782 | N | LYS+ | 93 | 15.779 | -15.711 | 9.678 | 1.00 | 0.00 |
| ATOM | 1783 | H | LYS+ | 93 | 14.866 | -15.619 | 9.335 | 1.00 | 0.00 |
| ATOM | 1784 | CA | LYS+ | 93 | 16.357 | -17.043 | 9.816 | 1.00 | 0.00 |
| ATOM | 1785 | HA | LYS+ | 93 | 17.348 | -16.934 | 10.230 | 1.00 | 0.00 |
| ATOM | 1786 | CB | LYS+ | 93 | 15.511 | -17.892 | 10.767 | 1.00 | o.oo |
| ATOM | 1787 | 1HB | LYS+ | 93 | 15.597 | -17.488 | 11.765 | 1.00 | 0.00 |
| ATOM | 1788 | 2HB | LYS+ | 93 | 15.893 | -18.903 | 10.764 | 1.00 | 0.00 |
| ATOM | 1789 | QB | LYS+ | 93 | 15.745 | -18.195 | 11.269 | 1.00 | 0.00 |
| ATOM | 1790 | CG | LYS+ | 93 | 14.038 | -17.938 | 10.396 | 1.00 | 0.00 |
| ATOM | 1791 | 1HG | LYS+ | 93 | 13.643 | -18.910 | 10.652 | 1.00 | 0.00 |
| ATOM | 1792 | 2HG | LYS+ | 93 | 13.940 | -17.775 | 9.333 | 1.00 | 0.00 |
| ATOM | 1793 | QG | LYS+ | 93 | 13.792 | -18.343 | 9.993 | 1.00 | 0.00 |
| ATOM | 1794 | CD | LYS+ | 93 | 13.245 | -16.872 | 11.133 | 1.00 | 0.00 |
| ATOM | 1795 | 1HD | LYS+ | 93 | 13.186 | -15.989 | 10.514 | 1.00 | 0.00 |
| ATOM | 1796 | 2HD | LYS+ | 93 | 13.753 | -16.633 | 12.056 | 1.00 | 0.00 |
| ATOM | 1797 | QD | LYS+ | 93 | 13.469 | -16.311 | 11.285 | 1.00 | 0.00 |
| ATOM | 1798 | CE | LYS+ | 93 | 11.837 | -17.348 | 11.455 | 1.00 | 0.00 |
| ATOM | 1799 | 1HE | LYS+ | 93 | 11.899 | -18.154 | 12.170 | 1.00 | 0.00 |
| ATOM | 1800 | 2HE | LYS+ | 93 | 11.377 | -17.707 | 10.547 | 1.00 | 0.00 |
| ATOM | 1801 | QE | LYS+ | 93 | 11.638 | -17.931 | 11.359 | 1.00 | 0.00 |
| ATOM | 1802 | NZ | LYS+ | 93 | 11.000 | -16.257 | 12.024 | 1.00 | 0.00 |
| ATOM | 1803 | 1HZ | LYS+ | 93 | 11.164 | -15.370 | 11.504 | 1.00 | 0.00 |
| ATOM | 1804 | 2HZ | LYS+ | 93 | 11.238 | -16.107 | 13.025 | 1.00 | 0.00 |
| ATOM | 1805 | 3HZ | LYS+ | 93 | 9.992 | -16.506 | 11.959 | 1.00 | 0.00 |
| ATOM | 1806 | QZ | LYS+ | 93 | 10.798 | -15.995 | 12.161 | 1.00 | 0.00 |
| ATOM | 1807 | C | LYS+ | 93 | 16.464 | -17.732 | 8.460 | 1.00 | 0.00 |
| ATOM | 1808 | O | LYS+ | 93 | 15.490 | -17.807 | 7.711 | 1.00 | 0.00 |
| ATOM | 1809 | N | ASP | 94 | 17.655 | -18.235 | 8.150 | 1.00 | 0.00 |
| ATOM | 1810 | H | ASP | 94 | 18.393 | -18.144 | 8.788 | 1.00 | 0.00 |
| ATOM | 1811 | CA | ASP | 94 | 17.891 | -18.918 | 6.884 | 1.00 | 0.00 |
| ATOM | 1812 | HA | ASP | 94 | 16.987 | -19.447 | 6.620 | 1.00 | 0.00 |
| ATOM | 1813 | CB | ASP | 94 | 18.213 | -17.905 | 5.784 | 1.00 | 0.00 |
| ATOM | 1814 | 1HB | ASP | 94 | 18.925 | -18.339 | 5.099 | 1.00 | 0.00 |
| ATOM | 1815 | 2HB | ASP | 94 | 18.644 | -17.022 | 6.233 | 1.00 | 0.00 |
| ATOM | 1816 | QB | ASP | 94 | 18.784 | -17.681 | 5.666 | 1.00 | 0.00 |
| ATOM | 1817 | CG | ASP | 94 | 16.982 | -17.491 | 5.001 | 1.00 | 0.00 |
| ATOM | 1818 | OD1 | ASP | 94 | 16.282 | -18.385 | 4.479 | 1.00 | 0.00 |

(fortgesetzt)

Atomkooerdinaten der Struktur der ADAP hSH3-1-Domäne, oxidierte Form

| ATOM | 1819 | OD2 | ASP | 94 | 16.718 | -16.274 | 4.908 | 1.00 | 0.00 |
|------|------|-----|-----|----|--------|---------|-------|------|------|
| ATOM | 1820 | C | ASP | 94 | 19.029 | -19.926 | 7.013 | 1.00 | 0.00 |
| ATOM | 1821 | O | ASP | 94 | 20.196 | -19.548 | 7.122 | 1.00 | 0.00 |
| ATOM | 1822 | N | LEU | 95 | 18.682 | -21.208 | 7.000 | 1.00 | 0.00 |
| ATOM | 1823 | H | LEU | 95 | 17.735 | -21.447 | 6.910 | 1.00 | 0.00 |
| ATOM | 1824 | CA | LEU | 95 | 19.674 | -22.271 | 7.116 | 1.00 | 0.00 |
| ATOM | 1825 | HA | LEU | 95 | 20.637 | -21.809 | 7.278 | 1.00 | 0.00 |
| ATOM | 1826 | CB | LEU | 95 | 19.349 | -23.174 | 8.307 | 1.00 | 0.00 |
| ATOM | 1827 | 1HB | LEU | 95 | 18.562 | -23.853 | 8.013 | 1.00 | 0.00 |
| ATOM | 1828 | 2HB | LEU | 95 | 18.984 | -22.554 | 9.112 | 1.00 | 0.00 |
| ATOM | 1829 | QB | LEU | 95 | 18.773 | -23.203 | 8.562 | 1.00 | 0.00 |
| ATOM | 1830 | CG | LEU | 95 | 20.523 | -24.002 | 8.832 | 1.00 | 0.00 |
| ATOM | 1831 | HG | LEU | 95 | 20.986 | -24.522 | 8.006 | 1.00 | 0.00 |
| ATOM | 1832 | QD1 | LEU | 95 | 21.818 | -22.887 | 9.616 | 1.00 | 0.00 |
| ATOM | 1833 | QD2 | LEU | 95 | 19.920 | -25.287 | 10.067 | 1.00 | 0.00 |
| ATOM | 1834 | CD1 | LEU | 95 | 21.570 | -23.101 | 9.466 | 1.00 | 0.00 |
| ATOM | 1835 | 1HD1 | LEU | 95 | 21.432 | -23.086 | 10.537 | 1.00 | 0.00 |
| ATOM | 1836 | 2HD1 | LEU | 95 | 21.467 | -22.099 | 9.076 | 1.00 | 0.00 |
| ATOM | 1837 | 3HD1 | LEU | 95 | 22.556 | -23.477 | 9.235 | 1.00 | 0.00 |
| ATOM | 1838 | CD2 | LEU | 95 | 20.035 | -25.041 | 9.830 | 1.00 | 0.00 |
| ATOM | 1839 | 1HD2 | LEU | 95 | 19.125 | -24.693 | 10.296 | 1.00 | 0.00 |
| ATOM | 1840 | 2HD2 | LEU | 95 | 20.790 | -25.196 | 10.586 | 1.00 | 0.00 |
| ATOM | 1841 | 3HD2 | LEU | 95 | 19.845 | -25.973 | 9.318 | 1.00 | 0.00 |
| ATOM | 1842 | QQD | LEU | 95 | 20.869 | -24.087 | 9.841 | 1.00 | 0.00 |
| ATOM | 1843 | C | LEU | 95 | 19.736 | -23.099 | 5.836 | 1.00 | 0.00 |
| ATOM | 1844 | O | LEU | 95 | 18.891 | -23.963 | 5.602 | 1.00 | 0.00 |
| ATOM | 1845 | N | GLU | 96 | 20.743 | -22.830 | 5.012 | 1.00 | 0.00 |
| ATOM | 1846 | H | GLU | 96 | 21.386 | -22.130 | 5.254 | 1.00 | 0.00 |
| ATOM | 1847 | CA | GLU | 96 | 20.916 | -23.551 | 3.756 | 1.00 | 0.00 |
| ATOM | 1848 | HA | GLU | 96 | 19.940 | -23.690 | 3.316 | 1.00 | 0.00 |
| ATOM | 1849 | CB | GLU | 96 | 21.784 | -22.739 | 2.792 | 1.00 | 0.00 |
| ATOM | 1850 | 1HB | GLU | 96 | 22.799 | -22.730 | 3.162 | 1.00 | 0.00 |
| ATOM | 1851 | 2HB | GLU | 96 | 21.413 | -21.725 | 2.760 | 1.00 | 0.00 |
| ATOM | 1852 | QB | GLU | 96 | 22.106 | -22.227 | 2.961 | 1.00 | 0.00 |
| ATOM | 1853 | CG | GLU | 96 | 21.797 | -23.287 | 1.376 | 1.00 | 0.00 |
| ATOM | 1854 | 1HG | GLU | 96 | 20.925 | -23.908 | 1.236 | 1.00 | 0.00 |
| ATOM | 1855 | 2HG | GLU | 96 | 22.687 | -23.883 | 1.241 | 1.00 | 0.00 |
| ATOM | 1856 | QG | GLU | 96 | 21.806 | -23.896 | 1.239 | 1.00 | 0.00 |
| ATOM | 1857 | CD | GLU | 96 | 21.785 | -22.192 | 0.326 | 1.00 | 0.00 |
| ATOM | 1858 | OE1 | GLU | 96 | 22.651 | -22.224 | -0.574 | 1.00 | 0.00 |
| ATOM | 1859 | OE2 | GLU | 96 | 20.912 | -21.303 | 0.406 | 1.00 | 0.00 |
| ATOM | 1860 | C | GLU | 96 | 21.547 | -24.919 | 3.996 | 1.00 | 0.00 |
| ATOM | 1861 | OT1 | GLU | 96 | 21.631 | -25.708 | 3.033 | 1.00 | 0.00 |
| ATOM | 1862 | OT2 | GLU | 96 | 21.953 | -25.188 | 5.147 | 1.00 | 0.00 |
| TER | | | | | | | | | |
| ENDMDL | | | | | | | | | |

**Patentansprüche**

1. Isoliertes Nukleinsäuremolekül ausgewählt aus der Gruppe umfassend:

   a) ein Nukleinsäuremolekül kodierend eine Aminosäuresequenz gemäß SEQ ID Nr. 1 oder deren komplementären Nukleotidsequenzen,
   b) ein Nukleinsäuremolekül, welches mit einer Nukleotidsequenz gemäß a) unter stringenten Bedingungen hybridisiert,
   c) ein Nukleinsäuremolekül umfassend eine Nukleotidsequenz, die eine ausreichende Homologie aufweist, um zu einer Nukleotidsequenz gemäß a) oder b) funktionsanalog zu sein,
   d) ein Nukleinsäuremolekül, das in Folge des genetisches Codes zu einer Nukleotidsequenz gemäß a) - c) degeneriert ist und
   e) ein Nukleinsäuremolekül gemäß einer Nukleotidsequenz nach a) - d), welches durch Deletionen, Additionen, Substitutionen, Translokationen, Inversionen und/oder Insertionen modifiziert und funktionsanalog zu einer Nukleotidsequenz gemäß a) bis d) ist.

2. Polypeptid, kodiert durch ein Nukleinsäuremolekül gemäß Anspruch 1.

3. Polypeptid nach Anspruch 2
   **dadurch gekennzeichnet, dass**
   die Positionen 32, 37, 48 und/oder 81 mindestens eines Aminosäurerestes gemäß der Aminosäuresequenz/des Polypeptids nach SEQ. Nr. 1 durch eine der zwanzig natürlichen Aminosäuren ersetzt sind.

4. Polypeptid-Bibliothek umfassend Polypeptide nach Anspruch 3.

5. Verfahren zur Detektion und Gewinnung von an Targetmolekülen bindenden Polypeptiden umfassend folgende Schritte

   a) Immobilisierung eines potentiellen Targetmoleküls auf einem Träger,
   b) Inkontaktbringen von Peptiden einer Polypeptid-Bibliothek nach Anspruch 4 mit den immobilisierten Targetmolekülen,
   c) Eliminieren der nicht an die Targetmoleküle gebundenen Polypeptide,
   d) Eluieren der gebundenen Polypeptide unter stringenten Bedingungen, wobei die bindenden Polypeptide gewonnen werden.

6. Verfahren nach dem vorhergehenden Anspruch,
   **dadurch gekennzeichnet, dass**
   es unter reduzierenden, insbesondere in Gegenwart von Dithiothreitol, oder oxidierenden, insbesondere im Gegenwart von Wasserstoffperoxid, Bedingungen durchgeführt wird.

**7.** Verfahren zur Detektion von Targetmolekülen, deren Inhibition Redox-Potential-abhängig schaltbar ist, umfassend die Schritte:

- Bereitstellung einer Probe umfassend mindestens einen Kandidaten des Targetmoleküls,
- Inkontaktbringen eines Polypeptids gemäß einem der Ansprüche 2 oder 3 oder der Polypeptid-Bibliothek nach Anspruch 4 mit der Probe und
- Detektion der Wechselwirkung, insbesondere der Bindung zwischen Targetmolekül und Polypeptid.

**8.** Kit umfassend ein Nukleinsäuremolekül gemäß Anspruch 1, ein Polypeptid gemäß Anspruch 2 oder 3 oder einer Polypeptid-Bibliothek nach Anspruch 4.

**9.** Verwendung des Kits gemäß dem vorangegangenen Anspruch zur Detektion von Targetmolekülen, deren Inhibition Redox-Potential-abhängig schaltbar ist oder zur Detektion von Polypeptiden, die mit diesen Targetmolekülen wechselwirken.

Abbildung 1

EP 1 695 981 A1

Abbildung 2

Abbildung 3

Abbildung 4

Abbildung 5

Abbildung 6

Abbildung 7

SH3-1, oxid.

Abbildung 8

Abbildung 9

Abbildung 10

Abbildung 11

Abbildung 12

Abbildung 13

Abbildung 14

149

Abbildung 15

Abbildung 16

EP 1 695 981 A1

Europäisches
Patentamt

**EUROPÄISCHER TEILRECHERCHENBERICHT**
der nach Regel 45 des Europäischen Patentübereinkommens für das weitere Verfahren als
europäischer Recherchenbericht gilt

**Nummer der Anmeldung**

EP 05 09 0045

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | WO 01/74858 A (MASSACHUSETTS INSTITUTE OF TECHNOLOGY; GESELLSCHAFT FUER BIOTECHNOLOGI) 11. Oktober 2001 (2001-10-11) SEQ ID NOs 2 and 3 ----- | 1-9 | C07K14/435 G01N33/50 |
| X | WO 03/048323 A (BRISTOL-MYERS SQUIBB COMPANY; CARMAN, JULIE; NADLER, STEVEN, G; BOWEN,) 12. Juni 2003 (2003-06-12) * Abbildungen 62,63 * ----- | 1-9 | |

**RECHERCHIERTE SACHGEBIETE (IPC)**

C12N
C07K
G01N

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

| Recherchenort | Abschlußdatum der Recherche | Prüfer |
|---|---|---|
| München | 3. März 2006 | Herrmann, K |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                            

& : Mitglied der gleichen Patentfamilie, übereinstimmendes Dokument

EPO FORM 1503 03.82 (P04C09)

Europäisches
Patentamt

**UNVOLLSTÄNDIGE RECHERCHE
ERGÄNZUNGSBLATT C**

Nummer der Anmeldung

EP 05 09 0045

Unvollständig recherchierte Ansprüche:
    2-9

Grund für die Beschränkung der Recherche:

Ein Polynukleotid kann durch eine Aminosäuresequenz klar definiert sein,
umgekehrt ist dies jedoch nicht der Fall. Von einem Polynukleotid können
praktisch unzählige Polypeptide abgeleitet werden (6 Leserahmen,
unterschiedliche Längen). Aus diesem Grund ist für den Gegenstand des
unabhängigen Anspruchs 2 keine sinnvolle Recherche möglich (Art. 84 EPÜ).
Folglich gilt dies auch für den Gegenstand der Ansprüche 3-9, welcher
sich auf das nicht hinreichend definierte Polypeptid von Anspruch 2
bezieht (Art. 84 EPÜ).

Eine Recherche ist aus diesem Grund nur für ein Polypeptid gemäß SEQ ID
NO:1 (siehe S. 6, Z. 2-4 und S. 17, Z. 5-7 der ursprünglichen
Beschreibung) durchgeführt worden. Außerdem sind Polynukleotide, welche
für ein Polypeptid gemäß SEQ ID NO:1 kodieren, recherchiert worden.

## ANHANG ZUM EUROPÄISCHEN RECHERCHENBERICHT
## ÜBER DIE EUROPÄISCHE PATENTANMELDUNG NR.

EP 05 09 0045

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-03-2006

| Im Recherchenbericht angeführtes Patentdokument | Datum der Veröffentlichung | Mitglied(er) der Patentfamilie | Datum der Veröffentlichung |
|---|---|---|---|
| WO 0174858 A | 11-10-2001 | AU 5310401 A | 15-10-2001 |
| WO 03048323 A | 12-06-2003 | AU 2002346613 A1 | 17-06-2003 |

EPO FORM P0461

Für nähere Einzelheiten zu diesem Anhang : siehe Amtsblatt des Europäischen Patentamts, Nr.12/82